# EUROPÄISCHE PATENTSCHRIFT

(11) **EP 3 089 972 B1**
(45) Veröffentlichungstag und Bekanntmachung des Hinweises auf die Patenterteilung: **16.05.2018**
(21) Anmeldenummer: 14824853.7
(22) Anmeldetag: 30.12.2014
(51) Int. Cl.: C07D 401/14, C07D 231/38, A01N 43/56

(54) **NEUE PYRAZOLYL-HETEROARYLAMIDE ALS SCHÄDLINGSBEKÄMPFUNGSMITTEL**
NOVEL PYRAZOLE HETEROARYLAMIDES AS PESTICIDES
NOUVEAUX PYRAZOL-HÉTÉROARYLAMIDES COMME MOYEN DE LUTTE CONTRE LES PARASITES

(30) Priorität: 03.01.2014 EP 14150153
(43) Veröffentlichungstag der Anmeldung: 09.11.2016
(73) Patentinhaber: Bayer Animal Health GmbH, 51373 Leverkusen (DE)
(72) Erfinder: SCHWARZ, Hans-Georg, 46282 Dorsten (DE); MAUE, Michael, 40764 Langenfeld (DE); ILG, Kerstin, 50670Köln (DE); GÖRGENS, Ulrich, 40882 Ratingen (DE); TURBERG, Andreas, 42781 Haan (DE); HORSTMANN, Sebastian, 51381 Leverkusen (DE); KÖBBERLING, Johannes, 41466 Neuss (DE); LINDNER, Niels, 42115 Wuppertal (DE)
(74) Vertreter: BIP Patents
(86) Internationale Anmeldenummer: PCT/EP2014/079440
(87) Internationale Veröffentlichungsnummer: WO 2015/101622

(56) Entgegenhaltungen:
- WO-A1-2012/080376
- WO-A1-2012/107434
- WO-A1-2014/122083

## Beschreibung

Die vorliegende Anmeldung betrifft neue Pyrazolyl-Heteroarylamide, Verfahren zu ihrer Herstellung und ihre Verwendung zur Bekämpfung von tierischen Schädlingen, vor allem von Arthropoden und insbesondere von Insekten, Spinnentieren und Nematoden.

Es ist bekannt, dass bestimmte Halogen-substituierte Verbindungen herbizid wirksam sind (vgl. J. Org. Chem. 1997, 62(17), 5908-5919, J. Heterocycl. Chem. 1998, 35(6), 1493-1499, WO 2004/035545, WO 2004/106324, US 2006/069132, WO 2008/029084).

Des Weiteren ist bekannt, dass bestimmte Halogen-substituierte Verbindungen insektizid wirksam sind (EP 1 911 751, WO 2010/051926, WO 2012/069366, WO 2012/080376, WO 2012/107434, WO 2014/122083, WO 2014/135439 und WO 2014/135437).

Ferner ist bekannt, dass bestimmte Halogen-substituierte Verbindungen Cytokin-inhibitorische Aktivitäten aufweisen (WO 2000/07980).

Moderne Pflanzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nie als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert und/oder ihre Aktivität verbessert wird.

Es wurde nun überraschenderweise gefunden, dass bestimmte Pyrazolyl-Heteroarylamide, sowie deren *N*-Oxide und Salze biologische Eigenschaften aufweisen und sich insbesondere zur Bekämpfung von tierischen Schädlingen eignen, und deshalb besonders gut im agrochemischen Bereich und im Bereich der Tiergesundheit einsetzbar sind.

Ähnliche Verbindungen sind bereits aus WO 2012/080376 bekannt geworden. Diese Anmeldung beschreibt 1,2,3-Triazol-benzanilide als insektizide Verbindungen, andere Heteroaroylbenzanilide werden nicht aufgeführt.

### Zusammenfassung

Die Erfindung betrifft Verbindungen der allgemeinen Formel (Ic), 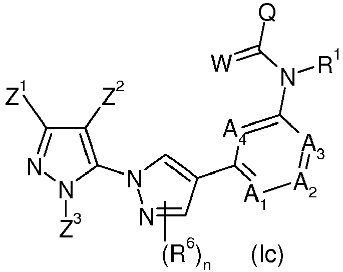 worin
- R¹: für Wasserstoff, gegebenenfalls substituiertes C₁-C₆-Alkyl, C₃-C₆-Alkenyl, C₃-C₆-Alkinyl, C₃-C₇-Cycloalkyl, C₁-C₆-Alkylcarbonyl, C₁-C₆-Alkoxycarbonyl, Aryl(C₁-C₃)-alkyl, Heteroaryl(C₁-C₃)-alkyl steht;
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Halogen, Cyano, Nitro, gegebenenfalls substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N*,*N*-Di-C₁-C₆-alkylamino, stehen; wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder wenn keine der Gruppierungen A₁ und A₂ für Stickstoff steht, können R² und R³ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält;
- W: für Sauerstoff oder Schwefel steht;
- Q: für Wasserstoff, Amino oder eine der gegebenenfalls substituierten Gruppierungen Alkyl, Alkyloxy, Alkenyl, Alkinyl, Cycloalkyl, Heterocyclyl, Cycloalkylalkyl, Arylalkyl, Heteroarylalkyl, Alkoxycarbonyl, oder für eine Gruppierung *N*-Alkylamino, *N*-Alkylcarbonylamino, *N*,*N*-Dialkylamino, Alkylsulfonylamino steht; oder
- Q: für ein gegebenenfalls ein- bis fünffach mit V substituiertes Aryl steht, oder für ein gegebenenfalls ein- bis fünffach mit V substituiertes Heterorayl steht, wobei
- V: für Halogen, Cyano, Nitro, gegebenenfalls substituiertes Alkyl, Alkenyl, Alkinyl, Cycloalkyl, Alkoxy, *N*-Alkoxyiminoalkyl, Alkylsulfanyl, Alkylsulfinyl, Alkylsulfonyl, *N,N*-Dialkylamino steht;
- R⁶: unabhängig voneinander für Halogen, Cyano, Nitro, Amino oder ein gegebenenfalls substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, und
- n: für die Werte 0-2 steht (wenn n = 0 dann ist R⁶ entsprechend H);
- Z¹: für ein gegebenenfalls substituiertes Alkyl und Cycloalkyl, und
- Z²: für Wasserstoff, Halogen, Cyano, Nitro, Amino oder ein gegebenenfalls substituiertes Alkyl, Alkylcarbonyl, Alkylsulfanyl, Alkylsulfinyl, Alkylsulfonyl, und
- Z³: für Wasserstoff oder ein gegebenenfalls substituiertes Alkyl, Cycloalkyl, Alkenyl, Alkinyl, Aryl oder Hetaryl stehen.

Eine bevorzugte Ausführungsform betrifft erfindungsgemäße Verbindungen wie in der Zusammenfassung zuvor beschrieben, wobei
- R¹: für Wasserstoff oder für ein gegebenenfalls ein- bis siebenfach unabhängig voneinander mit Halogen, Cyano, Alkoxy und Alkoxycarbonyl substituiertes C₁-C₆-Alkyl, C₃-C₆-Alkenyl, C₃-C₆-Alkinyl, C₃-C₇-Cycloalkyl, C₃-C₇-Cycloalkyl-C₁-C₃-alkyl, C₁-C₆-Alkylcarbonyl, C₁-C₆-Alkoxycarbonyl, Aryl(C₁-C₃)-alkyl, Heteroaryl(C₁-C₃)-alkyl steht;
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Halogen, Cyano, Nitro, oder für ein gegebenenfalls ein- bis siebenfach unabhängig voneinander mit Halogen, Cyano, Alkoxy und Alkoxycarbonyl substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N*-Di-C₁-C₆-alkylamino stehen;
- W: für Sauerstoff oder Schwefel steht;
- Q: für Wasserstoff, Amino oder eine der gegebenenfalls unabhängig von einander ein- bis siebenfach mit Hydoxy, Nitro, Amino, Halogen, Alkoxy, Cyano, Hydroxycarbonyl, Alkoxycarbonyl, Alkylcarbamoyl, Cycloalkylcarbamoyl, Phenyl substituierten Gruppierungen C₁-C₆-Alkyl, C₂-C₆-Alkenyl, C₂-C₆-Alkinyl, C₃-C₆-Cycloalkyl, C₂-C₅-Heterocyclyl, C₁-C₄-Alkoxy, C₁-C₆-Alkyl-C₃-C₆-cycloalkyl, C₃-C₆-Cycloalkyl-C₁-C₆-alkyl, C₁-C₆-Hydroxyalkyl, Aryl(C₁-C₃)-alkyl, Heteroaryl(C₁-C₃)-alkyl, C₁-C₄-Alkoxycarbonyl, *N*-C₁-C₄-Alkylamino, *N*-C₁-C₄-Alkylcarbonylamino, *N,N*-Di-C₁-C₄-alkylamino, C₁-C₄-Alkylsulfonylamino steht; oder
- Q: für Wasserstoff, Amino oder eine der gegebenenfalls unabhängig von einander mit 1, 2, 3, 4, 5, 6 oder 7 Substituenten ausgewählt aus der Gruppe bestehend aus Hydoxy, Nitro, Amino, Halogen, Oxo, Benzyloxy, C₃-C₆-Cycloalkyl, C₁-C₆-Alkoxy, C₁-C₆-Halogenalkoxy, Cyano, Hydroxycarbonyl, C₁-C₄-Alkoxycarbonyl, C₁-C₆-Alkylcarbamoyl, C₃-C₆-Cycloalkylcarbamoyl, *N,N*-Di-C₁-C₄-alkylamino, gegebenenfalls mit 1, 2 oder 3 Substituenten unabhängig voneinander ausgewählt aus der Gruppe von Substituenten bestehend aus Halogen, C₁-C₆-Alkoxy, C₁-C₆-Halogenalkoxy und C₁-C₆-Alkyl substituierten Phenyl, gegebenenfalls mit 1, 2 oder 3 Substituenten unabhängig voneinander ausgewählt aus der Gruppe von Substituenten bestehend aus Halogen, C₁-C₆-Alkoxy, C₁-C₆-Halogenalkoxy nd C₁-C₆-Alkyl substituierten Phenylthio, gegebenenfalls mit 1, 2 oder 3 Substituenten unabhängig voneinander ausgewählt aus der Gruppe von Substituenten bestehend aus Halogen, C₁-C₆-Alkoxy, C₁-C₆-Halogenalkoxy und C₁-C₆-Alkyl substituierten Phenyloxy, gegebenenfalls mit 1, 2 oder 3 Substituenten unabhängig voneinander ausgewählt aus der Gruppe von Substituenten bestehend aus Halogen, C₁-C₆-Alkoxy, C₁-C₆-Halogenalkoxy und C₁-C₆-Alkyl substituierten 5- oder 6-gliedrigen Heteroaryl (z. B. Pyrazolyl) substituierten Gruppierungen ausgewählt aus C₁-C₆-Alkyl, C₁-C₆-Alkyloxy, C₂-C₆-Alkenyl, C₂-C₆-Alkinyl, C₃-C₆-Cycloalkyl, 5 oder 6 gliedriges Heterocyclyl, C₁-C₆-Alkyl-C₃-C₆-cycloalkyl, C₃-C₆-Cycloalkyl-C₁-C₆-alkyl, C₆-C₁₀-Aryl-(C₁-C₃)-alkyl, 5 oder 6 gliedriges Heteroaryl-(C₁-C₃)-alkyl, C₁-C₄-Alkoxycarbonyl, oder für eine Gruppierung *N*-C₁-C₄-Alkylamino, *N*-C₁-C₄-Alkylcarbonylamino, *N,N*-Di-C₁-C₄-alkylamino, C₁-C₄-Alkylsulfonylamino steht; oder
- Q: für ein mit 0, 1, 2, 3 oder 4 Substituenten V substituiertes Aryl oder für ein mit 0, 1, 2, 3 oder 4 Substituenten V substituiertes 5- bzw. 6-gliedriges Heteroaryl steht, oder
- Q: für einen mit 0, 1, 2, 3 oder 4 Substituenten V substituierten bicyclischen Heterocyclus oder für einen mit 0, 1, 2, 3 oder 4 Substituenten V substituierten bicyclische Carbocyclus steht; wobei
- V: unabhängig voneinander für Halogen, Cyano, Nitro oder für ein gegebenenfalls ein- bis siebenfach unabhängig voneinander mit Halogen, Cyano, Alkoxy und Alkoxycarbonyl substituiertes C₁-C₆-Alkyl, C₁-C₆-Halogenalkyl, C₂-C₄-Alkenyl, C₂-C₄-Alkinyl, C₃-C₆-Cycloalkyl, C₁-C₆-Alkoxy, C₁-C₆-Halogenalkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N,N-*Di-(C₁-C₆-alkyl)amino steht;
- R⁶: unabhängig voneinander für Halogen, Cyano, Nitro, Amino oder ein gegebenenfalls ein- bis siebenfach mit Halogen substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, und
- n: für die Werte 0-1 steht;
- Z¹: für ein gegebenenfalls ein- bis siebenfach unabhängig voneinander mit Halogen, Cyano, Alkoxy und Alkoxycarbonyl substituiertes C₁-C₆-Alkyl, C₃-C₆-Cycloalkyl, und
- Z²: für Wasserstoff, Halogen, Cyano, Nitro, Amino oder ein ein- bis siebenfach unabhängig voneinander mit Halogen, Cyano, Alkoxy und Alkoxycarbonyl substituiertes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl, und
- Z³: für Wasserstoff oder für ein gegebenenfalls ein- bis siebenfach unabhängig voneinander mit Halogen, Cyano, Alkoxy und Alkoxycarbonyl substituiertes C₁-C₆-Alkyl, C₃-C₆-Cycloalkyl C₃-C₄-Alkenyl, C₃-C₄-Alkinyl, oder für ein gegebenenfalls ein- bis fünffach unabhängig voneinander mit Halogen, Cyano, Alkoxy und Alkoxycarbonyl substituiertes Aryl und Hetaryl stehen.

Eine weitere bevorzugte Ausführungsform betrifft erfindungsgemäße Verbindungen wie in der Zusammenfassung zuvor beschrieben, worin
- R¹: für Wasserstoff, C₁-C₄-Alkyl, C₃-C₄-Alkenyl, C₃-C₄-Alkinyl steht;
die chemischen Gruppierungen
- A₁: für CR²,
- A₂: für CR³ oder Stickstoff,
- A₃: für CR⁴, und
- A₄: für CR⁵ stehen;
- W: für Sauerstoff steht;
- R⁶: für C₁-C₄-Alkyl steht;
- n: für die Werte 0-1 steht;
- Z¹: für ein jeweils gegebenenfalls ein- bis siebenfach unabhängig voneinander mit Halogen oder Cyano substituiertes C₁-C₆-Alkyl oder C₃-C₆-Cycloalkyl steht;
- Z²: für ein jeweils gegebenenfalls ein- bis siebenfach unabhängig voneinander mit Halogen oder Cyano substituiertes C₁-C₆-Alkyl oder C₃-C₆-Cycloalkyl steht;
- Z³: für Wasserstoff oder C₁-C₆-Alkyl steht.

Eine weitere bevorzugte Ausführungsform betrifft erfindungsgemäße Verbindungen wie in der Zusammenfassung zuvor beschrieben, worin
- R¹: für Wasserstoff, Methyl, Ethyl, n-Propyl, Isopropyl, n-Butyl, Isobutyl, s-Butyl, oder t-Butyl steht, besonders bevorzugt für Wasserstoff steht;
die chemischen Gruppierungen
- A₁: für CR²,
- A₂: für CR³ oder Stickstoff,
- A₃: für CR⁴, und
- A₄: für CR⁵ stehen;
- W: für Sauerstoff steht;
- R⁶: für Wasserstoff steht (n = 0);
- Z¹: für ein jeweils gegebenenfalls ein- bis siebenfach unabhängig voneinander mit Halogen substituiertes C₁-C₆-Alkyl steht;
- Z²: für ein jeweils gegebenenfalls ein- bis siebenfach unabhängig voneinander mit Halogen substituiertes C₁-C₆-Alkyl steht;
- Z³: für C₁-C₆-Alkyl steht.

Eine weitere bevorzugte Ausführungsform betrifft erfindungsgemäße Verbindungen wie in der Zusammenfassung zuvor beschrieben, worin
R² und R⁵ unabhängig voneinander für Wasserstoff, Fluor Chlor, Brom, Iod, Methyl und Methoxy stehen;
R³ und R⁴ unabhängig voneinander für Wasserstoff, Fluor, Chlor, Brom, Iod, Cyano, Nitro, C₁-C₄-Alkyl, C₁-C₄-Haloalkyl stehen;

Eine weitere bevorzugte Ausführungsform betrifft erfindungsgemäße Verbindungen wie in der Zusammenfassung zuvor beschrieben, worin
R² und R⁵ unabhängig voneinander für Wasserstoff oder Fluor stehen;
R³ für Wasserstoff steht; und
R⁴ für Wasserstoff, Fluor, Chlor, oder Methyl, steht.

Eine weitere bevorzugte Ausführungsform betrifft erfindungsgemäße Verbindungen wie in der Zusammenfassung zuvor beschrieben, worin
- Q: für Wasserstoff; oder
C₁-C₆-Alkyl optional substituiert mit 1, 2, 3 oder 4 Substituenten unabhängig voneinander ausgewählt aus der Gruppe bestehend aus
Fluor,

Chlor,
Brom,
Iod,
Cyano,
Oxo,
Methoxy,
Benzyloxy,
Ethoxy,
*N,N*-Di-C₁-C₄-alkylamino,
optional unabhängig voneinander mit 1, 2, 3, oder 4 Subtituenten ausgewählt aus der Gruppe bestehend aus Fluor, Chlor, Brom, Iod, Methoxy, Methyl, und Trifluormethyl substituiertem Phenylthio,
optional unabhängig voneinander mit 1, 2, 3, oder 4 Subtituenten ausgewählt aus der Gruppe bestehend aus Fluor, Chlor, Brom, Iod, Methoxy, Methyl, und Trifluormethyl substituiertem Phenyloxy,
optional unabhängig voneinander mit 1, 2, 3, oder 4 Subtituenten ausgewählt aus der Gruppe bestehend aus Fluor, Chlor, Brom, Iod, Methoxy, Methyl, und Trifluormethyl substituiertem Phenyl,
optional unabhängig voneinander mit 1, 2, 3, oder 4 Subtituenten ausgewählt aus der Gruppe bestehend aus Fluor, Chlor, Brom, Iod, Methoxy, Methyl, und Trifluormethyl substituiertem Thiophenyl,
optional unabhängig voneinander mit 1, 2, 3, oder 4 Subtituenten ausgewählt aus der Gruppe bestehend aus Fluor, Chlor, Brom, Iod, Methoxy, Methyl, und Trifluormethyl substituiertem Pyrazolyl, und
C₃-C₆-Cycloalkyl;
oder
C₃-C₆-Cycloalkyl optional substituiert mit 1, 2, 3 oder 4 Substituenten unabhängig voneinander ausgewählt aus der Gruppe bestehend aus
Methoxy,
Fluor,
Chlor,
Brom
Iod,
Cyano,
Methyl,
optional unabhängig voneinander mit 1, 2, 3, oder 4 Subtituenten ausgewählt aus der Gruppe bestehend aus Fluor, Chlor, Brom, Iod, Methoxy, Methyl und Trifluormethyl substituiertem Phenyl;
oder
- Q: für ein mit 0, 1, 2, 3 oder 4 Substituenten V substituiertes Phenyl, Naphthyl, Pyridazinyl, Pyrazinyl, Pyrimidinyl, Triazinyl, Pyridinyl, Pyrazolyl, Thiazolyl, Isothiazolyl, Oxazolyl, Isoxazolyl, Triazolyl, Imidazolyl, Furanyl, Thiophenyl, Pyrrolyl, Oxadiazolyl, Thiadiazolyl, Benzothiophenyl, Benzofuranyl, [1,2,4]Triazolo[1,5-a]pyrimidinyl, 1,3-Benzodioxolyl oder Tetralinyl steht, wobei
- V: unabhängig voneinander für Fluor, Chlor, Brom, Iod, Cyano, Nitro, Methyl, Ethyl, Propyl, Butyl, Difluormethyl, , Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N*-Di-C₁-C₄-alkylamino (z. B. *N*,*N-*Dimethylamino) steht.

Eine weitere bevorzugte Ausführungsform betrifft erfindungsgemäße Verbindungen wie in der Zusammenfassung zuvor beschrieben, worin
- Q: C₁-C₆-Alkyl optional substituiert mit 1, 2, 3 oder 4 Substituenten unabhängig voneinander ausgewählt aus der Gruppe bestehend aus
Fluor,
Oxo,
Methoxy,
Benzyloxy,
Ethoxy,
*N,N*-Dimethylamino
Phenylthio,
Phenyloxy,
C₃-C₆-Cycloalkyl,
optional unabhängig voneinander mit 1, 2, 3, oder 4 Subtituenten ausgewählt aus der Gruppe bestehend aus Fluor, Chlor, und Trifluormethyl substituiertem Phenyl,
optional mit 1, 2, 3, oder 4 Trifluormethyl substituiertem Thiophenyl, und
optional unabhängig voneinander mit 1, 2, 3, oder 4 Subtituenten ausgewählt aus der Gruppe bestehend aus Methyl und Trifluormethyl substituiertem Pyrazolyl;
oder
C₃-C₆-Cycloalkyl optional substituiert mit 1, 2, 3 oder 4 Substituenten unabhängig voneinander ausgewählt aus der Gruppe bestehend aus
Methoxy,
Chlor,
Cyano,
Methyl, optional unabhängig voneinander mit 1, 2, 3, oder 4 Subtituenten ausgewählt aus der Gruppe bestehend aus Chlor, Methyl, und Trifluormethyl, substituiertem Phenyl;
oder
- Q: für ein mit 0, 1, 2, 3 oder 4 Substituenten V substituiertes Phenyl, Naphthyl, Pyridazinyl, Pyrazinyl, Pyrimidinyl, Triazinyl, Pyridinyl, Pyrazolyl, Thiazolyl, Isothiazolyl, Oxazolyl, Isoxazolyl, Triazolyl, Imidazolyl, Furanyl, Thiophenyl, Pyrrolyl, Oxadiazolyl, Thiadiazolyl, Benzothiophenyl, Benzofuranyl, [1,2,4]Triazolo[1,5-a]pyrimidinyl, 1,3-Benzodioxolyl, oder Tetralinyl steht, wobei
- V: unabhängig voneinander für Fluor, Chlor, Brom, Iod, Cyano, Nitro, Methyl, Ethyl, Propyl, Butyl, Difluormethyl, , Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N-*Di-C₁-C₄-alkylamino (z. B. *N*,*N*-Dimethylamino) steht.

Eine weitere bevorzugte Ausführungsform betrifft erfindungsgemäße Verbindungen wie in der Zusammenfassung zuvor beschrieben, worin die Verbindung eine Verbindung der Formel (Ic-1) ist

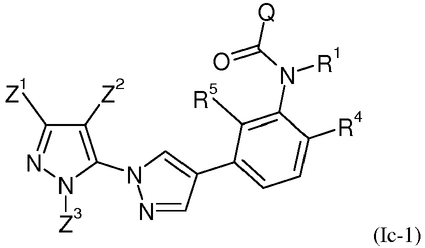

Eine weitere bevorzugte Ausführungsform betrifft erfindungsgemäße Verbindungen wie in der Zusammenfassung zuvor beschrieben, worin die Verbindung eine Verbindung der Formel (Ic-2) ist

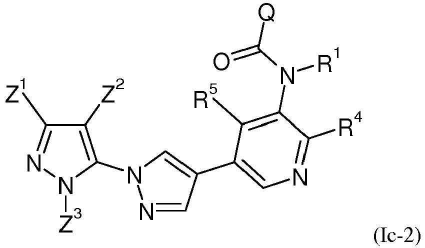

Gegenstand der Erfindung ist auch die Verwendung von erfindungsgemäßen Verbindungen (z. B. der Formel (Ic), (Ic-1) oder (Ic-2)) zur Bekämpfung von Insekten, Spinnentieren und Nematoden; Pharmazeutische Zusammensetzungen, enthaltend wenigstens eine erfindungsgemäße Verbindung; eine erfindungsgemäße Verbindungen zur Verwendung als Arzneimittel; die Verwendung von einer erfindungsgemäßen Verbindung zur Herstellung pharmazeutischer Zusammensetzungen zur Bekämpfung von Parasiten auf Tieren; ein Verfahren zur Herstellung von Pflanzenschutzmitteln enthaltend mindestens eine erfindungsgemäße Verbindung sowie übliche Streckmittel und/oder oberflächenaktive Substanzen; ein Verfahren zum Bekämpfen von Schädlingen, dadurch gekennzeichnet, dass man eine erfindungsgemäße Verbindung auf die Schädlinge und/oder ihren Lebensraum einwirken lässt; die Verwendung von einer erfindungsgemäßen Verbindung zum Schutz des Vermehrungsmaterials von Pflanzen.

### Detaillierte Beschreibung

Hierin beschrieben sind Pyrazolyl-Heteroarylamide der allgemeinen Formel (I) 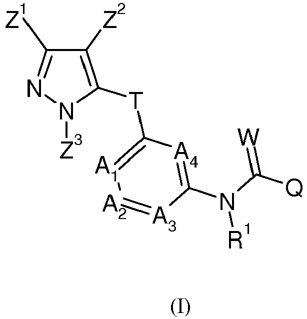 , in denen
- R¹: für Wasserstoff, gegebenenfalls substituiertes C₁-C₆-Alkyl, C₃-C₆-Alkenyl, C₃-C₆-Alkinyl, C₃-C₇-Cycloalkyl, C₁-C₆-Alkylcarbonyl, C₁-C₆-Alkoxycarbonyl, Aryl(C₁-C₃)-alkyl, Heteroaryl(C₁-C₃)-alkyl steht, ganz besonders bevorzugt für Wasserstoff steht;
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Halogen, Cyano, Nitro, gegebenenfalls substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N-*Di-C₁-C₆-alkylamino, stehen;
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält;
- W: für Sauerstoff oder Schwefel steht;
- Q: für Wasserstoff, Amino oder eine der gegebenenfalls substituierten Gruppierungen Alkyl, Alkyloxy, Alkenyl, Alkinyl, Cycloalkyl, Heterocyclyl, Cycloalkylalkyl, Arylalkyl, Heteroarylalkyl, Alkoxycarbonyl, oder für eine Gruppierung *N*-Alkylamino, *N-*Alkylcarbonylamino, *N,N*-Dialkylamino, Alkylsulfonylamino steht; oder
- Q: für ein gegebenenfalls ein- bis fünffach mit V substituiertes Aryl steht, oder für ein gegebenenfalls ein- bis fünffach mit V substituiertes Heterorayl steht, wobei
- V: für Halogen, Cyano, Nitro, gegebenenfalls substituiertes Alkyl, Alkenyl, Alkinyl, Cycloalkyl, Alkoxy, *N*-Alkoxyiminoalkyl, Alkylsulfanyl, Alkylsulfinyl, Alkylsulfonyl, *N,N-*Dialkylamino steht;
- T: für einen der nachfolgend aufgeführten 5-gliedrigen Heteroaromaten T1-T10 steht, wobei die Bindung zur Pyrazolkopfgruppe mit einem Sternchen gekennzeichnet ist, 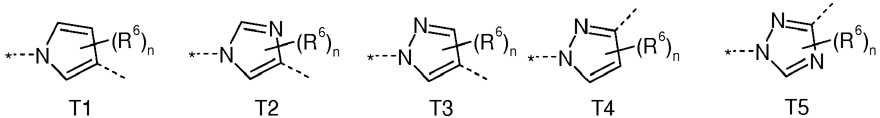 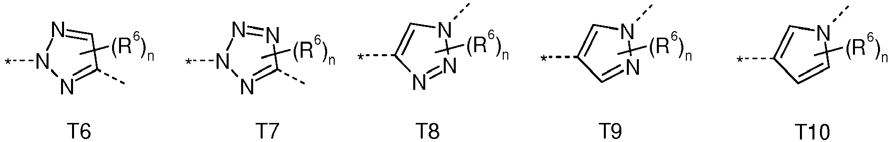
wobei
- R⁶: unabhängig voneinander für Halogen, Cyano, Nitro, Amino oder ein gegebenenfalls substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, und
- n: für die Werte 0-2 steht;
- Z¹: für ein gegebenenfalls substituiertes Alkyl und Cycloalkyl, und
- Z²: für Wasserstoff, Halogen, Cyano, Nitro, Amino oder ein gegebenenfalls substituiertes Alkyl, Alkylcarbonyl, Alkylsulfanyl, Alkylsulfinyl, Alkylsulfonyl, und
- Z³: für Wasserstoff oder ein gegebenenfalls substituiertes Alkyl, Cycloalkyl, Alkenyl, Alkinyl, Aryl oder Hetaryl stehen.

Hierin beschrieben sind weiterhin Verbindungen der Formel (I) 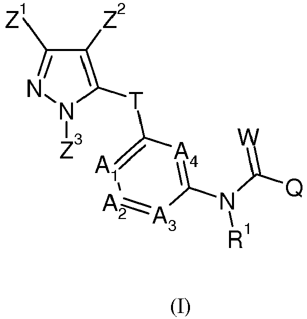 , in denen
- R¹: für Wasserstoff oder für ein gegebenenfalls ein- bis siebenfach unabhängig voneinander mit Halogen, Cyano, Alkoxy oder Alkoxycarbonyl substituiertes C₁-C₆-Alkyl, C₃-C₆-Alkenyl, C₃-C₆-Alkinyl, C₃-C₇-Cycloalkyl, C₁-C₆-Alkylcarbonyl, C₁-C₆-Alkoxycarbonyl, Aryl(C₁-C₃)-alkyl, Heteroaryl(C₁-C₃)-alkyl steht, ganz besonders bevorzugt für Wasserstoff steht;
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Halogen, Cyano, Nitro, oder für gegebenenfalls ein- bis siebenfach unabhängig voneinander mit Halogen, Cyano, Alkoxy oder Alkoxycarbonyl substituiertes C₁-C₆-Alkyl, C₃-C₆-Cycloalkyl, C₁-C₆-Alkoxy, *N-*Alkoxyiminoalkyl, C₁-C₆-Alkylsulfanyl, C₁-C₆-Alkylsulfinyl, C₁-C₆-Alkylsulfonyl, *N-*C₁-C₆-Alkylamino, *N,N*-Di-C₁-C₆alkylamino, stehen;
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 6-gliedrigen Ring bilden, der 0, 1 oder 2 Stickstoffatome enthält;
- W: für Sauerstoff oder Schwefel steht;
- Q: für Wasserstoff, Amino oder für gegebenenfalls ein- bis siebenfach unabhängig voneinander mit Halogen, Cyano, C₁-C₆-Alkoxy oder C₁-C₆-Alkoxycarbonyl substituierte Gruppierungen C₁-C₆-Alkyl, C₂-C₆-Alkenyl, C₂-C₆-Alkinyl, C₃-C₆-Cycloalkyl, C₂-C₅-Heterocyclyl, C₁-C₄-Alkoxy, C₁-C₆-Alkyl-C₃-C₆-cycloalkyl, C₃-C₆-Cycloalkyl-C₁-C₆-alkyl, C₆-Aryl-(C₁-C₃)-alkyl, 5- oder 6-gliedriges Heteroaryl, 5- oder 6-gliedriges Heteroaryl-(C₁-C₃)-alkyl, C₁-C₄-Alkoxycarbonyl, oder für eine Gruppierung *N*-C₁-C₄-Alkylamino, *N*-C₁-C₄-Alkylcarbonylamino, *N,N*-Di-C₁-C₄-alkylamino, C₁-C₄-Alkylsulfonylamino steht; oder
- Q: für ein mit 0, 1, 2, 3 oder 4 Substituenten V substituiertes Aryl oder für ein mit 0, 1, 2, 3 oder 4 Substituenten V substituiertes 5- bzw. 6-gliedriges Heteroaryl steht, wobei
- V: unabhängig voneinander für Halogen, Cyano, Nitro, oder für gegebenenfalls ein- bis siebenfach unabhängig voneinander mit Halogen, Cyano, Alkoxy oder Alkoxycarbonyl substituiertes C₁-C₆-Alkyl, C₂-C₄-Alkenyl, C₂-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N,N*-Di-(C₁-C₆-alkyl)amino steht;
- T: für einen der nachfolgend aufgeführten 5-gliedrigen Heteroaromaten T1-T10 steht, wobei die Bindung zur Pyrazolkopfgruppe mit einem Sternchen gekennzeichnet ist, 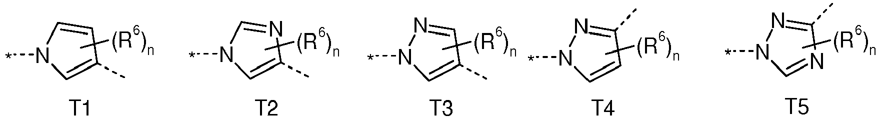 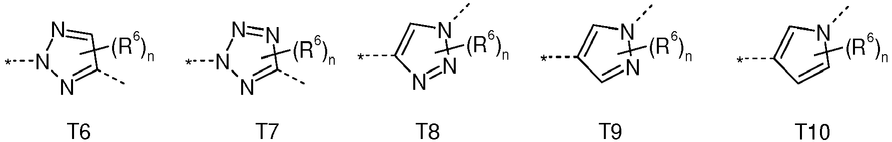
wobei
- R⁶: unabhängig voneinander für Halogen, Cyano, Nitro, Amino oder für ein gegebenenfalls ein- bis siebenfach mit Halogen substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, und
- n: für die Werte 0-1 steht;
- Z¹: für ein gegebenenfalls ein- bis siebenfach unabhängig voneinander mit Halogen, Cyano, Alkoxy und Alkoxycarbonyl substituiertes C1-C6-Alkyl, C3-C6-Cycloalkyl, und
- Z²: für Wasserstoff, Halogen, Cyano, Nitro, Amino oder für ein gegebenenfalls ein- bis siebenfach unabhängig voneinander mit Halogen, Cyano, Alkoxy oder Alkoxycarbonyl substituiertes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl, und
- Z³: für Wasserstoff oder für ein gegebenenfalls ein- bis siebenfach unabhängig voneinander mit Halogen, Cyano, Alkoxy oder Alkoxycarbonyl substituiertes C₁-C₆-Alkyl, C₃-C₆-Cycloalkyl, C₃-C₆-Alkenyl, C₃-C₆-Alkinyl, oder für ein gegebenenfalls ein- bis fünffach unabhängig voneinander mit Halogen, Cyano, Alkoxy und Alkoxycarbonyl substituiertes Aryl oder Hetaryl stehen;

Ferner hierin beschrieben sind Verbindungen der Formel (I) 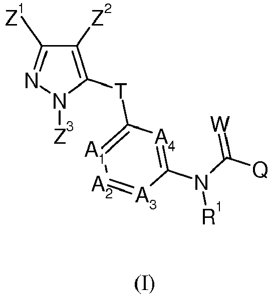 , in denen
- R¹: für Wasserstoff oder für ein gegebenenfalls ein- bis siebenfach unabhängig voneinander mit Halogen, Cyano, Alkoxy oder Alkoxycarbonyl substituiertes C₁-C₆-Alkyl, C₃-C₆-Alkenyl, C₃-C₆-Alkinyl, C₃-C₇-Cycloalkyl, C₁-C₆-Alkylcarbonyl, C₁-C₆-Alkoxycarbonyl, Aryl(C₁-C₃)-alkyl, Heteroaryl(C₁-C₃)-alkyl steht, ganz besonders bevorzugt für Wasserstoff steht;
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Halogen, Cyano, Nitro, oder für gegebenenfalls ein- bis siebenfach unabhängig voneinander mit Halogen, Cyano, Alkoxy oder Alkoxycarbonyl substituiertes C₁-C₆-Alkyl, C₃-C₆-Cycloalkyl, C₁-C₆-Alkoxy, *N-*Alkoxyiminoalkyl, C₁-C₆-Alkylsulfanyl, C₁-C₆-Alkylsulfinyl, C₁-C₆-Alkylsulfonyl, *N*-C₁-C₆-Alkylamino, *N,N*-Di-C₁-C₆alkylamino, stehen;
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder wenn keine der Gruppierungen A₁ und A₂ für Stickstoff steht, können R² und R³ gemeinsam mit dem Kohlenstoffatom, an das sie gebunden sind, einen 6-gliedrigen Ring bilden, der 0, 1 oder 2 Stickstoffatome enthält;
- W: für Sauerstoff oder Schwefel steht;
- Q: für Wasserstoff, Amino oder für gegebenenfalls ein- bis siebenfach unabhängig voneinander mit Halogen, Cyano, Alkoxy oder Alkoxycarbonyl substituierte Gruppierungen C₁-C₆-Alkyl, C₂-C₆-Alkenyl, C₂-C₆-Alkinyl, C₃-C₆-Cycloalkyl, C₂-C₅-Heterocyclyl, C₁-C₄-Alkoxy, C₁-C₆-Alkyl-C₃-C₆-cycloalkyl, C₃-C₆-Cycloalkyl-C₁-C₆-alkyl, Aryl-(C₁-C₃)-alkyl, Heteroaryl-(C₁-C₃)-alkyl, C₁-C₄-Alkoxycarbonyl, oder für eine Gruppierung *N*-C₁-C₄-Alkylamino, *N*-C₁-C₄-Alkylcarbonylamino, *N,N-*Di-C₁-C₄-alkylamino, C₁-C₄-Alkylsulfonylamino steht; oder
- Q: für ein gegebenenfalls ein- bis fünffach mit V substituiertes Aryl, oder für ein gegebenenfalls ein-bis fünffach mit V substituiertes Heteroaryl steht, wobei
- V: unabhängig voneinander für Halogen, Cyano, Nitro, oder für gegebenenfalls ein- bis siebenfach unabhängig voneinander mit Halogen, Cyano, Alkoxy oder Alkoxycarbonyl substituiertes C₁-C₆-Alkyl, C₂-C₄-Alkenyl, C₂-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N,N*-Di-(C₁-C₆-alkyl)amino steht;
- T: für einen der nachfolgend aufgeführten 5-gliedrigen Heteroaromaten T1-T10 steht, wobei die Bindung zur Pyrazolkopfgruppe mit einem Sternchen gekennzeichnet ist, 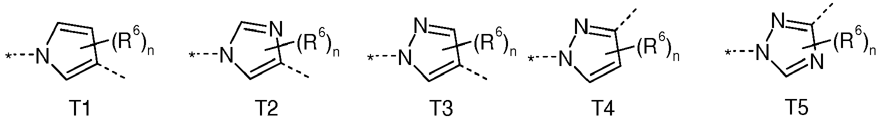 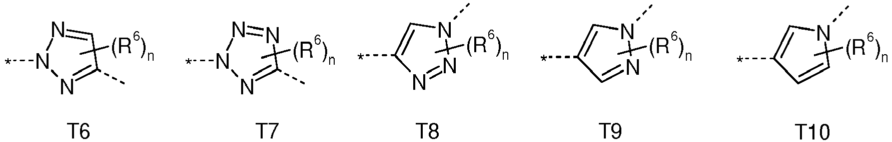
wobei
- R⁶: unabhängig voneinander für Halogen, Cyano, Nitro, Amino oder für ein gegebenenfalls ein- bis siebenfach mit Halogen substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, und
- n: für die Werte 0-1 steht;
- Z¹: für ein gegebenenfalls substituiertes C₁-C₆-Halogenalkyl, C₃-C₆-Cycloalkyl, C₃-C₆-Halogencycloalkyl, und
- Z²: für Wasserstoff, Halogen, Cyano, Nitro, Amino oder für ein gegebenenfalls ein- bis siebenfach unabhängig voneinander mit Halogen, Cyano, Alkoxy oder Alkoxycarbonyl substituiertes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl, und
- Z³: für Wasserstoff oder für ein gegebenenfalls ein- bis siebenfach unabhängig voneinander mit Halogen, Cyano, Alkoxy oder Alkoxycarbonyl substituiertes C₁-C₆-Alkyl, C₃-C₆-Cycloalkyl, C₃-C₆-Alkenyl, C₃-C₆-Alkinyl, oder für ein gegebenenfalls ein- bis fünffach unabhängig voneinander mit Halogen, Cyano, Alkoxy und Alkoxycarbonyl substituiertes Aryl oder Hetaryl stehen.

Weiterhin hierin beschrieben sind Verbindungen der Formel (I) 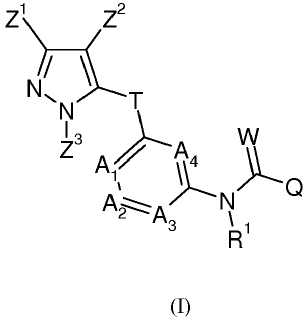 definiert, in denen
- R¹: für Wasserstoff, Methyl, Ethyl, n-Propyl, Isopropyl, n-Butyl, Isobutyl, s-Butyl, t-Butyl, Prop-2-enyl, 2-Methylprop-2-enyl, Prop-2-inyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht, ganz besonders bevorzugt für Wasserstoff steht;
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R² und R⁵: unabhängig voneinander für Wasserstoff, Fluor Chlor, Brom, Iod, Methyl und Methoxy stehen und
- R³ und R⁴: unabhängig voneinander für Wasserstoff, Fluor, Chlor, Brom, Iod, Cyano, Nitro, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Trifluormethylsulfanyl, Methylsulfonyl, Methylsulfinyl, Trifluormethylsulfonyl, Trifluormethylsulfinyl stehen;
- W: für Sauerstoff oder Schwefel steht;
- Q: für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1,1,2,2-Tetrafluorethyl, 1-Trifluormethylethyl, 2,2-Difluorpropyl, 3,3,3-Trifluropropyl, 2,2-Dimethyl-3-fluorpropyl, Cyclopropyl, 1-Cyano-cyclopropyl, 1-Chlor-cyclopropyl, 1-Methyl-cyclopropyl, 1-Methoxycarbonyl-cyclopropyl, 1-(*N*-Methylcarbamoyl)cyclopropyl, 1-(*N*-Cyclopropyl-carbamoyl)cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl, 3,3-Dichlor-prop-2-enyl, 3,3-Dichlor-1,1-dimethylprop-2-enyl, Oxetan-3-yl, Thietan-3-yl, 1-Oxido-thietan-3-yl, 1,1-Dioxido-thietan-3-yl, Isoxazol-3-ylmethyl, 3-Methyloxetan-3-ylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, Pyridin-2--ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N-*Allylamino, *N,N*-Dimethylamino, *N,N*-Diethylamino, Methylsulfonylamino steht; oder
- Q: für ein mit 0, 1, 2, 3, oder 4 Substituenten V substituiertes Phenyl, Naphthyl, Pyridazinyl, Pyrazinyl, Pyrimidinyl, Triazinyl, Pyridinyl, Pyrazolyl, Thiazolyl, Isothiazolyl, Oxazolyl, Isoxazolyl, Triazolyl, Imidazolyl, Furanyl, Thiophenyl, Pyrrolyl, Oxadiazolyl, Thiadiazolyl steht, wobei
- V: unabhängig voneinander für Fluor, Chlor, Brom, Iod, Cyano, Nitro, Methyl, Ethyl, Difluormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N*,*N*-Dimethylamino steht;
- T: für einen der nachfolgend aufgeführten 5-gliedrigen Heteroaromaten T1-T10 steht, wobei die Bindung zur Pyrazolkopfgruppe mit einem Sternchen gekennzeichnet ist, 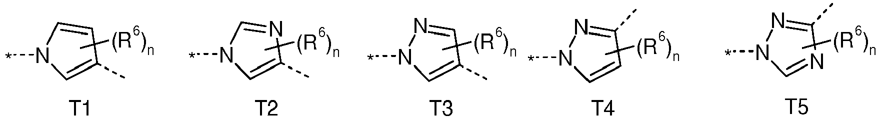 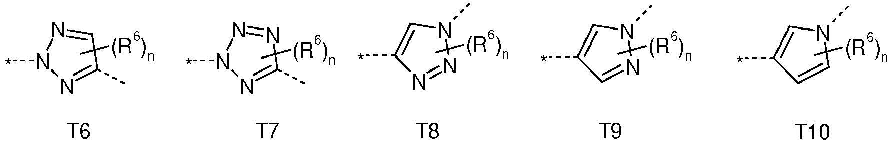
wobei
- R⁶: unabhängig voneinander für Halogen, Cyano, Nitro, Amino, Methyl, Ethyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluormethylsulfanyl, Trifluormethylsulfinyl, und
- n: für die Werte 0-1 steht;
- Z¹: für Methyl, Ethyl, 1,1-Dimethylethyl, Difluormethyl, , Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl, Pentafluor-t-butyl, Heptafluor-n-propyl, Heptafluor-isopropyl, Nonafluor-n-butyl, Cyclopropyl, 1-Chlorcyclopropyl, 1-Fluorcyclopropyl, 1-Bromcyclopropyl, 1-Cyan-cyclopropyl, 1-Trifluormehtyl-cyclopropyl, Cyclobutyl und 2,2-Difluor-1-methyl-cyclopropyl, und
- Z²: für Wasserstoff, Halogen, Cyano, Nitro, Amino, Methyl, Ethyl, 1,1-Dimethylethyl, Difluormethyl, , Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-t-butyl, Heptafluor-n-propyl, Heptafluor-isopropyl, Nonafluor-n-butyl, Methylsulfanyl, Methylsulfinyl, Methylsulfonyl, Ethylthio, Ethylsulfinyl, Ethylsulfonyl, Trifluormethylsulfanyl, Trifluormethylsulfinyl, Trifluormethylsulfonyl, Chlor-difluormethylsulfanyl, Chlor-difluormethylsulfinyl, Chlor-difluormethylsulfonyl, Dichlor-fluormethylsulfanyl, Dichlor-fluormethylsulfinyl, Dichlor-fluormethylsulfonyl und
- Z³: für Wasserstoff, Methyl, Ethyl, n-Propyl, Isopropyl, n-Butyl, Isobutyl, s-Butyl, t-Butyl, Ethenyl, 1-Propenyl, 2-Propenyl, 1-Propinyl, 1-Butinyl, Difluormethyl, , Chlordifluormethyl, Dichlorfluormethyl, Trifluormethyl, Chlormethyl, Brommethyl, 1-Fluorethyl, 1-Fluor-1-methylethyl, 2-Fluorethyl, 2,2-Difluorethyl, 2,2,2-Trifluorethyl, Phenyl, 2-Chlorphenyl, 3-Chlorphenyl. 4-Chlophenyl, 2,5-Dichlorphenyl, 2,4-Dichlorphenyl, 3,4-Dichlorphenyl, 2,6-Dichlorphenyl, 2,6-Dichlor-4-trifluormehtylphenyl, 3-Chlor-5-trifluormethylpyridin-2-yl stehen.

Weiterhin hierin beschrieben sind Verbindungen der allgemeinen Formel (I), in denen
- Z¹: für Trifluormethyl, 1-Chlor-cyclopropyl, 1-Fluor-cyclopropyl oder Pentafluorethyl steht;
- Z²: für Trifluormethyl, Nitro, Methylsulfanyl, Methylsulfinyl, Methylsulfonyl, Fluor, Chlor, Brom, Cyano oder Iod steht;
- Z³: für Methyl, Ethyl, n-Propyl oder Wasserstoff steht;
- R¹: für Wasserstoff, Methyl, Ethyl, n-Propyl, Isopropyl, n-Butyl, i-Butyl, s-Butyl, t-Butyl, Prop-2-enyl, 2-Methylprop-2-enyl, Prop-2-inyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht;
- A₁ und A₂: jeweils für CH stehen;
- A₃: für CR⁴ und
- A₄: für CR⁵ stehen;
- R⁴: für Wasserstoff, Fluor, Chlor, Brom, Iod oder Cyano steht;
- R⁵: für Wasserstoff, Fluor, Chlor, Brom, Iod, Methyl oder Methoxy steht;
- T: für einen der nachfolgend aufgeführten 5-gliedrigen Heteroaromaten T1-T10 steht, wobei die Bindung zur Pyrazolkopfgruppe mit einem Sternchen gekennzeichnet ist, 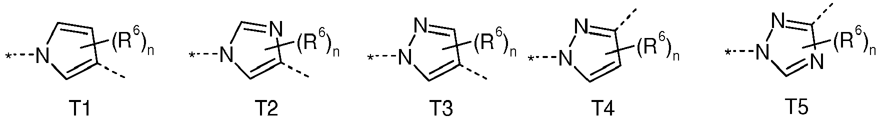 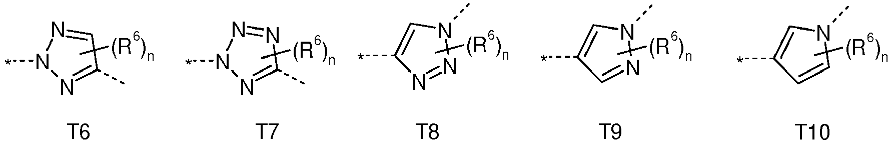
wobei
- R⁶: für Wasserstoff (n = 0), Methyl, Ethyl, 2-Methylethyl, 2,2-Dimethylethyl, Fluor, Chlor, Brom, Iod, Nitro, Trifluormethyl, Amino steht;
- W: für Sauerstoff steht;
- Q: für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1,1,2,2-Tetrafluorethyl, 1-Trifluormethylethyl, 2,2-Difluorpropyl, 3,3,3-Trifluropropyl, 2,2-Dimethyl-3-fluorpropyl, Cyclopropyl, 1-Cyano-cyclopropyl, 1-Chlor-cyclopropyl, 1-Methyl-cyclopropyl, 1-Methoxycarbonyl-cyclopropyl, 1-(*N*-Methylcarbamoyl)cyclopropyl, 1-(*N-*Cyclopropylcarbamoyl)cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl, 3,3-Dichlor-prop-2-enyl, 3,3-Dichlor-1,1-dimethylprop-2-enyl, Oxetan-3-yl, Thietan-3-yl, 1-Oxido-thietan-3-yl, 1,1-Dioxido-thietan-3-yl, Isoxazol-3-ylmethyl, 3-Methyloxetan-3-ylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, Pyridin-2--ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N-*Allylamino, *N*,*N*-Dimethylamino, *N*,*N*-Diethylamino, Methylsulfonylamino steht, ganz besonders bevorzugt für Wasserstoff steht; oder
- Q: für ein mit 0, 1, 2, 3, oder 4 Substituenten V substituiertes Phenyl, Naphthyl, Pyridazinyl, Pyrazinyl, Pyrimidinyl, Triazinyl, Pyridinyl, Pyrazolyl, Thiazolyl, Isothiazolyl, Oxazolyl, Isoxazolyl, Triazolyl, Imidazolyl, Furanyl, Thiophenyl, Pyrrolyl, Oxadiazolyl, Thiadiazolyl steht, wobei
- V: unabhängig voneinander für Fluor, Chlor, Brom, Iod, Cyano, Nitro, Methyl, Ethyl, Difluormethyl, , Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N*,*N*-Dimethylamino steht.

Weiterhin hierin beschrieben sind Verbindungen der allgemeinen Formel (I), in denen
- Z¹: für Trifluormethyl, 1-Chlor-cyclopropyl, 1-Fluor-cyclopropyl oder Pentafluorethyl steht;
- Z²: für Trifluormethyl, Nitro, Methylsulfanyl, Methylsulfinyl, Methylsulfonyl, Fluor, Chlor, Brom, Cyano oder Iod steht;
- Z³: für Methyl, Ethyl, n-Propyl oder Wasserstoff steht;
- R¹: für Wasserstoff, Methyl, Ethyl, n-Propyl, Isopropyl, n-Butyl, i-Butyl, s-Butyl, t-Butyl, Prop-2-enyl, 2-Methylprop-2-enyl, Prop-2-inyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht, ganz besonders bevorzugt für Wasserstoff steht;
- A₁: für CH steht;
- A₂: für Stickstoff steht;
- A₃: für CR⁴ und
- A₄: für CR⁵ stehen;
- R⁴: für Wasserstoff, Fluor, Chlor, Brom, Iod oder Cyano steht;
- R⁵: für Wasserstoff, Fluor, Chlor, Brom, Iod, Methyl oder Methoxy steht;
- T: für einen der nachfolgend aufgeführten 5-gliedrigen Heteroaromaten T1-T10 steht, wobei die Bindung zur Pyrazolkopfgruppe mit einem Sternchen gekennzeichnet ist, 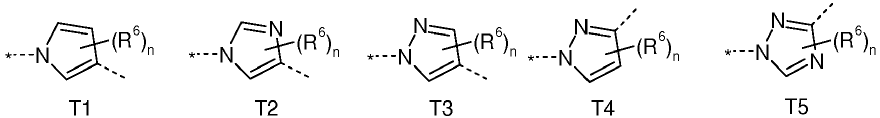 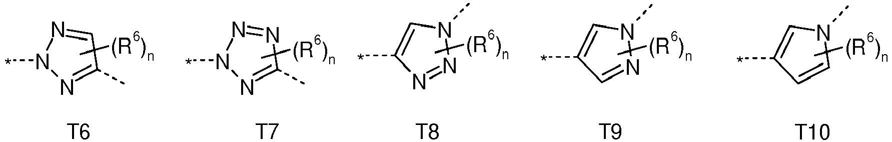
wobei
- R⁶: für Wasserstoff (n = 0), Methyl, Ethyl, 2-Methylethyl, 2,2-Dimethylethyl, Fluor, Chlor, Brom, Iod, Nitro, Trifluormethyl, Amino steht;
- W: für Sauerstoff steht;
- Q: für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1,1,2,2-Tetrafluorethyl, 1-Trifluormethylethyl, 2,2-Difluorpropyl, 3,3,3-Trifluropropyl, 2,2-Dimethyl-3-fluorpropyl, Cyclopropyl, 1-Cyano-cyclopropyl, 1-Chlor-cyclopropyl, 1-Methyl-cyclopropyl, 1-Methoxycarbonyl-cyclopropyl, 1-(*N*-Methylcarbamoyl)cyclopropyl, 1-(*N-*Cyclopropylcarbamoyl)cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl, 3,3-Dichlor-prop-2-enyl, 3,3-Dichlor-1,1-dimethylprop-2-enyl, Oxetan-3-yl, Thietan-3-yl, 1-Oxido-thietan-3-yl, 1,1-Dioxido-thietan-3-yl, Isoxazol-3-ylmethyl, 3-Methyloxetan-3-ylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, Pyridin-2--ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N-*Allylamino, *N*,*N*-Dimethylamino, *N*,*N*-Diethylamino, Methylsulfonylamino steht; oder
- Q: für ein mit 0, 1, 2, 3, oder 4 Substituenten V substituiertes Phenyl, Naphthyl, Pyridazinyl, Pyrazinyl, Pyrimidinyl, Triazinyl, Pyridinyl, Pyrazolyl, Thiazolyl, Isothiazolyl, Oxazolyl, Isoxazolyl, Triazolyl, Imidazolyl, Furanyl, Thiophenyl, Pyrrolyl, Oxadiazolyl, Thiadiazolyl steht, wobei
- V: unabhängig voneinander für Fluor, Chlor, Brom, Iod, Cyano, Nitro, Methyl, Ethyl, Difluormethyl, , Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N*,*N*-Dimethylamino steht.

Hierin beschrieben sind ferner die Verbindungen, die jeweils durch eine der allgemeinen Formeln (Ia), (Ib), (Ic), (Id), (Ie), (If), (Ig), (Ih), (Ii) und (Ij) definiert sind, in denen die Reste A₁-A₄, n, W, Q, R¹ und Z¹-Z³ die oben beschriebenen Bedeutungen haben.

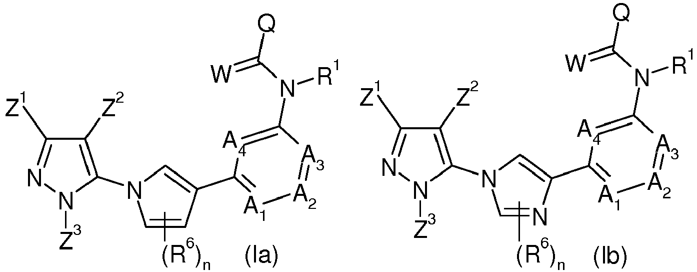

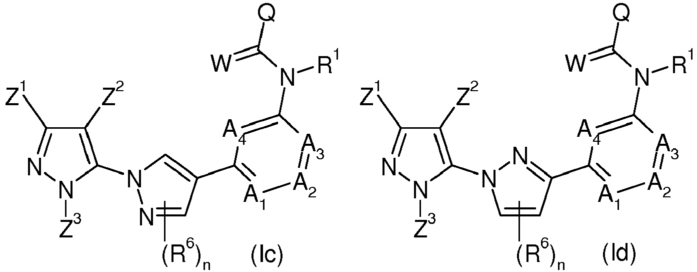

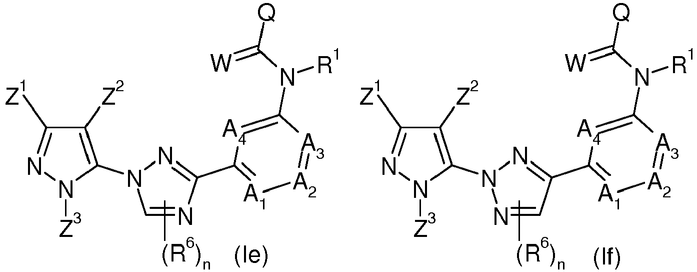

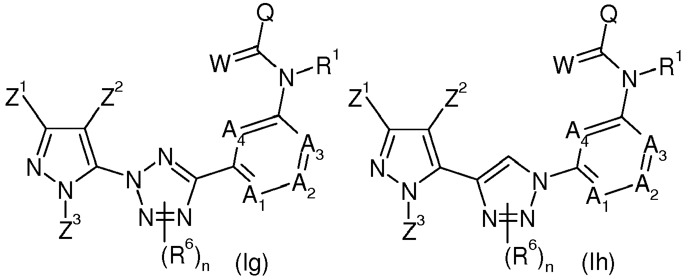

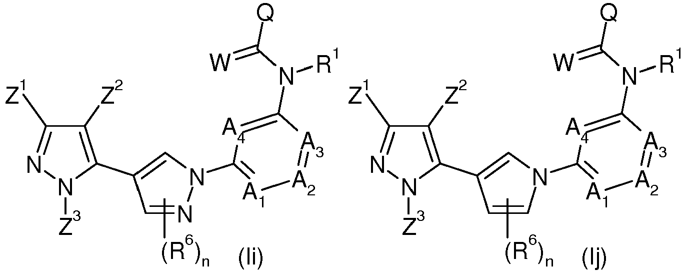

Ganz besonders bevorzugt sind Verbindungen der allgemeinen Formel (Ic), in denen Z¹ für CF₂CF₃ steht, Z² für CF_{3,} Z³ für CH₃, die Reste R¹, R⁶ für Wasserstoff (n= 0) stehen, A₁, A₂, für C-H, A₃ für C-H, C-Cl, C-F, A₄ für C-H, C-F, C-OMe stehen, W für Sauerstoff und Q für Methyl, Ethyl, Cyclopropyl, 1-Chlor-cyclopropyl, 1-Cyano-cyclopropyl, 2,2,2-Trifluorethyl, 1,1,2,2-Tetrafluorethyl, Phenyl, 4-Fluorphenyl, 2-Fluorphenyl, 3-Fluorphenyl, 3,5-Difluorphenyl, 2,6-Difluorphenyl, 2-Thiophenyl, 3-Thiophenyl, 3-Chlor-3-thiophenyl, 1-Methyl-4-pyrazolyl, 4-Pyridinyl, 3-Chlor-2-Pyridinyl, 2-Chlor-4-Pyridinyl, 3-Fluor-4-Pyridinyl, 2,6-Dichlor-4-Pyridinyl stehen.

Ganz besonders bevorzugt sind Verbindungen der allgemeinen Formel (Ic), in denen Z¹ für CF₂CF₃ steht, Z² für CF_{3,} Z³ für CH₃, die Reste R¹, R⁶ für Wasserstoff (n= 0) stehen, A₁ für C-H, A₂ für N, A₃ für C-H, C-C1, C-F, C-CH₃, A₄ für C-H, C-F, C-OMe stehen, W für Sauerstoff und Q für Methyl, Ethyl, Cyclopropyl, 1-Chlor-cyclopropyl, 1-Cyano-cyclopropyl, 2,2,2-Trifluorethyl, 1,1,2,2-Tetrafluorethyl, Phenyl, 4-Fluorphenyl, 2-Fluorphenyl, 3-Fluorphenyl, 3,5-Difluorphenyl, 2,6-Difluorphenyl, 2-Thiophenyl, 3-Thiophenyl, 3-Chlor-3-thiophenyl, 1-Methyl-4-pyrazolyl, 4-Pyridinyl, 3-Chlor-2-Pyridinyl, 2-Chlor-4-Pyridinyl, 3-Fluor-4-Pyridinyl, 2,6-Dichlor-4-Pyridinyl stehen.

Weiterhin hierin beschriebensind Verbindungen der allgemeinen Formel (Ih), in denen Z¹ für CF₂CF₃ steht, Z² für CF₃, Z³ für CH₃, die Reste R¹, R⁶ für Wasserstoff (n= 0) stehen, A₁, A₂, für C-H, A₃ für C-H, C-C1, C-F, A₄ für C-H, C-F, C-OMe stehen, W für Sauerstoff und Q für Methyl, Ethyl, Cyclopropyl, 1-Chlor-cyclopropyl, 1-Cyano-cyclopropyl, 2,2,2-Trifluorethyl, 1,1,2,2-Tetrafluorethyl, Phenyl, 4-Fluorphenyl, 2-Fluorphenyl, 3-Fluorphenyl, 3,5-Difluorphenyl, 2,6-Difluorphenyl, 2-, 3-Thiophenyl, 3-Chlor-3-Thiophenyl, 1-Methyl-4-pyrazolyl, 4-Pyridinyl, 3-Chlor-2-Pyridinyl, 2-Chlor-4-Pyridinyl, 3-Fluor-4-Pyridinyl, 2,6-Dichlor-4-Pyridinyl stehen.

Weiterhin hierin beschriebensind Verbindungen der allgemeinen Formel (Ih), in denen Z¹ für CF₂CF₃ steht, Z² für CF₃, Z³ für CH₃, die Reste R¹, R⁶ für Wasserstoff (n= 0) stehen, A₁ für C-H, A₂ für N, A₃ für C-H, C-C1, C-F, C-CH₃, A₄ für C-H, C-F, C-OMe stehen, W für Sauerstoff und Q für Methyl, Ethyl, Cyclopropyl, 1-Chlor-cyclopropyl, 1-Cyano-cyclopropyl, 2,2,2-Trifluorethyl, 1,1,2,2-Tetrafluorethyl, Phenyl, 4-Fluorphenyl, 2-Fluorphenyl, 3-Fluorphenyl, 3,5-Difluorphenyl, 2,6-Difluorphenyl, 2-Thiophenyl, 3-Thiophenyl, 3-Chlor-3-thiophenyl, 1-Methyl-4-pyrazolyl, 4-Pyridinyl, 3-Chlor-2-Pyridinyl, 2-Chlor-4-Pyridinyl, 3-Fluor-4-Pyridinyl, 2,6-Dichlor-4-Pyridinyl stehen.

### Definitionen

Erfindungsgemäß steht "Alkyl" - in Alleinstellung oder als Bestandteil einer chemischen Gruppe - für geradkettige oder verzweigte Kohlenstoffwasserstoffe, vorzugsweise mit 1 bis 6 Kohlenstoffatomen, wie beispielsweise Methyl, Ethyl, n-Propyl, Isopropyl, n-Butyl, Isobutyl, s-Butyl, t-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylpropyl, 1,3-Dimethylbutyl, 1,4-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl und 2-Ethylbutyl. Ferner bevorzugt sind Alkyle mit 1 bis 4 Kohlenstoffatomen, wie unter anderem Methyl, Ethyl, Ethyl, n-Propyl, Isopropyl, n-Butyl, Isobutyl, s-Butyl oder t-Butyl. Die erfindungsgemäßen Alkyle können mit einem oder mehreren, gleichen oder verschiedenen Resten substituiert sein.

Erfindungsgemäß steht "Alkenyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen und mindestens einer Doppelbindung, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-dimethyl-3-butenyl, 1,2-Dimethyl-2-butenyl, 1,2-Dimethyl-3-butenyl, 1,3-Dimethyl-2-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl. Ferner bevorzugt sind Alkenyle mit 2 bis 4 Kohlenstoffatomen, wie unter anderem 2-Propenyl, 2-Butenyl oder 1-Methyl-2-propenyl. Die erfindungsgemäßen Alkenyle können mit einem oder mehreren, gleichen oder verschiedenen Resten substituiert sein.

Erfindungsgemäß steht "Alkinyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen und mindestens einer Dreifachbindung wie beispielsweise 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-3-butinyl, 1,2-Dimethyl-3-butinyl, 2,2-Dimethyl-3-butinyl, 1-Ethyl-3-butinyl, 2-Ethyl-3-butinyl, 1-Ethyl-1-methyl-2-propinyl und 2,5-Hexadiynyl. Ferner bevorzugt sind Alkinyle mit 2 bis 4 Kohlenstoffatomen wie unter anderem Ethinyl, 2-Propinyl oder 2-Butinyl-2-propenyl. Die erfindungsgemäßen Alkinyle können mit einem oder mehreren, gleichen oder verschiedenen Resten substituiert sein.

Erfindungsgemäß steht "Cycloalkyl" - in Alleinstellung oder als Bestandteil einer chemischen Gruppe - für mono-, bi- oder tricyclische Kohlenwasserstoffe, vorzugsweise mit 3 bis 10 Kohlenstoffen wie beispielsweise Cyclopropyl, Cyclobutyl, Cyclopentyl, Cyclohexyl, Cycloheptyl, Cyclooctyl, Bicyclo[2.2.1]heptyl, Bicyclo[2.2.2]octyl oder Adamantyl. Ferner bevorzugt sind Cycloalkyle mit 3, 4, 5, 6 oder 7 Kohlenstoffatomen, wie unter anderem Cyclopropyl oder Cyclobutyl. Die erfindungsgemäßen Cycloalkyle können mit einem oder mehreren, gleichen oder verschiedenen Resten substituiert sein.

Erfindungsgemäß steht "Alkylcycloalkyl" für mono-, bi- oder tricyclisches Alkylcycloalkyl, vorzugsweise mit 4 bis 10 oder 4 bis 7 Kohlenstoffatomen, wie beispielsweise Ethylcyclopropyl, Isopropylcyclobutyl, 3-Methylcyclopentyl und 4-Methyl-cyclohexyl. Ferner bevorzugt sind Alkylcycloalkyle mit 4, 5 oder 7 Kohlenstoffatomen wie unter anderen Ethylcyclopropyl oder 4-Methyl-cyclohexyl. Die erfindungsgemäßen Alkylcycloalkyle können mit einem oder mehreren, gleichen oder verschiedenen Resten substituiert sein.

Erfindungsgemäß steht "Cycloalkylalkyl" für mono, bi- oder tricyclisches Cycloalkylalkyl, vorzugsweise mit 4 bis 10 oder 4 bis 7 Kohlenstoffatomen, wie beispielsweise Cyclopropylmethyl, Cyclobutylmethyl, Cyclopentylmethyl, Cyclohexylmethyl und Cyclopentylethyl. Ferner bevorzugt sind Cycloalkylalkyle mit 4, 5 oder 7 Kohlenstoffatomen wie unter anderen Cyclopropylmethyl oder Cyclobutylmethyl. Die erfindungsgemäßen Cycloalkylalkyle können mit einem oder mehreren, gleichen oder verschiedenen Resten substituiert sein.

Erfindungsgemäß steht "Halogen" für Fluor, Chlor, Brom oder Iod, insbesondere für Fluor, Chlor oder Brom.

Die erfindungsgemäßen mit Halogen substituierten chemischen Gruppen, wie beispielsweise Halogenalkyl, Halogencycloalkyl, Halogenalkyloxy, HalogenAlkylsulfanyl, Halogenalkylsulfinyl oder Halogenalkylsulfonyl sind einfach oder mehrfach bis zur maximal möglichen Substituentenzahl mit Halogen substituiert. Bei mehrfacher Substitution mit Halogen, können die Halogenatome gleich oder verschieden sein und können alle an eines oder an mehrere Kohlenstoffatome gebunden sein. Dabei steht Halogen insbesondere für Fluor, Chlor, Brom oder Iod, vorzugsweise für Fluor, Chlor oder Brom und besonders bevorzugt für Fluor.

Erfindungsgemäß steht "Halogencycloalkyl" für mono-, bi- oder tricyclisches Halogencycloalkyl, vorzugsweise mit 3 bis 10 Kohlenstoffatomen, wie unter anderen 1-Fluor-cyclopropyl, 2-Fluor-cyclopropyl oder 1-Fluor-cyclobutyl. Ferner bevorzugt ist Halogencycloalkyl mit 3, 5 oder 7 Kohlenstoffatomen. Die erfindungsgemäßen Halogencycloalkyle können mit einem oder mehreren, gleichen oder verschiedenen Resten substituiert sein.

Erfindungsgemäß steht "Halogenalkyl" "Halogenalkenyl" oder "Halogenalkinyl" für mit Halogen substituierte Alkyle, Alkenyle oder Alkinyle mit vorzugsweise 1 bis 9 gleichen oder verschiedenen Halogenatomen, wie beispielsweise Monohaloalkyl (= Monohalogenalkyl) wie CH2CH2Cl, CH2CH2F, CHClCH3, CHFCH3, CH2Cl, CH2F; Perhaloalkyl wie CCl3 oder CF3 oder CF2CF3; Polyhaloalkyl wie CHF2, CH2F, CH2CHFCl, CHCl2, CF2CF2H, CH2CF3. Entsprechendes gilt für Haloalkenyl und andere durch Halogen substituierten Reste. Haloalkoxy ist z.B. OCF3, OCHF2, OCH2F, OCF2CF3, OCH2CF3 und OCH2CH2Cl;

Weitere Beispiele für Halogenalkyle sind Chlordifluormethyl, Dichlorfluormethyl, Chlormethyl, Brommethyl, 1-Fluorethyl, 2-Fluorethyl, 2,2-Difluorethyl, 2,2,2-Trifluorethyl, 2,2,2-Trichlorethyl, 2-Chlor-2,2-difluorethyl, Pentafluorethyl und Pentafluor-t-butyl. Bevorzugt sind Halogenalkyle mit 1 bis 4 Kohlenstoffatomen und 1 bis 9, vorzugsweise 1 bis 5 gleichen oder verschiedenen Halogenatomen, die ausgewählt sind unter Fluor, Chlor oder Brom. Besonders bevorzugt sind Halogenalkyle mit 1 oder 2 Kohlenstoffatomen und mit 1 bis 5 gleichen oder verschiedenen Halogenatomen, die ausgewählt sind unter Fluor oder Chlor, wie unter anderen Difluormethyl, Trifluormethyl oder 2,2-Difluorethyl.

Erfindungsgemäß steht "Hydroxyalkyl" für geradkettigen oder verzweigten Alkohol, vorzugsweise mit 1 bis 6 Kohlenstoffatomen, wie beispielsweise Methanol, Ethanol, n-Propanol, Isopropanol, n-Butanol, Isobutanol, s-Butanol und t-Butanol. Ferner bevorzugt für Hydroxyalkylgruppen mit 1 bis 4 Kohlenstoffatomen. Die erfindungsgemäßen Hydroxyalkylgruppen können mit einem oder mehreren, gleichen oder verschiedenen Resten substituiert sein

Erfindungsgemäß steht "Alkoxy" für geradkettiges oder verzweigtes O-Alkyl, vorzugsweise mit 1 bis 6 Kohlenstoffatomen, wie beispielsweise Methoxy, Ethoxy, n-Propoxy, Isopropoxy, n-Butoxy, Isobutoxy, s-Butoxy und t-Butoxy. Ferner bevorzugt für Alkoxygruppen mit 1 bis 4 Kohlenstoffatomen. Die erfindungsgemäßen Alkoxygruppen können mit einem oder mehreren, gleichen oder verschiedenen Resten substituiert sein.

Erfindungsgemäß steht "Halogenalkoxy" für mit Halogen substituiertes geradkettiges oder verzweigtes O-Alkyl, vorzugsweise mit 1 bis 6 Kohlenstoffatomen, wie unter anderem Difluormethoxy, Trifluormethoxy, 2,2-Difluorethoxy, 1,1,2,2-Tetrafluorethoxy, 2,2,2-Trifluorethoxy und 2-Chlor-1,1,2-trifluorethoxy. Ferner bevorzugt für Halogenalkoxygruppen mit 1 bis 4 Kohlenstoffatomen. Die erfindungsgemäßen Halogenalkoxygruppen können mit einem oder mehreren, gleichen oder verschiedenen Resten substituiert sein.

Erfindungsgemäß steht "Alkylsulfanyl" für geradkettiges oder verzweigtes S-Alkyl, vorzugsweise mit 1 bis 6 Kohlenstoffatomen, wie beispielsweise Methylthio, Ethylthio, n-Propylthio, Isopropylthio, n-Butylthio, Isobutylthio, s-Butylthio und t-Butylthio. Ferner bevorzugt für Alkylsulfanylgruppen mit 1 bis 4 Kohlenstoffatomen. Die erfindungsgemäßen Alkylsulfanylgruppen können mit einem oder mehreren, gleichen oder verschiedenen Resten substituiert sein.

Beispiele für Halogenalkylsulfanyl, d.h. mit Halogen substituierte Alkylsulfanylgruppen, sind unter anderem Difluormethylthio, Trifluormethylthio, Chlordifluormethylthio, 1-Fluorethylthio, 2-Fluorethylthio, 2,2-Difluorethylthio, 1,1,2,2-Tetrafluorethylthio, 2,2,2-Trifluorethylthio oder 2-Chlor-1,1,2-trifluorethylthio.

Erfindungsgemäß steht "Alkylsulfinyl" für geradkettiges oder verzweigtes Alkylsulfinyl, vorzugsweise mit 1 bis 6 Kohlenstoffatomen wie beispielsweise Methylsulfinyl, Ethylsulfinyl, n-Propylsulfinyl, Isopropylsulfinyl, n-Butylsulfinyl, Isobutylsulfinyl, s-Butylsulfinyl und t-Butylsulfinyl. Ferner bevorzugt für Alkylsulfinylgruppen mit 1 bis 4 Kohlenstoffatomen. Die erfindungsgemäßen Alkylsulfinylgruppen können mit einem oder mehreren, gleichen oder verschiedenen Resten substituiert sein.

Beispiele für Halogenalkylsulfinylgrupen, d.h. mit Halogen substituierte Alkylsulfinylgruppen, sind unter anderem Difluormethylsulfinyl, Trifluormethylsulfinyl, Chlordifluormethylsulfinyl, 1-Fluorethylsulfinyl, 2-Fluorethylsulfinyl, 2,2-Difluorethylsulfinyl, 1,1,2,2-Tetrafluorethylsulfinyl, 2,2,2-Trifluorethylsulfinyl und 2-Chlor-1,1 ,2-trifluorethylsulfinyl.

Erfindungsgemäß steht "Alkylsulfonyl" für geradkettiges oder verzweigtes Alkylsulfonyl, vorzugsweise mit 1 bis 6 Kohlenstoffatomen wie beispielsweise Methylsulfonyl, Ethylsulfonyl, n-Propylsulfonyl, Isopropylsulfonyl, n-Butylsulfonyl, Isobutylsulfonyl, s-Butylsulfonyl und t-Butylsulfonyl. Ferner bevorzugt für Alkylsulfonylgruppen mit 1 bis 4 Kohlenstoffatomen. Die erfindungsgemäßen Alkylsulfonylgruppen können mit einem oder mehreren, gleichen oder verschiedenen Resten substituiert sein.

Beispiele für Halogenalkylsulfonylgrupen, d.h. mit Halogen substituierte Alkylsulfonylgruppen sind unter anderem Difluormethylsulfonyl, Trifluormethylsulfonyl, sulfonyl, Chlordifluormethylsulfonyl, 1-Fluorethylsulfonyl, 2-Fluorethylsulfonyl, 2,2-Difluorethylsulfonyl, 1,1,2,2-Tetrafluorethylsulfonyl, 2,2,2-Trifluorethyl-sulfonyl und 2-Chlor-1,1,2-trifluorethylsulfonyl.

Erfindungsgemäß steht "Alkylcarbonyl" für geradkettiges oder verzweigtes Alkyl-C(=O), vorzugsweise mit 2 bis 7 Kohlenstoffatomen, wie Methylcarbonyl, Ethylcarbonyl, n-Propylcarbonyl, Isopropylcarbonyl, s-Butylcarbonyl und t-Butylcarbonyl. Ferner bevorzugt für Alkylcarbonyle mit 1 bis 4 Kohlenstoffatomen. Die erfindungsgemäßen Alkylcarbonyle können mit einem oder mehreren, gleichen oder verschiedenen Resten substituiert sein.

Erfindungsgemäß steht "Cycloalkylcarbonyl" für geradkettiges oder verzweigtes Cycloalkylcarbonyl, vorzugsweise mit 3 bis 10 Kohlenstoffatomen im Cycloalkylteil, wie beispielsweise Cyclopropylcarbonyl, Cyclobutylcarbonyl, Cyclopentylcarbonyl, Cyclohexyl-carbonyl, Cycloheptyl-'carbonyl, Cyclooctylcarbonyl, Bicyclo[2.2.1]heptyl, Bycyclo[2.2.2]octylcarbonyl und Adamantylcarbonyl. Ferner bevorzugt ist Cycloalkylcarbonyl mit 3, 5 oder 7 Kohlenstoffatomen im Cycloalkylteil. Die erfindungsgemäßen Cycloalkylcarbonylgruppen können mit einem oder mehreren, gleichen oder verschiedenen Resten substituiert sein.

Erfindungsgemäß steht "Alkoxycarbonyl" - in Alleinstellung oder als Bestandteil einer chemischen Gruppe - für geradkettiges oder verzweigtes Alkoxycarbonyl, vorzugsweise mit 1 bis 6 Kohlenstoffatomen oder 1 bis 4 Kohlenstoffatomen im Alkoxyteil, wie beispielsweise Methoxycarbonyl, Ethoxycarbonyl, n-Propoxycarbonyl, Isopropoxycarbonyl, s-Butoxycarbonyl und t-Butoxycarbonyl. Die erfindungsgemäßen Alkoxycarbonylgruppen können mit einem oder mehreren, gleichen oder verschiedenen Resten substituiert sein.

Erfindungsgemäß steht "Alkylaminocarbonyl" für geradkettiges oder verzweigtes Alkylaminocarbonyl mit vorzugsweise 1 bis 6 Kohlenstoffatomen oder 1 bis 4 Kohlenstoffatomen im Alkylteil, wie beispielsweise Methylaminocarbonyl, Ethylaminocarbonyl, n-Propylaminocarbonyl, Isopropylaminocarbonyl, s-Butylaminocarbonyl und t-Butylaminocarbonyl. Die erfindungsgemäßen Alkylaminocarbonylgruppen können mit einem oder mehreren, gleichen oder verschiedenen Resten substituiert sein.

Erfindungsgemäß steht "N,N-Dialkylamino-carbonyl" für geradkettiges oder verzweigtes N,N-Dialkylaminocarbonyl mit vorzugsweise 1 bis 6 Kohlenstoffatomen oder 1 bis 4 Kohlenstoffatomen im Alkylteil, wie beispielsweise N,N-Dimethylamino-carbonyl, N.N-Diethylamino-carbonyl, N,N-Di(n-propylamino)-carbonyl, N,N-Di-(isopropylamino)-carbonyl und N,N-Di-(s-butylamino)-carbonyl. Die erfindungsgemäßen N,N-Dialkylamino-carbonylgruppen können mit einem oder mehreren, gleichen oder verschiedenen Resten substituiert sein.

Erfindungsgemäß steht "Aryl" oder für ein mono, bi- oder polycyclisches aromatisches System mit vorzugsweise 6 bis 14, insbesondere 6 bis 10 Ring-Kohlenstoffatomen, wie beispielsweise Phenyl, Naphthyl, Anthryl, Phenanthrenyl, vorzugsweise Phenyl. Ferner steht Aryl auch für mehrcyclische Systeme, wie Tetrahydronaphtyl, Indenyl, Indanyl, Fluorenyl, Biphenyl, wobei die Bindungsstelle am aromatischen System ist. Ebenfalls umfasst sind bicyclische Carbocyclen (nur Kohlenstoff als Ringatome) von denen ein Ring ein Aryl und der zweite Ring kein Aryl darstellt, z. B. Tetralinyl (C₁₀H₁₁) wobei die Bindungsstelle am aromatischen oder am nicht-aromatischen Ring liegen kann. Die erfindungsgemäßen Arylgruppen können mit einem oder mehreren, gleichen oder verschiedenen Resten substituiert sein.

Beispiele substitutierter Aryle stellen die Arylalkyle dar, die gleichfalls mit einem oder mehreren, gleichen oder verschiedenen Resten im Alkyl- und/oder Arylteil substituiert sein können. Beispiele solcher Arylalkyle sind unter anderem Benzyl und 1-Phenylethyl.

Erfindungsgemäß steht "Heterocyclus", "heterocyclischer Ring" oder "heterocyclisches Ringsystem" (Heterocyclyl) für ein carbocyclisches Ringsystem mit mindestens einem Ring, in dem mindestens ein Kohlenstoffatom durch ein Heteroatom ersetzt ist, vorzugsweise durch ein Heteroatom aus der Gruppe N, O, S, P, B, Si, Se und der gesättigt oder teilweise ungesättigt ist und dabei unsubstituiert oder mit einem weiteren Substituenten substituiert sein kann, wobei die Bindungsstelle an einem Ringatom lokalisiert ist. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen. Die heterocyclischen Ringe enthalten gewöhnlicherweise nicht mehr als 4 Stickstoffatome, und/oder nicht mehr als 2 Sauerstoffatome und/oder nicht mehr als 2 Schwefelatome. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden erfindungsgemäß auch gegebenenfalls substituierte mehrcyclische, bevorzugt bicyclischeHeterocyclen umfaßt, wie beispielsweise 8-Aza-bicyclo[3.2.1]octanyl oder l-Aza-bicyclo[2.2.1]heptyl, Benzothiophenyl z. B. Benzothiophen-2-yl, Benzofuranyl z. B. Benzofuran-2-yl , [1,2,4]Triazolo[1,5-a]pyrimidinyl z. B. [1,2,4]Triazolo[1,5-a]pyrimidin-2-yl, 1,3-Benzodioxolyl z. B. 1,3-Benzodioxol-5-yl. Im Falle von gegebenenfalls substituiertem Heterocyclyl werden erfindungsgemäß auch spirocyclische Systeme umfasst, wie beispielsweise 1-Oxa-5-aza-spiro[2.3]hexyl.

Erfindungsgemäße Heterocyclylgruppen sind beispielsweise Piperidinyl, Piperazinyl, Morpholinyl, Thiomorpholinyl, Dihydropyranyl, Tetrahydropyranyl, Dioxanyl, Pyrrolinyl, Pyrrolidinyl, Imidazolinyl, Imidazolidinyl, Thiazolidinyl, Oxazolidinyl, Dioxolanyl, Dioxolyl, Pyrazolidinyl, Tetrahydrofuranyl, Dihydrofuranyl, Oxetanyl, Oxiranyl, Azetidinyl, Aziridinyl, Oxazetidinyl, Oxaziridinyl, Oxazepanyl, Oxazinanyl, Azepanyl, Oxopyrrolidinyl, Dioxopyrrolidinyl, Oxomorpholinyl, Oxopiperazinyl und Oxepanyl.

Eine besondere Bedeutung kommt Heteroarylen, also heteroaromatischen Systemen zu. Erfindungsgemäß steht der Ausdruck Heteroaryl für heteroaromatische Verbindungen, das heißt vollständig ungesättigte aromatische heterocyclische Verbindungen, die unter die vorstehende Definiton von Heterocyclen fallen. Vorzugsweise für 5- bis 7-gliedrige Ringe mit 1 bis 3, vorzugsweise 1 oder 2 gleichen oder verschiedenen Heteroatomen aus der oben genannten Gruppe. Erfindungsgemäße Heteroaryle sind beispielsweise Furanyl, Thiophenyl, Pyrazolyl, Imidazolyl, 1,2,3- und 1,2,4-Triazolyl, Isoxazolyl, Thiazolyl, Isothiazolyl, 1,2,3-, 1,3,4-, 1,2,4- und 1,2,5-Oxadiazolyl, Azepinyl, Pyrrolyl, Pyridinyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl, 1,2,4-Triazolonyl und 1,2,4-Diazepinyl. Die erfindungsgemäßen Heteroarylgruppen können ferner mit einem oder mehreren, gleichen oder verschiedenen Resten substituiert sein.

Substituierte Gruppen, wie ein substituierter Alkyl-, Alkenyl-, Alkinyl-, Cycloalkyl , Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Oxo (O=), Alkoxy, Alkylsulfanyl, Hydroxy, Amino, Nitro, Carboxy oder eine der Carboxygruppe äquivalente Gruppe, Cyano, Isocyano, Azido, Alkoxycarbonyl, Alkylcarbonyl, Formyl, Carbamoyl, Mono und N,N-Dialkylamino-carbonyl, substituiertes Amino, wie Acylamino, Mono und N,N-Dialkylamino, Trialkylsilyl und gegebenenfalls substituiertes Cycloalkyl, gegebenenfalls substituiertes Aryl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, wobei beide Enantiomere der Alkylsulfinylgruppe umfasst sind, Alkylsulfonyl, Alkylphosphinyl, Alkylphosphonyl und, im Falle cyclischer Reste (= "cyclischer Grundkörper"), auch Alkyl, Haloalkyl, Alkylsulfanyl-alkyl, Alkoxy-alkyl, gegebenfalls substituiertes Mono- und N,N-Dialkyl-aminoalkyl und Hydroxyalkyl bedeuten.

Im Begriff "substituierte Gruppen" wie substituiertes Alkyl, Cycloalkyl, Aryl, Heteroaryl etc. sind als Substituenten zusätzlich zu den genannten gesättigten kohlenwasserstoffhaltigen Resten entsprechende ungesättigte aliphatische und aromatische Reste, wie jeweils gegebenenfalls substituiertes Alkenyl, Alkinyl, Oxo (O=), Alkoxy, Alkenyloxy, Alkinyloxy, Aryloxy (z. B. Phenyloxy (Phenyl-O-), Benzyloxy (C₆H₅-CH₂-O-) Alkylthio, Alkenylthio, Alkinylthio, Arylthio (z. B. Phenylthio (Phenyl-S-)), Alkenyloxycarbonyl, Alkinyloxycarbonyl, Alkenylcarbonyl, Alkinylcarbonyl, Mono und N,N-Dialkenylamino-carbonyl, Mono und Dialkinylaminocarbonyl, Mono und N,N-Dialkenylamino, Mono und N,N-Dialkinylamino, Trialkenylsilyl, Trialkinylsilyl, Cycloalkyl, Cycloalkenyl, Cycloalkinyl, Heteroaryl, Aryl wie Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Einfachbindung am Ring gebunden sind, einer Doppelbindung am Ring gebunden sind, z. B. mit einer Alkylidengruppe wie Methyliden oder Ethyliden oder einer Oxogruppe, Iminogruppe sowie einer substituierten Iminogruppe, oder auch cyclische Systeme mit solchen Substituenten umfaßt in denen ein zweiter Ring mit zwei verschiedenen Atomen an zwei verschiedene Atome des cyclischen Rest gebunden ist, z. B. Naphtyl oder Tetrahydronaphthalinyl (z. B. 1,2,3,4-Tetrahydronaphthalin-1-yl).

Wenn zwei oder mehrere Reste einen oder mehrere Ringe bilden, so können diese carbocyclisch, heterocyclisch, gesättigt, teilgesättigt, ungesättigt, beispielsweise auch aromatisch und weiter substituiert sein.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bevorzugte Substituenten für die Substituentenebenen sind beispielsweise

Amino, Hydroxy, Halogen, Nitro, Cyano, Isocyano, Mercapto, Isothiocyanato, Carboxy, Carbonamid, SF5, Aminosulfonyl, Alkyl, Cycloalkyl, Alkenyl, Cycloalkenyl, Alkinyl, N-Mono-alkyl-amino, N,N-Dialkylamino, N-Alkanoylamino, Alkoxy, Alkenyloxy, Alkinyloxy, Cycloalkoxy, Cycloalkenyloxy, Alkoxycarbonyl, Alkenyloxycarbonyl, Alkinyloxycarbonyl, Aryloxycarbonyl, Alkanoyl, Alkenylcarbonyl, Alkinylcarbonyl, Arylcarbonyl, Alkylsulfanyl, Cycloalkylsulfanyl, Alkenylthio, Cycloalkenylthio, Alkinylthio, Alkylsulfenyl und Alkylsulfinyl, wobei beide Enantiomere der Alkylsulfinylgruppe umfasst sind, Alkylsulfonyl, N-Mono-alkyl-aminosulfonyl, N,N-Dialkyl-aminosulfonyl, Alkylphosphinyl, Alkylphosphonyl, wobei für Alkylphosphinyl bzw. Alkylphosphonyl beide Enantiomere umfasst sind, N-Alkyl-aminocarbonyl, N,N-Dialkyl-amino-carbonyl, N-Alkanoylamino-carbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl und Trialkylsilyl.

Substituenten, die aus mehreren Substituentenebenen zusammengesetzt sind, sind bevorzugt Alkoxyalkyl, Alkylsulfanylalkyl, Alkylsulfanylalkoxy, Alkoxyalkoxy, Phenethyl, Benzyloxy, Halogenalkyl, Halogencycloalkyl, Halogenalkoxy, Halogenalkylsulfanyl, Halogenalkylsulfinyl, Halogenalkylsulfonyl, Halogenalkanoyl, Halogenalkylcarbonyl, Halogenalkoxycarbonyl, Halogenalkoxyalkoxy, Halogenalkoxyalkylsulfanyl, Halogenalkoxyalkanoyl, Halogenalkoxyalkyl.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Hydroxy, Amino, Alkoxy, Acyl und Aryl N-substituiert sind; vorzugsweise N-Mono- und N,N-Dialkylamino, (z.B. Methylamino, Ethylamino, N,N-Dimethylamino, N,N-Diethylamino, N,N-Di-n-propylamino, N,N-Diisopropylamino oder N,N-Dibutylamino), N-Mono- oder N,N-Dialkoxyalkylaminogruppen (z.B. N-Methoxymethylamino, N-Methoxyethylamino, N,N-Di-(methoxymethyl)-amino oder N,N-Di-(methoxyethyl)-amino), N-Mono- und N,N-Diarylamino, wie gegebenenfalls substituierte Aniline, Acylamino, N,N-diacylamino, N-Alkyl N-arylamino, N-Alkyl N-acylamino sowie gesättigte N-Heterocyclen; dabei sind Alkylreste mit 1 bis 4 C Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Erfindungsgemäß umfasst der Begriff "cyclische Aminogruppen" heteroaromatische oder aliphatische Ringsysteme mit einem oder mehreren Stickstoffatomen. Die Heterocyclen sind gesättigt oder ungesättigt, bestehen aus einem oder mehreren, gegebenenfalls kondensierten Ringsystemen und beinhalten gegebenenfalls weitere Heteroatome, wie beispielsweise ein oder zwei Stickstoff-, Sauerstoff- und/oder Schwefelatome. Ferner umfasst der Begriff auch solche Gruppen, die einen Spiroring oder verbrücktes Ringsystem aufweisen. Die Anzahl der Atome, die die cyclische Aminogruppe bilden, ist beliebig und kann z.B. im Falle eines Einringsystems aus 3 bis 8 Ringatomen und im Falle eines Zweiringsystems aus 7 bis 11 Atomen bestehen.

Beispielhaft für cyclische Aminogruppen mit gesättigten und ungesättigten monocyclischen Gruppen mit einem Stickstoffatom als Heteroatom seien 1-Azetidinyl, Pyrrolidino, 2-Pyrrolidin-1-yl, 1-Pyrrolyl, Piperidino, 1,4-Dihydropyrazin-1-yl, 1,2,5,6-Tetrahydropyrazin-1-yl, 1,4-Dihydropyridin-1-yl, 1,2,5,6-Tetrahydropyridin-1-yl, Homopiperidinyl genannt; beispielhaft für cyclische Aminogruppen mit gesättigten und ungesättigten monocyclischen Gruppen mit zwei oder mehreren Stickstoffatomen als Heteroatome seien 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydro-piperazin-1-yl, 1,2-Dihydro-pyrimidin-1-yl, Perhydropyrimidin-1-yl, 1,4-Diazacycloheptan-1-yl, genannt; beispielhaft für cyclische Aminogruppen mit gesättigten und ungesättigten monocyclischen Gruppen mit einem oder zwei Sauerstoffatomen und einem bis drei Stickstoffatomen als Heteroatome, wie beispielsweise Oxazolidin-3-yl, 2,3-Dihydroisoxazol-2-yl, Isoxazol-2-yl, 1,2,3-Oxadiazin-2-yl, Morpholino, beispielhaft für cyclische Aminogruppen mit gesättigten und ungesättigten monocyclischen Gruppen mit einem bis drei Stickstoffatomen und einem bis zwei Schwefelatomen als Heteroatome seien Thiazolidin-3-yl, Isothiazolin-2-yl, Thiomorpholino, oder Dioxothiomorpholino genannt; beispielhaft für cyclische Aminogruppen mit gesättigten und ungesättigten kondensierten cyclischen Gruppen seien Indol-1-yl, 1,2-Dihydrobenzimidazol-1-yl, Perhydropyrrolo[1,2-a]pyrazin-2-yl genannt; beispielhaft für cyclische Aminogruppen mit spirocyclischen Gruppen sei das 2-Azaspiro[4,5]decan-2-yl genannt; beispielhaft für cyclische Aminogruppen mit verbrückten heterocyclischen Gruppen sei das 2-Azabicyclo[2,2,1]heptan-7-yl genannt.

Substituiertes Amino schließt auch quartäre Ammoniumverbindungen (Salze) mit vier organischen Substituenten am Stickstoffatom ein.

Gegebenenfalls substituiertes Phenyl ist vorzugsweise Phenyl, das unsubstituiert oder ein oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl, (C₁-C₄)Halogenalkoxy, (C₁-C₄)Alkylsulfanyl, (C₁-C₄)Halogenalkylsulfanyl, Cyano, Isocyano und Nitro substituiert ist, z.B. o-, m- und p- Tolyl, Dimethylphenyle, 2-, 3- und 4-Chlorphenyl, 2-, 3- und 4-Fluorphenyl, 2-, 3- und 4-Trifluormethylphenyl, 2,4-, 3,5-, 2,5- und 2,3-Dichlorphenyl, o-, m- und p-Methoxyphenyl.

Gegebenenfalls substituiertes Cycloalkyl ist vorzugsweise Cycloalkyl, das unsubstituiert oder ein oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl und (C₁-C₄)Halogenalkoxy substituiert ist, insbesondere durch einen oder zwei (C₁-C₄)Alkylreste substituiert ist,

Gegebenenfalls substituiertes Heterocyclyl ist vorzugsweise Heterocyclyl, das unsubstituiert oder ein oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl, (C₁-C₄)Halogenalkoxy, Nitro und Oxo substituiert ist, insbesondere ein- oder mehrfach durch Reste aus der Gruppe Halogen, (C₁-C₄)Alkyl, (C₁-C₄)Alkoxy, (C₁-C₄)Halogenalkyl und Oxo, ganz besonders durch einen oder zwei (C₁-C₄)Alkyl-Reste substituiert ist.

Beispiele für Alkyl-substituierte Heteroaryle sind Furanylmethyl, Thiophenylmethyl, Pyrazolylmethyl, Imidazolylmethyl, 1,2,3- und 1,2,4-Triazolylmethyl, Isoxazolylmethyl, Thiazolylmethyl, Isothiazolylmethyl, 1,2,3-, 1,3,4-, 1,2,4- und 1,2,5-Oxadiazolylmethyl, Azepinylmethyl, Pyrrolylmethyl, Pyridinylmethyl, Pyridazinylmethyl, Pyrimidinylmethyl, Pyrazinylmethyl, 1,3,5-, 1,2,4- und 1,2,3-Triazinylmethyl, 1,2,4-, 1,3,2-, 1,3,6- und 1,2,6-Oxazinylmethyl, Oxepinylmethyl, Thiepinylmethyl und 1,2,4-Diazepinylmethyl.

Erfindungsgemäß geeignete Salze der erfindungsgemäßen Verbindungen, beispielsweise Salze mit Basen oder Säureadditionssalze, sind alle üblichen nicht toxischen Salze, vorzugsweise landwirtschaftlich und/oder physiologisch annehmbare Salze. Beispielsweise Salze mit Basen oder Säureadditionssalze. Bevorzugt werden Salze mit anorganischen Basen, wie beispielsweise Alkalimetallsalze (z.B. Natrium-, Kalium- oder Cäsiumsalze), Erdalkalimetallsalze (z.B. Calzium- oder Magnesiumsalze), Ammoniumsalze oder Salze mit organischen Basen, insbesondere mit organischen Aminen, wie beispielsweise Triethylammonium-, Dicyclohexylammonium-, N,N'-Dibenzylethylendiammonium-, Pyridinium-, Picolinium- oder Ethanolammoniumsalze, Salze mit anorganischen Säuren (z.B. Hydrochloride, Hydrobromide, Dihydrosulfate, Trihydrosulfate, oder Phosphate), Salze mit organischen Carbonsäuren oder organischen Sulfosäuren (z.b. Formiate, Acetate, Trifluoracetate, Maleate, Tartrate, Methansulfonate, Benzolsulfonate oder 4-Toluolsulfonate). Bekannterweise können t-Amine, wie beispielsweise manche der erfindungsgemäßen Verbindungen, N-Oxide bilden, welche ebenfalls erfindungsgemäße Salze darstellen.

Die erfindungsgemäßen Verbindungen können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

Die erfindungsgemäßen Verbindungen können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Ein Aspekt 1 bezieht sich auf Verbindungen der allgemeinen Formel (I), 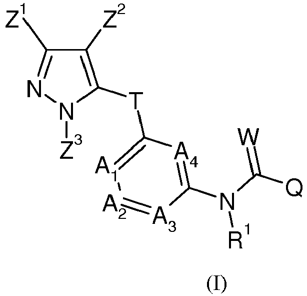 in denen
- R¹: für Wasserstoff, gegebenenfalls substituiertes C₁-C₆-Alkyl, C₃-C₆-Alkenyl, C₃-C₆-Alkinyl, C₃-C₇-Cycloalkyl, C₁-C₆-Alkylcarbonyl, C₁-C₆-Alkoxycarbonyl, Aryl-(C₁-C₃)-alkyl, Heteroaryl-(C₁-C₃)-alkyl steht, ganz besonders bevorzugt für Wasserstoff steht;
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Halogen, Cyano, Nitro, gegebenenfalls substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N*-Di-C₁-C₆-alkylamino, stehen;
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält;
- W: für Sauerstoff oder Schwefel steht;
- Q: für Wasserstoff, Amino oder eine der gegebenenfalls substituierten Gruppierungen Alkyl, Alkyloxy, Alkenyl, Alkinyl, Cycloalkyl, Heterocyclyl, Cycloalkylalkyl, Arylalkyl, Heteroarylalkyl, Alkoxycarbonyl, oder für eine Gruppierung *N*-Alkylamino, *N*-Alkylcarbonylamino, *N,N*-Dialkylamino, Alkylsulfonylamino steht; oder
- Q: für ein gegebenenfalls ein- bis fünffach mit V substituiertes Aryl steht, oder für ein gegebenenfalls ein- bis fünffach mit V substituiertes Heterorayl steht, wobei
- V: für Halogen, Cyano, Nitro, gegebenenfalls substituiertes Alkyl, Alkenyl, Alkinyl, Cycloalkyl, Alkoxy, *N*-Alkoxyiminoalkyl, Alkylsulfanyl, Alkylsulfinyl, Alkylsulfonyl, *N,N*-Dialkylamino steht;
- T: für einen der nachfolgend aufgeführten 5-gliedrigen Heteroaromaten T1-T10 steht, wobei die Bindung zur Pyrazolkopfgruppe mit einem Sternchen gekennzeichnet ist, 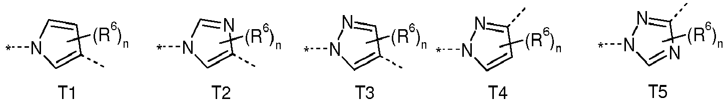 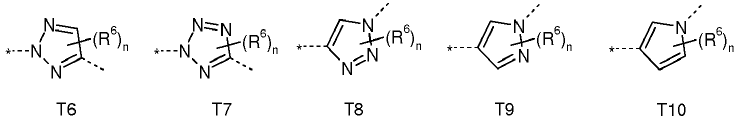
wobei
- R⁶: unabhängig voneinander für Halogen, Cyano, Nitro, Amino oder ein gegebenenfalls substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, und
- n: für die Werte 0-2 steht;
- Z¹: für ein gegebenenfalls substituiertes Alkyl und Cycloalkyl, und
- Z²: für Wasserstoff, Halogen, Cyano, Nitro, Amino oder ein gegebenenfalls substituiertes Alkyl, Alkylcarbonyl, Alkylsulfanyl, Alkylsulfinyl, Alkylsulfonyl, und
- Z³: für Wasserstoff oder ein gegebenenfalls substituiertes Alkyl, Cycloalkyl, Alkenyl, Alkinyl, Aryl oder Hetaryl stehen.

Ein Aspekt 2 bezieht sich auf Verbindungen gemäß Aspekt 1, in denen
- R¹: für Wasserstoff oder für ein gegebenenfalls ein- bis siebenfach unabhängig voneinander mit Halogen, Cyano, Alkoxy und Alkoxycarbonyl substituiertes C₁-C₆-Alkyl, C₃-C₆-Alkenyl, C₃-C₆-Alkinyl, C₃-C₇-Cycloalkyl, C₃-C₇-Cycloalkyl-C₁-C₃-alkyl, C₁-C₆-Alkylcarbonyl, C₁-C₆-Alkoxycarbonyl, Aryl(C₁-C₃)-alkyl, Heteroaryl(C₁-C₃)-alkyl steht, ganz besonders bevorzugt für Wasserstoff steht;
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Halogen, Cyano, Nitro, oder für ein gegebenenfalls ein- bis siebenfach unabhängig voneinander mit Halogen, Cyano, Alkoxy und Alkoxycarbonyl substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N*,*N*-Di-C₁-C₆-alkylamino stehen;
- W: für Sauerstoff oder Schwefel steht;
- Q: für Wasserstoff, Amino oder eine der gegebenenfalls unabhängig von einander ein- bis siebenfach mit Hydoxy, Nitro, Amino, Halogen, Alkoxy, Cyano, Hydroxycarbonyl, Alkoxycarbonyl, Alkylcarbamoyl, Cycloalkylcarbamoyl, Phenyl substituierten Gruppierungen C₁-C₆-Alkyl, C₂-C₆-Alkenyl, C₂-C₆-Alkinyl, C₃-C₆-Cycloalkyl, C₂-C₅-Heterocyclyl, C₁-C₄-Alkoxy, C₁-C₆-Alkyl-C₃-C₆-cycloalkyl, C₃-C₆-Cycloalkyl-C₁-C₆-alkyl, C₁-C₆-Hydroxyalkyl, Aryl(C₁-C₃)-alkyl, Heteroaryl(C₁-C₃)-alkyl, C₁-C₄-Alkoxycarbonyl, *N*-C₁-C₄-Alkylamino, *N*-C₁-C₄-Alkylcarbonylamino, *N,N*-Di-C₁-C₄-alkylamino, C₁-C₄-Alkylsulfonylamino steht; oder
- Q: für ein mit 0, 1, 2, 3 oder 4 Substituenten V substituiertes Aryl oder für ein mit 0, 1, 2, 3 oder 4 Substituenten V substituiertes 5- bzw. 6-gliedriges Heteroaryl steht, wobei
- V: unabhängig voneinander für Halogen, Cyano, Nitro oder für ein gegebenenfalls ein- bis siebenfach unabhängig voneinander mit Halogen, Cyano, Alkoxy und Alkoxycarbonyl substituiertes C₁-C₆-Alkyl, C₂-C₄-Alkenyl, C₂-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*,*N*-Di-(C₁-C₆-alkyl)amino steht;
- T: für einen der nachfolgend aufgeführten 5-gliedrigen Heteroaromaten T1-T10 steht, wobei die Bindung zur Pyrazolkopfgruppe mit einem Sternchen gekennzeichnet ist, 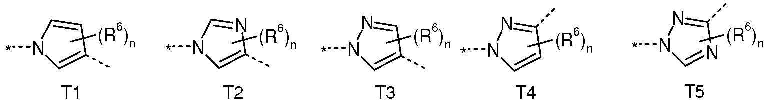 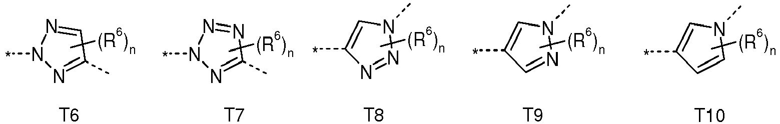 wobei
- R⁶: unabhängig voneinander für Halogen, Cyano, Nitro, Amino oder ein gegebenenfalls ein- bis siebenfach mit Halogen substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, und
- n: für die Werte 0-1 steht;
- Z¹: für ein gegebenenfalls ein- bis siebenfach unabhängig voneinander mit Halogen, Cyano, Alkoxy und Alkoxycarbonyl substituiertes C₁-C₆-Alkyl, C₃-C₆-Cycloalkyl, und
- Z²: für Wasserstoff, Halogen, Cyano, Nitro, Amino oder ein ein- bis siebenfach unabhängig voneinander mit Halogen, Cyano, Alkoxy und Alkoxycarbonyl substituiertes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl, und
- Z³: für Wasserstoff oder oder für ein gegebenenfalls ein- bis siebenfach unabhängig voneinander mit Halogen, Cyano, Alkoxy und Alkoxycarbonyl substituiertes C₁-C₆-Alkyl, C₃-C₆-Cycloalkyl C₃-C₄-Alkenyl, C₃-C₄-Alkinyl, oder für ein gegebenenfalls ein-bis fünffach unabhängig voneinander mit Halogen, Cyano, Alkoxy und Alkoxycarbonyl substituiertes Aryl und Hetaryl stehen.

Ein Aspekt 3 bezieht sich auf Verbindungen gemäß Aspekt 1 oder 2, in denen
- R¹: für Wasserstoff, Methyl, Ethyl, n-Propyl, Isopropyl, n-Butyl, Isobutyl, s-Butyl, t-Butyl, Prop-2-enyl, 2-Methylprop-2-enyl, Prop-2-inyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht, ganz besonders bevorzugt für Wasserstoff steht;
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R² und R⁵: unabhängig voneinander für Wasserstoff, Fluor Chlor, Brom, Iod, Methyl und Methoxy stehen und
- R³ und R⁴: unabhängig voneinander für Wasserstoff, Fluor, Chlor, Brom, Iod, Cyano, Nitro, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Trifluormethylsulfanyl, Methylsulfonyl, Methylsulfinyl, Trifluormethylsulfonyl, Trifluormethylsulfinyl stehen;
- W: für Sauerstoff oder Schwefel steht;
- Q: für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1,1,2,2-Tetrafluorethyl, 1-Trifluormethylethyl, 2,2-Difluorpropyl, 3,3,3-Trifluropropyl, 2,2-Dimethyl-3-fluorpropyl, Cyclopropyl, 1-Cyano-cyclopropyl, 1-Chlor-cyclopropyl, 1-Methyl-cyclopropyl, 1-Methoxycarbonyl-cyclopropyl, 1-(*N-*Methylcarbamoyl)cyclopropyl, 1-(*N*-Cyclopropyl-carbamoyl)cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl, 3,3-Dichlor-prop-2-enyl, 3,3-Dichlor-1,1-dimethylprop-2-enyl, Oxetan-3-yl, Thietan-3-yl, 1-Oxido-thietan-3-yl, 1,1-Dioxido-thietan-3-yl, Isoxazol-3-ylmethyl, 3-Methyloxetan-3-ylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, Pyridin-2--ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N*-Allylamino, *N,N-*Dimethylamino, *N*,*N*-Diethylamino, Methylsulfonylamino steht; oder
- Q: für ein mit 0, 1, 2, 3 oder 4 Substituenten V substituiertes Phenyl, Naphthyl, Pyridazinyl, Pyrazinyl, Pyrimidinyl, Triazinyl, Pyridinyl, Pyrazolyl, Thiazolyl, Isothiazolyl, Oxazolyl, Isoxazolyl, Triazolyl, Imidazolyl, Furanyl, Thiophenyl, Pyrrolyl, Oxadiazolyl, Thiadiazolyl steht, wobei
- V: unabhängig voneinander für Fluor, Chlor, Brom, Iod, Cyano, Nitro, Methyl, Ethyl, Difluormethyl, , Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N*,*N*-Dimethylamino steht;
- T: für einen der nachfolgend aufgeführten 5-gliedrigen Heteroaromaten T1-T10 steht, wobei die Bindung zur Pyrazolkopfgruppe mit einem Sternchen gekennzeichnet ist, 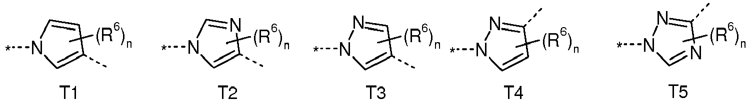 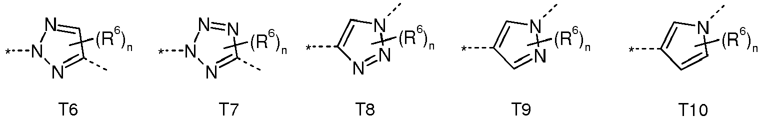 wobei
- R⁶: unabhängig voneinander für Halogen, Cyano, Nitro, Amino, Methyl, Ethyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluormethylsulfanyl, Trilfuormethylsulfinyl, und
- n: für die Werte 0-1 steht;
- Z¹: für Methyl, Ethyl, 1,1-Dimethylethyl, Difluormethyl, , Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl, Pentafluor-t-butyl, Heptafluor-n-propyl, Heptafluor-isopropyl, Nonafluor-n-butyl, Cyclopropyl, 1-Chlorcyclopropyl, 1-Fluorcyclopropyl, 1-Bromcyclopropyl, 1-Cyan-cyclopropyl, 1-Trifluormehtyl-cyclopropyl, Cyclobutyl und 2,2-Difluor-1-methyl-cyclopropyl, und
- Z²: für Wasserstoff, Halogen, Cyano, Nitro, Amino, Methyl, Ethyl, 1,1-Dimethylethyl, Difluormethyl, , Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-t-butyl, Heptafluor-n-propyl, Heptafluor-isopropyl, Nonafluor-n-butyl, Methylsulfanyl, Methylsulfinyl, Methylsulfonyl, Ethylthio, Ethylsulfinyl, Ethylsulfonyl, Trifluormethylsulfanyl, Trifluormethylsulfinyl, Trifluormethylsulfonyl, Chlor-difluormethylsulfanyl, Chlor-difluormethylsulfinyl, Chlor-difluormethylsulfonyl, Dichlor-fluormethylsulfanyl, Dichlor-fluormethylsulfinyl, Dichlor-fluormethylsulfonyl und
- Z³: für Wasserstoff, Methyl, Ethyl, n-Propyl, Isopropyl, n-Butyl, Isobutyl, s-Butyl, t-Butyl, Ethenyl, 1-Propenyl, 2-Propenyl, 1-Propinyl, 1-Butinyl, Difluormethyl, , Chlordifluormethyl, Dichlorfluormethyl, Trifluormethyl, Chlormethyl, Brommethyl, 1-Fluorethyl, 1-Fluor-1-methylethyl, 2-Fluorethyl, 2,2-Difluorethyl, 2,2,2-Trifluorethyl, Phenyl, 2-Chlorphenyl, 3-Chlorphenyl. 4-Chlorphenyl, 2,5-Dichlorphenyl, 2,4-Dichlorphenyl, 3,4-Dichlorphenyl, 2,6-Dichlorphenyl, 2,6-Dichlor-4-trifluormehtylphenyl, 3-Chlor-5-trifluormethylpyridin-2-yl stehen.

Ein Aspekt 4 bezieht sich auf Verbindungen gemäß einem der Aspekte 1 bis 3, in denen
- Z¹: für Trifluormethyl, 1-Chlor-cyclopropyl, 1-Fluor-cyclopropyl oder Pentafluorethyl steht;
- Z²: für Trifluormethyl, Nitro, Methylsulfanyl, Methylsulfinyl, Methylsulfonyl, Fluor, Chlor, Brom, Cyano oder Iod steht;
- Z³: für Methyl, Ethyl, n-Propyl oder Wasserstoff steht;
- R¹: für Wasserstoff, Methyl, Ethyl, n-Propyl, Isopropyl, n-Butyl, i-Butyl, s-Butyl, t-Butyl, Prop-2-enyl, 2-Methylprop-2-enyl, Prop-2-inyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht, ganz besonders bevorzugt für Wasserstoff steht;
- A₁ und A₂: jeweils für CH stehen;
- A₃: für CR⁴ und
- A₄: für CR⁵ stehen;
- R⁴: für Wasserstoff, Fluor, Chlor, Brom, Iod oder Cyano steht;
- R⁵: für Wasserstoff, Fluor, Chlor, Brom, Iod, Methyl oder Methoxy steht;
- T: für einen der nachfolgend aufgeführten 5-gliedrigen Heteroaromaten T1-T10 steht, wobei die Bindung zur Pyrazolkopfgruppe mit einem Sternchen gekennzeichnet ist, 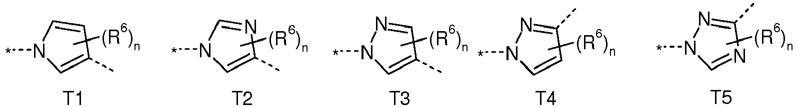 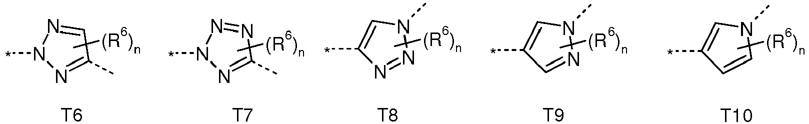 wobei
- R⁶: für Wasserstoff, Methyl, Ethyl, 2-Methylethyl, 2,2-Dimethylethyl, Fluor, Chlor, Brom, Iod, Nitro, Trifluormethyl, Amino steht;
- W: für Sauerstoff steht;
- Q: für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1,1,2,2-Tetrafluorethyl, 1-Trifluormethylethyl, 2,2-Difluorpropyl, 3,3,3-Trifluropropyl, 2,2-Dimethyl-3-fluorpropyl, Cyclopropyl, 1-Cyano-cyclopropyl, 1-Chlor-cyclopropyl, 1-Methyl-cyclopropyl, 1-Methoxycarbonyl-cyclopropyl, *1-*(*N-*Methylcarbamoyl)cyclopropyl, 1-(*N*-Cyclopropylcarbamoyl)cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl, 3,3-Dichlor-prop-2-enyl, 3,3-Dichlor-1,1-dimethylprop-2-enyl, Oxetan-3-yl, Thietan-3-yl, 1-Oxido-thietan-3-yl, 1,1-Dioxido-thietan-3-yl, Isoxazol-3-ylmethyl, 3-Methyloxetan-3-ylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, Pyridin-2--ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N*-Allylamino, *N,N-*Dimethylamino, *N*,*N*-Diethylamino, Methylsulfonylamino steht; oder
- Q: für ein mit 0, 1, 2, 3 oder 4 Substituenten V substituiertes Phenyl, Naphthyl, Pyridazinyl, Pyrazinyl, Pyrimidinyl, Triazinyl, Pyridinyl, Pyrazolyl, Thiazolyl, Isothiazolyl, Oxazolyl, Isoxazolyl, Triazolyl, Imidazolyl, Furanyl, Thiophenyl, Pyrrolyl, Oxadiazolyl, Thiadiazolyl steht, wobei
- V: unabhängig voneinander für Fluor, Chlor, Brom, Iod, Cyano, Nitro, Methyl, Ethyl, Difluormethyl, , Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N*,*N*-Dimethylamino steht.

Ein Aspekt 5 bezieht sich auf Verbindungen gemäß einem der Aspekte 1 bis 4, in denen
- Z¹: für Trifluormethyl, 1-Chlor-cyclopropyl, 1-Fluor-cyclopropyl oder Pentafluorethyl steht;
- Z²: für Trifluormethyl, Nitro, Methylsulfanyl, Methylsulfinyl, Methylsulfonyl, Fluor, Chlor, Brom, Cyano oder Iod steht;
- Z³: für Methyl, Ethyl, n-Propyl oder Wasserstoff steht;
- R¹: für Wasserstoff, Methyl, Ethyl, n-Propyl, Isopropyl, n-Butyl, i-Butyl, s-Butyl, t-Butyl, Prop-2-enyl, 2-Methylprop-2-enyl, Prop-2-inyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 4-Chlor-pyrid-3-yl-methyl steht, ganz besonders bevorzugt für Wasserstoff steht;
- A₁: für CH steht;
- A₂: für Stickstoff steht;
- A₃: für CR⁴ und
- A₄: für CR⁵ stehen;
- R⁴: für Wasserstoff, Fluor, Chlor, Brom, Iod oder Cyano steht;
- R⁵: für Wasserstoff, Fluor, Chlor, Brom, Iod, Methyl oder Methoxy steht;
- T: für einen der nachfolgend aufgeführten 5-gliedrigen Heteroaromaten T1-T10 steht, wobei die Bindung zur Pyrazolkopfgruppe mit einem Sternchen gekennzeichnet ist, 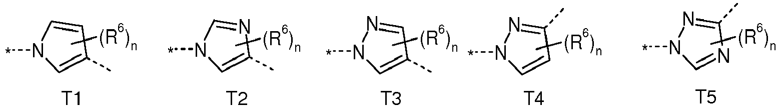 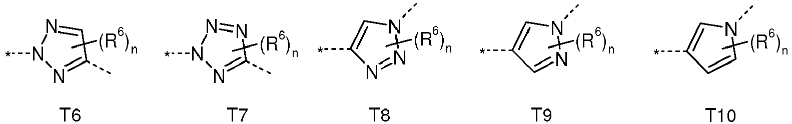 wobei
- R⁶: für Wasserstoff, Methyl, Ethyl, 2-Methylethyl, 2,2-Dimethylethyl, Fluor, Chlor, Brom, Iod, Nitro, Trifluormethyl, Amino steht;
- W: für Sauerstoff steht;
- Q: für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1,1,2,2-Tetrafluorethyl, 1-Trifluormethylethyl, 2,2-Difluorpropyl, 3,3,3-Trifluropropyl, 2,2-Dimethyl-3-fluorpropyl, Cyclopropyl, 1-Cyano-cyclopropyl, 1-Chlor-cyclopropyl, 1-Methyl-cyclopropyl, 1-Methoxycarbonyl-cyclopropyl, 1-(*N-*Methylcarbamoyl)cyclopropyl, 1-(*N*-Cyclopropylcarbamoyl)cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl, 3,3-Dichlor-prop-2-enyl, 3,3-Dichlor-1,1-dimethylprop-2-enyl, Oxetan-3-yl, Thietan-3-yl, 1-Oxido-thietan-3-yl, 1,1-Dioxido-thietan-3-yl, Isoxazol-3-ylmethyl, 3-Methyloxetan-3-ylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, Pyridin-2-ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N*-Allylamino, *N*,*N-*Dimethylamino, *N,N*-Diethylamino, Methylsulfonylamino steht; oder
- Q: für ein mit 0, 1, 2, 3 oder 4 Substituenten V substituiertes Phenyl, Naphthyl, Pyridazinyl, Pyrazinyl, Pyrimidinyl, Triazinyl, Pyridinyl, Pyrazolyl, Thiazolyl, Isothiazolyl, Oxazolyl, Isoxazolyl, Triazolyl, Imidazolyl, Furanyl, Thiophenyl, Pyrrolyl, Oxadiazolyl, Thiadiazolyl steht, wobei
- V: unabhängig voneinander für Fluor, Chlor, Brom, Iod, Cyano, Nitro, Methyl, Ethyl, Difluormethyl, , Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N*,*N*-Dimethylamino steht.

Ein Aspekt 6 bezieht sich auf Verbindungen gemäß einem der Aspekte 1 bis 5 worin eine Verbindung der allgemeinen Formel (I) eine Verbindung einer der Formeln (Ia), (Ib), (Ic), (Id), (Ie), (If), (Ig), (Ih), (Ii) und (Ij),

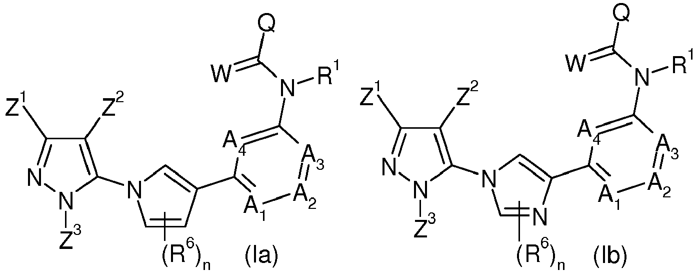

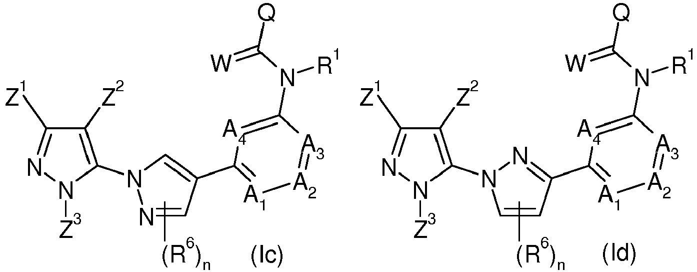

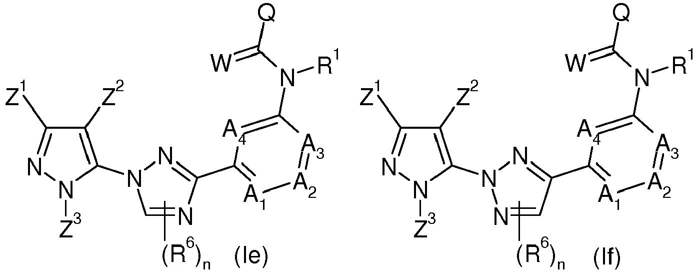

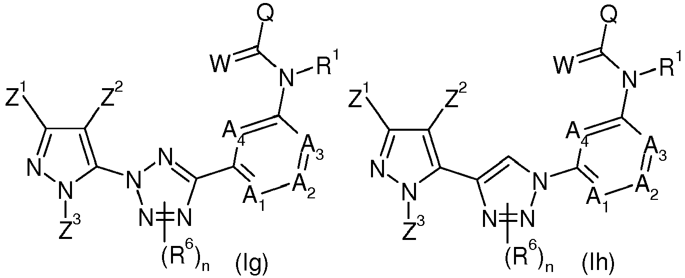

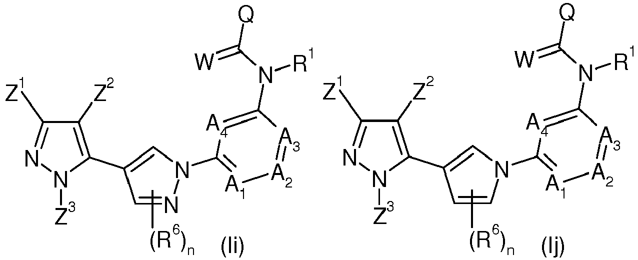

Darstellt, in denen die Reste A₁-A₄, n, W, Q, R¹ und Z¹-Z³ gemäß einem der Aspekte 1 bis 6 definiert sind.

Ein Aspekt 7 bezieht sich auf Verbindungen gemäß einem der Aspekte 1 bis 7 worin eine Verbindung der Formel (I) eine Verbindung der allgemeinen Formel (Ic) darstellt, 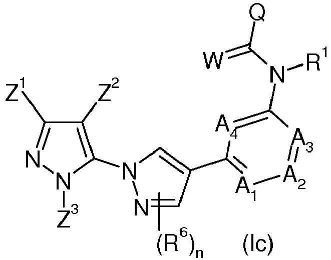 in denen
Z¹ für CF₂CF₃ steht,
Z² für CF₃,
Z³ für CH₃,
die Reste R¹, R⁶ für Wasserstoff (n= 0) stehen,
A₁, A₂, für C-H,
A₃ für C-H, C-Cl, C-F,
A₄ für C-H, C-F, C-OMe stehen,
W für Sauerstoff und
Q für Methyl, Ethyl, Cyclopropyl, 1-Chlor-cyclopropyl, 1-Cyano-cyclopropyl, 2,2,2-Trifluorethyl, 1,1,2,2-Tetrafluorethyl, Phenyl, 4-Fluorphenyl, 2-Fluorphenyl, 3-Fluorphenyl, 3,5-Difluorphenyl, 2,6-Difluorphenyl, 2-Thiophenyl, 3-Thiophenyl, 3-Chlor-3-thiophenyl, 1-Methyl-4-pyrazolyl, 4-Pyridinyl, 3-Chlor-2-Pyridinyl, 2-Chlor-4-Pyridinyl, 3-Fluor-4-Pyridinyl, 2,6-Dichlor-4-pyridinyl stehen.

Ein Aspekt 8 bezieht sich auf Verbindungen gemäß Aspekt 7, in denen
Z¹ für CF₂CF₃ steht,
Z² für CF₃,
Z³ für CH₃,
die Reste R¹, R⁶ für Wasserstoff (n= 0) stehen,
A₁ für C-H,
A₂ für N,
A₃ für C-H, C-Cl, C-F, C-CH₃,
A₄ für C-H, C-F, C-OMe stehen,
W für Sauerstoff und
Q für Methyl, Ethyl, Cyclopropyl, 1-Chlor-cyclopropyl, 1-Cyano-cyclopropyl, 2,2,2-Trifluorethyl, 1,1,2,2-Tetrafluorethyl, Phenyl, 4-Fluorphenyl, 2-Fluorphenyl, 3-Fluorphenyl, 3,5-Difluorphenyl, 2,6-Difluorphenyl, 2-Thiophenyl, 3-Thiophenyl, 3-Chlor-3-thiophenyl, 1-Methyl-4-pyrazolyl, 4-Pyridinyl, 3-Chlor-2-pyridinyl, 2-Chlor-4-pyridinyl, 3-Fluor-4-pyridinyl, 2,6-Dichlor-4-pyridinyl stehen.

Ein Aspekt 9 bezieht sich auf Verbindungen der allgemeinen Formel (Ih), 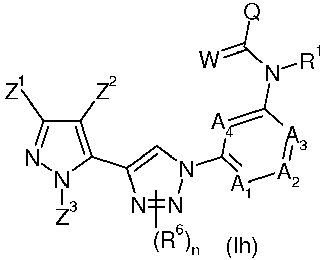 in denen
Z¹ für CF₂CF₃ steht,
Z² für CF₃,
Z³ für CH₃,
die Reste R¹, R⁶ für Wasserstoff (n= 0) stehen,
A₁, A₂, für C-H,
A₃ für C-H, C-Cl, C-F,
A₄ für C-H, C-F, C-OMe stehen,
W für Sauerstoff und
Q für Methyl, Ethyl, Cyclopropyl, 1-Chlor-cyclopropyl, 1-Cyano-cyclopropyl, 2,2,2-Trifluorethyl, 1,1,2,2-Tetrafluorethyl, Phenyl, 4-Fluorphenyl, 2-Fluorphenyl, 3-Fluorphenyl, 3,5-Difluorphenyl, 2,6-Difluorphenyl, 2-Thiophenyl, 3-Thiophenyl, 3-Chlor-3-thiophenyl, 1-Methyl-4-pyrazolyl, 4-Pyridinyl, 3-Chlor-2-pyridinyl, 2-Chlor-4-pyridinyl, 3-Fluor-4-pyridinyl, 2,6-Dichlor-4-pyridinyl stehen.

Ein Aspekt 10 bezieht sich auf Verbindungen gemäß Aspekt 9, in denen Z¹ für CF₂CF₃ steht,
Z² für CF₃,
Z³ für CH₃,
die Reste R¹, R⁶ für Wasserstoff (n= 0) stehen,
A₁ für C-H,
A₂ für N,
A₃ für C-H, C-Cl, C-F, C-CH₃,
A₄ für C-H, C-F, C-OMe stehen,
W für Sauerstoff und
Q für Methyl, Ethyl, Cyclopropyl, 1-Chlor-cyclopropyl, 1-Cyano-cyclopropyl, 2,2,2-Trifluorethyl, 1,1,2,2-Tetrafluorethyl, Phenyl, 4-Fluorphenyl, 2-Fluorphenyl, 3-Fluorphenyl, 3,5-Difluorphenyl, 2,6-Difluorphenyl, 2-Thiophenyl, 3-Thiophenyl, 3-Chlor-3-thiophenyl, 1-Methyl-4-pyrazolyl, 4-Pyridinyl, 3-Chlor-2-pyridinyl, 2-Chlor-4-pyridinyl, 3-Fluor-4-pyridinyl, 2,6-Dichlor-4-pyridinyl stehen.

Aspekt 11 bezieht sich auf die Verwendung von Verbindungen der allgemeinen Formel (I), (Ia), (Ib), (Ic), (Id), (Ie), (If), (Ig), (Ih), (Ii) und (Ij) gemäß einem der Aspekte 1 bis 10 zur nicht-therapeutischen Bekämpfung von Insekten, Spinnentieren und Nematoden.

Aspekt 12 bezieht sich auf Pharmazeutische Zusammensetzungen, enthaltend wenigstens eine Verbindung gemäß einem der Aspekte 1 bis 10.

Aspekt 13 bezeiht sich auf Verbindungen gemäß einem der Aspekte 1 bis 10 zur Verwendung als Arzneimittel.

Aspekt 14 bezeiht sich auf die Verwendung von Verbindungen gemäß einem der Aspekte 1 bis 10 zur Herstellung pharmazeutischer Zusammensetzungen zur Bekämpfung von Parasiten auf Tieren.

Aspekt 15 bezeiht sich auf Verfahren zur Herstellung von Pflanzenschutzmitteln enthaltend Verbindungen gemäß einem der Aspekte 1 bis 10, sowie übliche Streckmittel und/oder oberflächenaktive Substanzen.

Aspekt 16 bezeiht sich auf ein nicht-therapeutisches Verfahren zum Bekämpfen von Schädlingen, dadurch gekennzeichnet, dass man eine Verbindung gemäß einem der Aspekte 1 bis 10 auf die Schädlinge und/oder ihren Lebensraum einwirken lässt.

Aspekt 17 bezeiht sich auf Verwendung von Verbindungen gemäß einem der Aspekte 1 bis 10 zum Schutz des Vermehrungsmaterials von Pflanzen.

Weiterhin hierin beschrieben sind in einem Aspekt 18 Verbindungen der Formel (Ic) 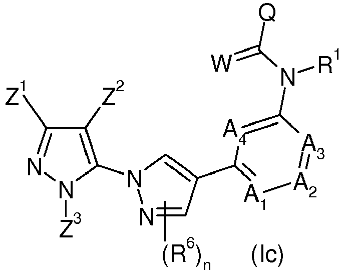 worin
- R¹: für Wasserstoff oder für ein gegebenenfalls ein- bis siebenfach unabhängig voneinander mit Halogen, Cyano, Alkoxy und Alkoxycarbonyl substituiertes C₁-C₆-Alkyl, C₃-C₆-Alkenyl, C₃-C₆-Alkinyl, C₃-C₇-Cycloalkyl, C₃-C₇-Cycloalkyl-C₁-C₃-alkyl, C₁-C₆-Alkylcarbonyl, C₁-C₆-Alkoxycarbonyl, Aryl(C₁-C₃)-alkyl, Heteroaryl(C₁-C₃)-alkyl steht, ganz besonders bevorzugt für Wasserstoff steht;
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Halogen, Cyano, Nitro, oder für ein gegebenenfalls ein- bis siebenfach unabhängig voneinander mit Halogen, Cyano, Alkoxy und Alkoxycarbonyl substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N*,*N*-Di-C₁-C₆-alkylamino stehen;
- W: für Sauerstoff oder Schwefel steht;
- Q: für Wasserstoff, Amino oder eine der gegebenenfalls unabhängig von einander ein- bis siebenfach mit Hydoxy, Nitro, Amino, Halogen, Alkoxy, Cyano, Hydroxycarbonyl, Alkoxycarbonyl, Alkylcarbamoyl, Cycloalkylcarbamoyl, Phenyl substituierten Gruppierungen C₁-C₆-Alkyl, C₂-C₆-Alkenyl, C₂-C₆-Alkinyl, C₃-C₆-Cycloalkyl, C₂-C₅-Heterocyclyl, C₁-C₄-Alkoxy, C₁-C₆-Alkyl-C₃-C₆-cycloalkyl, C₃-C₆-Cycloalkyl-C₁-C₆-alkyl, C₁-C₆-Hydroxyalkyl, Aryl(C₁-C₃)-alkyl, Heteroaryl(C₁-C₃)-alkyl, C₁-C₄-Alkoxycarbonyl, *N*-C₁-C₄-Alkylamino, *N*-C₁-C₄-Alkylcarbonylamino, *N*,*N*-Di-C₁-C₄-alkylamino, C₁-C₄-Alkylsulfonylamino steht; oder
- Q: für Wasserstoff, Amino oder eine der gegebenenfalls unabhängig von einander mit 1, 2, 3, 4, 5, 6 oder 7 Substituenten ausgewählt aus der Gruppe bestehend aus Hydoxy, Nitro, Amino, Halogen, Oxo, Benzyloxy, C₃-C₆-Cycloalkyl, C₁-C₆-Alkoxy, Cyano, Hydroxycarbonyl, C₁-C₄-Alkoxycarbonyl, C₁-C₆-Alkylcarbamoyl, C₃-C₆-Cycloalkylcarbamoyl, *N*,*N*-Di-C₁-C₄-alkylamino, gegebenenfalls mit 1, 2 oder 3 Substituenten unabhängig voneinander ausgewählt aus der Gruppe von Substituenten bestehend aus Halogen, C₁-C₆-Alkoxy, C₁-C₆-Halogenalkoxy und C₁-C₆-Alkyl substituierten Phenyl, gegebenenfalls mit 1, 2 oder 3 Substituenten unabhängig voneinander ausgewählt aus der Gruppe von Substituenten bestehend aus Halogen, C₁-C₆-Alkoxy, C₁-C₆-Halogenalkoxy nd C₁-C₆-Alkyl substituierten Phenylthio, gegebenenfalls mit 1, 2 oder 3 Substituenten unabhängig voneinander ausgewählt aus der Gruppe von Substituenten bestehend aus Halogen, C₁-C₆-Alkoxy, C₁-C₆-Halogenalkoxy und C₁-C₆-Alkyl substituierten 5- oder 6-gliedrigen Heteroaryl (z. B. Pyrazolyl) substituierten Gruppierungen ausgewählt aus C₁-C₆-Alkyl, C₁-C₆-Alkyloxy, C₂-C₆-Alkenyl, C₂-C₆-Alkinyl, C₃-C₆-Cycloalkyl, 5 oder 6 gliedriges Heterocyclyl, C₁-C₆-Alkyl-C₃-C₆-cycloalkyl, C₃-C₆-Cycloalkyl-C₁-C₆-alkyl, C₆-C₁₀-Aryl-(C₁-C₃)-alkyl, 5 oder 6 gliedriges Heteroaryl-(C₁-C₃)-alkyl, C₁-C₄-Alkoxycarbonyl, oder für eine Gruppierung *N*-C₁-C₄-Alkylamino, *N*-C₁-C₄-Alkylcarbonylamino, *N,N*-Di-C₁-C₄-alkylamino, C₁-C₄-Alkylsulfonylamino steht; oder
- Q: für ein mit 0, 1, 2, 3 oder 4 Substituenten V substituiertes Aryl oder für ein mit 0, 1, 2, 3 oder 4 Substituenten V substituiertes 5- bzw. 6-gliedriges Heteroaryl steht, oder
- Q: für einen mit 0, 1, 2, 3 oder 4 Substituenten V substituierten bicyclischen Heterocyclus oder für einen mit 0, 1, 2, 3 oder 4 Substituenten V substituierten bicyclische Carbocyclus steht; wobei
- V: unabhängig voneinander für Halogen, Cyano, Nitro oder für ein gegebenenfalls ein- bis siebenfach unabhängig voneinander mit Halogen, Cyano, Alkoxy und Alkoxycarbonyl substituiertes C₁-C₆-Alkyl, C₁-C₆-Haloalkyl, C₂-C₄-Alkenyl, C₂-C₄-Alkinyl, C₃-C₆-Cycloalkyl, C₁-C₆-Alkoxy, C₁-C₆-Halogenalkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N,N*-Di-(C₁-C₆-alkyl)amino steht;
- R⁶: unabhängig voneinander für Halogen, Cyano, Nitro, Amino oder ein gegebenenfalls ein- bis siebenfach mit Halogen substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, und
- n: für die Werte 0-1 steht;
- Z¹: für ein gegebenenfalls ein- bis siebenfach unabhängig voneinander mit Halogen, Cyano, Alkoxy und Alkoxycarbonyl substituiertes C₁-C₆-Alkyl, C₃-C₆-Cycloalkyl, und
- Z²: für Wasserstoff, Halogen, Cyano, Nitro, Amino oder ein ein- bis siebenfach unabhängig voneinander mit Halogen, Cyano, Alkoxy und Alkoxycarbonyl substituiertes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl, und
- Z³: für Wasserstoff oder oder für ein gegebenenfalls ein- bis siebenfach unabhängig voneinander mit Halogen, Cyano, Alkoxy und Alkoxycarbonyl substituiertes C₁-C₆-Alkyl, C₃-C₆-Cycloalkyl C₃-C₄-Alkenyl, C₃-C₄-Alkinyl, oder für ein gegebenenfalls ein-bis fünffach unabhängig voneinander mit Halogen, Cyano, Alkoxy und Alkoxycarbonyl substituiertes Aryl und Hetaryl stehen.

Weiterhin hierin beschrieben sindin einem Aspekt 19 Verbindungen der Formel (Ic) gemäß Aspekt 18, in der R⁶ für Wasserstoff oder C₁-C₆-Alkyl steht und n 0 oder 1 beträgt, mehr bevorzugt in der R⁶ für Wasserstoff steht.

Weiterhin hierin beschrieben sindin einem Aspekt 20 Verbindungen der Formel (Ic) gemäß Aspekt 18 oder 19 in der die chemischen Gruppierungen
- A₁: für CR²,
- A₂: für CR³ oder Stickstoff,
- A₃: für CR⁴, und
- A₄: für CR⁵ stehen, wobei

R² und R⁵ unabhängig voneinander für Wasserstoff, Fluor Chlor, Brom, Iod, C₁-C₄-Alkyl (z. B. Methyl) und C₁-C₄-Alkoxy (z. B. Methoxy) stehen und R³ und R⁴ unabhängig voneinander für Wasserstoff, Fluor, Chlor, Brom, Iod, Cyano, Nitro, C₁-C₄-Alkyl (z. B. Methyl), C₁-C₄-Haloalkyl stehen, mehr bevorzugt in der R² und R⁵ unabhängig voneinander für Wasserstoff, Fluor, Methyl und Methoxy stehen und R³ und R⁴ unabhängig voneinander für Wasserstoff, Fluor, Chlor, Methyl stehen.

Weiterhin hierin beschrieben sind in einem Aspekt 21 Verbindungen der Formel (Ic) gemäß einem der Aspekte 18 bis 21 in der die chemischen Gruppierungen
- A₁: für CR²,
- A₂: für CR³,
- A₃: für CR⁴, und
- A₄: für CR⁵ stehen, wobei

R² für Wasserstoff steht, R³ für Wasserstoff steht, R⁴ für Wasserstoff, Fluor, Chlor, Brom, Iod, Methyl oder Ethyl steht und R⁵ für Wasserstoff, Fluor, Chlor, Brom oder Iod steht, mehr bevorzugt R² für Wasserstoff steht, R³ für Wasserstoff steht, R⁴ für Wasserstoff, Fluor, Chlor oder Methyl steht und R⁵ für Wasserstoff oder Fluor steht.

Weiterhin hierin beschrieben sind in einem Aspekt 22 Verbindungen der Formel (Ic) gemäß einem der Aspekte 18 bis 21 in der die chemischen Gruppierungen
- A₁: für CR²,
- A₂: für Stickstoff,
- A₃: für CR⁴, und
- A₄: für CR⁵ stehen, wobei

R² für Wasserstoff steht, R⁴ für Wasserstoff, Fluor, Chlor, Brom, Iod, Methyl oder Ethyl steht und R⁵ für Wasserstoff, Fluor, Chlor, Brom oder Iod steht, mehr bevorzugt R² für Wasserstoff steht, R⁴ für Wasserstoff, Fluor, Chlor oder Methyl, bevorzugt für Fluor, Chlor oder Methyl, steht und R⁵ für Wasserstoff oder Fluor, bevorzugt für Wasserstoff, steht.

Weiterhin hierin beschrieben sindin einem Aspekt 23 Verbindungen der Formel (Ic) gemäß einem der Aspekte 18 bis 22 in der W für Sauerstoff steht.

Weiterhin hierin beschrieben sind in einem Aspekt 24 Verbindungen der Formel (Ic) gemäß einem der Aspekte 18 bis 23, in der
- Z¹: für ein jeweils gegebenenfalls ein- bis siebenfach unabhängig voneinander mit Halogen oder Cyano substituiertes C₁-C₆-Alkyl oder C₃-C₆-Cycloalkyl steht;
- Z²: für ein jeweils gegebenenfalls ein- bis siebenfach unabhängig voneinander mit Halogen oder Cyano substituiertes C₁-C₆-Alkyl oder C₃-C₆-Cycloalkyl steht;
- Z³: für Wasserstoff oder C₁-C₆-Alkyl steht,
mehr bevorzugt, in der
- Z¹: für ein jeweils gegebenenfalls ein- bis siebenfach unabhängig voneinander mit Halogen substituiertes C₁-C₆-Alkyl steht;
- Z²: für ein jeweils gegebenenfalls ein- bis siebenfach unabhängig voneinander mit Halogen substituiertes C₁-C₆-Alkyl steht;
- Z³: für C₁-C₆-Alkyl steht,
noch mehr bevorzugt, in der
- Z¹: für perfluoriertes C₁-C₃-Alkyl (z. B. C₂F₅) steht;
- Z²: für perfluoriertes C₁-C₃-Alkyl (z. B. CF₃) steht;
- Z³: für C₁-C₄-Alkyl (z. B. Methyl) steht.

Weiterhin hierin beschrieben sind in einem Aspekt 25 Verbindungen der Formel (Ic) gemäß einem der Aspekte 18 bis 24 worin Z¹ für C₂F₅ steht, Z² für CF₃ steht und Z³ für Methyl steht.

Weiterhin hierin beschrieben sind in einem Aspekt 26 Verbindungen der Formel (Ic) gemäß einem der Aspekte 18 bis 25 worin Q für Wasserstoff, Amino oder eine der gegebenenfalls unabhängig von einander ein- bis siebenfach mit Hydoxy, Nitro, Amino, Halogen, Alkoxy, Cyano, Hydroxycarbonyl, Alkoxycarbonyl, Alkylcarbamoyl, Cycloalkylcarbamoyl, Phenyl substituierten Gruppierungen C₁-C₆-Alkyl, C₂-C₆-Alkenyl, C₂-C₆-Alkinyl, C₃-C₆-Cycloalkyl, C₂-C₅-Heterocyclyl, C₁-C₄-Alkoxy, C₁-C₆-Alkyl-C₃-C₆-cycloalkyl, C₃-C₆-Cycloalkyl-C₁-C₆-alkyl, C₁-C₆-Hydroxyalkyl, Aryl(C₁-C₃)-alkyl, Heteroaryl(C₁-C₃)-alkyl, C₁-C₄-Alkoxycarbonyl, *N*-C₁-C₄-Alkylamino, *N*-C₁-C₄-Alkylcarbonylamino, *N*,*N*-Di-C₁-C₄-alkylamino, C₁-C₄-Alkylsulfonylamino steht oder für ein mit 0, 1, 2, 3 oder 4 Substituenten V substituiertes Aryl oder für ein mit 0, 1, 2, 3 oder 4 Substituenten V substituiertes 5-bzw. 6-gliedriges Heteroaryl steht wobei V unabhängig voneinander für Halogen, Cyano, Nitro oder für ein gegebenenfalls ein- bis siebenfach unabhängig voneinander mit Halogen, Cyano, Alkoxy und Alkoxycarbonyl substituiertes C₁-C₆-Alkyl, C₂-C₄-Alkenyl, C₂-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*,*N*-Di-(C₁-C₆-alkyl)amino steht.

Weiterhin hierin beschrieben sind in einem Aspekt 27 Verbindungen der Formel (Ic) gemäß einem der Aspekte 18 bis 25 worin Q für
für Wasserstoff, Amino oder eine der gegebenenfalls unabhängig von einander mit 1, 2, 3, 4, 5, 6 oder 7 Substituenten ausgewählt aus der Gruppe bestehend aus Hydoxy, Nitro, Amino, Halogen, Oxo, Benzyloxy, C₃-C₆-Cycloalkyl, C₁-C₆-Alkoxy, C₁-C₆-Halogenalkoxy, Cyano, Hydroxycarbonyl, C₁-C₄-Alkoxycarbonyl, C₁-C₆-Alkylcarbamoyl, C₃-C₆-Cycloalkylcarbamoyl, *N*,*N*-Di-C₁-C₄-alkylamino, gegebenenfalls mit 1, 2 oder 3 Substituenten unabhängig voneinander ausgewählt aus der Gruppe von Substituenten bestehend aus Halogen, C₁-C₆-Alkoxy, C₁-C₆-Halogenalkoxy und C₁-C₆-Alkyl substituierten Phenyl, gegebenenfalls mit 1, 2 oder 3 Substituenten unabhängig voneinander ausgewählt aus der Gruppe von Substituenten bestehend aus Halogen, C₁-C₆-Alkoxy, C₁-C₆-Halogenalkoxy nd C₁-C₆-Alkyl substituierten Phenylthio, gegebenenfalls mit 1, 2 oder 3 Substituenten unabhängig voneinander ausgewählt aus der Gruppe von Substituenten bestehend aus Halogen, C₁-C₆-Alkoxy, C₁-C₆-Halogenalkoxy und C₁-C₆-Alkyl substituierten Phenyloxy, gegebenenfalls mit 1, 2 oder 3 Substituenten unabhängig voneinander ausgewählt aus der Gruppe von Substituenten bestehend aus Halogen, C₁-C₆-Alkoxy, C₁-C₆-Halogenalkoxy und C₁-C₆-Alkyl substituierten 5- oder 6-gliedrigen Heteroaryl (z. B. Pyrazolyl oder Thiophenyl) substituierten Gruppierungen ausgewählt aus C₁-C₆-Alkyl, C₁-C₆-Alkyloxy, C₂-C₆-Alkenyl, C₂-C₆-Alkinyl, C₃-C₆-Cycloalkyl, 5 oder 6 gliedriges Heterocyclyl, C₁-C₆-Alkyl-C₃-C₆-cycloalkyl, C₃-C₆-Cycloalkyl-C₁-C₆-alkyl, C₆-C₁₀-Aryl-(C₁-C₃)-alkyl, 5 oder 6 gliedriges Heteroaryl-(C₁-C₃)-alkyl, C₁-C₄-Alkoxycarbonyl, oder für eine Gruppierung *N*-C₁-C₄-Alkylamino, *N*-C₁-C₄-Alkylcarbonylamino, *N*,*N*-Di-C₁-C₄-alkylamino, C₁-C₄-Alkylsulfonylamino steht; oder
- Q: für ein mit 0, 1, 2, 3 oder 4 Substituenten V substituiertes Aryl oder für ein mit 0, 1, 2, 3 oder 4 Substituenten V substituiertes 5- bzw. 6-gliedriges Heteroaryl steht, oder
- Q: für einen mit 0, 1, 2, 3 oder 4 Substituenten V substituierten bicyclischen Heterocyclus oder für einen mit 0, 1, 2, 3 oder 4 Substituenten V substituierten bicyclische Carbocyclus steht; wobei
- V: unabhängig voneinander für Halogen, Cyano, Nitro oder für ein gegebenenfalls ein- bis siebenfach unabhängig voneinander mit Halogen, Cyano, Alkoxy und Alkoxycarbonyl substituiertes C₁-C₆-Alkyl, C₁-C₆-Halogenalkyl, C₂-C₄-Alkenyl, C₂-C₄-Alkinyl, C₃-C₆-Cycloalkyl, C₁-C₆-Alkoxy, C₁-C₆-Halogenalkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*,*N*-Di-(C₁-C₆-alkyl)amino steht;

Weiterhin hierin beschrieben sind in einem Aspekt 28 Verbindungen der Formel (Ic) gemäß einem der Aspekte 18 bis 25 oder 27 in der Q für
- Wasserstoff;
- C₁-C₆-Alkyl, das optional substituiert ist mit 1, 2, 3 oder 4 Substituenten unabhängig voneinander ausgewählt aus der Gruppe bestehend aus
   Fluor, Chlor, Brom, Iod, Cyano, Oxo, C₁-C₄-Alkoxy, C₁-C₄-Halogenalkoxy, Benzyloxy, *N,N*-Di-C₁-C₄-alkylamino, C₃-C₆-Cycloalkyl, optional unabhängig voneinander mit 1, 2, 3, oder 4 Substituenten ausgewählt aus der Gruppe bestehend aus Fluor, Chlor, Brom, Iod, C₁-C₄-Alkoxy, C₁-C₄-Alkyl, und C₁-C₄-Halogenalkyl substituiertem Phenylthio, optional unabhängig voneinander mit 1, 2, 3, oder 4 Subtituenten ausgewählt aus der Gruppe bestehend aus Fluor, Chlor, Brom, Iod, C₁-C₄-Alkoxy, C₁-C₄-Alkyl, und C₁-C₄-Halogenalkyl substituiertem Phenyloxy, optional unabhängig voneinander mit 1, 2, 3, oder 4 Subtituenten ausgewählt aus der Gruppe bestehend aus Fluor, Chlor, Brom, Iod, C₁-C₄-Alkoxy, C₁-C₄-Alkyl, und C₁-C₄-Halogenalkyl substituiertem Phenyl, optional unabhängig voneinander mit 1, 2, 3, oder 4 Subtituenten ausgewählt aus der Gruppe bestehend aus Fluor, Chlor, Brom, Iod, C₁-C₄-Alkoxy, C₁-C₄-Alkyl, und C₁-C₄-Halogenalkyl substituiertem Pyrazolyl, optional unabhängig voneinander mit 1, 2, 3, oder 4 Substituenten ausgewählt aus der Gruppe bestehend aus Fluor, Chlor, Brom, Iod, C₁-C₄-Alkoxy, C₁-C₄-Alkyl, und C₁-C₄-Halogenalkyl substituiertem Thiophenyl;
- C₃-C₆-Cycloalkyl optional substituiert mit 1, 2, 3 oder 4 Substituenten unabhängig voneinander ausgewählt aus der Gruppe bestehend aus
   C₁-C₄-Alkoxy Fluor, Chlor, Brom, Iod, Cyano, C₁-C₄-Alkyl, optional unabhängig voneinander mit 1, 2, 3, oder 4 Subtituenten ausgewählt aus der Gruppe bestehend aus Fluor, Chlor, Brom, Iod, C₁-C₄-Alkoxy, C₁-C₄-Alkyl, und C₁-C₄-Halogenalkyl substituiertem Phenyl; oder
- für ein mit 0, 1, 2, 3 oder 4 Substituenten V substituiertes Phenyl, Naphthyl, Pyridazinyl, Pyrazinyl, Pyrimidinyl, Triazinyl, Pyridinyl, Pyrazolyl, Thiazolyl, Isothiazolyl, Oxazolyl, Isoxazolyl, Triazolyl, Imidazolyl, Furanyl, Thiophenyl, Pyrrolyl, Oxadiazolyl, Thiadiazolyl, Benzothiophenyl, Benzofuranyl, [1,2,4]Triazolo[1,5-a]pyrimidinyl, 1,3-Benzodioxolyl, oder Tetralinyl steht, wobei V unabhängig voneinander für Fluor, Chlor, Brom, Iod, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy, *N,N*-Di-C₁-C₄-alkylamino steht.

Weiterhin hierin beschrieben sind in einem Aspekt 29 Verbindungen der Formel (Ic) gemäß einem der Aspekte 18 bis 25 oder 27 bis 28 in der Q für
- C₁-C₆-Alkyl, das optional substituiert ist mit 1, 2, 3 oder 4 Substituenten unabhängig voneinander ausgewählt aus der Gruppe bestehend aus
   Fluor, Chlor, Brom, Iod, Oxo, C₁-C₄-Alkoxy, Benzyloxy, *N,N*-Di-C₁-C₄-alkylamino, C₃-C₆-Cycloalkyl, Phenylthio, Phenyloxy, optional unabhängig voneinander mit 1, 2, 3, oder 4 Substituenten ausgewählt aus der Gruppe bestehend aus Fluor, Chlor, Brom, Iod, C₁-C₄-Alkoxy, C₁-C₄-Alkyl, und C₁-C₄-Halogenalkyl substituiertem Phenyl, optional unabhängig voneinander mit 1, 2, 3, oder 4 Substituenten ausgewählt aus der Gruppe bestehend aus C₁-C₄-Alkyl und C₁-C₄-Halogenalkyl substituiertem Pyrazolyl und Thiophenyl;
- C₃-C₆-Cycloalkyl optional substituiert mit 1, 2, 3 oder 4 Substituenten unabhängig voneinander ausgewählt aus der Gruppe bestehend aus
   C₁-C₄-Alkoxy Fluor, Chlor, Brom, Iod, Cyano, C₁-C₄-Alkyl, optional unabhängig voneinander mit 1, 2, 3, oder 4 Subtituenten ausgewählt aus der Gruppe bestehend aus Fluor, Chlor, Brom, Iod und C₁-C₄-Alkyl, substituiertem Phenyl; oder
- für ein mit 0, 1, 2, 3 oder 4 Substituenten V substituiertes Phenyl, Pyrimidinyl, Pyridinyl, Pyrazolyl, Oxazolyl, Isoxazolyl, Furanyl, Thiophenyl, Benzothiophenyl, Benzofuranyl, [1,2,4]Ttriazolo[1,5-a]pyrimidinyl, 1,3-Benzodioxolyl oder Tetralinyl, wobei V unabhängig voneinander für Fluor, Chlor, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy *N*,*N*-Di-C₁-C₄-alkylamino steht.

Weiterhin hierin beschrieben sind in einem Aspekt 30 Verbindungen der Formel (Ic) gemäß einem der Aspekte 18 bis 25 oder 27 bis 29 in der Q für
- C₁-C₄-Alkyl, das optional substituiert ist mit 1, 2, 3 oder 4 Substituenten unabhängig voneinander ausgewählt aus der Gruppe bestehend aus
   Fluor, Chlor, Brom, Iod, Oxo, C₁-C₄-Alkoxy, Benzyloxy, *N,N*-Di-C₁-C₄-alkylamino, C₃-C₆-Cycloalkyl, Phenylthio, Phenyloxy, optional unabhängig voneinander mit 1, 2 oder 3 Substituenten ausgewählt aus der Gruppe bestehend aus Fluor, Chlor, Brom, Iod, C₁-C₄-Alkoxy, C₁-C₄-Alkyl, und C₁-C₄-Halogenalkyl substituiertem Phenyl, optional unabhängig voneinander mit 1 oder 2 Substituenten ausgewählt aus der Gruppe bestehend aus C₁-C₄-Alkyl und C₁-C₄-Halogenalkyl substituiertem Pyrazolyl und Thiophenyl;
- C₃-C₆-Cycloalkyl optional substituiert mit 1 oder 2 Substituenten unabhängig voneinander ausgewählt aus der Gruppe bestehend aus
   C₁-C₄-Alkoxy Fluor, Chlor, Brom, Iod, Cyano, C₁-C₄-Alkyl, optional unabhängig voneinander mit 1 oder 2, Substituenten ausgewählt aus der Gruppe bestehend aus Fluor, Chlor, Brom, Iod und C₁-C₄-Alkyl substituiertem Phenyl; oder
- für ein mit 0, 1, 2 oder 3 Substituenten V substituiertes Phenyl, Pyrimidinyl, Pyridinyl, Pyrazolyl, Oxazolyl, Isoxazolyl, Furanyl, Thiophenyl, Benzothiophenyl, Benzofuranyl, [1,2,4]Triazolo[1,5-a]pyrimidinyl, 1,3-Benzodioxolyl oder Tetralinyl, wobei V unabhängig voneinander für Fluor, Chlor, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy *N*,*N*-Di-C₁-C₂-alkylamino steht.

Weiterhin hierin beschrieben sind in einem Aspekt 31 Verbindungen der Formel (Ic) gemäß einem der Aspekte 18 bis 25 oder 27 bis 30 in der Q für
- C₁-C₄-Alkyl, das optional substituiert ist mit 1, 2, 3 oder 4 Substituenten unabhängig voneinander ausgewählt aus der Gruppe bestehend aus
   Fluor, Oxo, C₁-C₄-Alkoxy, Benzyloxy, *N,N*-Di-C₁-C₄-alkylamino, C₃-C₆-Cycloalkyl, Phenylthio, Phenyloxy, optional unabhängig voneinander mit 1, 2 oder 3 Substituenten ausgewählt aus der Gruppe bestehend aus Fluor, Chlor, C₁-C₄-Alkoxy, C₁-C₄-Alkyl, und C₁-C₄-Halogenalkyl substituiertem Phenyl, optional unabhängig voneinander mit 1 oder 2 Substituenten ausgewählt aus der Gruppe bestehend aus C₁-C₄-Alkyl und C₁-C₄-Halogenalkyl substituiertem Pyrazolyl, und Thiophenyl;
- C₃-C₆-Cycloalkyl optional substituiert mit 1 oder 2 Substituenten unabhängig voneinander ausgewählt aus der Gruppe bestehend aus
   C₁-C₄-Alkoxy, Chlor, Cyano, C₁-C₄-Alkyl, optional unabhängig voneinander mit 1 oder 2 Substituenten ausgewählt aus der Gruppe bestehend aus Chlor und C₁-C₄-Alkyl substituiertem Phenyl; oder
- für ein mit 0, 1, 2 oder 3 Substituenten V substituiertes Phenyl, Pyrimidinyl, Pyridinyl, Pyrazolyl, Oxazolyl, Isoxazolyl, Furanyl, Thiophenyl, Benzothiophenyl, Benzofuranyl, [1,2,4]triazolo[1,5-a]pyrimidinyl, 1,3-Benzodioxolyl, oder Tetralinyl, wobei V unabhängig voneinander für Fluor, Chlor, Cyano, Nitro, C₁-C₄-Alkyl, C₁-C₄-Halogenalkyl, C₃-C₆-Cycloalkyl, C₁-C₄-Alkoxy, C₁-C₄-Halogenalkoxy *N*,*N*-Di-C₁-C₂-alkylamino steht.

Weiterhin hierin beschrieben sind in einem Aspekt 32 Verbindungen der Formel (Ic) gemäß einem der Aspekte 18 bis 25 oder 27 bis 31 in der Q für
- C₁-C₆-Alkyl, das optional substituiert ist mit 1, 2, 3 oder 4 Substituenten unabhängig voneinander ausgewählt aus der Gruppe bestehend aus
   Fluor, Oxo, Methoxy, Ethoxy, Benzyloxy, *N,N*-Dimethylamino, C₃-C₆-Cycloalkyl, Phenylthio, Phenyloxy, optional unabhängig voneinander mit 1, 2 oder 3 Substituenten ausgewählt aus der Gruppe bestehend aus Fluor, Chlor, C₁-C₄-Alkoxy, C₁-C₄-Alkyl, und C₁-C₄-Halogenalkyl substituiertem Phenyl, optional unabhängig voneinander mit 1 oder 2 Substituenten ausgewählt aus der Gruppe bestehend aus C₁-C₄-Alkyl und C₁-C₄-Halogenalkyl substituiertem Pyrazolyl und Thiophenyl;
- C₃-C₆-Cycloalkyl optional substituiert mit 1 oder 2 Substituenten unabhängig voneinander ausgewählt aus der Gruppe bestehend aus
   C₁-C₄-Alkoxy, Chlor, Cyano, C₁-C₂-Alkyl, optional unabhängig voneinander mit 1 oder 2 Substituenten ausgewählt aus der Gruppe bestehend aus Chlor und C₁-C₂-Alkyl substituiertem Phenyl; oder
- für ein mit 0, 1, 2 oder 3 Substituenten V substituiertes Phenyl, Pyrimidinyl, Pyridinyl, Pyrazolyl, Oxazolyl, Isoxazolyl, Furanyl, Thiophenyl, Benzothiophenyl, Benzofuranyl, [1,2,4]triazolo[1,5-a]pyrimidinyl, 1,3-Benzodioxolyl oder Tetralinyl, wobei V unabhängig voneinander für Fluor, Chlor, Cyano, Nitro, C₁-C₄-Alkyl, C₁-C₂-Halogenalkyl, C₃-C₆-Cycloalkyl, C₁-C₂-Alkoxy, C₁-C₂-Halogenalkoxy *N*,*N*-Di-C₁-C₂-alkylamino steht.

Weiterhin hierin beschrieben sindin einem Aspekt 33 Verbindungen der Formel (Ic) gemäß einem der Aspekte 18 bis 25 oder 27 bis 32 worin Q für
- Gegebenenfalls mit Fluor, Methyl, Cyano, Chlor unabhängig voneinander ausgewählt einfach oder zweifach substituiertes Pyridinyl (z. B. 4-Pyridinyl, 3-F-4-Pyridinyl, 3-Cl-2-Pyridinyl, 2-Cl-4-Pyridinyl, 2,6-Cl₂-4-Pyridinyl, 2-Cl-3-Pyridinyl, 2-Cl-6-Me-4-Pyridinyl, 3-Pyridinyl, 3-F-5-Pyridinyl, 2-Me-4-Pyridinyl, 2-F-4-Pyridinyl, 2-CN-5-Pyridinyl, 2-Cl-5-Pyridinyl, 2-Cl-5-Pyridinyl, 2-F-5-Pyridinyl, 2-CN-5-Pyridinyl),
- Gegebenenfalls mit Cl, Methoxy, Methyl oder CN einfach substituiertes C₃-C₆-Cycloalkyl (z. B. Cyclopropyl, 1-CN-Cyclopropyl, 1-Cl-Cyclopropyl, 4-Methoxy-Cyclohex-1-yl, 1-Methyl-Cyclohexyl, 1-Methyl-Cyclopropyl, Cyclopentyl),
- Gegebenenfalls mit Fluor, Methoxy, Ethoxy, Trifluormethoxy, Cyano, Trifluormethyl, Dimethylamino, Methyl, Ethyl, Propyl unabhängig voneinander ausgewählt einfach oder zweifach oder dreifach substituiertes Phenyl (z. B. Phenyl, 2-F-Phenyl, 3- F-Phenyl, 3,5-F₂-Phenyl, 2,6-F₂-Phenyl, 4-F-Phenyl, 3-Methoxy-Phenyl, 3-CF₃-4-F-Phenyl, 2,6-Bismethoxyphenyl, 4-(Dimethylamino)-phenyl, 2,4,6-Trisisopropyl-phenyl, 3-CF₃-Phenyl, 4-Me-Phenyl, 2,3-F₂-Phenyl, 2-Ethoxy-phenyl, 2-(Trifluormethoxy)-phenyl, 3-Cyano-phenyl, 2,3-Dimethyl-phenyl, 4-CN-Phenyl, 3,4-Dimethyl-phenyl, 3-Me, 4-F-Phenyl, 3-F, 4-Me-Phenyl, 3-MeO, 4-F-Phenyl, 3-F, 4-MeO-Phenyl, 4-Cl, 3-F-Phenyl, 3-Methoxy-4-Me-Phenyl, 3-EtO-4-F-Phenyl),
- Gegebenenfalls mit Methoxy oder Ethoxy einfach substituiertes C₁-C₆-Alkyl oder C₁-C₆-Haloalkyl (z. B. CHF₂CF₂-, CF₃CH₂-, Methyl, Ethyl, Propyl, 1,1,2,2-Tetramethyl-ethyl, (1,1-Dimethyl)-2-F-ethyl, Methoxymethyl, 1-Methyl-propyl, 2-Methoxyethyl, (R)-1-F-Ethyl, 2-Me-prop-1-yl, Butyl, 2,2-Diemthyl-prop-1-yl, 1,1-Dimethyl-2-ethoxy-ethyl),
- Gegebenenfalls mit Methyl, Ethyl, Propyl, Butyl, Nitro unabhängig voneinander ausgewählt einfach, zweifach oder dreifach substituiertes Pyrazolyl (z. B. 1-Methyl-4-pyrazolyl, 1-Methyl-3-tBu-4-NO₂-Pyrazolyl),
- Thiazolyl (z. B. 1-Thiazolyl),
- Gegebenenfalls mit Chlor einfach substituiertes Thiophenyl (z. B. 3-Thiophenyl, 2-Thiophenyl, 3-Cl-2-Thiophenyl),
- Pyrimidinyl (z. B. Pyrimidin-3-yl, Pyrimidin-2-yl),
- Gegebenenfalls mit Halogenmethyl, Methyl unabhängig voneinander ausgewählt einfach oder zweifach substituiertes [1,2,4]Triazolo[1,5-a]pyrimidin-2-yl (z. B. 7-(Difluormethyl)-5-methyl-[1,2,4]Triazolo[1,5-a]pyrimidin-2-yl),
- Gegebenenfalls mit Chlor, Methyl unabhängig voneinander ausgewählt einfach oder zweifach substituiertes Benzothiophenyl (z. B. 3-Cl-Benzothiophen-2-yl, 3-Cl-6-Me-Benzothiophen-2-yl),
- Gegebenenfalls mit Methyl, Trifluormethyl unabhängig voneinander ausgewählt einfach oder zweifach substituiertes Furanyl (z. B. 3-Me-2-Benzofuranyl, 2-CF₃-4-Me-Furan-2-yl, 2-Me-3-Furanyl,),
- Gegebenenfalls mit Methyl, Trifluormethyl einfach oder zweifach substituiertes Thiophenyl (z. B. 5-Me-Thiophen-2-yl),
- Gegebenenfalls mit Methyl einfach substituiertes Isoxazolyl (z. B. 5-Methyl-isoxazol-4-yl),
- Gegebenenfalls mit Fluor einfach oder zweifach substituiertes 1,3-Benzodioxolyl (z. B. 2,2-Difluor-1,3-benzodioxol-5-yl oder 1,3-Benzodioxol-5-yl) steht.

Weiterhin hierin beschrieben sind in einem Aspekt 34 Verbindungen der Formel (Ic) gemäß einem der Aspekte 18 bis 25 oder 27 bis 32 worin Q für Phenylthiomethyl, 1-Phenyl-ethyl, Phenyl-cPentylmethyl, Benzyloxymethyl, Phenoxyethyl, 1-Me, 1-(3-Cl-Ph)-ethyl, 1-(4-Cl-Ph)-2-Me-prop-1-yl, 3-F-Benzyl, 3-CF₃-Benzyl, 3-CF3-4-Me-Pyrazol-1-yl-methyl rac-1-Phenoxy-ethyl, 1-Phenyl-prop-1-yl, 2-Phenyl-ethyl, 2-Cl-4-F-Benzyl oder Tetralinyl,

1-(4-Me-Ph)-Cyclohex-1-yl, 1-Ph-Cycloprop-1-yl, 1-(4-Cl-Ph)-Cyclobut-1-yl, 1-(4-Cl-Ph)-Cyclopent-1-yl, 2-Thiophenyl-methyl, (Me)₂NC(O)C(O)- steht.

Weiterhin hierin beschrieben sindin einem Aspekt 35 Verbindungen der Formel (Ic) gemäß einer der Aspekte 18 bis 34, die eine Verbindung der Formel (Ic-1) darstellen

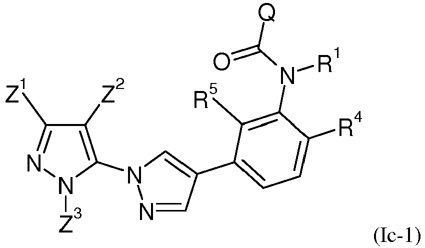

Verbindungen der Formel (Ic-1) sind Verbindungen der allgemeinen Formel (Ic), wobei W = O, A₁ = A₂ = CH, A₃ = CR⁴, A₄ = CR⁵ und R⁶ = H (bzw. n = 0) sind.

Weiterhin hierin beschrieben sind in einem Aspekt 36 Verbindungen der Formel (Ic-1) gemäß Aspekt 35 worin Q für
- Gegebenenfalls mit Fluor, Methyl, Cyano, Chlor unabhängig voneinander ausgewählt einfach oder zweifach substituiertes Pyridinyl (z. B. 4-Pyridinyl, 3-F-4-Pyridinyl, 3-Cl-2-Pyridinyl, 2-Cl-4-Pyridinyl, 2,6-Cl₂-4-Pyridinyl, 2-Cl-3-Pyridinyl, 2-Cl-6-Me-4-Pyridinyl, 3-Pyridinyl, 3-F-5-Pyridinyl, 2-Me-4-Pyridinyl, 2-F-4-Pyridinyl, 2-CN-5-Pyridinyl, 2-Cl-5-Pyridinyl, 2-Cl-5-Pyridinyl, 2-F-5-Pyridinyl, 2-CN-5-Pyridinyl),
- Gegebenenfalls mit Cl, Methoxy, Methyl oder CN einfach substituiertes C₃-C₆-Cycloalkyl (z. B. Cyclopropyl, 1-CN-Cyclopropyl, 1-Cl-Cyclopropyl, 4-Methoxy-Cyclohex-1-yl, 1-MethylCyclohexyl, 1-Methyl-Cyclopropyl, Cyclopentyl),
- Gegebenenfalls mit Fluor, Methoxy, Ethoxy, Trifluormethoxy, Cyano, Trifluormethyl, Dimethylamino, Methyl, Ethyl, Propyl unabhängig voneinander ausgewählt einfach oder zweifach oder dreifach substituiertes Phenyl (z. B. Phenyl, 2-F-Phenyl, 3- F-Phenyl, 3,5-F₂-Phenyl, 2,6-F₂-Phenyl, 4-F-Phenyl, 3-Methoxy-Phenyl, 3-CF₃-4-F-Phenyl, 2,6-Bismethoxyphenyl, 4-(Dimethylamino)-phenyl, 2,4,6-Trisisopropyl-phenyl, 3-CF₃-Phenyl, 4-Me-Phenyl, 2,3-F₂-Phenyl, 2-Ethoxy-phenyl, 2-(Trifluormethoxy)-phenyl, 3-Cyano-phenyl, 2,3-Dimethyl-phenyl, 4-CN-Phenyl, 3,4-Dimethyl-phenyl, 3-Me, 4-F-Phenyl, 3-F, 4-Me-Phenyl, 3-MeO, 4-F-Phenyl, 3-F, 4-MeO-Phenyl, 4-Cl, 3-F-Phenyl, 3-Methoxy-4-Me-Phenyl, 3-EtO-4-F-Phenyl),
- Gegebenenfalls mit Methoxy oder Ethoxy einfach substituiertes C₁-C₆-Alkyl oder C₁-C₆-Haloalkyl (z. B. CHF₂CF₂-, CF₃CH₂-, Methyl, Ethyl, Propyl, 1,1,2,2-Tetramethyl-ethyl, (1,1-Dimethyl)-2-F-ethyl, Methoxymethyl, 1-Methyl-propyl, 2-Methoxyethyl, (R)-1-F-Ethyl, 2-Me-prop-1-yl, Butyl, 2,2-Diemthyl-prop-1-yl, 1,1-Dimethyl-2-ethoxy-ethyl),
- Gegebenenfalls mit Methyl, Ethyl, Propyl, Butyl, Nitro unabhängig voneinander ausgewählt einfach, zweifach oder dreifach substituiertes Pyrazolyl (z. B. 1-Methyl-4-pyrazolyl, 1-Methyl-3-tBu-4-NO₂-Pyrazolyl),
- Thiazolyl (z. B. 1-Thiazolyl),
- Gegebenenfalls mit Chlor einfach substituiertes Thiophenyl (z. B. 3-Thiophenyl, 2-Thiophenyl, 3-Cl-2-Thiophenyl),
- Pyrimidinyl (z. B. Pyrimidin-3-yl, Pyrimidin-2-yl),
- Gegebenenfalls mit Halogenmethyl, Methyl unabhängig voneinander ausgewählt einfach oder zweifach substituiertes [1,2,4]triazolo[1,5-a]pyrimidin-2-yl (z. B. 7-(Difluormethyl)-5-methyl-[1,2,4]triazolo[1,5-a]pyrimidin-2-yl),
- Gegebenenfalls mit Chlor, Methyl unabhängig voneinander ausgewählt einfach oder zweifach substituiertes Benzothiophenyl (z. B. 3-Cl-Benzothiophen-2-yl, 3-Cl-6-Me-Benzothiophen-2-yl(,
- Gegebenenfalls mit Methyl, Trifluormethyl unabhängig voneinander ausgewählt einfach oder zweifach substituiertes Furanyl (z. B. 3-Me-2-Benzofuranyl, 2-CF₃-4-Me-Furan-2-yl, 2-Me-3-Furanyl,),
- Gegebenenfalls mit Methyl, Trifluormethyl einfach oder zweifach substituiertes Thiophenyl (z. B. 5-Me-Thiophen-2-yl),
- Gegebenenfalls mit Methyl einfach substituiertes Isoxazolyl (z. B. 5-Methyl-isoxazol-4-yl),
- Gegebenenfalls mit Fluor einfach oder zweifach substituiertes 1,3-Benzodioxolyl (z. B. 2,2-Difluor-1,3-benzodioxol-5-yl oder 1,3-Benzodioxol-5-yl) steht.

Weiterhin hierin beschrieben sind in einem Aspekt 37 Verbindungen der Formel (Ic-1) gemäß Aspekt 35 worin Q für
Phenylthiomethyl, 1-Phenyl-ethyl, Phenyl-cPentylmethyl, Benzyloxymethyl, Phenoxyethyl, 1-Me, 1-(3-Cl-Ph)-ethyl, 1-(4-Cl-Ph)-2-Me-prop-1-yl, 3-F-Benzyl, 3-CF₃-Benzyl, 3-CF3-4-Me-Pyrazol-1-yl-methyl rac-1-Phenoxy-ethyl, 1-Phenyl-prop-1-yl, 2-Phenyl-ethyl, 2-Cl-4-F-Benzyl oder Tetralinyl,
1-(4-Me-Ph)-Cyclohex-1-yl, 1-Ph-Cycloprop-1-yl, 1-(4-Cl-Ph)-Cyclobut-1-yl, 1-(4-Cl-Ph)-Cyclopent-1-yl, 2-Thiophenyl-methyl, (Me)₂NC(O)C(O)- steht.

Weiterhin hierin beschrieben sind in einem Aspekt 38 Verbindungen der Formel (Ic-1) gemäß Aspekt 35 worin Q für einen Rest wie in Aspekten 36 und 37 angegeben steht.

Weiterhin hierin beschrieben sind in einem Aspekt 39 Verbindungen der Formel (Ic) gemäß Aspekt 35 worin Q für 4-F-Phenyl, 3-MeO-4-F-Phenyl, 3-Cl-2-Pyridinyl, 2-CN-5-Pyridinyl, 4-Pyridinyl steht. Weiterhin hierin beschrieben sind in einem Aspekt 40 Verbindungen der Formel (Ic-1) gemäß Aspekt 35 worin Q für einen Rest wie in Aspekten 36, 37 und 38 angegeben steht.

Weiterhin hierin beschrieben sind in einem Aspekt 41 Verbindungen der Formel (Ic) gemäß einem der Aspekte 18 bis 34, die eine Verbindung der Formel (Ic-2) darstellen

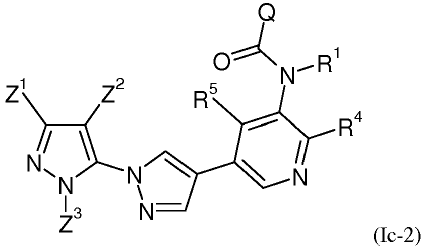

Verbindungen der Formel (Ic-2) sind Verbindungen der allgemeinen Formel (Ic), wobei W = O, A₁ = CH, A₂ = N, A₃ = CR⁴, A₄ = CR⁵ und R⁶ = H (bzw. n = 0) sind.

Weiterhin hierin beschrieben sindVerbindungen der Formel (Ic-2) gemäß Aspekt 41 worin Q für 4-F-Phenyl, 3-MeO, 4-F-Phenyl, 3-Cl-2-Pyridinyl, 2-CN-5-Pyridinyl, 4-Pyridinyl steht.

### Verwendung:

Die Erfindung betrifft auch nicht-therapeutische Verfahren zur Bekämpfung von tierischen Schädlingen, bei dem man erfindungsgemäße Verbindungen der allgemeinen Formel (Ic) auf tierische Schädlinge und/oder ihren Lebensraum einwirken lässt. Die erfindungsgemäßen Verbindungen der allgemeinen Formel (Ic) werden zur Bekämpfung einer Vielzahl verschiedener Schädlinge einschließlich beispielsweise schädlicher saugender Insekten, beißender Insekten und anderen an Pflanzen parasitierenden Schädlingen, Vorratsschädlingen, Schädlingen, die industrielle Materialien zerstören und Hygieneschädlingen einschließlich Parasiten im Bereich Tiergesundheit verwendet.

### Pflanzenschutz

Die erfindungsgemäßen Verbindungen der Formel (Ic) eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., Aceria kuko, Aceria sheldoni, Aculops spp., Aculus spp., Amblyomma spp., Amphitetranychus viennensis, Argas spp., Boophilus spp., Brevipalpus spp., Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., Epitrimerus pyri, Eutetranychus spp., Eriophyes spp., Glycyphagus domesticus, Halotydeus destructor, Hemitarsonemus spp., Hyalomma spp., Ixodes spp., Latrodectus spp., Loxosceles spp., Metatetranychus spp., Neutrombicula autumnalis, Nuphersa spp., Oligonychus spp., Ornithodorus spp., Ornithonyssus spp., Panonychus spp., Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., Tetranychus spp., Trombicula alfreddugesi, Vaejovis spp., Vasates lycopersici.;
aus der Klasse der Chilopoda z.B. Geophilus spp., Scutigera spp.;
aus der Ordnung oder der Klasse der Collembola z.B. Onychiurus armatus;
aus der Klasse der Diplopoda z.B. Blaniulus guttulatus;
aus der Klasse der Insecta, z.B. aus der Ordnung der Blattodea z.B. Blattella asahinai, Blattella germanica, Blatta orientalis, Leucophaea maderae, Panchlora spp., Parcoblatta spp., Periplaneta spp., Supella longipalpa;
aus der Ordnung der Coleoptera z.B. Acalymma vittatum, Acanthoscelides obtectus, Adoretus spp., Agelastica alni, Agriotes spp., Alphitobius diaperinus, Amphimallon solstitialis, Anobium punctatum, Anoplophora spp., Anthonomus spp., Anthrenus spp., Apion spp., Apogonia spp., Atomaria spp., Attagenus spp., Baris caerulescens, Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptolestes ferrugineus, Cryptorhynchus lapathi, Cylindrocopturus spp., Dermestes spp., Diabrotica spp., Dichocrocis spp., Dicladispa armigera, Diloboderus spp., Epilachna spp., Epitrix spp., Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., Lachnosterna consanguinea, Lasioderma serricorne, Latheticus oryzae, Lathridius spp., Lema spp., Leptinotarsa decemlineata, Leucoptera spp., Lissorhoptrus oryzophilus, Lixus spp., Luperomorpha xanthodera, Luperodes spp., Lyctus spp., Megascelis spp., Melanotus spp., Meligethes aeneus, Melolontha spp., Migdolus spp., Monochamus spp., Naupactus xanthographus, Necrobia spp., Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllophaga helleri, Phyllotreta spp., Popillia japonica, Premnotrypes spp., Prostephanus truncatus, Psylliodes spp., Ptinus spp., Rhizobius ventralis, Rhizopertha dominica, Sitophilus spp., Sitophilus oryzae, Sphenophorus spp., Stegobium paniceum, Sternechus spp., Symphyletes spp., Tanymecus spp., Tenebrio molitor, Tenebrioides mauretanicus, Tribolium spp., Trogoderma spp., Tychius spp., Xylotrechus spp., Zabrus spp.;
aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Calliphora vicina, Ceratitis capitata, Chironomus spp., Chrysomyia spp., Chrysops spp., Chrysozona pluvialis, Cochliomyia spp., Contarinia spp., Cordylobia anthropophaga, Cricotopus sylvestris, Culex spp., Culicoides spp., Culiseta spp., Cuterebra spp., Dacus oleae, Dasyneura spp., Delia spp., Dermatobia hominis, Drosophila spp., z.B. Drosophila suzukii, Echinocnemus spp., Fannia spp., Gasterophilus spp., Glossina spp., Haematopota spp., Hydrellia spp., Hydrellia griseola, Hylemya spp., Hippobosca spp., Hypoderma spp., Liriomyza spp., Lucilia spp., Lutzomyia spp., Mansonia spp., Musca spp., Oestrus spp.,
Oscinella frit, Paratanytarsus spp., Paralauterborniella subcincta, Pegomyia spp., Phlebotomus spp., Phorbia spp., Phormia spp., Piophila casei, Prodiplosis spp., Psila rosae, Rhagoletis spp., Sarcophaga spp., Simulium spp., Stomoxys spp., Tabanus spp., Tetanops spp., Tipula spp.;
aus der Ordnung der Heteroptera z.B. Anasa tristis, Antestiopsis spp., Boisea spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Homoptera z.B. Acizzia acaciaebaileyanae, Acizzia dodonaeae, Acizzia uncatoides, Acrida turrita, Acyrthosipon spp., Acrogonia spp., Aeneolamia spp., Agonoscena spp., Aleyrodes proletella, Aleurolobus barodensis, Aleurothrixus floccosus, Allocaridara malayensis, Amrasca spp., Anuraphis cardui, Aonidiella spp., Aphanostigma piri, Aphis spp., Arboridia apicalis, Arytainilla spp., Aspidiella spp., Aspidiotus spp., Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., Calligypona marginata, Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus ficus, Cicadulina mbila, Coccomytilus halli, Coccus spp., Cryptomyzus ribis, Cryptoneossa spp., Ctenarytaina spp., Dalbulus spp., Dialeurodes citri, Diaphorina citri, Diaspis spp., Drosicha spp., Dysaphis spp., Dysmicoccus spp., Empoasca spp., Eriosoma spp., Erythroneura spp., Eucalyptolyma spp., Euphyllura spp., Euscelis bilobatus, Ferrisia spp., Geococcus coffeae, Glycaspis spp., Heteropsylla cubana, Heteropsylla spinulosa, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Macrosteles facifrons, Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nettigoniclla spectra, Nilaparvata lugens, Oncometopia spp., Orthezia praelonga, Oxya chinensis, Pachypsylla spp., Parabemisia myricae, Paratrioza spp., Parlatoria spp., Pemphigus spp., Peregrinus maidis, Phenacoccus spp., z.B. Phenacoccus madeirensis, Phloeomyzus passerinii, Phorodon humuli, Phylloxera spp., Pinnaspis aspidistrae, Planococcus spp., Prosopidopsylla flava, Protopulvinaria pyriformis, Pseudaulacaspis pentagona, Pseudococcus spp., z.B. Pseudococcus viburni, Psyllopsis spp., Psylla spp., Pteromalus spp., Pyrilla spp., Quadraspidiotus spp., Quesada gigas, Rastrococcus spp., Rhopalosiphum spp., Saissetia spp., Scaphoideus titanus, Schizaphis graminum, Selenaspidus articulatus, Sogata spp., Sogatella furcifera, Sogatodes spp., Stictocephala festina, Siphoninus phillyreae, Tenalaphara malayensis,
Tetragonocephela spp., Tinocallis caryaefoliae, Tomaspis spp., Toxoptera spp., Trialeurodes vaporariorum, Trioza spp., Typhlocyba spp., Unaspis spp., Viteus vitifolii, Zygina spp.;
aus der Ordnung der Hymenoptera z.B. Acromyrmex spp., Athalia spp., Atta spp., Diprion spp., Hoplocampa spp., Lasius spp., Monomorium pharaonis, Sirex spp., Solenopsis invicta, Tapinoma spp., Urocerus spp., Vespa spp., Xeris spp.;
aus der Ordnung der Isopoda z.B. Armadillidium vulgare, Oniscus asellus, Porcellio scaber;
aus der Ordnung der Isoptera z.B. Coptotermes spp., Cornitermes cumulans, Cryptotermes spp., Incisitermes spp., Microtermes obesi, Odontotermes spp., Reticulitermes spp.;
aus der Ordnung der Lepidoptera z.B. Achroia grisella, Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnaphalocrocis medinalis, Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia spp., Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamstra brassicae, Melanitis leda, Mocis spp., Monopis obviella, Mythimna separata, Nemapogon cloacellus, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plodia interpunctella, Plusia spp., Plutella xylostella, Prays spp., Prodenia spp., Protoparce spp., Pseudaletia spp., Pseudaletia unipuncta, Pseudoplusia includens, Pyrausta nubilalis, Rachiplusia nu, Schoenobius spp., Scirpophaga spp., Scirpophaga innotata, Scotia segetum, Sesamia spp., Sesamia inferens, Sparganothis spp., Spodoptera spp., Spodoptera praefica, Stathmopoda spp., Stomopteryx subsecivella, Synanthedon spp., Tecia solanivora, Thermesia gemmatalis, Tinea cloacella, Tinea pellionella, Tineola bisselliella, Tortrix spp., Trichophaga tapetzella, Trichoplusia spp., Tryporyza incertulas, Tuta absoluta, Virachola spp.;
aus der Ordnung der Orthoptera oder Saltatoria z.B. Acheta domesticus, Dichroplus spp., Gryllotalpa spp., Hieroglyphus spp., Locusta spp., Melanoplus spp., Schistocerca gregaria;
aus der Ordnung der Phthiraptera z.B. Damalinia spp., Haematopinus spp., Linognathus spp., Pediculus spp., Phylloera vastatrix, Phtirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., Pulex irritans, Tunga penetrans, Xenopsylla cheopsis;
aus der Ordnung der Thysanoptera z.B. Anaphothrips obscurus, Baliothrips biformis, Drepanothrips reuteri, Enneothrips flavens, Frankliniella spp., Heliothrips spp., Hercinothrips femoralis, Rhipiphorothrips cruentatus, Scirtothrips spp., Taeniothrips cardamomi, Thrips spp.;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp.;
Schädlinge aus dem Stamm der Mollusca, insbesondere aus der Klasse der Bivalvia, z.B. Dreissena spp., sowie aus der Klasse der Gastropoda z.B. Arion spp., Biomphalaria spp., Bulinus spp., Deroceras spp., Galba spp., Lymnaea spp., Oncomelania spp., Pomacea spp., Succinea spp.;

### Formulierungen

Die vorliegende Erfindung betrifft weiterhin Formulierungen und daraus bereitete Anwendungsformen als Pflanzenschutzmittel und/oder Schädlingsbekämpfungsmittel wie z. B. Drench-, Drip- und Spritzbrühen, umfassend mindestens einen der erfindungsgemäßen Wirkstoffe. Gegebenenfalls enthalten die Anwendungsformen weitere Pflanzenschutzmittel und/oder Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren erfindungsgemäßen Wirkstoffen weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Wirkstoffe mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung des Wirkstoffs oder den aus diesen Formulierungen bereiteten Anwendungsformen (wie z.B. gebrauchsfähigen Pflanzenschutzmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasser-stoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn einer der Wirkstoff und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer. Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natür-liche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Wirkstoffe mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% Wirkstoff oder, besonders bevorzugt zwischen 0,01 und 95 Gew.-% Wirkstoff, besonders bevorzugt zwischen 0,5 und 90 Gew.-% Wirkstoff, bezogen auf das Gewicht der Formulierung.

Der Wirkstoffgehalt der aus den Formulierungen bereiteten Anwendungsformen (Pflanzenschutzmittel) kann in weiten Bereichen variieren. Die Wirkstoffkonzentration der Anwendungsformen kann üblicherweise zwischen 0,00000001 und 95 Gew.-% Wirkstoff, vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (Ic) können auch in Mischung mit einem oder mehreren geeigneten Fungiziden, Bakteriziden, Akariziden, Molluskiziden, Nematiziden, Insektiziden, Mikrobiologika, Nützlingen, Herbizide, Düngemitteln, Vogelrepellentien, Phytotonics, Sterilantien, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern.

Weiterhin können die Verbindungen der Formel (Ic) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (Ic) in Mischungen mit Mitteln zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (Ic) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann sind auch diese Formen mit umfasst, auch wenn sie sie nicht in jedem Fall explizit genannt wurden.

### Mischungen mit Insektiziden / Akariziden / Nematiziden

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Momfluorothrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin oder DDT oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor oder Flupyradifurone.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene oder Fenoxycarb oder Pyriproxyfen.
(8) Wirkstoffe mit unbekannten oder nicht spezifischen Wirkmechanismen, wie beispielsweise Alkylhalide, z.B. Methylbromid und andere Alkylhalide; oderChloropicrin oder Sulfurylfluorid oder Borax oder Brechweinstein.
(9) Selektive Fraßhemmer, z.B. Pymetrozine oder Flonicamid.
(10) Milbenwachstumsinhibitoren, z.B. Clofentezine, Hexythiazox und Diflovidazin oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, CrylAc, CrylFa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungshemmer (insbesondere bei Dipteren, d.h.Zweiflüglern), wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon oder Acequinocyl oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).
(22) Spannungsabhängige Natriumkanal-Blocker, z.B. Indoxacarb oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise Diamide, z.B. Chlorantraniliprole, Cyantraniliprole und Flubendiamide.

Weitere Wirkstoffe mit unbekanntem oder nicht eindeutigem Wirkmechanismus, wie beispielsweise Afidopyropen, Afoxolaner, Azadirachtin, Benclothiaz, Benzoximate, Bifenazate, Bromopropylate, Chinomethionat, Cryolite, Cyclaniliprole, Cycloxaprid, Cyhalodiamide Dicloromezotiaz, Dicofol, Diflovidazin, Flometoquin, Fluensulfone, Flufenerim, Flufenoxystrobin, Flufiprole, Fluhexafon, Fluopyram, Fluralaner, Fufenozide, Guadipyr, Heptafluthrin, Imidaclothiz, Iprodione, Meperfluthrin, Paichongding, Pyflubumide, Pyridalyl, Pyrifluquinazon, Pyriminostrobin, Tetramethylfluthrin, Tetraniliprole, Tetrachlorantraniliprole, Tioxazafen, Triflumezopyrim und Iodmethan; desweiteren Präparate auf Basis von Bacillus firmus (I-1582, BioNeem, Votivo), sowie folgende bekannte wirksame Verbindungen: 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), Methyl-2-[2-({[3-brom-1-(3-chlorpyridin-2-yl)-1H-pyrazol-5-yl]carbonyl} amino)-5-chlor-3-methylbenzoyl] -2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-(1{[3-brom-1-(3-chlorpyridin-2-yl)-1H-pyrazol-5-yl]carbonyl}amino)benzoyl]-2-ethylhydrazincarboxylat (bekannt aus WO2005/085216), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 8-Chlor-N-[(2-chlor-5-methoxyphenyl)sulfonyl]-6-(trifluormethyl)imidazo[1,2-a]pyridin-2-carboxamid (bekannt aus WO2010/129500), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2009/080250), N-[(2E)-1-[(6-Chlorpyridin-3-yl)methyl]pyridin-2(1H)-yliden]-2,2,2-trifluoracetamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-pyrido[1,2-a]pyrimidin-1-ium-2-olat (bekannt aus WO2009/099929), 4-(3-{2,6-Dichlor-4-[(3,3-dichlorprop-2-en-1-yl)oxy]phenoxy}propoxy)-2-methoxy-6-(trifluormethyl)pyrimidin (bekannt aus CN101337940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969, 3-[Benzoyl(methyl)amino]-N-[2-brom-4-[1,2,2,2-tetrafluor-1-(trifluormethyl)ethyl]-6-(trifluormethyl)phenyl]-2-fluor-benzamid (bekannt aus WO 2010018714), Butyl-[2-(2,4-dichlorphenyl)-3-oxo-4-oxaspiro[4.5]dec-1-en-1-yl]-carbonat (bekannt aus CN 102060818), 4-[5-(3,5-Dichlorphenyl)-5-(trifluormethyl)-4H-isoxazol-3-yl]-N-[(Z)-methoxyiminomethyl]-2-methyl-benzamid (bekannt aus WO2007/026965), 3E)-3-[1-[(6-Chlor-3-Pyridinyl)methyl]-2-Pyridinyliden]-1,1,1-trifluor-propan-2-on (bekannt aus WO2013/144213, N-(Methylsulfonyl)-6-[2-(pyridin-3-yl)-1,3-thiazol-5-yl]pyridin-2-carboxamid (bekannt aus WO2012/000896), N-[3-(Benzylcarbamoyl)-4-chlorphenyl]-1-methyl-3-(pentafluorethyl)-4-(trifluormethyl)-1H-pyrazol-5-carboxamid bekannt aus WO2010/051926).

### Mischungen mit Fungiziden

Die hier mit ihrem "common name" spezifizierten Wirkstoffe sind bekannt, beispielsweise beschrieben im "Pesticide Manual" oder im Internet (beispielsweise: http://www.alanwood.net/pesticides).

Alle aufgeführten fungiziden Mischpartner der Klassen (1) bis (15) können optional Salze mit entsprechenden Basen oder Säuren bilden, sofern geeignete funktionelle Gruppen vorliegen. Außerdem sind für die aufgeführten fungiziden Mischpartner der Klassen (1) bis (15) auch tautomere Formen eingeschlossen, sofern Tautomerie möglich ist.
1) Inhibitoren der Ergosterolbiosynthese, zum Beispiel (1.01) Aldimorph, (1.02) Azaconazol, (1.03) Bitertanol, (1.04) Bromuconazol, (1.05) Cyproconazol, (1.06) Diclobutrazol, (1.07) Difenoconazol, (1.08) Diniconazol, (1.09) Diniconazol-M, (1.10) Dodemorph, (1.11) Dodemorphacetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-cis, (1.25) Hexaconazol, (1.26) Imazalil, (1.27) Imazalilsulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) 1-(2,2-Dimethyl-2,3-dihydro-1H-inden-1-yl)-1H-imidazol-5-carbonsäuremethylester, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid, (1.64) O-[1-(4-Methoxyphenoxy)-3,3-dimethylbutan-2-yl]-1H-imidazol-1-carbothioat, (1.65) Pyrisoxazol, (1.66) 2-{[3-(2-Chlorphenyl)-2-(2,4-difluorphenyl)oxiran-2-yl]methyl}-2,4-dihydro-3H-1,2,4-triazol-3-thion, (1.67) 1-{[3-(2-Chlorphenyl)-2-(2,4-difluorphenyl)oxiran-2-yl]methyl}-1H-1,2,4-triazol-5-ylthiocyanat, (1.68) 5-(Allylsulfanyl)-1-{[3-(2-chlorphenyl)-2-(2,4-difluorphenyl)oxiran-2-yl]methyl}-1H-1,2,4-triazol, (1.69) 2-[1-(2,4-Dichlorphenyl)-5-hydroxy-2,6,6-trimethylheptan-4-yl]-2,4-dihydro-3H-1,2,4-triazol-3-thion, (1.70) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.71) 2-{[rel(2R,3R)-3-(2-Chlorphenyl)-2-(2,4-difluorphenyl)oxiran-2-yl]methyl}-2,4-dihydro-3H-1,2,4-triazol-3-thion, (1.72) 1-{[rel(2R,3S)-3-(2-Chlorphenyl)-2-(2,4-difluorphenyl)oxiran-2-yl]methyl}-1H-1,2,4-triazol-5-ylthiocyanat, (1.73) 1-{[rel(2R,3R)-3-(2-Chlorphenyl)-2-(2,4-difluorphenyl)oxiran-2-yl]methyl}-1H-1,2,4-triazol-5-ylthiocyanat, (1.74) 5-(Allylsulfanyl)-1-{[rel(2R,3S)-3-(2-chlorphenyl)-2-(2,4-difluorphenyl)oxiran-2-yl]methyl}-1H-1,2,4-triazol, (1.75) 5-(Allylsulfanyl)-1-{[rel(2R,3R)-3-(2-chlorphenyl)-2-(2,4-difluorphenyl)oxiran-2-yl]methyl}-1H-1,2,4-triazol, (1.76) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.77) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.78) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.79) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.80) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (1.81) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (1.82) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.83) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (1.84) 2-[4-(4-Chlorphenoxy)-2-(trifluormethyl)phenyl]-1-(1H-1,2,4-triazol-1-yl)propan-2-ol, (1.85) 2- [4-(4-Chlorphenoxy)-2-(trifluormethyl)phenyl]-1-(1H-1,2,4-triazol-1-yl)butan-2-ol, (1.86) 2-[4-(4-Chlorphenoxy)-2-(trifluormethyl)phenyl]-1-(1H-1,2,4-triazol-1-yl)pentan-2-ol, (1.87) 2- [2-Chlor-4-(4-chlorphenoxy)phenyl] -1-(1H-1,2,4-triazol-1-yl)butan-2-ol, (1.88) 2-[2-Chlor-4-(2,4-dichlorphenoxy)phenyl] -1-(1H-1,2,4-triazol-1-yl)propan-2-ol, (1.89) (2R)-2-(1-Chlorcyclopropyl)-4-[(1R)-2,2-dichlorcyclopropyl]-1-(1H-1,2,4-triazol-1-yl)butan-2-ol, (1.90) (2R)-2-(1-Chlorcyclopropyl)-4-[(1S)-2,2-dichlorcyclopropyl] -1-(1H-1,2,4-triazol-1-yl)butan-2-ol, (1.91) (2S)-2-(1-Chlorcyclo-propyl)-4-[(1S)-2,2-dichlorcyclopropyl]-1-(1H-1,2,4-triazol-1-yl)butan-2-ol, (1.92) (2S)-2-(1-Chlor-cyclopropyl)-4-[(1R)-2,2-dichlorcyclopropyl]-1-(1H-1,2,4-triazol-1-yl)butan-2-ol, (1.93) (1S,2R,5R)-5-(4-Chlorbenzyl)-2-(chlormethyl)-2-methyl-1-(1H-1,2,4-triazol-1-ylmethyl)cyclopentanol, (1.94) (1R,2S,5S)-5-(4-Chlorbenzyl)-2-(chlormethyl)-2-methyl-1-(1H-1,2,4-triazol-1-ylmethyl)cyclopentanol, (1.95) 5-(4-Chlorbenzyl)-2-(chlormethyl)-2-methyl-1-(1H-1,2,4-triazol-1-ylmethyl)cyclopentanol.
2) Inhibitoren der Atmungskette am Komplex I oder II, zum Beispiel (2.01) Bixafen, (2.02) Boscalid, (2.03) Carboxin, (2.04) Diflumetorim, (2.05) Fenfuram, (2.06) Fluopyram, (2.07) Flutolanil, (2.08) Fluxapyroxad, (2.09) Furametpyr, (2.10) Furmecyclox, (2.11) Isopyrazam (Mischung von syn-epimerem Racemat 1RS,4SR,9RS und anti-epimerem Racemat 1RS,4SR,9SR), (2.12) Isopyrazam (anti-epimeres Racemat 1RS,4SR,9SR), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Racemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxan, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{ [4-(trifluormethyl)pyridin-2-yl]oxy}phenyl)ethyl]chinazolin-4-amin, (2.29) Benzovindiflupyr, (2.30) N-[(1S,4R)-9-(Dichlormethylen)-1,2,3,4-tetrahydro-1,4-methanonaphthalin-5-yl]-3-(difluormethyl)-1-methyl-1H-pyrazol-4-carboxamid, (2.31) N-[(1R,4S)-9-(Dichlormethylen)-1,2,3,4-tetrahydro-1,4-methanonaphthalin-5-yl]-3-(difluormethyl)-1-methyl-1H-pyrazol-4-carboxamid, (2.32) 3-(Difluormethyl)-1-methyl-N-(1,1,3-trimethyl-2,3-dihydro-1H-inden-4-yl)-1H-pyrazol-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridin-3-carboxamid, (2.43) Isofetamid, (2.44) 1-Methyl-3-(trifluormethyl)-N-[2'-(trifluormethyl)biphenyl-2-yl]-1H-pyrazol-4-carboxamid, (2.45) N-(4'-Chlorbiphenyl-2-yl)-3-(difluormethyl)-1-methyl-1H-pyrazol-4-carboxamid, (2.46) N-(2',4'-Dichlorbiphenyl-2-yl)-3-(difluormethyl)-1-methyl-1H-pyrazol-4-carboxamid, (2.47) 3-(Difluormethyl)-1-methyl-N- [4'-(trifluormethyl)biphenyl-2-yl]-1H-pyrazol-4-carboxamid, (2.48) N-(2',5'-Difluorbiphenyl-2-yl)-1-methyl-3-(trifluormethyl)-1H-pyrazol-4-carboxamid, (2.49) 3-(Difluormethyl)-1-methyl-N-[4'-(prop-1-in-1-yl)biphenyl-2-yl]-1H-pyrazol-4-carboxamid, (2.50) 5-Fluor-1,3-dimethyl-N-[4'-(prop-1-in-1-yl)biphenyl-2-yl]-1H-pyrazol-4-carboxamid, (2.51) 2-Chlor-N-[4'-(prop-1-in-1-yl)biphenyl-2-yl]nicotinamid, (2.52) 3-(Difluormethyl)-N-[4'-(3,3-dimethylbut-1-in-1-yl)biphenyl-2-yl]-1-methyl-1H-pyrazol-4-carboxamid, (2.53) N-[4'-(3,3-Dimethylbut-1-in-1-yl)biphenyl-2-yl]-5-fluor-1,3-dimethyl-1H-pyrazol-4-carboxamid, (2.54) 3-(Difluormethyl)-N-(4'-ethinylbiphenyl-2-yl)-1-methyl-1H-pyrazol-4-carboxamid, (2.55) N-(4'-Ethinylbiphenyl-2-yl)-5-fluor-1,3-dimethyl-1H-pyrazol-4-carboxamid, (2.56) 2-Chlor-N-(4'-ethinylbiphenyl-2-yl)nicotinamid, (2.57) 2-Chlor-N-[4'-(3,3-dimethylbut-1-in-1-yl)biphenyl-2-yl]nicotinamid, (2.58) 4-(Difluormethyl)-2-methyl-N-[4'-(trifluormethyl)biphenyl-2-yl]-1,3-thiazol-5-carboxamid, (2.59) 5-Fluor-N-[4'-(3-hydroxy-3-methylbut-1-in-1-yl)biphenyl-2-yl] -1,3-dimethyl-1H-pyrazol-4-carboxamid, (2.60) 2-Chlor-N-[4'-(3-hydroxy-3-methylbut-1-in-1-yl)biphenyl-2-yl]nicotinamid, (2.61) 3-(Difluormethyl)-N-[4'-(3-methoxy-3-methylbut-1-in-1-yl)biphenyl-2-yl] -1-methyl-1H-pyrazol-4-carboxamid, (2.62) 5-Fluor-N-[4'-(3-methoxy-3-methylbut-1-in-1-yl)biphenyl-2-yl]-1,3-dimethyl-1H-pyrazol-4-carboxamid, (2.63) 2-Chlor-N-[4'-(3-methoxy-3-methylbut-1-in-1-yl)biphenyl-2-yl]nicotinamid, (2.64) 1,3-Dimethyl-N-(1,1,3-trimethyl-2,3-dihydro-1H-inden-4-yl)-1H-pyrazol-4-carboxamid, (2.65) 1,3-Dimethyl-N-[(3R)-1,1,3-trimethyl-2,3-dihydro-1H-inden-4-yl]-1H-pyrazol-4-carboxamid, (2.66) 1,3-Dimethyl-N-[(3S)-1,1,3-trimethyl-2,3-dihydro-1H-inden-4-yl]-1H-pyrazol-4-carboxamid, (2.67) 3-(Difluormethyl)-N-methoxy-1-methyl-N-[1-(2,4,6-trichlorphenyl)propan-2-yl]-1H-pyrazol-4-carboxamid, (2.68) 3-(Difluormethyl)-N-(7-fluor-1,1,3-trimethyl-2,3-dihydro-1H-inden-4-yl)-1-methyl-1H-pyrazol-4-carboxamid, (2.69) 3-(Difluormethyl)-N-[(3R)-7-fluor-1,1,3-trimethyl-2,3-dihydro-1H-inden-4-yl]-1-methyl-1H-pyrazol-4-carboxamid, (2.70) 3-(Difluormethyl)-N-[(3S)-7-fluor-1,1,3-trimethyl-2,3-dihydro-1H-inden-4-yl]-1-methyl-1H-pyrazol-4-carboxamid.
3) Inhibitoren der Atmungskette am Komplex III, zum Beispiel (3.01) Ametoctradin, (3.02) Amisulbrom, (3.03) Azoxystrobin, (3.04) Cyazofamid, (3.05) Coumethoxystrobin, (3.06) Coumoxystrobin, (3.07) Dimoxystrobin, (3.08) Enoxastrobin, (3.09) Famoxadon, (3.10) Fenamidon, (3.11) Flufenoxystrobin, (3.12) Fluoxastrobin, (3.13) Kresoxim-methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methylacetamid, (3.24) (2E)-2-(Methoxyimino)-N-methyl-2-(2-{[({(1E)-1-[3-(trifluormethyl)phenyl]ethyliden}amino)oxy]methyl}phenyl)acetamid, (3.25) (2E)-2-(Methoxyimino)-N-methyl-2-{2-[(E)-({1-[3-(trifluormethyl)phenyl]ethoxy}imino)methyl]phenyl}acetamid, (3.26) (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, (3.27) Fenaminostrobin, (3.28) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.29) (2E)-2-{2-[({Cyclopropyl[(4-methoxyphenyl)imino]methyl}sulfanyl)methyl]phenyl}-3-methoxyacrylsäuremethylester, (3.30) N-(3-Ethyl-3,5,5-trimethylcyclohexyl)-3-formamido-2-hydroxybenzamid, (3.31) 2-{2-[(2,5-Dimethylphenoxy)methyl]phenyl}-2-methoxy-N-methylacetamid, (3.32) 2-{2-[(2,5-Dimethylphenoxy)methyl]phenyl}-2-methoxy-N-methylacetamid, (3.33) (2E,3Z)-5-{[1-(4-Chlorphenyl)-1H-pyrazol-3-yl]oxy}-2-(methoxyimino)-N,3-dimethylpent-3-enamid.
4) Inhibitoren der Mitose und Zellteilung, zum Beispiel (4.01) Benomyl, (4.02) Carbendazim, (4.03) Chlorfenazol, (4.04) Diethofencarb, (4.05) Ethaboxam, (4.06) Fluopicolid, (4.07) Fuberidazol, (4.08) Pencycuron, (4.09) Thiabendazol, (4.10) Thiophanat-methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin, (4.14) 3-Chlor-5-(6-chlorpyridin-3-yl)-6-methyl-4-(2,4,6-trifluorphenyl)pyridazin.
5) Verbindungen, die dazu fähig sind, an mehreren Stellen anzugreifen ("Multisite Action"), zum Beispiel (5.01) Bordeaux-Mischung, (5.02) Captafol, (5.03) Captan, (5.04) Chlorothalonil, (5.05) Kupferhydroxid, (5.06) Kupfernaphthenat, (5.07) Kupferoxid, (5.08) Kupferoxychlorid, (5.09) Kupfer(2+)-sulfat, (5.10) Dichlofluanid, (5.11) Dithianon, (5.12) Dodin, (5.13) Dodin freie Base, (5.14) Ferbam, (5.15) Fluorofolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mancopper, (5.23) Mancozeb, (5.24) Maneb, (5.25) Metiram, (5.26) Metiram-Zink, (5.27) Oxin-Kupfer, (5.28) Propamidin, (5.29) Propineb, (5.30) Schwefel und Schwefelzubereitungen einschließlich Calciumpolysulfid, (5.31) Thiram, (5.32) Tolylfluanid, (5.33) Zineb, (5.34) Ziram, (5.35) Anilazin.
6) Verbindungen, die dazu fähig sind, eine Abwehrreaktion des Wirtes zu induzieren, zum Beispiel (6.01) Acibenzolar-S-methyl, (6.02) Isotianil, (6.03) Probenazol, (6.04) Tiadinil, (6.05) Laminarin.
7) Inhibitoren der Aminosäure- und/oder Proteinbiosynthese, zum Beispiel (7.01) Andoprim, (7.02) Blasticidin-S, (7.03) Cyprodinil, (7.04) Kasugamycin, (7.05) Kasugamycinhydrochlorid-hydrat, (7.06) Mepanipyrim, (7.07) Pyrimethanil, (7.08) 3-(5-Fluor-3,3,4,4-tetramethyl-3,4-dihydroisochinolin-1-yl)chinolin, (7.09) Oxytetracyclin, (7.10) Streptomycin.
8) Inhibitoren der ATP-Produktion, zum Beispiel (8.01) Fentinacetat, (8.02) Fentinchlorid, (8.03) Fentinhydroxid, (8.04) Silthiofam.
9) Inhibitoren der Zellwandsynthese, zum Beispiel (9.01) Benthiavalicarb, (9.02) Dimethomorph, (9.03) Flumorph, (9.04) Iprovalicarb, (9.05) Mandipropamid, (9.06) Polyoxine, (9.07) Polyoxorim, (9.08) Validamycin A, (9.09) Valifenalat, (9.10) Polyoxin B, (9.11) (2E)-3-(4-tert.-Butylphenyl)-3-(2-chlorpyridin-4-yl)-1-(morpholin-4-yl)prop-2-en-1-on, (9.12) (2Z)-3-(4-tert.-Butylphenyl)-3-(2-chlorpyridin-4-yl)-1-(morpholin-4-yl)prop-2-en-1-on.
10) Inhibitoren der Lipid- und Membransynthese, zum Beispiel (10.01) Biphenyl, (10.02) Chloroneb, (10.03) Dicloran, (10.04) Edifenphos, (10.05) Etridiazol, (10.06) Iodocarb, (10.07) Iprobenfos, (10.08) Isoprothiolan, (10.09) Propamocarb, (10.10) Propamocarbhydrochlorid, (10.11) Prothiocarb, (10.12) Pyrazophos, (10.13) Quintozen, (10.14) Tecnazen, (10.15) Tolclofos-methyl.
11) Inhibitoren der Melaninbiosynthese, zum Beispiel (11.01) Carpropamid, (11.02) Diclocymet, (11.03) Fenoxanil, (11.04) Phthalid, (11.05) Pyroquilon, (11.06) Tricyclazol, (11.07) 2,2,2-Trifluorethyl {3-methyl-1-[(4-methylbenzoyl)amino]butan-2-yl}carbamat.
12) Inhibitoren der Nukleinsäuresynthese, zum Beispiel (12.01) Benalaxyl, (12.02) Benalaxyl-M (Kiralaxyl), (12.03) Bupirimat, (12.04) Clozylacon, (12.05) Dimethirimol, (12.06) Ethirimol, (12.07) Furalaxyl, (12.08) Hymexazol, (12.09) Metalaxyl, (12.10) Metalaxyl-M (Mefenoxam), (12.11) Ofurace, (12.12) Oxadixyl, (12.13) Oxolinsäure, (12.14) Octhilinon.
13) Inhibitoren der Signalvermittlung, zum Beispiel (13.01) Chlozolinat, (13.02) Fenpiclonil, (13.03) Fludioxonil, (13.04) Iprodion, (13.05) Procymidon, (13.06) Quinoxyfen, (13.07) Vinclozolin, (13.08) Proquinazid.
14) Verbindungen, die als Entkoppler wirken können, zum Beispiel (14.01) Binapacryl, (14.02) Dinocap, (14.03) Ferimzon, (14.04) Fluazinam, (14.05) Meptyldinocap.
15) Weitere Verbindungen, zum Beispiel (15.001) Benthiazol, (15.002) Bethoxazin, (15.003) Capsimycin, (15.004) Carvon, (15.005) Chinomethionat, (15.006) Pyriofenon (Chlazafenon), (15.007) Cufraneb, (15.008) Cyflufenamid, (15.009) Cymoxanil, (15.010) Cyprosulfamid, (15.011) Dazomet, (15.012) Debacarb, (15.013) Dichlorophen, (15.014) Diclomezin, (15.015) Difenzoquat, (15.016) Difenzoquatmetilsulfat, (15.017) Diphenylamin, (15.018) Ecomat, (15.019) Fenpyrazamin, (15.020) Flumetover, (15.021) Fluoroimid, (15.022) Flusulfamid, (15.023) Flutianil, (15.024) Fosetyl-Aluminium, (15.025) Fosetyl-Calcium, (15.026) Fosetyl-Natrium, (15.027) Hexachlorbenzol, (15.028) Irumamycin, (15.029) Methasulfocarb, (15.030) Methylisothiocyanat, (15.031) Metrafenon, (15.032) Mildiomycin, (15.033) Natamycin, (15.034) Nickeldimethyldithiocarbamat, (15.035) Nitrothal-isopropyl, (15.036) Oxamocarb, (15.037) Oxyfenthiin, (15.038) Pentachlorphenol und Salze, (15.039) Phenothrin, (15.040) phosphorige Säure und deren Salze, (15.041) Propamocarb-fosetylat, (15.042) Propanosin-Natrium, (15.043) Pyrimorph, (15.044) Pyrrolnitrin, (15.045) Tebufloquin, (15.046) Tecloftalam, (15.047) Tolnifanid, (15.048) Triazoxid, (15.049) Trichlamid, (15.050) Zarilamid, (15.051) 2-Methylpropansäure-(3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-ylester, (15.052) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.053) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-l-yl]ethanon, (15.054) Oxathiapiprolin, (15.055) 1H-Imidazol-1-carbonsäure-1-(4-methoxyphenoxy)-3,3-dimethylbutan-2-ylester, (15.056) 2,3,5,6-Tetrachlor-4-(methylsulfonyl)pyridin, (15.057) 2,3-Dibutyl-6-chlorthieno[2,3-d]pyrimidin-4(3H)-on, (15.058) 2,6-Dimethyl-1H,5H-[1,4]dithiino[2,3-c:5,6-c']dipyrrol-1,3,5,7(2H,6H)-tetron, (15.059) 2-[5-Methyl-3-(trifluormethyl)-1H-pyrazol-1-yl]-1-(4-14-[(5R)-5-phenyl-4,5-dihydro-1,2-oxazol-3-yl]-1,3-thiazol-2-yl}piperidin-1-yl)ethanon, (15.060) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, (15.061) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.062) 2-Butoxy-6-iod-3-propyl-4H-chromen-4-on, (15.063) 2-Chlor-5-[2-chlor-1-(2,6-difluor-4-methoxyphenyl)-4-methyl-1H-imidazol-5-yl]pyridin, (15.064) 2-Phenylphenol und Salze, (15.065) 3-(4,4,5-Trifluor-3,3-dimethyl-3,4-dihydroisochinolin-1-yl)chinolin, (15.066) 3,4,5-Trichlorpyridin-2,6-dicarbonsäurenitril, (15.067) 3-Chlor-5-(4-chlorphenyl)-4-(2,6-difluorphenyl)-6-methylpyridazin, (15.068) 4-(4-Chlorphenyl)-5-(2,6-difluorphenyl)-3,6-dimethylpyridazin, (15.069) 5-Amino-1,3,4-thiadiazol-2-thiol, (15.070) 5-Chlor-N'-phenyl-N'-(prop-2-in-1-yl)thiophen-2-sulfonohydrazid, (15.071) 5-Fluor-2- [(4-fluorbenzyl)oxy]pyrimidin-4-amin, (15.072) 5-Fluor-2-[(4-methylbenzyl)oxy]pyrimidin-4-amin, (15.073) 5-Methyl-6-octyl[1,2,4]triazolo[1,5-a]pyrimidin-7-amin, (15.074) (2Z)-3-Amino-2-cyano-3-phenylacrylsäureethylester, (15.075) N'-(4-{[3-(4-Chlorbenzyl)-1,2,4-thiadiazol-5-yl]oxyl-2,5-dimethylphenyl)-N-ethyl-N-methylimidoformamid, (15.076) N-(4-Chlorbenzyl)-3-[3-methoxy-4-(prop-2-in-1-yloxy)phenyl]propanamid, (15.077) N-[(4-Chlorphenyl)(cyano)methyl]-3-[3-methoxy-4-(prop-2-in-l-yloxy)phenyl]propanamid, (15.078) N-[(5-Brom-3-chlorpyridin-2-yl)methyl]-2,4-dichlornicotinamid, (15.079) N-[1-(5-Brom-3-chlorpyridin-2-yl)ethyl]-2,4-dichlornicotinamid, (15.080) N-[1-(5-Brom-3-chlorpyridin-2-yl)ethyl]-2-fluor-4-iodnicotinamid, (15.081) N-{(E)-[(Cyclopropylmethoxy)imino][6-(difluormethoxy)-2,3-difluorphenyl]methyl}-2-phenylacetamid, (15.082) N-{(Z)-[(Cyclopropylmethoxy)imino][6-(difluormethoxy)-2,3-difluorphenyl]methyl}-2-phenylacetamid, (15.083) N'-{4-[(3-tert.-Butyl-4-cyano-1,2-thiazol-5-yl)oxy]-2-chlor-5-methylphenyl}-N-ethyl-N-methylimidoformamid, (15.084) N-Methyl-2-(1-{[5-methyl-3-(trifluormethyl)-1H-pyrazol-1-yl]acetyl}piperidin-4-yl)-N-(1,2,3,4-tetrahydronaphthalin-1-yl)-1,3-thiazol-4-carboxamid, (15.085) N-Methyl-2-(1-{[5-methyl-3-(trifluormethyl)-1H-pyrazol-1-yl]acetyl}piperidin-4-yl)-N-[(1R)-1,2,3,4-tetrahydronaphthalin-1-yl]-1,3-thiazol-4-carboxamid, (15.086) N-Methyl-2-(1-{[5-methyl-3-(trifluormethyl)-1H-pyrazol-1-yl]acetyl}piperidin-4-yl)-N-[(1S)-1,2,3,4-tetrahydronaphthalin-1-yl]-1,3-thiazol-4-carboxamid, (15.087) {6-[({[(1-Methyl-1H-tetrazol-5-yl)(phenyl)methylen]amino}oxy)methyl]pyridin-2-yl}carbamidsäurepentylester, (15.088) Phenazin-1-carbonsäure, (15.089) Chinolin-8-ol, (15.090) Chinolin-8-olsulfat (2:1), (15.091) {6-[({[(1-Methyl-1H-tetrazol-5-yl)(phenyl)methylen]amino}oxy)methyl]pyridin-2-yl}carbamidsäure-tert.-butylester, (15.092) (5-Brom-2-methoxy-4-methylpyridin-3-yl)(2,3,4-trimethoxy-6-methylphenyl)methanon, (15.093) N-[2-(4-{[3-(4-Chlorphenyl)prop-2-in-1-yl]oxy}-3-methoxyphenyl)ethyl]-N2-(methylsulfonyl)valinamid, (15.094) 4-Oxo-4-[(2-phenylethyl)amino]butansäure, (15.095) {6-[({[(Z)-(1-Methyl-1H-tetrazol-5-yl)(phenyl)methylen]amino}oxy)methyl]pyridin-2-yl}carbamidsäurebut-3-in-1-ylester, (15.096) 4-Amino-5-fluorpyrimidin-2-ol (tautomere Form: 4-Amino-5-fluorpyrimidin-2(1H)-on), (15.097) 3,4,5-Trihydroxybenzoesäurepropylester, (15.098) [3-(4-Chlor-2-fluorphenyl)-5-(2,4-difluorphenyl)-1,2-oxazol-4-yl](pyridin-3-yl)methanol, (15.099) (S)-[3-(4-Chlor-2-fluorphenyl)-5-(2,4-difluorphenyl)-1,2-oxazol-4-yl](pyridin-3-yl)methanol, (15.100) (R)-[3-(4-Chlor-2-fluorphenyl)-5-(2,4-difluorphenyl)-1,2-oxazol-4-yl](pyridin-3-yl)methanol, (15.101) 2-Fluor-6-(trifluormethyl)-N-(1,1,3-trimethyl-2,3-dihydro-1H-inden-4-yl)benzamid, (15.102) 2-(6-Benzylpyridin-2-yl)chinazolin, (15.103) 2-[6-(3-Fluor-4-methoxyphenyl)-5-methylpyridin-2-yl]chinazolin, (15.104) 3-(4,4-Difluor-3,3-dimethyl-3,4-dihydroisochinolin-1-yl)chinolin, (15.105) Abscisinsäure, (15.106) N'-[5-Brom-6-(2,3-dihydro-1H-inden-2-yloxy)-2-methylpyridin-3-yl]-N-ethyl-N-methylimidoformamid, (15.107) N'-{5-Brom-6-[1-(3,5-difluorphenyl)ethoxy] -2-methylpyridin-3-yl} -N-ethyl-N-methylimidoformamid, (15.108) N'-{5-Brom-6-[(1R)-1-(3,5-difluorphenyl)ethoxy]-2-methylpyridin-3-yl}-N-ethyl-N-methylimidoformamid, (15.109) N'-{5-Brom-6-[(1S)-1-(3,5-difluorphenyl)ethoxy]-2-methylpyridin-3-yl}-N-ethyl-N-methylimidoformamid, (15.110) N'-{5-Brom-6-[(cis-4-isopropylcyclohexyl)oxy]-2-methylpyridin-3-yl}-N-ethyl-N-methylimidoformamid, (15.111) N'- {5-Brom-6- [(trans-4-isopropylcyclohexyl)oxy] -2-methylpyridin-3-yl}-N-ethyl-N-methylimidoformamid, (15.112) N-Cyclopropyl-3-(difluormethyl)-5-fluor-N-(2-isopropylbenzyl)-1-methyl-1H-pyrazo1-4-carboxamid, (15.113) N-Cyclopropyl-N-(2-cyclopropylbenzyl)-3-(difluormethyl)-5-fluor-1-methyl-1H-pyrazol-4-carboxamid, (15.114) N-(2-tert.-Butylbenzyl)-N-cyclopropyl-3-(difluormethyl)-5-fluor-1-methyl-1H-pyrazol-4-carboxamid, (15.115) N-(5-Chlor-2-ethylbenzyl)-N-cyclopropyl-3-(difluormethyl)-5-fluor-1-methyl-1H-pyrazol-4-carboxamid, (15.116) N-(5-Chlor-2-isopropylbenzyl)-N-cyclopropyl-3-(difluormethyl)-5-fluor-1-methyl-1H-pyrazol-4-carboxamid, (15.117) N-Cyclopropyl-3-(difluormethyl)-N-(2-ethyl-5-fluorbenzyl)-5-fluor-1-methyl-1H-pyrazol-4-carboxamid, (15.118) N-Cyclopropyl-3-(difluormethyl)-5-fluor-N-(5-fluor-2-isopropylbenzyl)-1-methyl-1H-pyrazol-4-carboxamid, (15.119) N-Cyclopropyl-N-(2-cyclopropyl-5-fluorbenzyl)-3-(difluormethyl)-5-fluor-1-methyl-1H-pyrazol-4-carboxamid, (15.120) N-(2-Cyclopentyl-5-fluorbenzyl)-N-cyclopropyl-3-(difluormethyl)-5-fluor-1-methyl-1H-pyrazol-4-carboxamid, (15.121) N-Cyclopropyl-3-(difluormethyl)-5-fluor-N-(2-fluor-6-isopropylbenzyl)-1-methyl-1H-pyrazol-4-carboxamid, (15.122) N-Cyclopropyl-3-(difluormethyl)-N-(2-ethyl-5-methylbenzyl)-5-fluor-1-methyl-1H-pyrazol-4-carboxamid, (15.123) N-Cyclopropyl-3-(difluormethyl)-5-fluor-N-(2-isopropyl-5-methylbenzyl)-1-methyl-1H-pyrazol-4-carboxamid, (15.124) N-Cyclopropyl-N-(2-cyclopropyl-5-methylbenzyl)-3-(difluormethyl)-5-fluor-1-methyl-1H-pyrazol-4-carboxamid, (15.125) N-(2-tert.-Butyl-5-methylbenzyl)-N-cyclopropyl-3-(difluormethyl)-5-fluor-1-methyl-1H-pyrazol-4-carboxamid, (15.126) N-[5-Chlor-2-(trifluormethyl)benzyl]-N-cyclopropyl-3-(difluormethyl)-5-fluor-1-methyl-1H-pyrazol-4-carboxamid, (15.127) N-Cyclopropyl-3-(difluormethyl)-5-fluor-1-methyl-N-[5-methyl-2-(trifluormethyl)benzyl]-1H-pyrazol-4-carboxamid, (15.128) N-[2-Chlor-6-(trifluormethyl)benzyl]-N-cyclopropyl-3-(difluormethyl)-5-fluor-1-methyl-1H-pyrazol-4-carboxamid, (15.129) N-[3-Chlor-2-fluor-6-(trifluormethyl)benzyl]-N-cyclopropyl-3-(difluormethyl)-5-fluor-1-methyl-1H-pyrazol-4-carboxamid, (15.130) N-Cyclopropyl-3-(difluormethyl)-N-(2-ethyl-4,5-dimethylbenzyl)-5-fluor-1-methyl-1H-pyrazol-4-carboxamid, (15.131) N-Cyclopropyl-3-(difluormethyl)-5-fluor-N-(2-isopropylbenzyl)-1-methyl-1H-pyrazol-4-carbothioamid, (15.132) N'-(2,5-Dimethyl-4-phenoxyphenyl)-N-ethyl-N-methylimidoformamid, (15.133) N'-{4-[(4,5-Dichlor-1,3-thiazol-2-yl)oxy]-2,5-dimethylphenyl}-N-ethyl-N-methylimidoformamid, (15.134) N-(4-Chlor-2,6-difluorphenyl)-4-(2-chlor-4-fluorphenyl)-1,3-dimethyl-1H-pyrazol-5-amin, (15.135) 9-Fluor-2,2-dimethyl-5-(chinolin-3-yl)-2,3-dihydro-1,4-benzoxazepin, (15.136) 2-{2-Fluor-6-[(8-fluor-2-methylchinolin-3-yl)oxy]phenyl }propan-2-ol, (15.137) 2-{2-[(7,8-Difluor-2-methylchinolin-3-yl)oxy]-6-fluorphenyl}propan-2-ol, (15.138) 4-(2-Chlor-4-fluorphenyl)-N-(2-fluorphenyl)-1,3-dimethyl-1H-pyrazol-5-amin, (15.139) 4-(2-Chlor-4-fluorphenyl)-N-(2,6-difluorphenyl)-1,3-dimethyl-1H-pyrazol-5-amin, (15.140) 4-(2-Chlor-4-fluorphenyl)-N-(2-chlor-6-fluorphenyl)-1,3-dimethyl-1H-pyrazol-5-amin, (15.141) 4-(2-Brom-4-fluorphenyl)-N-(2-chlor-6-fluorphenyl)-1,3-dimethyl-1H-pyrazol-5-amin, (15.142) N-(2-Brom-6-fluorphenyl)-4-(2-chlor-4-fluorphenyl)-1,3-dimethyl-1H-pyrazol-5-amin, (15.143) 4-(2-Brom-4-fluorphenyl)-N-(2-bromphenyl)-1,3-dimethyl-1H-pyrazol-5-amin, (15.144) 4-(2-Brom-4-fluorphenyl)-N-(2-brom-6-fluorphenyl)-1,3-dimethyl-1H-pyrazol-5-amin, (15.145) 4-(2-Brom-4-fluorphenyl)-N-(2-chlorphenyl)-1,3-dimethyl-1H-pyrazol-5-amin, (15.146) N-(2-Bromphenyl)-4-(2-chlor-4-fluorphenyl)-1,3-dimethyl-1H-pyrazol-5-amin, (15.147) 4-(2-Chlor-4-fluorphenyl)-N-(2-chlorphenyl)-1,3-dimethyl-1H-pyrazol-5-amin, (15.148) 4-(2-Brom-4-fluorphenyl)-N-(2,6-difluorphenyl)-1,3-dimethyl-1H-pyrazol-5-amin, (15.149) 4-(2-Brom-4-fluorphenyl)-N-(2-fluorphenyl)-1,3-dimethyl-1H-pyrazol-5-amin, (15.150) N'-(4-{3-[(Difluormethyl)sulfanyl]phenoxy}-2,5-dimethylphenyl)-N-ethyl-N-methylimidoformamid, (15.151) N'-(2,5-Dimethyl-4-{3-[(1,1,2,2-tetrafluorethyl)sulfanyl]phenoxy}phenyl)-N-ethyl-N-methylimidoformamid, (15.152) N'-(2,5-Dimethyl-4-{3-[(2,2,2-trifluorethyl)sulfanyl]phenoxy}phenyl)-N-ethyl-N-methylimidoformamid, (15.153) N'-(2,5-Dimethyl-4-{3-[(2,2,3,3-tetrafluorpropyl)sulfanyl]phenoxy}phenyl)-N-ethyl-N-methylimidoformamid, (15.154) N'-(2,5-Dimethyl-4-{3-[(pentafluorethyl)sulfanyl]phenoxy}phenyl)-N-ethyl-N-methylimidoformamid, (15.155) N'-(4-{[3-(Difluormethoxy)phenyl]sulfanyl}-2,5-dimethylphenyl)-N-ethyl-N-methylimidoformamid, (15.156) N'-(2,5-Dimethyl-4- {[3-(1,1,2,2-tetrafluorethoxy)phenyl]sulfanyl}phenyl)-N-ethyl-N-methylimidoformamid, (15.157) N'-(2,5-Dimethyl-4-{[3-(2,2,2-trifluorethoxy)phenyl]sulfanyl}phenyl)-N-ethyl-N-methylimidoformamid, (15.158) N'-(2,5-Dimethyl-4-{[3-(2,2,3,3-tetrafluorpropoxy)phenyl]sulfanyl}phenyl)-N-ethyl-N-methylimidoformamid, (15.159) N'-(2,5-Dimethyl-4-{[3-(pentafluorethoxy)phenyl]sulfanyl}phenyl)-N-ethyl-N-methylimidoformamid, (15.160) 2-[3,5-Bis(difluormethyl)-1H-pyrazol-1-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon, (15.161) 2-[3,5-Bis(difluormethyl)-1H-pyrazol-1-yl]-1-[4-(4-{5-[2-fluor-6-(prop-2-in-1-yloxy)phenyl]-4,5-dihydro-1,2-oxazol-3-yl}-1,3-thiazol-2-yl)piperidin-1-yl]ethanon, (15.162) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.163) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.164) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat, (15.165) 2-[3,5-Bis(difluormethyl)-1H-pyrazol-1-yl]-1-[4-(4-{(5S)-5-[2-(prop-2-in-1-yloxy)phenyl]-4,5-dihydro-1,2-oxazol-3-yl}-1,3-thiazol-2-yl)piperidin-1-yl]ethanon, (15.166) 2-[3,5-Bis(difluormethyl)-1H-pyrazol-1-yl]-1-[4-(4-{(5R)-5-[2-(prop-2-in-1-yloxy)phenyl]-4,5-dihydro-1,2-oxazol-3-yl}-1,3-thiazol-2-yl)piperidin-1-yl]ethanon, (15.167) 2-[3,5-Bis(difluormethyl)-1H-pyrazol-1-yl]-1-[4-(4-{(5S)-5-[2-fluor-6-(prop-2-in-1-yloxy)phenyl]-4,5-dihydro-1,2-oxazol-3-yl}-1,3-thiazol-2-yl)piperidin-1-yl]ethanon, (15.168) 2-[3,5-Bis(difluormethyl)-1H-pyrazol-1-yl]-1-[4-(4-{(5R)-5-[2-fluor-6-(prop-2-in-1-yloxy)phenyl]-4,5-dihydro-1,2-oxazol-3-yl}-1,3-thiazol-2-yl)piperidin-1-yl]ethanon, (15.169) 2-[3,5-Bis(difluormethyl)-1H-pyrazol-1-yl]-1-[4-(4-{(5S)-5-[2-chlor-6-(prop-2-in-1-yloxy)phenyl]-4,5-dihydro-1,2-oxazol-3-yl}-1,3-thiazol-2-yl)piperidin-1-yl]ethanon, (15.170) 2-[3,5-Bis(difluormethyl)-1H-pyrazol-1-yl]-1-[4-(4-{(5R)-5-[2-chlor-6-(prop-2-in-1-yloxy)phenyl]-4,5-dihydro-1,2-oxazol-3-yl}-1,3-thiazol-2-yl)piperidin-1-yl]ethanon, (15.171) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.172) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.173) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat, (15.174) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat.

### Biologische Schädlingsbekämpfungsmittel als Mischungspartner

Die Verbindungen der Formel (Ic) können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
Bacillus amyloliquefaciens, Stamm FZB42 (DSM 231179), oder Bacillus cereus, insbesondere B. cereus Stamm CNCM I-1562 oder Bacillus firmus, Stamm I-1582 (Accession number CNCM I-1582) oder Bacillus pumilus, insbesondere Stamm GB34 (Accession No. ATCC 700814) und Stamm QST2808 (Accession No. NRRL B-30087), oder Bacillus subtilis, insbesondere Stamm GB03 (Accession No. ATCC SD-1397), oder Bacillus subtilis Stamm QST713 (Accession No. NRRL B-21661) oder Bacillus subtilis Stamm OST 30002 (Accession No. NRRL B-50421) Bacillus thuringiensis, insbesondere B. thuringiensis subspecies israelensis (serotype H-14), Stamm AM65-52 (Accession No. ATCC 1276), oder B. thuringiensis subsp. aizawai, insbesondere Stamm ABTS-1857 (SD-1372), oder B. thuringiensis subsp. kurstaki Stamm HD-1, oder B. thuringiensis subsp. tenebrionis Stamm NB 176 (SD-5428), Pasteuria penetrans, Pasteuria spp. (Rotylenchulus reniformis nematode)-PR3 (Accession Number ATCC SD-5834), Streptomyces microflavus Stamm AQ6121 (= QRD 31.013, NRRL B-50550), Streptomyces galbus Stamm AQ 6047 (Acession Number NRRL 30232).

Beispiele für Pilze und Hefen, die als biologische Schädlingsbekämpfungsmittel eingesetzt werden bzw. verwendet werden können, sind:
Beauveria bassiana, insbesondere Stamm ATCC 74040, Coniothyrium minitans, insbesondere Stamm CON/M/91-8 (Accession No. DSM-9660), Lecanicillium spp., insbesondere Stamm HRO LEC 12, Lecanicillium lecanii, (ehemals bekannt als Verticillium lecanii), insbesondere Stamm KV01, Metarhizium anisopliae, insbesondere Stamm F52 (DSM3884/ ATCC 90448), Metschnikowia fructicola, insbesondere Stamm NRRL Y-30752, Paecilomyces fumosoroseus (neu: Isaria fumosorosea), insbesondere Stamm IFPC 200613, oder Stamm Apopka 97 (Accesion No. ATCC 20874), Paecilomyces lilacinus, insbesondere P. lilacinus Stamm 251 (AGAL 89/030550), Talaromyces flavus, insbesondere Stamm V117b, Trichoderma atroviride, insbesondere Stamm SC1 (Accession Number CBS 122089), Trichoderma harzianum, insbesondere T. harzianum rifai T39. (Accession Number CNCM I-952).

Beispiele für Viren, die als biologische Schädlingsbekämpfungsmittel eingesetzt werden bzw. verwendet werden können, sind:
Adoxophyes orana (Apfelschalenwickler) Granulosevirus (GV), Cydia pomonella (Apfelwickler) Granulosevirus (GV), Helicoverpa armigera (Baumwollkapselwurm) Nuklear Polyhedrosis Virus (NPV), Spodoptera exigua (Zuckerrübeneule) mNPV, Spodoptera frugiperda (Heerwurm) mNPV, Spodoptera littoralis (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
Agrobacterium spp., Azorhizobium caulinodans, Azospirillum spp., Azotobacter spp., Bradyrhizobium spp., Burkholderia spp., insbesondere Burkholderia cepacia (ehemals bekannt als Pseudomonas cepacia), Gigaspora spp., oder Gigaspora monosporum, Glomus spp., Laccaria spp., Lactobacillus buchneri, Paraglomus spp., Pisolithus tinctorus, Pseudomonas spp., Rhizobium spp., insbesondere Rhizobium trifolii, Rhizopogon spp., Scleroderma spp., Suillus spp., Streptomyces spp..

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense,Fortune Aza, Fungastop, Heads Up (Chenopodium quinoa-Saponinextrakt), Pyrethrum/Pyrethrins, Quassia amara, Quercus, Quillaja, Regalia,,,Requiem™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischpartner

Die Verbindungen der Formel (Ic) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloroacetyl)-1-oxa-4-azaspiro[4.5]decane (CAS 71526-07-3), 2,2,5-Trimethyl-3-(dichloroacetyl)-1,3-oxazolidine (CAS 52836-31-4).

### Planzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Auf¬streichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflan-zensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

### Transgene Pflanze, Saatgutbehandlung und Integrationsereignisse

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegen¬über hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Be¬schleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kom-binationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

### Transgene Pflanzen, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie Insekten, Spinnentiere, Nematoden, Milben, Schnecken, bewirkt z.B. durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z.B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinonen, Sulfonyl-harnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den erfindungsgemäßen Verbindungen der Formel (Ic) erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Injizieren, Gießen (Drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Eine bevorzugte direkte Behandlung der Pflanzen ist die Blattapplikation, d.h. erfindungsgemäße Verbindungen der Formel (Ic) werden auf das Blattwerk aufgebracht, wobei die Behandlungsfrequenz und die Aufwandmenge auf den Befallsdruck des jeweiligen Pathogens, Schädlings, Unkrauts abgestimmt sein kann.

Bei systemisch wirksamen Verbindungen gelangen die erfindungsgemäßen Verbindungen der Formel (Ic) auch über das Wurzelwerk in die Pflanzen. Die Behandlung der Pflanzen erfolgt dann durch Einwirkung der erfindungsgemäßen Verbindungen der Formel (Ic) auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der erfindungsgemäßen Verbindungen der Formel (Ic) getränkt, oder durch die Bodenapplikation, d.h. die erfindungsgemäßen Verbindungen der Formel (Ic) werden in fester Form, (z.B. in Form eines Granulats) in den Standort der Pflanzen eingebracht. Bei Wasserreiskulturen kann das auch durch Zudosieren einer Verbindung der Formel (Ic) in einer festen Anwendungsform (z.B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch Tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer erfindungsgemäßen Verbindung der Formel (Ic) behandelt wird. Das erfindungsgemäße Verfahren zum Schutz von Saatgut und keimenden Pflanzen vor dem Befall von Schädlingen umfasst ein Verfahren, in dem das Saatgut gleichzeitig in einem Vorgang oder sequentiell mit einem Wirkstoff der Formel I und Mischungspartner behandelt wird. Es umfasst auch ein Verfahren, in dem

das Saatgut zu unterschiedlichen Zeiten mit einem Wirkstoff der Formel I und Mischungspartner behandelt wird.

Die Erfindung bezieht sich ebenfalls auf die Verwendung der erfindungsgemäßen Verbindungen der Formel (Ic) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor Tierischen Schädlingen mit einer erfindungsgemäßen Verbindung der Formel (Ic) behandelt wurde. Die Erfindung bezieht sich auch auf Saatgut, welches zur gleichen Zeit mit einem Wirkstoff der Formel (Ic) und Mischungspartner behandelt wurde. Die Erfindung bezieht sich weiterhin auf Saatgut, welches zu unterschiedlichen Zeiten mit einem Wirkstoff der Formel (Ic) und Mischungspartner behandelt wurde. Bei Saatgut, welches zu unterschiedlichen Zeiten mit einem Wirkstoff der Formel (Ic) und Mischungs-partner behandelt wurde, können die einzelnen Wirkstoffe des erfindungsgemäßen Mittels in unterschiedlichen Schichten auf dem Saatgut enthalten sein. Dabei können die Schichten, die einen Wirkstoff der Formel (Ic) und Mischungspartner enthalten, gegebenenfalls durch eine Zwischenschicht getrennt sein. Die Erfindung bezieht sich auch auf Saatgut, bei dem ein Wirkstoff der Formel (Ic) und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit der erfindungsgemäßen Verbindung der Formel (Ic) einem Filmcoating - Verfahren unterzogen wird, um Staubabrieb am Saatgut zu vermeiden.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit der erfindungsgemäßen Verbindung der Formel (Ic) Keimung und Auflauf des behandelten Saatguts gefördert werden können.

Ebenso ist es als vorteilhaft anzusehen, dass erfindungsgemäße Verbindungen der Formel (Ic) insbesondere auch bei transgenem Saatgut eingesetzt werden können.

Zu nennen ist auch, dass erfindungsgemäße Verbindungen der Formel (Ic) in Kombination mit Mitteln der Signaltechnologie eingesetzt werden können, wodurch beispielhaft eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu. Wie vorstehend bereits erwähnt, kommt auch der Behandlung von transgenem Saatgut mit einer erfindungsgemäßen Verbindung der Formel (Ic) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die erfindungsgemäße Verbindung der Formel (Ic) allein oder in einer geeigneten Formulierung auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden

z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Verbindungen der Formel (Ic) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (Ic) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der erfindungsgemäßen Verbindungen der Formel (Ic) in den Formulierungen und nach dem Saatgut. Die Aufwandmengen bei der Verbindung der Formel (Ic) liegen im Allgemeinen zwischen 0,001 und 50 g pro Kilogramm Saatgut, vorzugsweise zwischen 0,01 und 15 g pro Kilogramm Saatgut.

### Tiergesundheit

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder, in einer weiteren Ausführungsform auch Endoparasiten. Der Begriff Endoparasiten schließt insbesondere Helminthen wie Cestoden, Nematoden oder Trematoden, und Protozoen wie Kokzidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen sowie aquatische Ektoparasiten wie Copepoden.

Auf dem Gebiet der Tiermedizin eignen sich die Verbindungen der Formel (Ic), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Gemäß einer bevorzugten Ausführungsform werden die Verbindungen der Formel (Ic) an Säugetiere verabreicht.

Gemäß einer weiteren bevorzugten Ausführungsform werden die Verbindungen der Formel (Ic) an Vögel, nämlich Stubenvögel und insbesondere Geflügel, verabreicht.

Durch Verwendung der Verbindungen der Formel (Ic) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Verbindungen der Formel (Ic) wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (Ic) den jeweiligen Parasiten abtöten, sein Wachstum verhindern oder seine Vermehrung verhindern kann.

Zu diesen Parasiten gehören:
Aus der Ordnung der Anoplurida z.B. Haematopinus spp., Linognathus spp., Pediculus spp., Phthirus spp., Solenopotes spp.; spezielle Beispiele sind: Linognathus setosus, Linognathus vituli, Linognathus ovillus, Linognathus oviformis, Linognathus pedalis, Linognathus stenopsis, Haematopinus asini macrocephalus, Haematopinus eurysternus, Haematopinus suis, Pediculus humanus capitis, Pediculus humanus corporis, Phylloera vastatrix, Phthirus pubis, Solenopotes capillatus;
Aus der Ordnung der Mallophagida und den Unterordnungen Amblycerina und Ischnocerina z.B. Trimenopon spp., Menopon spp., Trinoton spp., Bovicola spp., Werneckiella spp., Lepikentron spp., Damalina spp., Trichodectes spp., Felicola spp.; spezielle Beispiele sind: Bovicola bovis, Bovicola ovis, Bovicola limbata, Damalina bovis, Trichodectes canis, Felicola subrostratus, Bovicola caprae, Lepikentron ovis, Werneckiella equi;
Aus der Ordnung der Diptera und den Unterordnungen Nematocerina und Brachycerina z.B. Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; spezielle Beispiele sind: Aedes aegypti, Aedes albopictus, Aedes taeniorhynchus, Anopheles gambiae, Anopheles maculipennis, Calliphora erythrocephala, Chrysozona pluvialis, Culex quinquefasciatus, Culex pipiens, Culex tarsalis, Fannia canicularis, Sarcophaga carnaria, Stomoxys calcitrans, Tipula paludosa, Lucilia cuprina, Lucilia sericata, Simulium reptans, Phlebotomus papatasi, Phlebotomus longipalpis, Odagmia ornata, Wilhelmia equina, Boophthora erythrocephala, Tabanus bromius, Tabanus spodopterus, Tabanus atratus, Tabanus sudeticus, Hybomitra ciurea, Chrysops caecutiens, Chrysops relictus, Haematopota pluvialis, Haematopota italica, Musca autumnalis, Musca domestica, Haematobia irritans irritans, Haematobia irritans exigua, Haematobia stimulans, Hydrotaea irritans, Hydrotaea albipuncta, Chrysomya chloropyga, Chrysomya bezziana, Oestrus ovis, Hypoderma bovis, Hypoderma lineatum, Przhevalskiana silenus, Dermatobia hominis, Melophagus ovinus, Lipoptena capreoli, Lipoptena cervi, Hippobosca variegata, Hippobosca equina, Gasterophilus intestinalis, Gasterophilus haemorroidalis, Gasterophilus inermis, Gasterophilus nasalis, Gasterophilus nigricornis, Gasterophilus pecorum, Braula coeca;
Aus der Ordnung der Siphonapterida z.B. Pulex spp., Ctenocephalides spp., Tunga spp., Xenopsylla spp., Ceratophyllus spp.; spezielle Beispiele sind: Ctenocephalides canis, Ctenocephalides felis, Pulex irritans, Tunga penetrans, Xenopsylla cheopis;
Aus der Ordnung der Heteropterida z.B. Cimex spp., Triatoma spp., Rhodnius spp., Panstrongylus spp.
Aus der Ordnung der Blattarida z.B. Blatta orientalis, Periplaneta americana, Blattela germanica, Supella spp. (z.B. Suppella longipalpa);
Aus der Unterklasse der Acari (Acarina) und den Ordnungen der Meta- und Mesostigmata z.B. Argas spp., Ornithodorus spp., Otobius spp., Ixodes spp., Amblyomma spp., Rhipicephalus (Boophilus) spp., Dermacentor spp., Haemophysalis spp., Hyalomma spp., Dermanyssus spp., Rhipicephalus spp. (der ursprünglichen Gattung der Mehrwirtszecken), Ornithonyssus spp., Pneumonyssus spp., Raillietia spp., Pneumonyssus spp., Sternostoma spp., Varroa spp., Acarapis spp.; spezielle Beispiele sind: Argas persicus, Argas reflexus, Ornithodorus moubata, Otobius megnini, Rhipicephalus (Boophilus) microplus, Rhipicephalus (Boophilus) decoloratus, Rhipicephalus (Boophilus) annulatus, Rhipicephalus (Boophilus) calceratus, Hyalomma anatolicum, Hyalomma aegypticum, Hyalomma marginatum, Hyalomma transiens, Rhipicephalus evertsi, Ixodes ricinus, Ixodes hexagonus, Ixodes canisuga, Ixodes pilosus, Ixodes rubicundus, Ixodes scapularis, Ixodes holocyclus, Haemaphysalis concinna, Haemaphysalis punctata, Haemaphysalis cinnabarina, Haemaphysalis otophila, Haemaphysalis leachi, Haemaphysalis longicorni, Dermacentor marginatus, Dermacentor reticulatus, Dermacentor pictus, Dermacentor albipictus, Dermacentor andersoni, Dermacentor variabilis, Hyalomma mauritanicum, Rhipicephalus sanguineus, Rhipicephalus bursa, Rhipicephalus appendiculatus, Rhipicephalus capensis, Rhipicephalus turanicus, Rhipicephalus zambeziensis, Amblyomma americanum, Amblyomma variegatum, Amblyomma maculatum, Amblyomma hebraeum, Amblyomma cajennense, Dermanyssus gallinae, Ornithonyssus bursa, Ornithonyssus sylviarum, Varroa jacobsoni;
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.; spezielle Beispiele sind: Cheyletiella yasguri, Cheyletiella blakei, Demodex canis, Demodex bovis, Demodex ovis, Demodex caprae, Demodex equi, Demodex caballi, Demodex suis, Neotrombicula autumnalis, Neotrombicula desaleri, Neoschöngastia xerothermobia, Trombicula akamushi, Otodectes cynotis, Notoedres cati, Sarcoptis canis, Sarcoptes bovis, Sarcoptes ovis, Sarcoptes rupicaprae (=S. caprae), Sarcoptes equi, Sarcoptes suis, Psoroptes ovis, Psoroptes cuniculi, Psoroptes equi, Chorioptes bovis, Psoergates ovis, Pneumonyssoidic mange, Pneumonyssoides caninum, Acarapis woodi.

Aus Unterklasse der Copepoden mit der Ordnung der Siphonostomatoida insbesondere die Gattungen Lepeophtheirus und Caligus, beispielhaft und besonders bevorzugt seien die Arten Lepeophtheirus salmonis, Caligus elongatus und Caligus clemensi genannt.

Im allgemeinen können die erfindungsgemäßen Wirkstoffe, wenn sie für die Behandlung von Tieren eingesetzt werden, direkt angewendet werden. Vorzugsweise werden sie als pharmazeutische Zusammensetzungen angewendet, die im Stand der Technik bekannte pharmazeutisch unbedenkliche Exzipienten und/oder Hilfsstoffe enthalten können.

Die Anwendung (= Verabreichung) der Wirkstoffe im Bereich Tiergesundheit und in der Tierhaltung erfolgt in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subkutan, intravenös, intraperitoneal u.a.), Implantate, durch nasale Applikation, durch dermale Applikation in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw. Die Wirkstoffe können als Shampoo oder als geeignete, in Aerosolen oder drucklosen Sprays, z.B. Pumpsprays und Zerstäubersprays, anwendbare, Formulierungen formuliert werden.

Bei der Anwendung für Nutztiere, Geflügel, Haustiere etc. kann man die erfindungsgemäßen Wirkstoffe als Formulierungen (beispielsweise Pulver, Spritzpulver [wettable powders, "WP"], Emulsionen, Emulsionskonzentrate [emulsifiable concentrates ,"EC"], fließfähige Mittel, homogene Lösungen und Suspensionskonzentrate [suspension concentrates, "SC"]), die die Wirkstoffe in einer Menge von 1 bis 80 Gew.-% enthalten, direkt oder nach Verdünnung (z.B. 100- bis 10 000facher Verdünnung) anwenden oder sie als chemisches Bad verwenden.

Beim Einsatz im Bereich Tiergesundheit können die erfindungsgemäßen Wirkstoffe zur Erweiterung des Wirkspektrums in Kombination mit geeigneten Synergisten, Repellentien oder anderen Wirkstoffen wie beispielsweise Akariziden, Insektiziden, Anthelmintika, Mitteln gegen Protozoen, verwendet werden. Potentielle Mischpartner für erfindungsgemäße Verbindungen der Formel (Ic) können bei Anwendungen in der Tiergesundheit eine oder mehrere Verbindungen der Gruppen (INS-1) bis (INS-25) sein.

(INS-1) Acetylcholinesterase (AChE) Inhibitoren, wie beispielsweise Carbamate, z. B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; besonders bevorzugt seien hier für Anwendungen gegen Bendiocarb, Carbaryl, Methomyl, Promacyl und Propoxur genannt; oder

Organophosphate, z. B. Acephate, Azamethiphos, Azinphos (-methyl, -ethyl), Cadusafos, Chlorethoxyfos, Chlorfenvinphos, Chlormephos, Chlorpyrifos (-methyl), Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion (-methyl), Phenthoate, Phorate, Phosalone, Phosmet, Phosphamidon, Phoxim, Pirimiphos (-methyl), Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion; besonders bevorzugt seien hier für Anwendungen gegen Ektoparasiten Azamethiphos, Chlorfenvinphos, Chlorpyrifos, Coumaphos, Cythioate, Diazinon (Dimpylate), Dichlorvos (DDVP), Dicrotophos, Dimethoate, Ethion (Diethion), Famphur (Famophos), Fenitrothion, Fenthion (MPP), Heptenophos, Malathion, Naled, Phosmet (PMP, Phtalofos) Phoxim, Propetamphos, Temephos, Tetrachlorvinphos (CVMP) und Triclorfon/Metrifonate genannt.

(INS-2) GABA-gesteuerte Chlorid-Kanal-Antagonisten, wie beispielsweise Organochlorine, z. B. Bromocyclene, Chlordane und Endosulfan (alpha-), Heptachlor, Lindan, und Toxaphene; besonders bevorzugt seien hier für Anwendungen gegen Ektoparasiten Endosulphan (alpha-) und Lindan genannt; oder
Fiprole (Phenylpyrazole), z. B. Acetoprole, Ethiprole, Fipronil, Pyrafluprole und Pyriprole, Rizazole; besonders. bevorzugt seien hier für Anwendungen gegen Ektoparasiten Fipronil und Pyriprole genannt; oder
Arylisoxazoline, Arylpyrroline, Arylpyrrolidine z. B. Fluralaner (bekannt aus WO2009/2024541, Bsp . 11-1; aber auch Verbindungen aus WO2012007426, WO2012042006, WO2012042007, WO2012107533, WO2012120135, WO2012165186, WO2012155676, WO2012017359, WO2012127347, WO2012038851, WO2012120399, WO2012156400, WO2012163959, WO2011161130, WO2011073444, WO2011092287, WO2011075591, WO2011157748, WO 2007/075459, WO 2007/125984, WO 2005/085216, WO 2009/002809), Afoxolaner (z.B. in WO2011149749) und strukturell verwandte Arylpyrroline (bekannt z.B. aus WO2009/072621, WO 2010020522, WO 2009112275, WO 2009097992, WO 2009072621, JP 2008133273, JP 2007091708), oder Arylpyrrolidine (z.B. in WO2012004326, WO2012035011, WO2012045700, WO 2010090344, WO 2010043315, WO 2008128711, JP 2008110971), und Verbindungen aus der Gruppe der sogenannten Metadiamide (bekannt z.B. aus WO2012020483, WO2012020484, WO2012077221, WO2012069366, WO2012175474, WO2011095462, WO2011113756, WO2011093415, WO2005073165) besonders bevorzugt sei hier für Anwendungen gegen Ektoparasiten Afoxolaner und Fluaralaner genannt.

(INS-3) Natrium-Kanal-Modulatoren / Spannungsabhängige Natrium-Kanal-Blocker, wie beispielsweise Pyrethroide, z. B. Acrinathrin, Allethrin (d-cis-trans, d-trans), Bifenthrin, Bioallethrin, Bioallethrin-S-cyclopentenyl, Bioresmethrin, Cycloprothrin, Cyfluthrin (beta-), Cyhalothrin (gamma-, lambda-), Cypermethrin (alpha-, beta-, theta-, zeta-), Cyphenothrin [(1*R*)-*trans*-Isomere], Deltamethrin, Dimefluthrin, Empenthrin [(*EZ*)-(1*R*)-Isomere], Esfenvalerate, Etofenprox, Fenpropathrin, Fenvalerate, Flucythrinate, Flumethrin, Fluvalinate (tau-), Halfenprox, Imiprothrin, Metofluthrin, Permethrin, Phenothrin [(1*R*)-trans-Isomer], Prallethrin, Profluthrin, Pyrethrine (pyrethrum), Resmethrin, RU 15525, Silafluofen, Tefluthrin, Tetramethrin [(1*R*)- Isomere], Tralomethrin, Transfluthrin und ZXI 8901; besonders bevorzugt seien hier für Anwendungen gegen Ektoparasiten die Typ I Pyrethroide Allethrin, Bioallethrin, Permethrin, Phenothrin, Resmethrin, Tetramethrin und die Typ II Pyrethroide (Alphacyanopyrethroide) Alpha-cypermethrin, Cyfluthrin (beta-), Cyhalothrin (lambda-), Cypermethrin (alpha-, zeta-), Deltamethrin, Fenvalerate, Flucythrinate, Flumethrin, Fluvalinate (tau-), und die esterfreien Pyrethroide Etofenprox und Silafluofen genannt; oder Organochlorverbindungen z. B. DDT; oder Methoxychlor. Wirkstoffe aus dieser Klasse eignen sich ganz besonders als Mischpartner da sie eine längeranhaltende Kontakt-repellierende Wirkung haben und somit das Wirkspektrum um diese Komponente erweitern.

(INS-4) Nikotinerge Acetylcholin-Rezeptor-Agonisten, wie beispielsweise Neonikotinoide, z. B. Acetamiprid, Clothianidin, Dinotefuran, Imidacloprid, Imidaclothiz, Nitenpyram, Thiacloprid, Thiamethoxam; besonders bevorzugt seien hier für Anwendungen gegen Ektoparasiten Chlothianidin, Dinotefuran, Imidacloprid, Nitenpyram, und Thiacloprid genannt; oder Nikotin.

(INS-5) Allosterische Acetylcholin-Rezeptor-Modulatoren (Agonisten), wie beispielsweise Spinosyne, z. B. Spinetoram und Spinosad; besonders bevorzugt seien hier für Anwendungen gegen Ektoparasiten Spinosad und Spinetoram genannt.

(INS-6) Chlorid-Kanal-Aktivatoren, wie beispielsweise Avermectine/Milbemycine, z. B. Abamectin, Doramectin, Emamectin-benzoate, Eprinomectin, Ivermectin, Latidectin, Lepimectin, Milbemycin oxime, Milbemectin, Moxidectin, und Selamectin; Indolterpenoide wie z.B. Nodulisporinsäurederivate insbesondere Nodulisporinsäure A; besonders bevorzugt seien hier für Anwendungen gegen Ektoparasiten Doramectin, Eprinomectin, Ivermectin, Milbemycin oxime, Moxidectin, Selamectin und Nodulisporinsäure A genannt.

(INS-7) Juvenilhormon-Analoge, z. B. Hydroprene (S-), Kinoprene, Methoprene (S-); oder Fenoxycarb; Pyriproxyfen; besonders bevorzugt seien hier für Anwendungen gegen Ektoparasiten Methoprene (S-) und Pyriproxyfen genannt.

(INS-8) Milbenwachstumsinhibitoren, z. B. Clofentezine, Diflovidazin, Hexythiazox, Etoxazole; besonders bevorzugt sei hier für Anwendungen gegen Ektoparasiten Etoxazole genannt.

(INS-9) Slo-1- und Latrophilin-Rezeptor Agonisten, wie beispielsweise zyklische Depsipeptide, z. B. Emodepsid sowie seine Ausgangsverbindung PF1022A (bekannt aus EP 382173, compound I); besonders bevorzugt sei hier für Anwendungen gegen Ektoparasiten Emodepsid genannt.

(INS-10) Inhibitoren der oxidativen Phosphorylierung, ATP-Disruptoren, wie beispielsweise Diafenthiuron.

(INS-12) Nikotinerge Acetylcholin-Rezeptor-Antagonisten, wie beispielsweise Bensultap, Cartap (-Hydrochlorid), Thiocylam, und Thiosultap (-sodium).

(INS-13) Inhibitoren der Chitinbiosynthese, Typ 0, wie beispielsweise Benzoylharnstoffe, z. B. Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron; besonders bevorzugt seien hier für Anwendungen gegen Ektoparasiten Diflubenzuron, Fluazuron, Lufenuron und Triflumuron genannt.

(INS-14) Inhibitoren der Chitinbiosynthese, Typ 1, wie beispielsweise Buprofezin.

(INS-15) Häutungsstörende Wirkstoffe, wie beispielsweise Cyromazin und Dicyclanil; besonders bevorzugt seien hier für Anwendungen gegen Ektoparasiten Cyromazin und Dicyclanil genannt.

(INS-16) Ecdysonagonisten/-disruptoren, wie beispielsweise Diacylhydrazine, z. B. Chromafenozide, Halofenozide, Methoxyfenozide und Tebufenozide.

(INS-17) Oktopaminerge Agonisten, wie beispielsweise Amitraz, Cymiazole, Chlordimeform und Demiditraz; besonders bevorzugt seien hier für Anwendungen gegen Ektoparasiten Amitraz, Cymiazole und Demiditraz genannt.

(INS-18) Komplex-III-Elektronentransportinhibitoren, wie beispielsweise Hydramethylnon; Acequinocyl; Fluacrypyrim.

(INS-19) Komplex-I-Elektronentransportinhibitoren, wie beispielsweise aus der Gruppe der METI-Akarizide, z. B. Fenazaquin, Fenpyroximate, Pyrimidifen, Pyridaben, Tebufenpyrad, Tolfenpyrad; besonders bevorzugt seien hier für Anwendungen gegen Ektoparasiten Fenpyroximate, Pyrimidifen und Tolfenpyrad genannt;

(INS-20) Blocker des spannungsabhängigen Natriumkanals, wie beispielsweise Indoxacarb und Metaflumizone; besonders bevorzugt seien hier für Anwendungen gegen Ektoparasiten Indoxacarb und Metaflumizone genannt.

(INS-21) Inhibitoren der Acetyl-CoA-Carboxylase, wie beispielsweise Tetronsäure-Derivate, z. B. Spirodiclofen und Spiromesifen; oder Tetramsäure-Derivate, z. B. Spirotetramat.

(INS-22) Komplex-II-Elektronentransportinhibitoren, wie beispielsweise Cyenopyrafen.

(INS-23) Ryanodinrezeptor-Effektoren, wie beispielsweise Diamide, z. B. Flubendiamide, Chlorantraniliprole (Rynaxypyr), Cyantraniliprole (Cyazypyr) sowie 3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) oder Methyl-2-[3,5-dibrom-2-({[3-brom-1-(3-chlorpyridin-2-yl)-1H-pyrazol-5-yl]carbonyl}amino)benzoyl]-1,2-dimethylhydrazincarboxy-lat (bekannt aus WO2007/043677), sowie 1-(3-Chloropyridin-2-yl)-N-[4-cyano-2-methyl-6-(methylcarbamoyl)phenyl]-3-[[5-(trifluoromethyl)-2H-tetrazol-2-yl]methyl]-1H-pyrazole-5-carboxamide (CAS-No. 1229654-66-3 - mixtures may contain also 1-(3-chloro-2-pyridinyl)-N-[4-cyano-2-methyl-6-[(methylamino)carbonyl]phenyl] -3-[[5-(trifluoromethyl)-1H-tetrazol-1-yl]methyl]-1H-pyrazole-5-carboxamide, CAS-No. 1229656-17-0)

(INS-24) Weitere Wirkstoffe mit unbekanntem Wirkmechanismus, wie beispielsweise Azadirachtin, Amidoflumet, Benzoximate, Bifenazate, Chinomethionat, Cryolite, Cyflumetofen, Dicofol, Fluensulfone (5-chloro-2-[(3,4,4-trifluorobut-3-en-1-yl)sulfonyl]-1,3-thiazole), Flufenerim, Pyridalyl und Pyrifluquinazon; desweiteren Präparate auf Basis von Bacillus firmus (I-1582, BioNeem, Votivo) sowie folgende bekannte wirksame Verbindungen 4-{[(6-Brompyrid-3-yl)methyl](2-fluorethyl)amino}furan-2(5H)-on (bekannt aus WO 2007/115644), 4-{[(6-Fluorpyrid-3-yl)methyl](2,2-difluorethyl)-amino}furan-2(5H)-on (bekannt aus WO 2007/115644), 4-1[(2-Chlor-1,3-thiazol-5-yl)methyl](2-fluorethyl)amino}furan-2(5H)-on (bekannt aus WO 2007/115644), 4-{[(6-Chlorpyrid-3-yl)methyl](2-fluorethyl)amino}furan-2(5H)-on (bekannt aus WO 2007/ 115644), 4-{[(6-Chlorpyrid-3-yl)methyl](2,2-difluorethyl)amino}furan-2(5H)-on (bekannt aus WO 2007/115644), 4-{[(6-Chlor-5-fluorpyrid-3-yl)methyl](methyl)amino}furan-2(5H)-on (bekannt aus WO 2007/115643), 4-{[(5,6-Dichlorpyrid-3-yl)methyl](2-fluorethyl)amino}furan-2(5H)-on (bekannt aus WO 2007/115646), 4-{[(6-Chlor-5-fluorpyrid-3-yl)methyl](cyclopropyl)amino}furan-2(5H)-on (bekannt aus WO 2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), [(6-Chlorpyridin-3-yl)methyl](methyl)oxido-λ⁴-sulfanylidencyanamid (bekannt aus WO 2007/149134), [1-(6-Chlor-pyridin-3-yl)ethyl](methyl)oxido-λ⁴-sulfanylidencyanamid (bekannt aus WO 2007/149134), [(6-Trifluormethylpyridin-3-yl)methyl](methyl)oxido-λ⁴-sulfanylidencyanamid (bekannt aus WO 2007/095229), Sulfoxaflor (ebenfalls bekannt aus WO 2007/149134), 11-(4-Chlor-2,6-dimethylphenyl)-12-hydroxy-1,4-dioxa-9-azadispiro[4.2.4.2]tetradec-11-en-10-on (bekannt aus WO 2006/089633), 3-(4'-Fluor-2,4-dimethylbiphenyl-3-yl)-4-hydroxy-8-oxa-1-azaspiro[4.5]dec-3-en-2-on (bekannt aus WO 2008/067911), 1-[2-fluoro-4-methyl-5-[(2,2,2-trifluoroethyl)sulfinyl]phenyl]-3-(trifluoromethyl)-1H-1,2,4-Triazol-5-amine (bekannt aus WO 2006/ 043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclo-propancarboxylat (bekannt aus WO 2006/129714), 2-Cyano-3-(difluoromethoxy)-N-ethyl-benzenesulfonamide (bekannt aus WO 2005/035486), N-[1-(2,3-Dimethylphenyl)-2-(3,5-dimethylphenyl)ethyl]-4,5-dihydro-2-thiazolamine (bekannt aus WO 2008/104503); Penigequinolone A (bekannt aus EP 2248422 (compound I) und WO 2009/060015 (compound No. 11).

(INS-25) Als geeignete Synergisten bei Verwendung mit Ektoparasitiziden seien hier MGK264 (N-Octylbicycloheptencarboxamid), Piperonylbutoxid (PBO) und Verbutin genannt; besonders bevorzugt seien hier Piperonylbutoxid und MGK264 genannt.

Zusätzlich zu diesen Gruppen können in Mischungen oder in Kombinationsanwendung auch Kurzzeitrepellentien verwendet werden. Beispiele sind DEET (N,N-diethyl-3-methylbenzamide), icaridin (1-Piperidine carboxylic acid), (1S, 20S)-2-methylpiperidinyl-3-cyclohexene-1-carboxamide (SS220), indalone (butyl 3,4-dihydro-2, 2-dimethyl-4-oxo-2H-pyran-6-carboxylate), dihydronepetalactones, nootkatone, IR3535 (3-[N-butyl-N-acetyl]-aminopropionic acid ethyl ester), 2-Ethylhexane-1,3-diol, (1R,2R,5R)-2-(2-Hydroxypropan-2-yl)-5-methyl-cyclohexan-1-ol, Dimethyl benzene-1,2-dicarboxylate, Dodecanoic acid, Undecan-2-one, N,N-Diethyl-2-phenyl-acetamide und aetherische Öle oder andere Pflanzeninhaltsstofef mit bekannter repellierender Wirkung wie z.B. Borneol, Callicarpenal, 1,8-Cineol (eucalyptol), Carvacrol, β-Citronellol, α-Copaene, Coumarin (oder seine synthetischen Derviate bekannt aus US20120329832), Beim Einsatz gegen Ectoparasiten besonders bevorzugt sind icaridin, indalone und IR3535 (3-[N-butyl-N-acetyl]-aminopropionic acid ethyl ester).

Von den vorstehend genannten Gruppen (INS-1) bis (INS-25) sind die folgenden Gruppen als Mischpartner bevorzugt: (INS-2), (INS-3), (INS-4), (INS-5), (INS-6), (INS-17), (INS-25).

Besonders bevorzugte Beispiele für insektizid oder akarizid wirksame Verbindungen, Synergisten oder Repellentien als Mischpartner der efindungsgemäßen Verbindungen der Formel (Ic) sind Afoxolaner, Allethrin, Amitraz, Bioallethrin, Chlothianidin, Cyfluthrin (beta-), Cyhalothrin (lambda-), Cymiazole, Cypermethrin (alpha-, zeta-), Cyphenothrin, Deltamethrin, Demiditraz, Dinotefuran, Doramectin, Eprinomectin, Etofenprox, Fenvalerate, Fipronil, Fluazuron, Flucythrinate, Flumethrin, Fluralaner, Fluvalinate (tau-), Icaridin, Imidacloprid, Ivermectin, MGK264, Milbemycin oxime, Moxidectin, Nitenpyram, Permethrin, Phenothrin, Piperonylbutoxid, Pyriprole, Resmethrin, Selamectin, Silafluofen, Spinetoram, Spinosad, Tetramethrin, Thiacloprid.

### Vektorkontrolle

Die erfindungsgemäßen Verbindungen der allgemeinen Formel (Ic) können auch in der Vektorkontrolle eingesetzt werden. Ein Vektor im Sinne der vorliegenden Erfindung ist ein Arthropode, insbesondere ein Insekt oder Arachnide, der in der Lage ist Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhaut-entzündung (FSME), Krim-Kongo-Fieber, Fleckfieber, Borreliose;
6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (Ic) Resistenz-brechend sind.

Verbindungen der vorliegenden Erfindung sind zur Verwendung in der Prävention von Krankheiten bzw. vor Krankheitserregern, die durch Vektoren übertragen werden, geeignet. Somit ist ein weiter Aspekt der vorliegenden Erfindung die Verwendung von erfindungsgemäßen Verbindungen zur Vektorkontrolle, z.B. in der Landwirtschaft, im Gartenbau, in Forsten, in Gärten und Freizeiteinrichtungen sowie im Vorrats- und Materialschutz.

### Schutz von technischen Materialen

Die erfindungsgemäßen Verbindungen der Formel (Ic) eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer erfindungsgemäßen Ausführungsform enthalten die erfindungsgemäßen Zusammensetzungen oder Mittel noch mindestens ein weiteres Insektizid und/oder mindestens ein Fungizid.

In einer weiteren Ausführungsform ist diese erfindungsgemäße Zusammensetzung eine anwendungsfertige (ready-to-use) Zusammensetzung, d.h., sie kann ohne weitere Änderungen auf das entsprechende Material aufgebracht werden. Als weitere Insektizide oder als Fungizide kommen die oben genannten in Frage.

Überraschenderweise wurde auch gefunden, dass die erfindungsgemäßen Wirkstoffe und Zusammensetzungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die erfindungsgemäßen Wirkstoffe und Zusammensetzungen allein oder in Kombinationen mit anderen Wirkstoffen als Antifouling-Mittel eingesetzt werden.

### Bekämpfung von tierischen Schädlingen auf dem Hygienesektor

Die erfindungsgemäßen Verbindungen der Formel (Ic) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Wirkstoffe oder Zusammensetzungen allein oder in Kombination mit anderen Wirk- und/oder Hilfsstoffen verwendet. Bevorzugt werden sie in Haushaltsinsektizid-Produkten verwendet. Die erfindungsgemäßen Wirkstoffe sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsverfahren

Die erfindungsgemäßen Verbindungen können nach üblichen, dem Fachmann bekannten Methoden hergestellt werden.

Im Reaktionsschema 1 ist das allgemeine Darstellungsverfahren A für die erfindungsgemäßen Verbindungen (I-1) abgebildet.

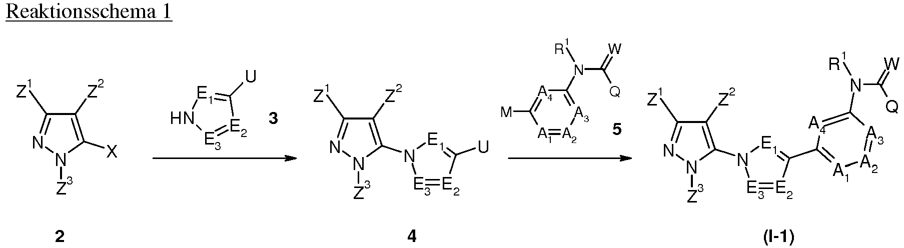

Die Reste A₁-A₄, Q, W, R¹ und Z¹-Z³ haben die oben beschriebenen Bedeutungen. Die fünfgliedrigen Zyklen aus E1-E3, Kohlenstoff und Stickstoff stehen für die unter T definierten 5-gliedrigen Heterozyklen. X steht für ein Halogen. U steht für Brom, Iod oder Triflat, wenn M für eine Boronsäure, Boronsäureester oder Trifluorboronat steht. U steht für eine Boronsäure, Boronsäureester oder Trifluorboronat, wenn M für Brom, Iod oder Triflat steht.

Erfindungsgemäße Verbindungen der allgemeinen Struktur (I-1) können nach literaturbekannten Verfahren mittels Palladium katalysierten Reaktionen aus den Reaktionspartnern 4 und 5 hergestellt werden [WO 2005/040110; WO 2009/089508]. Die Verbindungen der allgemeinen Struktur 5 sind entweder kommerziell erhältlich oder können nach dem Fachmann bekannten Verfahren hergestellt werden. Die Verbindungen der allgemeinen Struktur **4** lassen sich nach literaturbekannten Verfahren entweder durch eine nucleophile Substitution am Aromaten (X = Chlor oder Fluor) [WO 2007/107470; Tetrahedron Letters 2003, 44, 7629-7632] oder durch eine Übergangsmetall-katalysierte Reaktion (X = Brom oder Iod) [WO 2012/003405; WO 2009/158371] aus den entsprechenden Ausgangsmaterialien **2** und **3** herstellen.

Alternativ können die erfindungsgemäßen Verbindungen (I-1) durch das allgemeine Darstellungsverfahren B (Reaktionsschema 2) dargestellt werden.

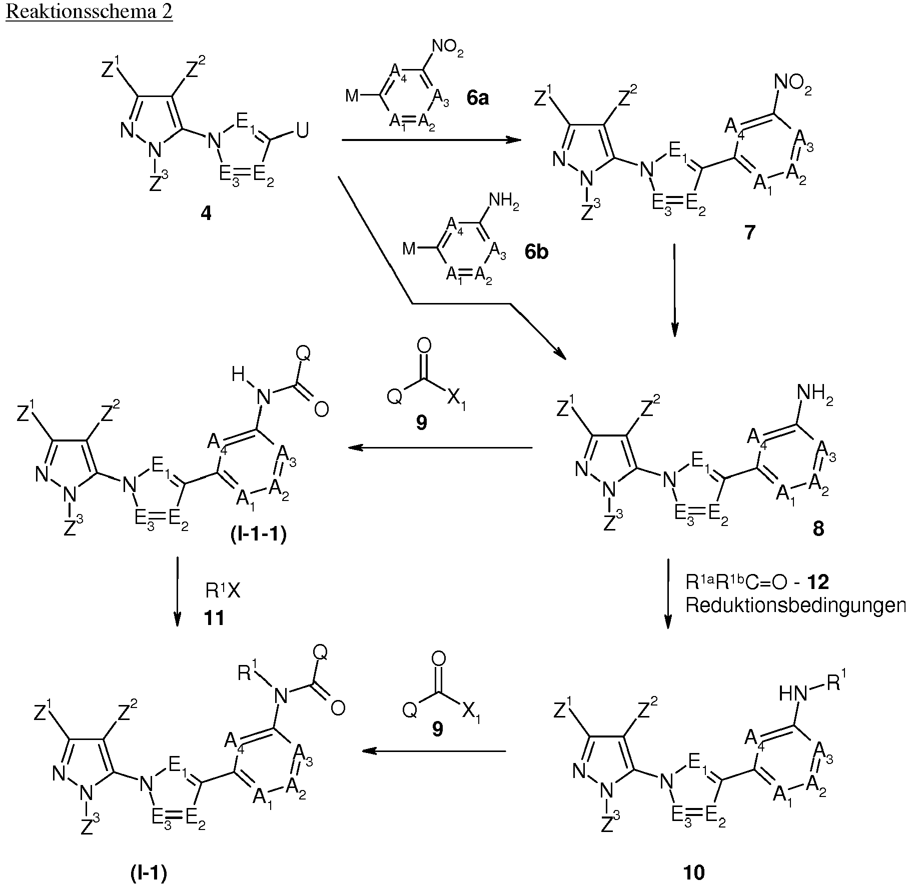

Die Reste A₁-A₄, Q, R¹ und Z¹-Z³ haben die oben beschriebenen Bedeutungen. Die fünfgliedrigen Zyklen aus E1-E3, Kohlenstoff und Stickstoff stehen für die unter T definierten 5-gliedrigen Heterozyklen. X steht für ein Halogen, X₁ für Halogen oder OH. U steht für Brom, Iod oder Triflat, wenn M für eine Boronsäure, Boronsäureester oder Trifluorboronat steht. U steht für eine Boronsäure, Boronsäureester oder Trifluorboronat, wenn M für Brom, Iod oder Triflat steht.

Erfindungsgemäße Verbindungen der allgemeinen Struktur (I-1) und (I-1-1) können in Analogie zu literaturbekannten Peptidkupplungsmethoden aus den Ausgangsmaterialien **8** oder **10** umgesetzt mit **9** hergestellt werden [WO 2010/051926; WO 2010/133312]. Verbindungen der allgemeinen Struktur **8** lassen sich in Analogie zu literaturbekannten Verfahren durch Reduktion aus Verbindungen der allgemeinen Struktur **7** herstellen [WO 2012/080376] oder auf direktem Kupplungswege aus der allgemeinen Struktur **4.** Verbindungen der allgemeinen Struktur **7** können in Analogie zur literaturbekannten Verfahren mittels Palladium-katalysierter Reaktionen hergestellt werden [WO 2005/040110; WO 2009/089508]. Erfindungsgemäße Verbindungen der allgemeinen Struktur (I-1) können nach dem Fachmann hinlänglich bekannten Alkylierungsreaktionen von Amiden der Struktur (I-1-1) mit Alkylierungsreagentien **11,** bevorzugt in Gegenwart von basischen Reaktionshilfsmitteln, erzeugt werden. Alternativ können unter reduktiven Bedingungen Intermediate der allgemeinen Struktur **8** mit geeignet substituierten (R^{1a}, R^{1b}) Ketonen oder Aldehyden **12** zu Verbindungen der allgemeinen Struktur **10** umgesetzt werden [WO 2012/080376], um anschließend in Analogie zu literaturbekannten Peptidkupplungsmethoden [WO 2012/080376] die erfindungsgemäße Verbindungen der allgemeinen Struktur (I-1) zu erhalten.

Erfindungsgemäße Verbindungen der allgemeinen Struktur (I-2) lassen sich nach dem in Reaktionsschema 3 dargestellten Darstellungsverfahren C synthetisieren.

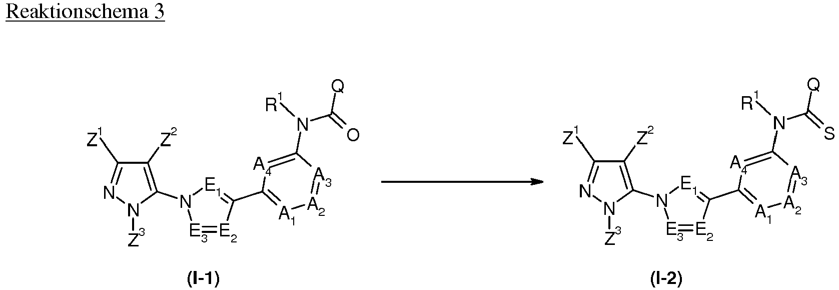

Die Reste A₁-A₄, Q, R¹ und Z¹-Z³ haben die oben beschriebenen Bedeutungen. Die fünfgliedrigen Zyklen aus E1-E3, Kohlenstoff und Stickstoff stehen für die unter T definierten 5-gliedrigen Heterozyklen.

Erfindungsgemäße Verbindungen der allgemeinen Struktur (I-2) lassen sich in Analogie zu literaturbekannten Verfahren aus Verbindungen der allgemeinen Struktur (I-1) darstellen [WO 2012/056372; WO 2003/066050].

Die Verbindungen der allgemeinen Struktur **2** sind entweder kommerziell erhältlich oder können nach dem Fachmann bekannten Verfahren bzw. in Analogie zu diesen Verfahren hergestellt werden [WO 2010/051926; WO 2011/131615; WO 2006/018725; WO 2012/065932; WO 2007/077961; US2012-0115903; WO 2010/017902; WO 2010/127856; Tetrahedron Letters 2011, 44, 8451-8457].

Die Verbindungen der allgemeinen Struktur **3** sind entweder kommerziell erhältlich oder können nach dem Fachmann bekannten Verfahren bzw. in Analogie zu diesen Verfahren hergestellt werden [WO2009/155527; WO2007/138072].

Die Verbindungen der allgemeinen Struktur **5** sind entweder kommerziell erhältlich oder können nach dem Fachmann bekannten Verfahren bzw. in Analogie zu diesen Verfahren hergestellt werden [WO 2005/041904].

Die Verbindungen der allgemeinen Struktur **6** sind entweder kommerziell erhältlich oder können nach dem Fachmann bekannten Verfahren bzw. in Analogie zu diesen Verfahren hergestellt werden [Journal of Organic Chemistry 2013, 78(5), 1923-1933].

Im Reaktionsschema 4 ist das allgemeine Darstellungsverfahren D für die Verbindungen (Ih) abgebildet.

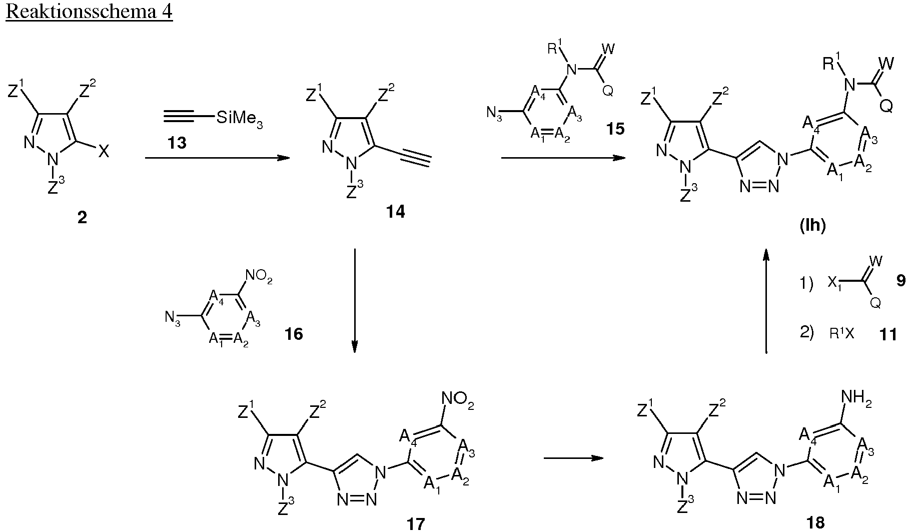

Die Reste Z¹, Z², Z³, A¹, A², A³, A⁴ in den oben genannten Formeln 2, 14, 15, 16, 17 und 18 haben die oben genannten allgemeinen Bedeutungen gemäß der Restedefinitionen für die allgemeine Formel (Ih) oder der allgemeinen Formel (I), dies gilt insbesondere für die bevorzugten oder besonders bevorzugten Restedefinition der Formel (Ih).

Ausgehend von Pyrazolen mit der Abgangsgruppe X = Halogen oder X = Mesylat (2) können entsprechende Pyrazol-acetylene **14** hergestellt werden [US2011/275628 A], ggf. Übergangsmetallkatalysiert.

Verbindungen der allgemeinen Struktur **17** werden durch eine [3+2]-Cycloaddition der Acetylene **14** mit entsprechenden (Het)aryl-aziden **16** synthetisiert [analog Tetrahedron Letters, 2006, 47, (19) 3209 - 3212] und dann weiter zu den Aminen/Anilinen **18** reduziert bzw. zu den Verbindungen (Ih) umgesetzt. Alternativ können die Verbindungen (Ih) direkt aus den Acetylenen **14** mit entsprechenden (Het)aryl-aziden **15** im Zuge einer [3+2]-Cycloaddition synthetisiert werden.

Oxidationsmittel für die Oxidation alkoholischer Gruppen sind bekannt (vgl. z. B. Oxidationsreagenzien in Organic Synthesis by Oxidation with Metal Compounds, Mijs, de Jonge, Plenum Verlag, New York, 1986; Manganese Compounds as Oxidizing Agens in Organic Chemistry, Arndt, Open Court Publishing Company, La Salle, IL, 1981; The Oxidation of Organic Compounds by Permanganate Ion and Hexavalent Chromium, Lee, Open Court Publishing Company, La Salle, IL, 1980). Eine Oxidation kann beispielsweise in Gegenwart von Permanganaten (z.B. Kaliumpermanganat), Metalloxiden (z.B. Mangandioxid, Chromoxide die beispielsweise in Dipyridin-chrom(VI)-oxid als Collins Reagenz (vgl. J. C. Collins et al., Tetrahedron Lett. 30, 3363-3366, 1968) verwendet werden) durchgeführt werden. Ebenfalls in Gegenwart von Pyridiniumchlorochromat (z.B. Corey's Reagenz) (vgl. auch R. O. Hutchins et al., Tetrahedron Lett. 48, 4167-4170, 1977; D. Landini et al. Synthesis 134-136, 1979) oder Ruthenium-tetroxid (vgl. S.-I. Murahashi, N. Komiya Ruthenium-catalyzed Oxidation of Alkenes, Alcohols, Amines, Amides, β-Lactams, Phenols and Hydrocarbons, in: Modern Oxidation Methods, Baeckvall, Jan-Erling (Eds.), Wiley-VCH-Verlag GmbH & Co. KGaA, 2004). Ebenfalls geeignet sind Ultraschall-induzierte Oxidationsreaktionen, sowie die Verwendung von Kaliumpermanganat (vgl. J. Yamawaki et al., Chem. Lett. 3, 379-380, 1983).

Zur Deblockierung/Abspaltung von Schutzgruppen (SG) können alle bekannten geeigneten sauren oder basischen Reaktionshilfsmittel nach den in der Literatur beschriebenen Verfahrensweises verwendet werden. Bei Verwendung von Schutzgruppen für Aminogruppen des Carbamat-Typs werden bevorzugt saure Reaktionshilfsmittel verwendet. Bei Verwendung der t-Butylcarbamat-Schutzgruppe (BOC-Gruppe) werden beispielsweise Mischungen von Mineralsäuren wie Salzsäure, Bromwasserstoffsäure, Salpetersäure, Schwefelsäure, Phosphorsäure oder organischen Säuren wie Benzoesäure, Ameisensäure, Essigsäure, Trifluoressigsäure, Methansulfonsäure, Benzolsulfonsäure oder Toluolsulfonsäure und einem geeigneten Verdünnungsmittel wie Wasser und/oder einem organischen Lösungsmittel wie Tetrahydrofuran, Dioxan, Dichlormethan, Chloroform, Essigester, Ethanol oder Methanol verwendet. Bevorzugt sind Mischungen von Salzsäure oder Essigsäure mit Wasser und/oder einem organischen Lösungsmittel wie Essigester.

Es ist bekannt, dass manche Reaktionen und Herstellungsverfahren besonders gut in Gegenwart von Verdünnungs- bzw. Lösungsmittel und basischer oder saurer Reaktionshilfsmitteln durchführbar sind. Mischungen der Verdünnungs- bzw. Lösungsmittel sind ebenfalls einsetzbar. Die Verdünnungs- bzw. Lösungsmittel werden vorteilhafterweise in einer solchen Menge eingesetzt, dass das Reaktionsgemisch während des ganzen Verfahrens gut rührbar ist.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Halogenkohlenwasserstoffe (z.B. Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-t-butylether, n-Butylether, Anisol, Phenetol, Cyclohexylmethylether, Dimethylether, Diethylether, Diproplether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids), Amine (z.B. Trimethyl-, Triethyl-, Tripropyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril), Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40°C bis 250°C, Cymol, Benzinfraktionen innerhalb eines Siedeinterwalles von 70°C bis 190°C, Cyclohexan, Methylcyclohexan, Petrolether, Ligroin, Octan, Benzol, Toluol, Chlorbenzol, Brombenzol, Nitrobenzol, Xylol, Ester (z.B. Methyl-, Ethyl-, Butyl-, Isobutylacetat, Dimethyl-, Dibutyl-, Ethylencarbonat); Amide (z.B. Hexamethylenphosphorsäuretriamid, Formamid, N-Methyl-formamid, *N*,*N*-Dimethyl-formamid, *N*,*N*-Dipropyl-formamid, *N*,*N*-Dibutyl-formamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, *N*,*N*'-1,4-Diformyl-piperazin) und Ketone (z.B. Aceton, Acetophenon, Methylethylketon, Methylbutylketon).

Als basische Reaktionshilfsmittel zur Durchführung der erfindungsgemäßen Verfahren können alle geeigneten Säurebindemittel eingesetzt werden. Als Beispiele sind zu nennen: Erdalkali- oder Alkalimetallverbindungen (z.B. Hydroxide, Hydride, Oxide und Carbonate des Lithiums, Na-triums, Kaliums, Magnesiums, Calciums und Bariums), Amidinbasen oder Guanidinbasen (z.B. 7-Methyl-1,5,7-triaza-bicyclo(4.4.0)dec-5-en (MTBD); Diazabicyclo(4.3.0)nonen (DBN), Diazabicyclo(2.2.2)octan (DABCO), 1,8-Diazabicyclo(5.4.0)undecen (DBU), Cyclohexyltetrabutyl-guanidin (CyTBG), Cyclohexyltetramethylguanidin (CyTMG), *N,N,N,N-*Tetramethyl-1,8-naphthalindiamin, Pentamethylpiperidin) und Amine, insbesondere tertiäre Amine, (z.B. Triethylamin, Trimethylamin, Tribenzylamin, Triisopropylamin, Tributylamin, Tricyclohexylamin, Triamylamin, Trihexylamin, *N,N-*Dimethylanilin, *N,N*-Dimethyl-toluidin, *N,N*-Dimethyl-p-aminopyridin, N-Methyl-pyrrolidin, N-Methylpiperidin, N-Methyl-imidazol, N-Methyl-pyrazol, N-Methyl-morpholin, N-Methyl-hexamethylendiamin, Pyridin, 4-Pyrrolidinopyridin, 4-Dimethylamino-pyridin, Chinolin, α-Picolin, β-Picolin, Isochinolin, Pyrimidin, Acridin, *N,N*,N',N'-Tetramethylendiamin, *N,N*,N',N'-Tetraethylendiamin, Chinoxalin, N-Propyl-diisopropylamin, N-Ethyl-diisopropylamin, *N,N*'-Dimethyl-cyclohexylamin, 2,6-Lutidin, 2,4-Lutidin oder Triethylendiamin).

Als saure Reaktionshilfsmittel zur Durchführung der erfindungsgemäßen Verfahren können alle Mineralsäuren (z.B. Halogenwasserstoff-säuren wie Fluorwasserstoffsäure, Chlorwasserstoffsäure, Bromwasserstoffsäure oder Iodwasserstoffsäure sowie Schwefelsäure, Phosphorsäure, Phosphorige Säure, Salpetersäure), Lewis Säuren (z.B. Aluminium(III)-chlorid, Bortrifluorid oder sein Etherat, Titan-(V)chlorid, Zinn(V)-chlorid, und organische Säuren (z.B. Ameisensäure, Essigsäure, Propionsäure, Malonsäure, Milchsäure, Oxalsäure, Fumarsäure, Adipinsäure, Stearinsäure, Weinsäure, Ölsäure, Methansulfonsäure, Benzoesäure, Benzolsulfonsäure oder para-Toluolsulfonsäure eingesetzt werden.

Sofern in den Reaktionsschemata Schutzgruppen vorgesehen sind, können alle allgemein bekannten Schutzgruppen verwendet werden. Insbesondere solche, die von Greene T. W., Wuts P. G. W. in Protective Groups in Organic Synthesis; John Wiley & Sons, Inc. 1999, "Protection for the hydroxyl group including 1,2- and 1,3-diols" beschrieben sind.

Weiterhin eignen sich auch Schutzgruppen
vom Typ eines substituierten Methylethers (z.B. Methoxymethylether (MOM), Methylthiomethylether (MTM), (Phenyl-dimethylsilyl)methoxymethylether (SNOM-OR), Benzyloxymethylether (BOM-OR) para-Methoxybenzyloxymethylether (PMBM-OR), para-Nitrobenzyloxymethyl-ether, ortho-Nitrobenzyloxymethylether (NBOM-OR), (4-Methoxyphenoxy)-methylether (p-AOM-OR), Guaiacolmethylether (GUM-OR), t-Butoxymethylether, 4-Pentyloxy-methylether (POM-OR), Silyloxymethylether, 2-Methoxy-ethoxy-methylether (MEM-OR), 2,2,2-Trichlorethoxymethylether, Bis(2-chlorethoxy)-methylether, 2-(Trimethyl-silyl)ethoxymethylether (SEM-OR), Methoxymethylether (MM-OR));
vom Typ eines substituierten Ethylethers (z.B. 1-Ethoxyethylether (EE-OR), 1-(2-Chlorethoxy)ethylether (CEE-OR), 1-[2-(Trimethylsilyl)ethoxy]ethylether (SEE-OR), 1-Methyl-1-methoxyethylether (MIP-OR), 1-Methyl-1-benzyloxyethylether (MBE-OR), 1-Methyl-1-benzyloxy-2-fluor-ethylether (MIP-OR), 1-Methyl-1-phenoxyethylether, 2,2,-Trichlorethylether, 1,1-Dianisyl-2,2,2-trichlorethylether (DATE-OR), 1,1,1,3,3,3-Hexafluor-2-phenylisopropylether (HIP-OR), 2-Trimethylsilylethylether, 2-(Benzylthio)ethylether, 2-(Phenylselenyl)ethylether), eines Ethers (z.B. Tetrahydropyranylether (THP-OR), 3-Brom-tetrahydropyranylether (3-BrTHP-OR), Tetrahydrothiopyranylether, 1-Methoxy-cyclohexylether, 2- und 4-Picolylether, 3-Methyl-2-picolyl-N-oxido-ether, 2-Quinolinylmethylether (Qm-OR), 1-Pyrenylmethylether, Dipenylmethylether (DPM-OR), para, para'-Dinitrobenzhydrylether (DNB-OR), 5-Dibenzosuberylether, Triphenylmethylether (Tr-OR), alpha-Naphthyldiphenylmethylether, para-Methoxy-phenyldiphenylmethylether (MMTrOR), Di(para-methoxy-phenyl)phenylmethylether (DMTr-OR), Tri(para-methoxy-phenyl)phenylmethylether (TMTr-OR), 4-(4'-Brom-phenacyloxy) phenyldiphenylmethylether, 4,4',4"-Tris(4,5-dichlorphthalimido-phenyl)methylether (CPTr-OR), 4,4',4"-Tris(benzoyloxyphenyl)-methylether (TBTr-OR), 4,4'-Dimethoxy-3"-[N-(imidazolylmethyl)]-tritylether (IDTr-OR), 4,4'-Dimethoxy-3"-[N-(imidazolylethyl)carbamoyl]tritylether (IETr-OR), 1,1-Bis(4-methoxy-phenyl)-1'-pyrenyl-methylether (Bmpm-OR), 9-Anthrylether, 9-(9-Phenyl)xanthenylether (Pixyl-OR), 9-(9-Phenyl-10-oxo)anthryl (Tritylon-Ether), 4-Methoxy-tetrahydropyranylether (MTHP-OR), 4-Methoxy-tetrahydrothiopyranylether, 4-Methoxy-tetrahydrothiopyranyl-S,S-dioxid, 1-[(2-Chlor-4-methyl)phenyl]-4-methoxypiperidin-4-yl-ether (CTMP-OR), 1-(2-Fluorphenyl)-4-methoxy-piperidin-4-yl-ether (Fpmp-OR), 1,4-Dioxan-2-yl-ether, Tetrahydrofuranylether, Tetrahydrothiofuranylether, 2,3,3a,4,5,6,7,7a-Octahydro-7,8,8-trimethyl-4,7-methanbenzofuran-2-yl-ether (MBF-OR), t-Butylether, Allylether, Propargylether, para-Chlorphenylether, para-Methoxy-phenylether, para-Nitro-phenylether, para-2,4-Dinitro-phenylether (DNP-OR), 2,3,5,6-Tetrafluor-4-(trifluormethyl)phenylether, Benzylether (Bn-OR));
vom Typ eines sustituierten Benzylethers (z.B. para-Methoxy-benzylether (MPM-OR), 3,4-Dimethoxybenzylether (DMPM-OR), ortho-Nitro-benzylether, para-Nitro-benzylether, para-Halo-benzylether, 2,6-Dichlor-benzylether, para-Aminoacyl-benzylether (PAB-OR), para-Azido-benzylether (Azb-OR), 4-Azido-3-chlor-benzylether, 2-Trifluormethyl-benzylether, para-(Methylsulfinyl)benzylether (Msib-OR));
vom Typ eines Silylethers (z.B. Trimethylsilylether (TMS-OR), Triethylsilylether (TES-OR), Triisopropylsilylether (TIPS-OR), Dimethylisopropylsilylether (IPDMS-OR), Diethylisopropylsilylether (DEIPS-OR), Dimethylhexylsilylether (TDS-OR), t-Butyldimethylsilylether (TBDMS-OR), t-Butyldiphenylsilylether(TBDPS-OR), Tribenzylsilylether, Tri-para-xylylsilylether, Triphenylsilylether (TPS-OR), Diphenylmethylsilylether (DPMS-OR), Di-t-butylmethylsilylether (DTBMS-OR), Tris(trimethylsilyl)silylether (Sisylether), Di-t-butylmethylsilylether (DTBMS-OR), Tris(trimethylsilyl)silylether (Sisylether), (2-Hydroxystyryl)-dimethylsilylether (HSDMS-OR), (2-Hydroxystyryl)diisopropylsilylether (HSDIS-OR), t-Butylmethoxyphenyl-silylether (TBMPS-OR), t-Butoxydiphenylsilylether (DPTBOS-OR));
vom Typ eines Esters (z.B. Formiatester, Benzoylformiatester, Acetatester (Ac-OR), Chloracetatester, Dichloracetatester, Trichloracetatester, Trifluoracetatester, (TFA-OR), Methoxyacetatester, Triphenylmethoxyacetatester, Phenoxyacetatester, para-Chlor- phenoxyacetatester, Phenylacetatester, Diphenylacetatester (DPA-OR), Nicotinatester, 3-Phenyl-propionatester, 4-Pentoatester, 4-Oxopentoatester (Levulinate) (Lev-OR) 4,4-(Ethylendithio)-pentanoatester (LevS-OR), 5-[3-Bis(4-methoxyphenyl)hydroxy-methoxyphenoxy] -levulinatester, Pivaloatester (Pv-OR), 1-Adamantanoatester, Crotonatester, 4-Methoxy-crotonatester, Benzoatester (Bz-OR), para-Phenyl-benzoatester, 2,4,6-Trimethyl-benzoatester (Mesitoate), 4-(Methylthiomethoxy)-butyratester (MTMB-OR), 2-(Methylthiomethoxymethyl)-benzoatester (MTMT-OR),
vom Typ eines Esters (z.B. Methylcarbonat, Methoxymethylcarbonat, 9-Fluorenylmethylcarbonat (Fmoc-OR), Ethylcarbonat, 2,2,2-Trichlorethylcarbonat (Troc-OR), 1,1-Dimethyl-2,2,2-trichlorethylcarbonat (TCBOC-OR), 2-(Trimethylsilyl)ethylcarbonat (TMS-OR), 2-(Phenylsulfonyl)-ethylcarbonat (Ps-OR), 2-(Triphenylphosphonio)-ethylcarbonat (Peoc-OR), t-Butylcarbonat (Boc-OR), Isobutylcarbonat, Vinylcarbonat, Allylcarbonat (Alloc-OR), para-Nitro-phenylcarbonat, Benzylcarbonat (Z-OR), para-Methoxy-benzylcarbonat, 3,4-Dimethoxy-benzylcarbonat, ortho-Nitro-benzylcarbonat, para-Nitro-benzylcarbonat, 2-Dansylethylcarbonat (Dnseoc-OR), 2-(4-Nitrophenyl)ethylcarbonat (Npeoc-OR), 2-(2,4-Dinitrophenyl)ethylcarbonat (Dnpeoc)), und vom Typ eines Sulfats (z.B. Allylsulfonat (Als-OR), Methansulfonat (Ms-OR), Benzylsulfonat, Tosylat (Ts-OR), 2-[(4-Nitrophenyl)ethyl]sulfonat (Npes-OR)).

Als Katalysatoren zur Durchführung einer katalytischen Hydrierung im erfindungsgemäßen Verfahren sind alle üblichen Hydrierkatalysatoren, wie beispielsweise Platin-Katalysatoren (z.B. Platin-Platte, Platin-Schwamm, Platin-Schwarz, kolloidales Platin, Platinoxid, Platindraht), Palladium-Katalysatoren (z.B. Palladium-Schwamm, Palladium-Schwarz, Palladiumoxid, Palladium-Kohle, kolloidales Palladium, Palladium-Bariumsulfat, Palladium-Bariumcarbonat, Palladium-Hydroxid , Nickel-Katalysatoren (z.B. reduziertes Nickel, Nickeloxid, Raney-Nickel), Ruthenium-Katalysatoren, Cobalt-Katalysatoren (z.B. reduziertes Cobalt, Raney-Cobalt), Kupfer-Katalysatoren (z.B. reduziertes Kupfer, Raney-Kupfer, Ullmann-Kupfer) geeignet. Bevorzugt werden Edelmetalllkatalysatoren (z.B. Platin- und Palladium- oder Ruthenium-Katalysatoren) verwendet, die gegebenenfalls auf einem geeigneten Träger (z.B. Kohlenstoff oder Silizium) aufgebraucht sind, Rhodium-Katalysatoren (z.B. Tris(triphenylphosphin)rhodium(I)-chlorid in Gegenwart von Triphenylphosphin). Ferner können "chiralen Hydrierkatalysatoren" (z. B. solche die chirale Diphosphinliganden enhalten wie (2S,3S)-(-)-2,3-Bis(diphenylphosphino)-butan [(S,S)-Chiraphos] oder (R)-(+)-2,2'- bzw. (S)-(-)-2,2'-Bis(diphenylphosphino)-1,1'-binaphthalin [R(+)-BINAP bzw. S(-)-BINAP]) verwendet werden, wodurch der Anteil eines Isomers im Isomerengemisch erhöht wird bzw. das Entstehen eines anderen Isomers nahezu vollständig unterdrückt wird.

Die Herstellung von Salzen der erfindungsgemäßen Verbindungen erfolgt nach Standardverfahren. Repräsentative Säureadditionssalze sind beispielsweise solche die durch Reaktion mit anorganischen Säuren, wie beispielsweise Schwefelsäure, Salzsäure, Bromwasserstoffsäure, Phosphorsäure oder organischen Carbonsäuren wie Essigsäure, Trifluoressigsäure, Zitronensäure, Bernsteinsäure, Buttersäure, Milchsäure, Ameisensäure, Fumarsäure, Maleinsäure, Malonsäure, Camphersäure, Oxalsäure, Phthalsäure, Propionsäure, Glycolsäure, Glutarsäure, Stearinsäure, Salicylsäure, Sorbinsäure, Weinsäure, Zimtsäure, Valeriansäure, Pikrinsäure, Benzoesäure oder organischen Sulfonsäuren wie Methansulfonsäure und 4-Toluolsulfonsäure gebildet werden.

Repräsentativ sind auch Salze von erfindungsgemäßen Verbindungen, die aus organischen Basen, wie beispielsweise Pyridin oder Triethylamine gebildet werden oder solche, die aus anorganischen Basen, wie beispielsweise Hydride, Hydroxide oder Karbonate des Natriums, Lithiums, Calciums, Magnesiums oder Bariums, gebildet werden, wenn die Verbindungen der allgemeinen Formel (Ic) ein zu dieser Salzbildung geeignetes Strukturelement aufweist.

Synthesemethoden zur Darstellung heterocyclischer N-Oxide und t-Aminen sind bekannt. Sie können mit Peroxysäuren (z.B. Peressigsäure und meta-Chlor-perbenzoesäure (MCPBA), Wasserstoffperoxid), Alkylhydroperoxide (z.B. t-Butylhydroperoxid), Natriumperborat und Dioxiranen (z.B. Dimethyldioxiran) erhalten werden. Diese Methoden sind beispielsweise von T. L. Gilchrist, in Comprehensive Organic Synthesis, Vol. 7, S. 748-750, 1992, S. V. Ley, (Ed.), Pergamon Press; M. Tisler, B. Stanovnik, in Comprehensive Heterocyclic Chemistry, Vol. 3, S. 18-20, 1984, A. J. Boulton, A. McKillop, (Eds.), Pergamon Press; M. R. Grimmett, B. R. T. Keene in Advances in Heterocyclic Chemistry, Vol. 43, S. 149-163, 1988, A. R. Katritzky, (Ed.), Academic Press; M. Tisler, B. Stanovnik, in Advances in Heterocyclic Chemistry, Vol. 9, S. 285-291, 1968, A. R. Katritzky, A. J. Boulton (Eds.), Academic Press; G. W. H. Cheeseman, E. S. G. Werstiuk in Advances in Heterocyclic Chemistry, Vol. 22, S. 390-392, 1978, A. R. Katritzky, A. J. Boulton, (Eds.), Academic Press beschrieben.

### Herstellungsbeispiele

Erfindungsgemäße Beispiele sind solche, die unter die Patentansprüche fallen.

### ¹H-NMR Daten

Die ¹H-NMR-Daten wurden mit einem Bruker Avance 400, ausgestattet mit einer Flusszelle (60 µl Volumen), oder mit einem Bruker AVIII 400, ausgestattet mit einem 1.7 mm Kryo-CPTCI Probenkopf, oder mit einem Bruker AVII 600 (600.13 MHz), ausgestattet mit einem 5 mm Kryo-TCI Probenkopf, oder mit einem Bruker AVIII 600 (601.6 MHz), ausgestattet mit einem 5 mm Kryo-CPMNP Probenkopf, aufgenommen. Dabei wurde Tetramethylsilan als Referenz (0.0 ppm) und CD₃CN, CDCl₃ oder D₆-DMSO als deuterierte Lösungsmittel verwendet.

### Synthese von N-{3-[2'-Methyl-5'-(pentafluorethyl)-4'-(trifluormethyl)-2'H-1,3'-bipyrazol-4-yl]-phenyl}isonicotinamid

### Stufe 1

### 4-Brom-2'-methyl-5'-(pentafluorethyl)-4'-(trifluormethyl)-2'H-1,3'-bipyrazol

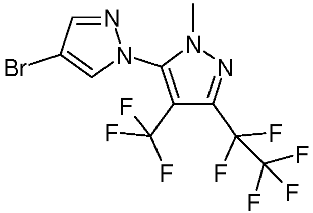

8 g (27,9 mmol) 5-Fluor-1-methyl-3-(pentafluorethyl)-4-(trifluormethyl)-1H-pyrazol (Nippon Kagaku Kaishi, 1985, (10), 1995-2000), 4,11 g (27,9 mmol) 4-Brompyrazol und 7,73 g (55,9 mmol) Kaliumcarbonat wurden in 140 mL THF 12 Stunden unter Rückfluß gerührt. Anschließend wurde das Kaliumcarbonat abfiltriert, das Lösungsmittel am Rotationsverdampfer abdestilliert und über Kieselgel (Cyclohexan-Essigester-Gradient) chromatographiert. Es wurden 6,83 g (55,3 % d.Th.) als wachsartiger Feststoff isoliert.
¹H-NMR, 400 MHz, d₆-DMSO, δ 8.58 (s, 1H), 8.16 (s, 1H), 3.77 (s, 3H).

### Stufe 2

### 2'-Methyl-4-(3-nitrophenyl)-5'-(pentafluorethyl)-4'-(trifluormethyl)-2'H-1,3'-bipyrazol

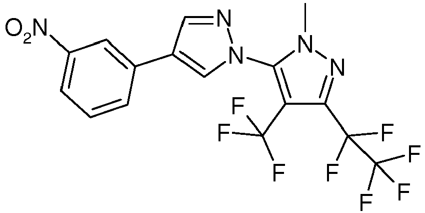

5,5 g (13,3 mmol) des Bipyrazoles aus Stufe 1 und 2,22 g (13,3 mmol) 3-Nitrophenylboronsäure wurden in 110 mL 1,4-Dioxan vorgelegt und mit 0,98 g (1,33 mmol) 1,1'-Bis(diphenylphosphino)ferrocenpalladium(II)chlorid und 86,5 mL einer 2M wässrigen Na₂CO₃-Lösung versetzt. Der Ansatz wurde bei 100 °C bis zur vollständigen Umsetzung gerührt. Nach Abkühlen des Reaktionsgemisches wurde der komplette Ansatz auf Kieselgel am Rotationsverdampfer eingeengt und anschließend über Kieselgel (Cyclohexan-Essigester-Gradient) chromatographiert. Es wurden 5,21 g (78,4 % d. Th.) als farbloser Feststoff erhalten.
¹H-NMR, 400 MHz, d₆-DMSO, δ 8.99 (s, 1H), 8.70 (s, 1H), 8.56 (s, 1H), 8.20 -8.14 (dd, 2H), 7.75 (t, 1H), 3.84 (s, 3H).

### Stufe 3

### 3-[2'-Methyl-5'-(pentafluorethyl)-4'-(trifluormethyl)-2'H-1,3'-bipyrazol-4-yl]anilin

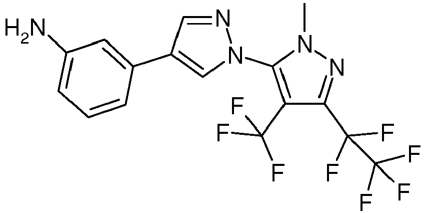

4,9 g (10,7 mmol) der Nitro-Verbindung aus Stufe 2 wurden in 36 mL Isopropanol gelöst und bei Raumtempertaur wurden 6,3 g (27,9 mmol) Zinn(II)chlorid-Dihydrat zugegeben. Die Reaktionsmischung wurde auf 0 °C gekühlt, dann wurden langsam 9 mL konz. HCl zugetropft und anschließend 2 Stunden unter Rückfluss gerührt. Nach Beendigung der Reaktion wurde das Volumen des Reaktionsgemisch am Rotationsverdampfer auf zwei Drittel eingeengt, dann wurden 500 mL Wasser hinzugefügt und anschließend wurde das wässrige Gemisch mit 30%iger NaOH auf pH 8-9 eingestellt. Das wässrige Gemisch wurde mehrfach mit Essigester extrahiert, die vereinigten organischen Phasen wurden über Magnesiumsulfat getrocknet, filtriert und eingeengt, der Rückstand wurde über Kieselgel (Cyclohexan-Essigester-Gradient) chromatographiert. Es wurden 3,2 g (68,2 % d. Th.) eines gelblichen Öls erhalten.
¹H-NMR, 400 MHz, d₆-DMSO, δ 8.55 (s, 1H), 8.30 (s, 1H), 7.07 (t, 1H), 6.83 - 6.81 (s, d, 2H), 6.53 (d, 1H), 5.16 (bs, 1H, NH₂), 3.81 (s, 3H).

### Stufe 4

### N-{3-[2'-Methyl-5'-(pentafluorethyl)-4'-(trifluormethyl)-2'H-1,3'-bipyrazol-4-yl]-phenyl}isonicotin-amid

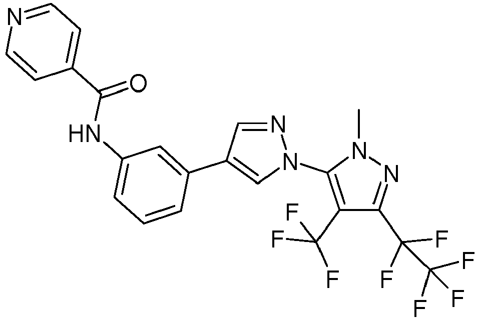

300 mg (0,7 mmol) des Anilins aus Stufe 3 wurden in 2 mL THF gelöst und mit 126 mg (0,7 mmol) Isonicotinsäurechlorid-Hydrochlorid und 86 mg (0,84 mmol) Triethylamin bei Raumtemperatur versetzt und unter Dünnschichtkontrolle bis zur vollständigen Umsetzung unter Rückfluss gerührt. Nach dem Abkühlen wurde mit 5 mL Wasser versetzt und mehrfach mit Dichlormethan extrahiert. Die vereinigten organischen Phasen wurden über Magnesiumsulfat getrocknet, filtriert und eingeengt, der Rückstand wurde über Kieselgel (Cyclohexan-Essigester-Gradient) chromatographiert. Es wurden 300 mg (80,2 % d.Th.) der Zielverbindung als gelbliches Öl erhalten.
¹H-NMR, 400 MHz, d₆-DMSO, δ 10.59 (1H, s, NH), 8.82 (d, 2H), 8.71 (s, 1H), 8.43 (s, 1H), 8.09 (s, 1H), 7.89 (d, 2H), 7.70 (d, 1H), 7.50 - 7.44 (m, 2H), 3.83 (s, 3H).

Mit Hilfe der oben beschriebenen Herstellungsverfahren wurden die in der Tabelle la, 1b und 2 aufgeführten Verbindungen dargestellt.

**Tabelle 1a**

| | | | | | | | |
|---|---|---|---|---|---|---|---|
| 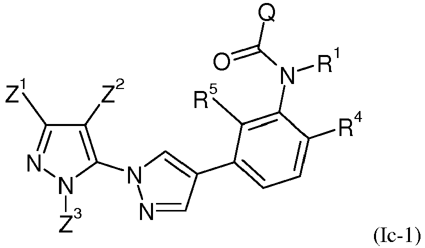 | | | | | | | |
| e Verbindungen der Formel (Ic-1) sind Verbindungen der allgemeinen Formel (Ic), wobei W = O, A₁ = A₂ = CH, A₃ = CR⁴, A₄ = CR⁵ und R⁶ = H (bzw. n = 0) sind. | | | | | | | |

| **Bsp.-Nr.** | **Z¹** | **Z²** | **Z³** | **R¹** | **R⁴** | **R⁵** | **Q** |
|---|---|---|---|---|---|---|---|
| 1-001 | CF₂CF₃ | CF₃ | CH₃ | H | H | H | 4-Pyridinyl |
| 1-002 | CF₂CF₃ | CF₃ | CH₃ | H | H | H | Cyclopropyl |
| 1-003 | CF₂CF₃ | CF₃ | CH₃ | H | H | H | 4-F-Phenyl |
| 1-004 | CF₂CF₃ | CF₃ | CH₃ | H | H | H | 3-F-4-Pyridinyl |
| 1-005 | CF₂CF₃ | CF₃ | CH₃ | H | H | H | 3-Cl-2-Pyridinyl |
| 1-006 | CF₂CF₃ | CF₃ | CH₃ | H | H | H | 2-Cl-4-Pyridinyl |
| 1-007 | CF₂CF₃ | CF₃ | CH₃ | H | H | H | CF₃CH₂- |
| 1-008 | CF₂CF₃ | CF₃ | CH₃ | H | H | H | Ethyl |
| 1-009 | CF₂CF₃ | CF₃ | CH₃ | H | H | H | CHF₂CF₂- |
| 1-010 | CF₂CF₃ | CF₃ | CH₃ | H | H | H | Phenyl |
| 1-011 | CF₂CF₃ | CF₃ | CH₃ | H | H | H | 2,6-Cl₂-4-Pyridinyl |
| 1-012 | CF₂CF₃ | CF₃ | CH₃ | H | H | H | 2-F-Phenyl |
| 1-013 | CF₂CF₃ | CF₃ | CH₃ | H | H | H | 3-F-Phenyl |
| 1-014 | CF₂CF₃ | CF₃ | CH₃ | H | H | H | 3,5-F₂-Phenyl |
| 1-015 | CF₂CF₃ | CF₃ | CH₃ | H | H | H | 2,6-F₂-Phenyl |
| 1-016 | CF₂CF₃ | CF₃ | CH₃ | H | H | H | 3-Thiophenyl |
| 1-017 | CF₂CF₃ | CF₃ | CH₃ | H | H | H | 2-Thiophenyl |
| 1-018 | CF₂CF₃ | CF₃ | CH₃ | H | H | H | 3-Cl-2-Thiophenyl |
| 1-019 | CF₂CF₃ | CF₃ | CH₃ | H | H | H | 1-Methyl-4-pyrazolyl |
| 1-020 | CF₂CF₃ | CF₃ | CH₃ | H | H | F | 3-Cl-2-Pyridinyl |
| 1-021 | CF₂CF₃ | CF₃ | CH₃ | H | Cl | H | 3-Cl-2-Pyridinyl |
| 1-022 | CF₂CF₃ | CF₃ | CH₃ | H | Cl | H | 4-F-Phenyl |
| 1-023 | CF₂CF₃ | CF₃ | CH₃ | H | Cl | H | Phenyl |
| 1-024 | CF₂CF₃ | CF₃ | CH₃ | H | Cl | H | 4-Pyridinyl |
| 1-025 | CF₂CF₃ | CF₃ | CH₃ | H | F | H | 4-Pyridinyl |
| 1-026 | CF₂CF₃ | CF₃ | CH₃ | H | F | H | Phenyl |
| 1-027 | CF₂CF₃ | CF₃ | CH₃ | H | F | H | 4-F-Phenyl |
| 1-028 | CF₂CF₃ | CF₃ | CH₃ | H | H | H | 2-Cl-3-Pyridinyl |
| 1-029 | CF₂CF₃ | CF₃ | CH₃ | H | H | H | 1-CN-cPropyl |
| 1-030 | CF₂CF₃ | CF₃ | CH₃ | H | H | H | 1-Cl-cPropyl |
| 1-031 | CF₂CF₃ | CF₃ | CH₃ | H | H | H | 1-Oxazolyl |
| 1-032 | CF₂CF₃ | CF₃ | CH₃ | H | F | H | 1-Cl-cPropyl |
| 1-033 | CF₂CF₃ | CF₃ | CH₃ | H | F | H | CHF₂CF₂- |
| 1-034 | CF₂CF₃ | CF₃ | CH₃ | H | F | H | 4-Pyrimidinyl |
| 1-035 | CF₂CF₃ | CF₃ | CH₃ | H | F | H | 1-Me-4-pyrazolyl |
| 1-036 | CF₂CF₃ | CF₃ | CH₃ | H | F | H | 2-Pyrimidinyl |
| 1-037 | CF₂CF₃ | CF₃ | CH₃ | CH3 | F | H | 4-F-Phenyl |
| 1-038 | CF₂CF₃ | CF₃ | CH₃ | H | CH₃ | H | 4-F-Phenyl |
| 1-039 | CF₂CF₃ | CF₃ | CH₃ | H | F | F | 4-F-Phenyl |
| 1-040 | CF₂CF₃ | CF₃ | CH₃ | H | CH₃ | H | 4-Pyridinyl |
| 1-041 | CF₂CF₃ | CF₃ | CH₃ | H | F | H | 3-Thiophenyl |
| 1-042 | CF₂CF₃ | CF₃ | CH₃ | H | F | H | 3-MeO-Phenyl |
| 1-043 | CF₂CF₃ | CF₃ | CH₃ | H | F | H | 2-Cl-6-Me-4-Pyridinyl |
| 1-044 | CF₂CF₃ | CF₃ | CH₃ | H | F | H | 1-Me-3-tBu-4-NO₂-5-Pyrazolyl |
| 1-045 | CF₂CF₃ | CF₃ | CH₃ | H | F | H | 3-C1-Benzthiophen-2-yl |
| 1-046 | CF₂CF₃ | CF₃ | CH₃ | H | F | H | (1,1-Dimethyl)-2-fluor-ethyl |
| 1-047 | CF₂CF₃ | CF₃ | CH₃ | H | F | H | cis/trans-4-MeO-Cyclohex-1-yl |
| 1-048 | CF₂CF₃ | CF₃ | CH₃ | H | F | H | MeOCH₂- |
| 1-049 | CF₂CF₃ | CF₃ | CH₃ | H | F | H | 2-Thiophenyl |
| 1-050 | CF₂CF₃ | CF₃ | CH₃ | H | F | H | 3-CF₃-4-F-Phenyl |
| 1-051 | CF₂CF₃ | CF₃ | CH₃ | H | F | H | 2,6-(MeO)₂-Phenyl |
| 1-052 | CF₂CF₃ | CF₃ | CH₃ | H | F | H | (rac)-1-Methylpropyl |
| 1-053 | CF₂CF₃ | CF₃ | CH₃ | H | F | H | 1-Me-Cyclohex-1-yl |
| 1-054 | CF₂CF₃ | CF₃ | CH₃ | H | F | H | 4-(Me₂N)-Phenyl |
| 1-055 | CF₂CF₃ | CF₃ | CH₃ | H | F | H | Cyclopropyl |
| 1-056 | CF₂CF₃ | CF₃ | CH₃ | H | F | H | 2,4,6-(iPr)₃-Phenyl |
| 1-057 | CF₂CF₃ | CF₃ | CH₃ | H | F | H | 3-CF₃-Phenyl |
| 1-058 | CF₂CF₃ | CF₃ | CH₃ | H | F | H | 1-Me-Cyclopropyl |
| 1-059 | CF₂CF₃ | CF₃ | CH₃ | H | F | H | 3-Me-2-Benzofuranyl |
| 1-060 | CF₂CF₃ | CF₃ | CH₃ | H | F | H | 4-Me-Phenyl |
| 1-061 | CF₂CF₃ | CF₃ | CH₃ | H | F | H | MeOCH₂CH₂- |
| 1-062 | CF₂CF₃ | CF₃ | CH₃ | H | F | H | 7-(Difluormethyl)-5-methyl-[1,2,4]triazolo[1,5 -a]pyrimidin-2-yl |
| 1-063 | CF₂CF₃ | CF₃ | CH₃ | H | F | H | 2-CF3-4-Me-Furan-2-yl |
| 1-064 | CF₂CF₃ | CF₃ | CH₃ | H | F | H | iPropyl |
| 1-065 | CF₂CF₃ | CF₃ | CH₃ | H | F | H | 1,1,2,2-Tetramethyl-ethyl |
| 1-066 | CF₂CF₃ | CF₃ | CH₃ | H | F | H | (R)-1-F-Ethyl |
| 1-067 | CF₂CF₃ | CF₃ | CH₃ | H | F | H | 2-Me-3-Furanyl |
| 1-068 | CF₂CF₃ | CF₃ | CH₃ | H | F | H | 2-Me-Prop-1-yl |
| 1-069 | CF₂CF₃ | CF₃ | CH₃ | H | F | H | 5-Me-Thien-2-yl |
| 1-070 | CF₂CF₃ | CF₃ | CH₃ | H | F | H | 2,3-F₂-Phenyl |
| 1-071 | CF₂CF₃ | CF₃ | CH₃ | H | F | H | 2-F-Phenyl |
| 1-072 | CF₂CF₃ | CF₃ | CH₃ | H | F | H | 2-EtO-Phenyl |
| 1-073 | CF₂CF₃ | CF₃ | CH₃ | H | F | H | 3-F-Phenyl |
| 1-074 | CF₂CF₃ | CF₃ | CH₃ | H | F | H | 2-CF₃O-Phenyl |
| 1-075 | CF₂CF₃ | CF₃ | CH₃ | H | F | H | 3-Cyano-Phenyl |
| 1-076 | CF₂CF₃ | CF₃ | CH₃ | H | F | H | Ethyl |
| 1-077 | CF₂CF₃ | CF₃ | CH₃ | H | F | H | Cyclopentyl |
| 1-078 | CF₂CF₃ | CF₃ | CH₃ | H | F | H | tButyl |
| 1-079 | CF₂CF₃ | CF₃ | CH₃ | H | F | H | 2,2-Dimethylprop-l-yl |
| 1-080 | CF₂CF₃ | CF₃ | CH₃ | H | F | H | 2,3-Dimethylphenyl |
| 1-081 | CF₂CF₃ | CF₃ | CH₃ | H | F | H | 5-Methyl-isoxazol-4-yl |
| 1-082 | CF₂CF₃ | CF₃ | CH₃ | H | F | H | 1,1-Dimethyl-2-ethoxy-ethyl |
| 1-083 | CF₂CF₃ | CF₃ | CH₃ | H | F | H | 3-Cl-6-Me-Benzothiophen-2-yl |
| 1-084 | CF₂CF₃ | CF₃ | CH₃ | H | F | H | 2,2-Difluor-13-benzodioxol-5-yl |
| 1-085 | CF₂CF₃ | CF₃ | CH₃ | H | F | H | 3-Pyridinyl |
| 1-086 | CF₂CF₃ | CF₃ | CH₃ | H | F | H | 3-F-5-Pyridinyl |
| 1-087 | CF₂CF₃ | CF₃ | CH₃ | H | F | H | 2-Me-4-Pyridinyl |
| 1-088 | CF₂CF₃ | CF₃ | CH₃ | H | F | H | 4-CN-Phenyl |
| 1-089 | CF₂CF₃ | CF₃ | CH₃ | H | F | H | 2-F-4-Pyridinyl |
| 1-090 | CF₂CF₃ | CF₃ | CH₃ | H | F | H | 3,4-Dimethylphenyl |
| 1-091 | CF₂CF₃ | CF₃ | CH₃ | H | F | H | 3-Me, 4-F-Phenyl |
| 1-092 | CF₂CF₃ | CF₃ | CH₃ | H | F | H | 3-F, 4-Me-Phenyl |
| 1-093 | CF₂CF₃ | CF₃ | CH₃ | H | F | H | 1,3-Benzodioxol-5-yl |
| 1-094 | CF₂CF₃ | CF₃ | CH₃ | H | F | H | 3-MeO, 4-F-Phenyl |
| 1-095 | CF₂CF₃ | CF₃ | CH₃ | H | F | H | 3-F, 4-MeO-Phenyl |
| 1-096 | CF₂CF₃ | CF₃ | CH₃ | H | F | H | 4-C1, 3-F-Phenyl |
| 1-097 | CF₂CF₃ | CF₃ | CH₃ | H | F | H | 3-MeO, 4-Me-Ph |
| 1-098 | CF₂CF₃ | CF₃ | CH₃ | H | F | H | 2-CN-5-Pyridinyl |
| 1-099 | CF₂CF₃ | CF₃ | CH₃ | H | F | H | 2-Cl-5-Pyridinyl |
| 1-100 | CF₂CF₃ | CF₃ | CH₃ | H | F | H | 2-F-5-Pyridinyl |
| 1-101 | CF₂CF₃ | CF₃ | CH₃ | H | F | H | 3-EtO, 4-F-Phenyl |
| 1-102 | CF₂CF₃ | CF₃ | CH₃ | H | F | F | 2-CN-5-Pyridinyl |
| 1-103 | CF₂CF₃ | CF₃ | CH₃ | CH₃ | F | F | 2-CN-5-Pyridinyl |
| 1-104 | CF₂CF₃ | CF₃ | CH₃ | H | H | F | 2-CN-5-Pyridinyl |
| 1-105 | CF₂CF₃ | CF₃ | CH₃ | CH₃ | H | F | 2-CN-5-Pyridinyl |
| 1-106 | CF₂CF₃ | CF₃ | CH₃ | CH₃ | F | H | 2-CN-5-Pyridinyl |
| 1-128 | CF₂CF₃ | CF₃ | CH₃ | H | F | F | 2-Thiophenyl |
| 1-129 | CF₂CF₃ | CF₃ | CH₃ | H | F | F | 3-MeO-4-F-Phenyl |
| 1-130 | CF₂CF₃ | CF₃ | CH₃ | H | F | F | 3-F-4-MeO-Phenyl |
| 1-131 | CF₂CF₃ | CF₃ | CH₃ | CH₃ | H | F | 3-MeO-4-F-Phenyl |
| 1-132 | CF₂CF₃ | CF₃ | CH₃ | H | F | F | 2-F-5-Pyridinyl |
| 1-133 | CF₂CF₃ | CF₃ | CH₃ | CH₃ | F | F | 2-F-5-Pyridinyl |
| 1-334 | CF₂CF₃ | CF₃ | CH₃ | H | H | F | 2-F-5-Pyridinyl |

Insbesondere bevorzugte Verbindungen der Formel (Ic-1) sind auch solche, die sich aus beliebigen Kombinationen der in der Tabelle la aufgeführten Restedefinitionen für Z¹, Z², Z³, R¹, R⁴, R⁵ und Q ergeben.

**Tabelle 1b**

| | | | | | | | |
|---|---|---|---|---|---|---|---|
| 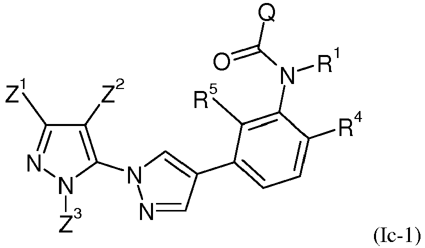 | | | | | | | |
| Verbindungen der Formel (Ic-1) sind Verbindungen der allgemeinen Formel (Ic), wobei W = O, A₁ = A₂ = CH, A₃ = CR⁴, A₄ = CR⁵ und R⁶ = H (bzw. n = 0) sind. | | | | | | | |

| **Bsp.-Nr.** | **Z¹** | **Z²** | **Z³** | **R¹** | **R⁴** | **R⁵** | **Q** |
|---|---|---|---|---|---|---|---|
| 1-107 | CF₂CF₃ | CF₃ | CH₃ | H | F | H | Phenyl-thiomethyl |
| 1-108 | CF₂CF₃ | CF₃ | CH₃ | H | F | H | (rac)-1-Phenyl-ethyl |
| 1-109 | CF₂CF₃ | CF₃ | CH₃ | H | F | H | (rac)-Phenyl-cPentylmethyl |
| 1-110 | CF₂CF₃ | CF₃ | CH₃ | H | F | H | (rac)-Tetralin |
| 1-111 | CF₂CF₃ | CF₃ | CH₃ | H | F | H | 1-(4-Me-Phenyl)-cyclohex-1-yl |
| 1-112 | CF₂CF₃ | CF₃ | CH₃ | H | F | H | 3-CF3-4-Me-Pyrazol-1-yl-methyl |
| 1-113 | CF₂CF₃ | CF₃ | CH₃ | H | F | H | 1-Phenyl-cycloprop-1-yl |
| 1-114 | CF₂CF₃ | CF₃ | CH₃ | H | F | H | Benzyloxymethyl |
| 1-115 | CF₂CF₃ | CF₃ | CH₃ | H | F | H | 1-(4-Cl-Phenyl)-cyclobut-1-yl |
| 1-116 | CF₂CF₃ | CF₃ | CH₃ | H | F | H | 3-CF₃-Benzyl |
| 1-117 | CF₂CF₃ | CF₃ | CH₃ | H | F | H | 2-Thiophenylmethyl |
| 1-118 | CF₂CF₃ | CF₃ | CH₃ | H | F | H | Phenoxyethyl |
| 1-119 | CF₂CF₃ | CF₃ | CH₃ | H | F | H | 1-Methyl-1-(3-Cl-phenyl)-ethyl |
| 1-120 | CF₂CF₃ | CF₃ | CH₃ | H | F | H | (rac)-1-(4-Cl-Phenyl)-2-methyl-prop-l-yl |
| 1-121 | CF₂CF₃ | CF₃ | CH₃ | H | F | H | 3-F-Benzyl |
| 1-122 | CF₂CF₃ | CF₃ | CH₃ | H | F | H | 1-(4-Cl-Phenyl)-cyclopent-1-yl |
| 1-123 | CF₂CF₃ | CF₃ | CH₃ | H | F | H | (rac)-1-Phenoxyethyl |
| 1-124 | CF₂CF₃ | CF₃ | CH₃ | H | F | H | (rac)-1-Phenyl-prop-l-yl |
| 1-125 | CF₂CF₃ | CF₃ | CH₃ | H | F | H | 2-Phenyl-ethyl |
| 1-126 | CF₂CF₃ | CF₃ | CH₃ | H | F | H | 2-Cl-4-F-Benzyl |
| 1-127 | CF₂CF₃ | CF₃ | CH₃ | H | F | H | N',N'-Dimethyloxalylamid |

Insbesondere bevorzugte Verbindungen der Formel (Ic-1) sind auch solche, die sich aus beliebigen Kombinationen der in der Tabelle 1b aufgeführten Restedefinitionen für Z¹, Z², Z³, R¹, R⁴, R⁵ und Q ergeben.

**Tabelle 2**

| | | | | | | | |
|---|---|---|---|---|---|---|---|
| 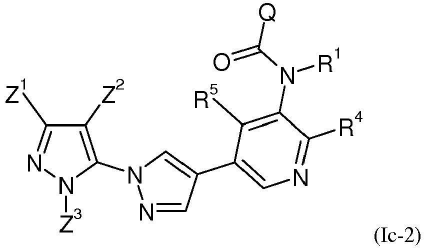 | | | | | | | |
| Verbindungen der Formel (Ic-2) sind Verbindungen der allgemeinen Formel (Ic), wobei W = O, A₁ = CH, A₂ = N, A₃ = CR⁴, A₄ = CR⁵ und R⁶ = H (bzw. n = 0) sind. | | | | | | | |

| **Bsp.-Nr.** | **Z¹** | **Z²** | **Z³** | **R¹** | **R⁴** | **R⁵** | **Q** |
|---|---|---|---|---|---|---|---|
| 2-001 | CF₂CF₃ | CF₃ | CH₃ | H | Cl | H | 4-F-Phenyl |
| 2-002 | CF₂CF₃ | CF₃ | CH₃ | H | Cl | H | 3-Cl-2-Pyridinyl |
| 2-003 | CF₂CF₃ | CF₃ | CH₃ | H | CH₃ | H | 4-F-Phenyl |
| 2-004 | CF₂CF₃ | CF₃ | CH₃ | H | CH₃ | H | 4-Pyridinyl |
| 2-005 | CF₂CF₃ | CF₃ | CH₃ | H | F | H | 4-Pyridinyl |
| 2-006 | CF₂CF₃ | CF₃ | CH₃ | H | Cl | H | 4-Pyridinyl |
| 2-007 | CF₂CF₃ | CF₃ | CH₃ | H | Cl | H | 3-MeO, 4-F-Phenyl |
| 2-008 | CF₂CF₃ | CF₃ | CH₃ | H | Cl | H | 2-CN-5-Pyridinyl |
| 2-009 | CF₂CF₃ | CF₃ | CH₃ | CH₃ | Cl | H | 2-CN-5-Pyridinyl |

Insbesondere bevorzugte Verbindungen der Formel (Ic-2) sind auch solche, die sich aus beliebigen Kombinationen der in der Tabelle 2 aufgeführten Restedefinitionen für Z¹, Z², Z³, R¹, R⁴, R⁵ und Q ergeben.

### Analytische Daten

### NMR-Peak-Listenverfahren

Die ¹H-NMR-Daten ausgewählter Beispiele werden in Form von ¹H-NMR-Peaklisten notiert. Zu jedem Signalpeak wird erst der δ -Wert in ppm und dann die Signalintensität in runden Klammern aufgeführt. Die δ -Wert - Signalintensitäts- Zahlenpaare von verschiedenen Signalpeaks werden durch Semikolons voneinander getrennt aufgelistet.

Die Peakliste eines Beispieles hat daher die Form:
δ₁ (IntenSität₁⁾; δ₂ (Intensität₂);........; δᵢ (Intensitätᵢ⁾; ......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation.

Weitere Details zu ¹H-NMR-Peaklisten können der Research Disclosure Database Number 564025 entnommen werden.

| |
|---|
| Beispiel 1-001: ¹H-NMR(400,0 MHz, DMSO): δ = 10,591(2,2); 8,817(3,5); 8,813(2,2); 8,806(2,3); 8,802(3,7); 8,707(3,9); 8,426(4,3); 8,317(0,4); 8,087(2,1); 7,954(2,2); 7,896(3,5); 7,892(2,2); 7,884(2,2); 7,880(3,4); 7,699(1,0); 7,680(1,2); 7,503(0,7); 7,500(0,5); 7,484(2,1); 7,480(1,3); 7,474(1,9); 7,455(1,9); 7,436(0,6); 4,039(0,3); 4,021(0,3); 3,829(10,2); 3,329(25,9); 2,892(16,0); 2,733(14,0); 2,525(0,5); 2,512(12,9); 2,508(25,5); 2,503(33,3); 2,499(24,2); 1,990(1,4); 1,193(0,4); 1,175(0,8); 1,158(0,4); 0,008(1,3); 0,000(30,9); -0,008(1,5) |
| Beispiel 1-002: ¹H-NMR(400,0 MHz, DMSO): δ = 10,280(0,5); 8,656(1,2); 8,371(1,3); 7,954(2,2); 7,927(0,6); 7,364(1,0); 7,355(0,5); 7,351(0,8); 3,813(3,2); 3,330(8,0); 2,892(16,0); 2,733(13,6); 2,512(3,2); 2,508(6,4); 2,503(8,4); 2,499(6,0); 2,494(2,8); 1,235(0,3); 0,814(1,5); 0,806(1,1); 0,798(0,7); 0,793(0,9); 0,787(0,6); 0,781(0,4); 0,773(0,4); 0,769(0,5); 0,008(0,5); 0,000(14,1); -0,009(0,5) |
| Beispiel 1-003: ¹H-NMR(400,0 MHz, DMSO): δ = 10,350(2,4); 8,694(4,2); 8,416(4,7); 8,084(3,2); 8,080(3,1); 8,071(2,4); 8,063(2,3); 8,054(0,9); 8,049(2,1); 8,021(0,4); 8,007(0,5); 7,999(0,5); 7,985(0,4); 7,954(2,2); 7,693(0,7); 7,688(1,2); 7,682(0,7); 7,675(0,8); 7,670(1,4); 7,665(0,8); 7,466(0,5); 7,462(0,4); 7,447(3,9); 7,428(1,9); 7,420(0,4); 7,412(2,2); 7,390(3,9); 7,373(0,7); 7,368(1,9); 7,343(0,4); 7,321(0,8); 7,298(0,4); 3,827(11,1); 3,328(27,3); 2,891(16,0); 2,732(13,6); 2,512(16,7); 2,507(32,9); 2,503(42,8); 2,498(30,8); 2,494(15,0); 0,008(1,6); |
| 0,000(37,7); -0,008(1,4) |
| Beispiel 1-004: ¹H-NMR(400,0 MHz, DMSO): δ = 10,775(3,3); 8,781(3,7); 8,709(5,7); 8,617(2,2); 8,605(2,3); 8,421(6,2); 8,024(3,3); 7,953(0,6); 7,735(1,6); 7,721(2,3); 7,708(1,6); 7,613(1,5); 7,594(1,9); 7,509(1,2); 7,489(2,9); 7,473(2,5); 7,453(2,7); 7,434(0,9); 5,757(2,7); 3,824(16,0); 3,326(86,8); 2,891(4,8); 2,731(4,1); 2,676(0,5); 2,671(0,7); 2,667(0,5); 2,511(41,6); 2,507(80,7); 2,502(104,0); 2,498(74,1); 2,493(35,3); 2,334(0,5); 2,329(0,7); 2,325(0,5); 1,259(0,4); 1,235(0,4); 0,146(0,6); 0,008(6,0); 0,000(134,9); -0,009(5,0); -0,150(0,6) |
| Beispiel 1-005: ¹H-NMR(400,0 MHz, DMSO): δ = 10,741(3,4); 8,714(6,1); 8,653(2,4); 8,650(2,6); 8,641(2,6); 8,638(2,6); 8,426(6,6); 8,139(2,4); 8,136(2,4); 8,118(2,6); 8,115(2,6); 8,091(3,4); 7,669(1,6); 7,664(1,1); 7,649(2,1); 7,645(3,8); 7,633(2,5); 7,624(2,4); 7,613(2,3); 7,490(1,0); 7,486(0,7); 7,471(3,2); 7,461(2,9); 7,442(2,9); 7,423(1,0); 4,056(1,2); 4,038(3,7); 4,020(3,7); 4,003(1,3); 3,825(16,0); 3,806(0,6); 3,329(54,8); 2,891(0,9); 2,732(0,7); 2,676(0,3); 2,672(0,4); 2,667(0,3); 2,525(1,2); 2,511(26,9); 2,507(54,4); 2,503(71,2); 2,498(51,5); 2,494(24,9); 2,334(0,3); 2,330(0,4); 2,325(0,3); 1,990(16,0); 1,398(15,9); 1,193(4,3); 1,175(8,5); 1,157(4,2); 0,008(0,6); 0,000(18,3); -0,009(0,6) |
| Beispiel 1-006: ¹H-NMR(400,0 MHz, DMSO): δ = 10,665(2,0); 8,709(3,6); 8,652(1,9); 8,639(2,0); 8,4273(3,9); 8,4265(3,9); 8,062(1,9); 8,025(2,4); 8,024(2,4); 7,903(1,5); 7,899(1,4); 7,890(1,4); 7,887(1,3); 7,695(0,9); 7,690(0,6); 7,675(1,1); 7,516(0,7); 7,512(0,5); 7,500(1,2); 7,497(1,9); 7,493(1,1); 7,483(1,6); 7,464(1,8); 7,444(0,6); 4,038(0,8); 4,020(0,8); 3,828(9,5); 3,569(4,9); 3,332(36,1); 2,525(0,6); 2,512(13,6); 2,508(27,3); 2,503(35,3); 2,499(24,9); 2,494(11,6); 1,990(3,7); 1,398(16,0); 1,193(1,0); 1,175(2,0); 1,157(1,0); 0,008(0,6); 0,000(18,1); - 0,009(0,6) |
| Beispiel 1-007: ¹H-NMR(400,0 MHz, d₆-DMSO): δ= 10,402(3,3);8,703(5,9);8,411(6,4);7,892(3,6);7,468(1,2);7,462(0,9);7,450(3,1);7,4 42(3,5);7,438(3,5);7,427(3,4);7,408(1,8);7,389(0,6);3,817(16,0);3,581(1,3);3,553(3,9);3,525(4,1);3,497(1,4);3,343(141,3);3,314(0,4);2 ,543(14,8);2,508(29,0);2,504(37,3);2,500(28,6);0,000(5,0) |
| Beispiel 1-008: ¹H-NMR(400,0 MHz, d₆-DMSO): δ= 9,947(2,7);8,657(6,0);8,373(6,3);7,919(2,9);7,498(0,7);7,493(0,8);7,486(1,2);7,48 2(1,5);7,476(1,1);7,471(1,1);7,362(5,1);7,350(4,1);3,816(16,0);3,334(77,4);2,543(7,9);2,525(0,5);2,512(12,9);2,508(26,7);2,503(35,8); 2,499(26,7);2,368(1,6);2,350(5,1);2,331(5,4);2,312(1,7);1,113(5,4);1,094(11,2);1,075(5,2);0,000(5,1) |
| Beispiel 1-009: ¹H-NMR(400,0 MHz, d₆-DMSO): δ= 11,137(2,2);8,720(6,2);8,436(6,7);7,968(2,1);7,964(3,6);7,959(2,3);7,614(1,5);7,5 94(1,9);7,589(1,7);7,575(1,6);7,555(2,4);7,494(2,5);7,474(3,3);7,454(1,3);7,006(0,7);6,993(0,3);6,891(0,7);6,878(1,4);6,864(0,7);6,76 2(0,3);6,749(0,7);6,735(0,4);5,756(1,3);3,822(16,0);3,326(30,7);2,525(0,7);2,512(16,3);2,508(33,5);2,503(44,8);2,498(33,2);2,494(16, 8);0,008(0,8);0,000(24,6);-0,008(1,1) |
| Beispiel 1-010: ¹H-NMR(400,0 MHz, d₆-DMSO): δ= 10,340(3,7);8,697(6,1);8,419(6,7);8,106(3,5);7,994(4,1);7,977(4,4);7,708(1,7);7,7 02(1,0);7,690(1,9);7,685(1,2);7,631(0,6);7,613(2,3);7,607(0,7);7,595(2,0);7,570(3,6);7,551(4,5);7,534(1,6);7,463(0,6);7,460(0,6);7,44 6(5,9);7,428(2,5);7,409(0,7);3,829(16,0);3,334(74,1);2,542(19,0);2,507(29,3);2,503(37,3);2,499(28,0);0,000(3,4) |
| Beispiel 1-011: ¹H-NMR(400,0 MHz, d₆-DMSO): δ= 10,710(3,6);8,707(5,8);8,423(6,3);8,039(16,0);7,687(1,5);7,668(1,8);7,526(1,1);7, 506(2,8);7,491(2,4);7,471(2,7);7,452(0,9);3,826(15,3);3,332(239,3);2,675(0,5);2,671(0,7);2,542(34,9);2,506(77,5);2,502(102,4);2,498 (78,3);2,329(0,7);2,325(0,5);1,234(0,6);0,000(7,6) |
| Beispiel 1-012: ¹H-NMR(400,0 MHz, d₆-DMSO): δ= 10,496(3,3);8,695(5,8);8,411(6,0);8,059(2,7);7,712(0,8);7,708(1,0);7,693(1,6);7,6 90(1,9);7,674(0,9);7,671(1,0);7,634(1,4);7,629(1,1);7,615(1,8);7,608(1,6);7,593(1,0);7,587(1,2);7,582(0,7);7,573(0,6);7,569(0,6);7,47 0(0,9);7,466(0,7);7,451(3,5);7,446(3,6);7,426(2,5);7,407(0,8);7,392(1,3);7,367(3,6);7,348(3,6);7,331(1,3);7,329(1,2);3,824(16,0);3,33 4(99,1);2,542(13,5);2,525(0,6);2,512(16,0);2,507(33,0);2,503(43,8);2,498(32,2);2,494(15,9);1,235(0,4);0,000(4,9) |
| Beispiel 1-013: ¹H-NMR(400,0 MHz, d₆-DMSO): δ= 10,405(3,7);8,702(6,2);8,423(6,7);8,088(3,4);7,855(1,9);7,836(2,3);7,816(1,2);7,8 10(1,5);7,806(1,1);7,791(1,1);7,786(1,4);7,782(1,1);7,705(1,0);7,701(1,7);7,696(1,1);7,687(1,2);7,683(2,0);7,678(1,2);7,642(0,9);7,62 7(1,0);7,621(1,7);7,607(1,7);7,602(1,2);7,587(1,0);7,494(0,9);7,487(1,1);7,482(1,0);7,473(1,7);7,467(3,6);7,463(4,2);7,459(4,8);7,447( 0,9);7,440(2,9);7,421(0,9);3,829(16,0);3,337(68,0);2,543(23,1);2,526(0,5);2,513(11,0);2,508(22,3);2,504(29,5);2,500(21,7);2,495(10, 9);0,000(3,1) |
| Beispiel 1-014: ¹H-NMR(400,0 MHz, d₆-DMSO): δ= 10,453(3,8);8,705(6,1);8,425(6,6);8,068(3,6);7,735(0,5);7,723(2,5);7,718(3,2);7,7 02(3,3);7,697(3,6);7,674(2,1);7,582(0,6);7,576(0,9);7,570(0,5);7,559(1,2);7,553(1,8);7,547(0,9);7,536(0,7);7,530(0,9);7,525(0,5);7,49 8(1,1);7,494(0,8);7,479(3,5);7,475(2,5);7,470(3,1);7,451(2,8);7,432(0,9);3,829(16,0);3,338(76,8);2,544(23,7);2,509(23,6);2,505(29,8); 2,500(22,4);0,000(2,3) |
| Beispiel 1-015: ¹H-NMR(400,0 MHz, d₆-DMSO): δ= 10,894(3,6);8,714(6,2);8,425(6,8);8,021(3,5);7,647(0,4);7,630(1,0);7,626(0,9);7,6 09(1,8);7,589(2,4);7,571(2,5);7,500(1,2);7,497(0,9);7,481(3,1);7,465(2,8);7,445(3,0);7,426(1,0);7,296(0,6);7,289(3,1);7,269(4,4);7,24 9(2,6);7,242(0,6);3,825(16,0);3,337(74,0);2,543(35,4);2,526(0,4);2,513(10,5);2,508(21,6);2,504(28,6);2,499(21,1);2,495(10,6);0,000( 3,3) |
| Beispiel 1-016: ¹H-NMR(400,0 MHz, d₆-DMSO): δ= 10,134(3,6);8,691(6,1);8,412(6,7);8,377(2,3);8,374(2,6);8,370(2,8);8,367(2,5);8,3 16(0,8);8,049(3,3);7,683(1,3);7,676(2,0);7,670(4,6);7,663(4,3);7,654(5,3);7,644(1,6);7,641(1,4);7,445(0,6);7,434(5,5);7,418(2,6);7,39 9(0,6);3,824(16,0);3,323(116,1);2,675(0,9);2,671(1,2);2,666(1,0);2,506(134,0);2,502(180,9);2,497(139,8);2,333(0,8);2,328(1,1);2,324 (0,9);1,989(0,6);1,398(7,7);0,146(0,4);0,008(2,9);0,000(80,9);-0,008(4,7);-0,150(0,4) |
| Beispiel 1-017: ¹H-NMR(400,0 MHz, d₆-DMSO): δ= 10,324(3,7);8,707(6,2);8,429(6,6);8,056(2,5);8,054(2,8);8,047(2,8);8,045(2,9);8,0 34(3,5);7,886(2,6);7,884(2,7);7,873(2,7);7,871(2,7);7,658(1,0);7,653(1,8);7,648(1,1);7,640(1,2);7,635(2,1);7,630(1,3);7,467(0,7);7,46 4(0,6);7,449(5,8);7,430(2,6);7,411(0,7);7,258(2,4);7,249(2,6);7,246(2,7);7,237(2,3);3,828(16,0);3,339(69,5);2,544(25,0);2,527(0,4);2, |
| 509(20,6);2,504(27,4);2,500(20,6);0,000(2,7) |
| Beispiel 1-018: ¹H-NMR(400,0 MHz, d₆-DMSO): δ= 10,261(1,3);8,707(2,2);8,425(2,4);7,999(1,2);7,994(0,8);7,936(1,8);7,923(1,9);7,6 06(0,5);7,601(0,4);7,586(0,7);7,582(0,5);7,485(0,4);7,469(0,7);7,466(1,1);7,462(0,6);7,449(1,0);7,429(1,1);7,410(0,4);7,235(1,9);7,22 2(1,8);3,822(5,6);3,325(13,7);2,525(0,3);2,511(8,2);2,507(17,3);2,502(23,4);2,498(17,4);2,493(8,7);1,989(0,4);1,398(16,0);0,008(0,7); 0,000(22,6);-0,009(1,0) |
| Beispiel 1-019: ¹H-NMR(400,0 MHz, d₆-DMSO): δ= 9,900(3,2);8,685(5,2);8,406(5,7);8,320(5,0);8,035(5,5);8,006(2,8);7,642(0,8);7,63 7(0,9);7,626(1,5);7,619(1,0);7,614(1,0);7,410(4,4);7,398(3,5);3,905(16,0);3,825(13,4);3,342(106,7);2,543(30,5);2,526(0,5);2,512(12,0 );2,508(23,5);2,504(30,2);2,499(22,3);0,000(1,8) |
| Beispiel 1-020: ¹H-NMR(400,0 MHz, d₆-DMSO): δ= 10,567(3,4);8,766(5,8);8,662(2,4);8,659(2,5);8,650(2,5);8,647(2,5);8,484(6,2);8,3 17(0,5);8,219(1,6);8,214(1,7);8,201(1,7);8,196(1,6);8,139(2,3);8,135(2,3);8,118(2,6);8,115(2,5);7,661(2,5);7,650(2,4);7,641(2,2);7,62 9(2,3);7,617(0,8);7,612(0,9);7,606(1,0);7,600(1,0);7,596(1,1);7,590(1,1);7,584(1,1);7,579(0,9);7,439(1,7);7,417(1,6);7,413(2,0);7,391( 1,4);3,826(16,0);3,327(199,1);2,676(0,8);2,671(1,1);2,667(0,8);2,542(22,6);2,524(2,8);2,511(61,5);2,507(123,4);2,502(162,4);2,498(1 17,5);2,493(56,4);2,333(0,8);2,329(1,1);2,324(0,8);0,008(0,5);0,000(15,9);-0,008(0,5) |
| Beispiel 1-021: ¹H-NMR(400,0 MHz, d₆-DMSO): δ= 10,596(3,7);8,839(5,4);8,695(2,2);8,692(2,3);8,684(2,3);8,681(2,2);8,530(5,7);8,3 18(3,5);8,314(3,4);8,162(2,0);8,159(2,1);8,141(2,3);8,138(2,2);7,699(2,0);7,687(2,0);7,678(1,9);7,666(2,3);7,643(4,7);7,624(2,8);7,62 0(2,6);7,603(1,0);7,598(1,0);3,827(16,0);3,327(95,8);2,676(0,5);2,671(0,7);2,667(0,5);2,542(2,1);2,511(42,1);2,507(81,7);2,502(106,3 );2,498(78,3);2,494(38,9);2,334(0,5);2,329(0,7);2,325(0,5);1,234(0,6);0,008(0,3);0,000(8,8);-0,008(0,4) |
| Beispiel 1-022: ¹H-NMR(601,6 MHz, d₆-DMSO): δ= 19,972(0,5);10,243(4,5);8,814(6,1);8,553(6,6);8,320(0,4);8,108(2,4);8,098(2,8);8, 093(2,9);8,084(2,4);7,909(3,4);7,906(3,5);7,678(1,2);7,664(2,6);7,661(2,7);7,642(4,9);7,628(2,3);7,415(2,6);7,400(5,0);7,386(2,5);3,8 31(16,0);3,339(61,6);2,615(0,8);2,521(1,5);2,518(1,5);2,506(111,4);2,503(152,4);2,500(114,0);2,387(0,8);1,397(1,7);0,096(0,5);0,000 (96,7);-0,100(0,4) |
| Beispiel 1-023: ¹H-NMR(601,6 MHz, d₆-DMSO): δ= 10,198(4,7);8,819(6,0);8,557(6,4);8,030(4,2);8,018(4,4);7,927(3,6);7,924(3,7);7,6 76(1,2);7,662(2,8);7,659(2,9);7,642(5,3);7,629(4,4);7,616(1,7);7,573(3,1);7,560(4,8);7,548(2,1);3,834(16,0);3,342(46,2);2,615(0,3);2, 503(61,3);2,388(0,4);1,398(2,4);0,000(31,9) |
| Beispiel 1-024: ¹H-NMR(601,6 MHz, d₆-DMSO): δ= 10,544(4,4);8,833(4,5);8,823(10,5);8,561(6,8);7,932(3,6);7,929(3,8);7,922(3,7);7, 913(3,5);7,711(1,4);7,707(1,2);7,697(2,5);7,693(2,5);7,666(4,6);7,652(2,5);5,762(0,5);3,835(16,0);3,344(18,4);2,507(24,0);2,505(31,9 );2,502(23,2);0,000(24,7);-0,006(0,9) |
| Beispiel 1-026: ¹H-NMR(601,6 MHz, d₆-DMSO): δ= 10,237(4,1);8,744(6,0);8,495(6,7);8,322(0,5);8,014(3,7);8,002(4,1);8,000(3,3);7,9 18(1,5);7,914(1,6);7,906(1,6);7,902(1,5);7,637(1,8);7,625(3,0);7,615(1,7);7,613(1,9);7,567(3,1);7,554(4,7);7,542(2,0);7,412(1,5);7,39 8(1,7);7,395(1,8);7,381(1,4);3,830(16,0);3,341(59,7);2,615(0,4);2,524(0,7);2,521(0,8);2,518(0,8);2,509(25,0);2,506(54,8);2,503(75,7); 2,500(54,7);2,497(25,3);2,388(0,4);1,990(0,6);1,398(4,8);0,005(1,8);0,000(54,9);-0,006(2,0) |
| Beispiel 1-027: ¹H-NMR(601,6 MHz, d₆-DMSO): δ= 10,274(2,4);8,743(3,4);8,494(3,8);8,099(1,4);8,096(0,7);8,090(1,6);8,084(1,6);8,0 79(0,7);8,075(1,5);7,913(0,9);7,910(0,9);7,901(0,9);7,898(0,9);7,646(0,5);7,642(0,5);7,638(0,6);7,634(0,5);7,632(0,6);7,628(0,6);7,62 4(0,6);7,620(0,5);7,414(1,0);7,409(1,6);7,395(3,3);7,383(1,3);7,380(1,6);4,036(0,3);4,024(0,3);3,832(9,3);3,345(11,3);2,511(4,7);2,50 8(10,2);2,505(14,1);2,502(10,4);2,500(4,9);1,991(1,4);1,397(16,0);1,188(0,4);1,176(0,7);1,164(0,4);0,005(0,4);0,000(12,4);-0,006(0,5) |
| Beispiel 1-028: ¹H-NMR(400,0 MHz, d₆-DMSO): δ= 10,738(3,6);8,705(6,0);8,559(2,1);8,554(2,4);8,547(2,4);8,542(2,4);8,413(6,5);8,1 11(2,1);8,106(2,3);8,092(2,5);8,088(2,4);8,032(3,5);7,599(3,4);7,586(3,4);7,580(3,9);7,568(2,3);7,493(1,1);7,489(0,8);7,474(3,2);7,46 2(2,8);7,443(2,8);7,424(0,9);5,757(5,2);4,056(0,4);4,038(1,2);4,021(1,2);4,003(0,4);3,822(16,0);3,327(46,4);2,525(0,7);2,511(17,8);2, 507(37,1);2,503(49,9);2,498(37,4);2,494(19,1);1,989(5,2);1,193(1,4);1,175(2,8);1,158(1,4);0,008(0,9);0,000(26,5);-0,008(1,1) |
| Beispiel 1-029: ¹H-NMR(400,0 MHz, d₆-DMSO): δ= 10,082(3,5);8,687(6,3);8,648(0,4);8,414(6,8);7,880(3,6);7,527(1,5);7,507(2,0);7,4 76(1,5);7,457(2,7);7,421(2,6);7,402(3,1);7,382(1,1);4,515(0,3);3,823(16,0);3,333(30,2);2,953(0,3);2,514(30,0);2,510(40,2);2,505(30,8 );2,318(0,4);1,996(0,5);1,706(1,7);1,696(14,9);1,686(2,0);1,673(0,5);1,664(0,4);1,405(7,0);1,183(0,4) |
| Beispiel 1-030: ¹H-NMR(400,0 MHz, d₆-DMSO): δ= 9,954(1,2);8,679(2,4);8,411(2,6);7,939(0,8);7,935(1,3);7,931(0,9);7,584(0,6);7,58 0(0,5);7,564(0,7);7,563(0,7);7,560(0,6);7,472(0,3);7,469(0,6);7,466(0,4);7,453(0,7);7,450(1,0);7,447(0,7);7,413(1,0);7,394(1,3);7,374( 0,5);3,817(6,0);3,325(6,9);2,512(4,0);2,508(8,4);2,503(11,4);2,498(8,3);2,494(4,1);1,989(0,6);1,625(0,8);1,612(1,9);1,604(1,9);1,592( 1,0);1,398(16,0);1,385(2,1);1,377(2,1);1,363(0,8);1,176(0,3) |
| Beispiel 1-031: ¹H-NMR(400,0 MHz, d₆-DMSO): δ= 10,882(3,2);8,690(6,1);8,433(6,3);8,431(6,5);8,4143(6,5);8,4137(6,5);8,131(3,1);7 ,760(1,4);7,740(1,6);7,574(6,4);7,572(6,4);7,499(1,3);7,496(0,9);7,480(2,7);7,456(2,4);7,437(3,1);7,417(1,2);5,756(6,1);3,867(0,4);3,8 27(16,0);3,810(1,8);3,325(29,9);2,923(1,1);2,870(1,1);2,676(0,4);2,672(0,5);2,668(0,7);2,525(1,3);2,520(1,9);2,512(26,8);2,507(55,1); 2,503(73,4);2,498(53,5);2,494(25,9);2,330(0,5);2,325(0,3);1,259(0,4);1,235(1,2);0,146(0,5);0,008(3,3);0,000(99,8);-0,009(3,6);-0,150(0,5) |
| Beispiel 1-032: ¹H-NMR(400,0 MHz, d₆-DMSO): δ= 9,871(3,6);8,726(5,5);8,479(6,2);7,808(1,5);7,802(1,6);7,790(1,5);7,784(1,5);7,64 3(0,8);7,638(0,8);7,632(0,9);7,626(1,0);7,622(1,1);7,616(1,0);7,610(1,0);7,605(0,9);7,385(1,6);7,363(1,7);7,360(1,9);7,338(1,4);3,821( 14,4);3,327(10,1);2,511(13,7);2,507(26,4);2,503(34,2);2,498(25,6);1,612(1,6);1,598(4,1);1,590(4,5);1,578(2,1);1,425(2,3);1,413(4,7); 1,405(5,4);1,398(16,0);0,008(1,0);0,000(20,7);-0,008(0,9) |
| Beispiel 1-033: ¹H-NMR(400,0 MHz, d₆-DMSO): δ= 11,154(3,7);8,771(6,2);8,518(6,9);7,768(1,4);7,763(2,1);7,750(2,4);7,745(2,8);7,7 38(1,5);7,729(1,2);7,723(1,0);7,717(1,2);7,712(0,8);7,463(1,8);7,441(1,8);7,438(2,0);7,416(1,5);7,007(0,6);6,892(0,6);6,878(1,3);6,86 5(0,6);6,750(0,7);6,736(0,3);3,826(16,0);3,329(14,5);2,526(0,5);2,512(12,5);2,508(25,6);2,503(34,2);2,499(25,3);2,494(12,6);1,990(0, 9);1,398(2,9);1,176(0,5);0,008(0,7);0,000(21,6);-0,008(0,8) |
| Beispiel 1-034: ¹H-NMR(400,0 MHz, d₆-DMSO): δ= 10,655(3,0);9,399(6,1);9,299(11,6);8,739(5,9);8,480(6,4);8,015(1,7);8,010(1,8);7, 997(1,8);7,992(1,8);7,671(0,9);7,666(1,0);7,660(1,1);7,654(1,2);7,650(1,3);7,644(1,3);7,639(1,2);7,633(1,0);7,456(1,6);7,431(2,1);7,4 09(1,4);3,827(16,0);3,325(69,7);2,671(1,3);2,506(154,5);2,502(193,0);2,498(152,5);2,329(1,3);1,989(1,2);1,192(0,3);1,175(0,6);1,157 (0,4);0,146(1,4);0,000(263,3);-0,150(1,4) |
| Beispiel 1-035: ¹H-NMR(400,0 MHz, d₆-DMSO): δ= 9,802(3,0);8,729(5,0);8,475(5,6);8,330(4,9);8,024(5,1);7,927(1,3);7,922(1,4);7,90 9(1,4);7,903(1,4);7,597(0,7);7,591(0,7);7,585(0,8);7,579(0,8);7,576(0,9);7,570(0,9);7,564(0,9);7,558(0,7);7,391(1,5);7,370(1,4);7,365( 1,7);7,344(1,2);3,902(16,0);3,825(13,1);3,335(17,0);3,333(16,5);3,328(16,0);2,525(0,9);2,511(17,6);2,507(35,3);2,503(46,5);2,498(34 |
| ,0);2,494(16,8);0,146(0,4);0,008(3,3);0,000(89,2);-0,009(3,5);-0,150(0,4) |
| Beispiel 1-036: ¹H-NMR(400,0 MHz, d₆-DMSO): δ= 10,643(3,0);9,465(4,3);9,462(4,4);9,183(4,5);9,170(4,6);8,751(6,1);8,481(6,8);8,2 15(1,5);8,209(1,6);8,196(1,6);8,191(1,6);8,168(2,6);8,165(2,7);8,155(2,6);8,152(2,6);7,652(0,8);7,646(0,9);7,640(1,0);7,634(1,0);7,63 0(1,1);7,624(1,1);7,618(1,1);7,613(1,0);7,468(1,8);7,447(1,7);7,442(2,1);7,421(1,5);3,830(16,0);3,326(34,1);2,672(0,5);2,667(0,3);2,5 25(1,3);2,512(25,2);2,507(51,5);2,503(68,6);2,498(50,4);2,494(24,8);2,329(0,4);1,989(0,5);1,398(2,8);0,008(1,4);0,000(40,9);-0,009(1,5) |
| Beispiel 1-037: ¹H-NMR(400,0 MHz, d₆-DMSO): δ= 8,701(5,6);8,482(6,5);7,941(1,6);7,936(1,7);7,923(1,7);7,917(1,7);7,615(0,7);7,60 9(0,8);7,603(0,8);7,597(0,9);7,594(0,9);7,588(0,9);7,582(0,9);7,577(0,7);7,407(1,6);7,391(1,2);7,258(0,8);7,234(1,3);7,211(0,8);7,130( 1,1);7,108(1,9);7,087(1,0);5,758(1,8);3,820(16,0);3,367(18,3);3,327(20,9);2,672(0,4);2,525(1,0);2,520(1,5);2,512(22,7);2,507(46,1);2, 503(60,8);2,498(44,6);2,494(22,1);2,330(0,4);1,989(0,6);1,398(0,5);0,000(8,7) |
| Beispiel 1-038: ¹H-NMR(400,0 MHz, d₆-DMSO): δ= 10,019(3,9);8,712(6,2);8,473(6,9);8,099(2,4);8,094(1,1);8,085(2,7);8,077(2,9);8,0 68(1,1);8,063(2,5);7,656(3,2);7,653(3,5);7,544(1,7);7,540(1,6);7,525(2,0);7,520(2,0);7,404(2,9);7,399(1,0);7,382(5,7);7,359(5,3);7,33 8(2,4);3,824(16,0);3,324(93,0);2,675(0,8);2,671(1,2);2,666(0,9);2,524(2,8);2,519(4,3);2,511(65,7);2,506(136,0);2,502(182,1);2,497(1 34,5);2,493(67,2);2,337(0,4);2,333(0,9);2,329(1,2);2,324(0,9);2,249(14,0);1,398(9,9);0,146(0,8);0,008(5,8);0,000(177,8);-0,008(6,8);-0,150(0,8) |
| Beispiel 1-039: ¹H-NMR(400,0 MHz, d₆-DMSO): δ= 10,293(4,1);8,741(2,9);8,736(3,1);8,533(5,6);8,316(2,6);8,110(2,6);8,097(3,1);8,0 88(3,2);8,075(2,8);7,986(0,9);7,963(0,5);7,955(2,9);7,950(1,2);7,941(3,1);7,933(3,4);7,925(1,3);7,919(3,1);7,911(0,5);7,904(0,7);7,88 2(1,1);7,867(1,1);7,846(0,6);7,430(2,6);7,408(5,5);7,395(1,9);7,385(3,6);7,379(2,2);7,355(2,0);7,331(0,9);7,306(0,4);7,299(3,1);7,294( 1,0);7,282(1,1);7,277(5,9);7,260(0,9);7,255(3,0);4,037(0,7);4,019(0,7);3,813(16,0);3,323(533,1);2,675(5,6);2,671(8,0);2,666(6,0);2,52 4(19,7);2,510(444,0);2,506(919,6);2,502(1233,6);2,497(916,3);2,493(458,2);2,333(5,7);2,328(8,0);2,324(6,1);1,989(2,9);1,234(0,6);1, 193(0,8);1,175(1,6);1,157(0,8);1,148(0,8);0,146(5,4);0,008(37,2);0,000(1198,0);-0,008(45,3);-0,065(0,5);-0,074(0,4);-0,150(5,7) |
| Beispiel 1-040: ¹H-NMR(400,0 MHz, d₆-DMSO): δ= 10,284(3,7);8,816(4,2);8,802(4,6);8,721(5,8);8,480(6,2);7,916(3,8);7,901(3,8);7,6 81(3,2);7,571(1,6);7,568(1,6);7,552(2,0);7,548(2,0);7,378(2,8);7,358(2,3);5,757(5,0);4,056(0,9);4,038(2,7);4,021(2,7);4,003(1,0);3,82 6(16,0);3,329(20,0);2,507(32,6);2,503(43,0);2,498(32,7);2,260(14,1);1,989(11,4);1,398(0,4);1,193(3,0);1,175(5,8);1,158(2,9);0,008(1, 6);0,000(43,4);-0,008(2,0) |
| Beispiel 1-041: ¹H-NMR(400,0 MHz, d₆-DMSO): δ= 10,049(4,0);8,736(6,2);8,485(6,8);8,393(2,5);8,390(2,7);8,386(2,8);8,382(2,6);8,3 17(0,4);7,906(1,5);7,900(1,7);7,887(1,6);7,882(1,6);7,686(1,7);7,679(1,7);7,674(3,3);7,666(3,3);7,647(3,3);7,644(3,4);7,635(2,6);7,63 1(2,2);7,623(1,0);7,617(1,0);7,613(1,1);7,607(1,1);7,601(1,0);7,596(0,9);7,411(1,8);7,390(1,7);7,386(2,1);7,364(1,5);3,826(16,0);3,32 5(124,6);2,675(0,9);2,671(1,3);2,666(0,9);2,524(3,2);2,519(4,8);2,511(70,9);2,506(146,7);2,502(195,5);2,497(142,8);2,493(70,0);2,33 3(0,9);2,329(1,3);2,324(0,9);2,320(0,5);1,398(7,1);0,146(0,9);0,008(6,6);0,000(202,2);-0,009(7,3);-0,150(0,9) |
| Beispiel 1-085: ¹H-NMR(400,0 MHz, d₆-DMSO): δ= 10,462(4,1);9,152(3,0);9,147(3,0);8,802(2,1);8,798(2,3);8,790(2,3);8,786(2,3);8,7 40(6,0);8,489(6,6);8,350(1,1);8,345(1,7);8,340(1,2);8,330(1,2);8,325(1,8);8,320(1,2);7,958(1,6);7,952(1,7);7,940(1,7);7,934(1,7);7,66 4(0,9);7,658(0,9);7,652(1,0);7,646(1,0);7,643(1,1);7,637(1,2);7,631(1,1);7,625(1,0);7,612(1,6);7,600(1,6);7,592(1,6);7,580(1,5);7,437( 1,7);7,415(1,8);7,412(2,1);7,390(1,5);3,828(16,0);3,331(246,9);2,676(0,6);2,671(0,8);2,667(0,6);2,525(2,1);2,507(93,6);2,502(123,0); 2,498(92,8);2,334(0,6);2,329(0,8);2,325(0,6);1,989(1,2);1,193(0,3);1,175(0,7);1,157(0,3);0,008(0,4);0,000(11,8);-0,008(0,5) |
| Beispiel 1-086: ¹H-NMR(400,0 MHz, d₆-DMSO): δ= 10,550(3,0);9,033(3,4);8,843(3,5);8,836(3,6);8,739(6,1);8,534(0,5);8,485(6,7);8,2 88(0,6);8,255(1,1);8,250(1,3);8,248(1,4);8,244(1,1);8,231(1,1);8,227(1,4);8,225(1,3);8,220(1,1);7,963(1,6);7,958(1,7);7,945(1,7);7,94 0(1,7);7,675(0,9);7,669(0,9);7,664(1,0);7,658(1,0);7,654(1,1);7,648(1,1);7,642(1,1);7,637(0,9);7,449(1,8);7,427(1,8);7,424(2,1);7,402( 1,5);5,225(0,4);4,038(0,5);4,020(0,5);3,828(16,0);3,803(1,5);3,324(112,5);2,676(0,8);2,671(1,1);2,667(0,8);2,524(3,0);2,511(60,6);2,5 07(121,7);2,502(160,0);2,497(116,9);2,493(57,3);2,338(0,4);2,333(0,8);2,329(1,0);2,324(0,8);1,989(1,9);1,398(0,5);1,193(0,5);1,175( 1,1);1,157(0,5);0,146(0,7);0,008(5,3);0,000(155,6);-0,009(5,8);-0,150(0,7) |
| Beispiel 2-001: ¹H-NMR(400,0 MHz, DMSO): δ= 10,386(2,6); 8,926(6,2); 8,752(4,4); 8,746(4,5); 8,650(7,0); 8,404(4,6); 8,398(4,5); 8,316(0,3); 8,124(2,7); 8,119(1,1); 8,110(3,0); 8,102(3,1); 8,094(1,2); 8,088(2,9); 7,442(3,0); 7,437(0,9); 7,420(5,7); 7,403(0,9); 7,398(2,8); 3,840(16,0); 3,325(59,1); 2,676(0,4); 2,672(0,5); 2,667(0,4); 2,525(1,3); 2,520(2,1); 2,511(30,6); 2,507(62,7); 2,502(83,3); 2,498(60,2); 2,493(28,6); 2,334(0,4); 2,329(0,5); 2,325(0,4); 1,989(1,1); 1,398(3,1); 1,175(0,6); 0,146(0,3); 0,008(2,8); 0,000(83,9); -0,009(2,7); -0,150(0,4) |
| Beispiel 2-002: ¹H-NMR(400,0 MHz, DMSO): δ = 10,706(3,5); 8,972(4,6); 8,714(4,1); 8,710(4,5); 8,703(2,4); 8,700(2,3); 8,693(3,9); 8,688(2,5); 8,651(5,0); 8,316(1,7); 8,176(1,7); 8,158(1,8); 8,155(1,8); 7,721(1,5); 7,709(1,5); 7,700(1,4); 7,689(1,3); 4,038(0,3); 4,020(0,4); 3,837(16,0); 3,323(410,7); 2,680(1,2); 2,675(2,6); 2,671(3,7); 2,666(2,6); 2,661(1,2); 2,524(8,8); 2,519(14,0); 2,511(201,5); 2,506(413,9); 2,502(552,3); 2,497(399,1); 2,492(189,6); 2,338(1,3); 2,333(2,7); 2,328(3,7); 2,324(2,7); 2,319(1,3); 1,989(1,4); 1,398(1,6); 1,235(0,5); 1,193(0,4); 1,175(0,8); 1,157(0,4); 0,951(0,3); 0,935(0,3); 0,146(2,4); 0,008(18,0); 0,000(568,5); - 0,009(19,2); -0,022(1,0); -0,046(0,4); -0,150(2,4) |
| Beispiel 2-001: ¹H-NMR(400,0 MHz, DMSO): δ= 10,386(2,6); 8,926(6,2); 8,752(4,4); 8,746(4,5); 8,650(7,0); 8,404(4,6); 8,398(4,5); 8,316(0,3); 8,124(2,7); 8,119(1,1); 8,110(3,0); 8,102(3,1); 8,094(1,2); 8,088(2,9); 7,442(3,0); 7,437(0,9); 7,420(5,7); 7,403(0,9); 7,398(2,8); 3,840(16,0); 3,325(59,1); 2,676(0,4); 2,672(0,5); 2,667(0,4); 2,525(1,3); 2,520(2,1); 2,511(30,6); 2,507(62,7); 2,502(83,3); 2,498(60,2); 2,493(28,6); 2,334(0,4); 2,329(0,5); 2,325(0,4); 1,989(1,1); 1,398(3,1); 1,175(0,6); 0,146(0,3); 0,008(2,8); 0,000(83,9); -0,009(2,7); -0,150(0,4) |

| **Bsp.-Nr.** | **NMR Peakliste** |
|---|---|
| 1-049 | 1H-NMR (400 MHz, DMSO-d6) δ [ppm]: -0.008 (0.44), 0.008 (0.40), 3.826 (16.00), 7.233 (2.29), 7.242 (2.50), 7.245 (2.50), 7.255 (2.42), 7.372 (1.56), 7.393 (2.08), 7.397 (1.83), 7.419 (1.85), 7.607 (0.95), 7.613 (1.10), 7.619 (1.15), 7.625 (1.14), 7.629 (1.04), 7.635 (1.04), 7.640 (0.93), 7.646 (0.90), 7.879 (3.45), 7.882 (3.96), 7.892 (2.69), 7.895 (3.80), 7.901 (1.79), 8.030 (2.52), 8.033 (2.68), 8.040 (2.60), 8.043 (2.47), 8.489 (6.71), 8.739 (6.14), 10.241 (4.28). |
| 1-054 | 1H-NMR (500 MHz, CHLOROFORM-d) δ [ppm]: 0.000 (0.54), 3.059 (16.00), 3.831 (6.12), 6.714 (1.78), 6.719 (0.58), 6.728 (0.63), 6.733 (1.80), 7.157 (0.86), 7.177 (1.11), 7.183 (0.54), 7.189 (0.57), 7.193 (0.55), 7.794 (1.94), 7.798 (0.61), 7.808 (0.63), 7.812 (1.84), 7.899 (1.75), 8.037 (0.56), 8.044 (0.55), 8.135 (2.18), 8.136 (2.05), 8.768 (0.60), 8.773 (0.62), 8.783 (0.61), 8.788 (0.58). |
| 1-058 | 1H-NMR (400 MHz, DMSO-d6) δ [ppm]: -0.008 (1.75), 0.008 (1.51), 0.655 (1.51), 0.665 (4.77), 0.672 (4.84), 0.681 (1.70), 1.085 (1.51), 1.093 (4.07), 1.101 (3.87), 1.110 (1.32), 1.412 (16.00), 2.366 (0.40), 2.710 (0.41), 3.814 (13.84), 7.293 (1.38), 7.314 (1.82), 7.318 (1.53), 7.339 (1.61), 7.536 (0.84), 7.542 (0.98), 7.547 (0.98), 7.553 (0.99), 7.557 (0.89), 7.563 (0.89), 7.569 (0.80), 7.575 (0.76), 7.751 (1.51), 7.757 (1.48), 7.769 (1.53), 7.775 (1.38), 8.450 (5.54), 8.452 (5.32), 8.702 (5.35), 9.137 (3.29). |
| 1-066 | 1H-NMR (400 MHz, DMSO-d6) δ [ppm]: -0.008 (1.26), 0.008 (1.04), 1.507 (4.92), 1.523 (5.24), 1.568 (5.05), 1.585 (5.05), 3.802 (1.86), 3.817 (16.00), 5.195 (0.44), 5.211 (1.73), 5.228 (1.38), 5.245 (0.42), 5.316 (0.42), 5.333 (1.34), 5.350 (1.31), 5.754 (1.89), 7.343 (1.46), 7.364 (2.04), 7.369 (1.68), 7.390 (1.75), 7.566 (0.98), 7.572 (1.11), 7.577 (1.14), 7.584 (1.20), 7.588 (1.05), 7.593 (1.00), 7.599 (0.90), 7.605 (0.83), 7.924 (1.68), 7.930 (1.72), 7.942 (1.74), 7.948 (1.64), 8.284 (0.62), 8.452 (6.34), 8.531 (0.59), 8.715 (6.07), 9.971 (3.13). |
| 1-084 | 1H-NMR (400 MHz, DMSO-d6) δ [ppm]: -0.008 (2.04), 0.008 (1.90), 2.524 (0.51), 3.826 (16.00), 7.378 (1.61), 7.399 (2.16), 7.403 (1.81), 7.425 (1.87), 7.595 (3.76), 7.616 (4.94), 7.620 (1.44), 7.626 (1.24), 7.632 (1.16), 7.636 (1.06), 7.641 (1.08), 7.647 (0.95), 7.653 (0.91), 7.890 (1.81), 7.895 (1.81), 7.908 (1.92), 7.913 (1.98), 7.918 (2.66), 7.922 (2.69), 7.939 (2.05), 7.943 (2.38), 7.993 (4.31), 7.998 (3.89), 8.480 (6.38), 8.481 (6.54), 8.732 (6.18), 10.290 (4.27). |
| 1-025 | 1H-NMR (400 MHz, DMSO-d6) delta [ppm]: -0.149 (1.32), -0.008 (16.00), 0.008 (10.39), 0.146 (1.32), 1.148 (0.82), 2.327 (1.73), 2.366 (2.14), 2.670 (1.83), 2.710 (2.24), 3.826 (10.29), 7.394 (0.92), 7.416 (1.43), 7.441 (1.12), 7.888 (3.16), 7.903 (3.16), 7.918 (1.22), 7.936 (1.22), 8.488 (3.97), 8.739 (3.67), 8.807 (3.36), 8.822 (3.36), 10.527 (2.75). |
| 1-087 | 1H-NMR (400 MHz, DMSO-d6) δ [ppm]: -0.008 (2.82), 0.008 (2.63), 2.327 (0.43), 2.366 (0.62), 2.523 (1.06), 2.584 (16.00), 2.670 (0.43), 2.710 (0.65), 3.285 (0.60), 3.826 (13.51), 7.387 (1.30), 7.409 (1.76), 7.413 (1.55), 7.434 (1.55), 7.629 (0.81), 7.634 (0.95), 7.640 (0.95), 7.646 (0.95), 7.650 (0.89), 7.656 (0.87), 7.661 (0.81), 7.667 (0.87), 7.678 (1.49), 7.680 (1.44), 7.693 (1.49), 7.764 (2.63), 7.766 (2.98), 7.899 (1.38), 7.905 (1.44), 7.917 (1.44), 7.923 (1.36), 8.482 (5.32), 8.484 (5.48), 8.656 (2.31), 8.667 (2.20), 8.735 (5.23), 10.458 (3.58). |
| 1-088 | 1H-NMR (400 MHz, DMSO-d6) δ [ppm]: -0.008 (2.53), 0.008 (2.23), 3.825 (16.00), 5.754 (1.02), 7.387 (1.48), 7.408 (2.02), 7.413 (1.78), 7.434 (1.74), 7.627 (0.95), 7.632 (1.09), 7.638 (1.13), 7.644 (1.11), 7.648 (1.02), 7.654 (1.01), 7.660 (0.91), 7.665 (0.86), 7.914 (1.69), 7.920 (1.73), 7.932 (1.76), 7.938 (1.62), 8.039 (4.08), 8.044 (1.66), 8.056 (2.11), 8.061 (6.58), 8.135 (6.02), 8.140 (2.08), 8.152 (1.73), 8.157 (3.86), 8.481 (6.11), 8.483 (6.43), 8.733 (6.06), 10.494 (4.24). |
| 1-089 | 1H-NMR (400 MHz, DMSO-d6) δ [ppm]: -0.008 (3.67), 0.008 (2.13), 2.366 (0.64), 2.670 (0.45), 2.711 (0.73), 3.826 (16.00), 7.404 (1.54), 7.425 (2.07), 7.430 (1.79), 7.451 (1.74), 7.645 (1.15), 7.651 (1.26), 7.657 (1.35), 7.662 (1.40), 7.666 (1.37), 7.673 (2.38), 7.678 (3.95), 7.684 (2.52), 7.854 (1.20), 7.859 (1.71), 7.871 (1.74), 7.928 (1.79), 7.934 (1.77), 7.946 (1.79), 7.952 (1.63), 8.475 (2.55), 8.477 (3.45), 8.481 (6.50), 8.483 (6.73), 8.490 (2.86), 8.736 (6.02), 10.616 (3.67). |
| 1-090 | 1H-NMR (400 MHz, DMSO-d6) δ [ppm]: -0.008 (3.86), 0.008 (2.81), 1.235 (0.39), 2.306 (12.07), 2.310 (16.00), 2.312 (14.43), 2.327 (0.82), 2.366 (0.85), 2.669 (0.52), 2.711 (0.85), 3.825 (12.34), 7.291 (1.90), 7.311 (2.06), 7.353 (1.24), 7.374 (1.67), 7.378 (1.44), 7.399 (1.41), 7.586 (0.82), 7.592 (0.92), 7.597 (0.92), 7.603 (0.92), 7.607 (0.88), 7.613 (0.82), 7.618 (0.75), 7.624 (0.69), 7.724 (1.28), 7.729 (1.47), 7.749 (1.34), 7.797 (2.39), 7.801 (2.45), 7.876 (1.37), 7.882 (1.37), 7.895 (1.37), 7.901 (1.28), 8.477 (4.38), 8.479 (5.01), 8.729 (4.65), 10.065 (3.24). |
| 1-091 | 1H-NMR (400 MHz, DMSO-d6) δ [ppm]: -0.149 (0.72), -0.008 (6.34), 0.008 (5.67), 0.146 (0.77), 2.321 (9.47), 2.327 (9.39), 2.367 (0.91), 2.710 (0.94), 3.826 (16.00), 7.292 (1.51), 7.315 (2.45), 7.338 (1.66), 7.364 (1.39), 7.385 (1.99), 7.410 (1.68), 7.599 (0.89), 7.638 (0.77), 7.874 (2.26), 7.881 (2.40), 7.893 (2.64), 7.898 (2.45), 7.953 (1.47), 7.972 (1.51), 8.479 (6.73), 8.728 (6.05), 10.183 (4.04). |
| 1-092 | 1H-NMR (400 MHz, DMSO-d6) δ [ppm]: -0.008 (1.55), 0.008 (1.36), 2.325 (9.00), 2.330 (8.62), 2.367 (0.78), 2.670 (0.45), 2.710 (0.78), 3.280 (0.52), 3.364 (0.65), 3.825 (16.00), 7.368 (1.43), 7.390 (2.01), 7.394 (1.81), 7.415 (1.75), 7.458 (1.04), 7.479 (2.20), 7.499 (1.10), 7.607 (0.84), 7.614 (1.10), 7.619 (1.10), 7.625 (1.10), 7.629 (1.04), 7.635 (0.97), 7.641 (0.91), 7.647 (0.84), 7.742 (1.55), 7.746 (1.88), 7.764 (2.07), 7.768 (4.08), 7.774 (1.55), 7.784 (2.14), 7.788 (1.55), 7.881 (1.75), 7.886 (1.81), 7.899 (1.81), 7.905 (1.68), 8.482 (6.67), 8.731 (6.02), 10.228 (4.02). |
| 1-093 | 1H-NMR (400 MHz, DMSO-d6) δ [ppm]: -0.008 (2.08), 0.008 (2.13), 2.366 (0.66), 2.710 (0.64), 3.287 (0.49), 3.824 (16.00), 6.143 (15.89), 7.061 (3.92), 7.081 (4.21), 7.351 (1.45), 7.373 (2.02), 7.377 (1.77), 7.398 (1.75), 7.526 (3.98), 7.531 (4.49), 7.586 (0.92), 7.592 (1.09), 7.598 (1.19), 7.603 (1.32), 7.607 (3.45), 7.612 (3.00), 7.619 (1.09), 7.628 (2.53), 7.632 (2.23), 7.864 (1.68), 7.870 (1.74), 7.882 (1.74), 7.888 (1.64), 8.477 (6.81), 8.726 (6.11), 10.027 (4.21). |
| 1-094 | 1H-NMR (400 MHz, DMSO-d6) δ [ppm]: -0.008 (0.52), 0.008 (0.42), 2.074 (0.74), 3.314 (15.90), 3.939 (16.00), 7.370 (1.25), 7.378 (1.11), 7.392 (1.58), 7.399 (2.48), 7.403 (1.28), 7.419 (1.55), 7.425 (1.29), 7.616 (0.73), 7.621 (1.33), 7.626 (1.41), 7.632 (1.36), 7.634 (1.16), 7.637 (1.46), 7.642 (1.21), 7.647 (1.16), 7.653 (1.03), 7.655 (0.99), 7.658 (0.74), 7.766 (1.21), 7.772 (1.16), 7.787 (1.24), 7.792 (1.14), 7.878 (1.12), 7.883 (1.12), 7.896 (1.15), 7.901 (1.08), 8.488 (4.32), 8.490 (4.24), 8.739 (4.10), 10.236 (2.82). |
| 1-095 | 1H-NMR (400 MHz, DMSO-d6) δ [ppm]: -0.008 (2.20), 0.008 (1.66), 2.367 (0.53), 2.524 (1.83), 2.710 (0.49), 3.825 (11.09), 3.937 (16.00), 7.316 (1.12), 7.337 (1.90), 7.359 (1.13), 7.363 (1.40), 7.384 (1.47), 7.388 (1.27), 7.410 (1.24), 7.599 (0.72), 7.605 (0.81), 7.611 (0.83), 7.617 (0.81), 7.621 (0.76), 7.626 (0.73), 7.632 (0.65), 7.638 (0.61), 7.840 (1.29), 7.846 (1.58), 7.872 (4.89), 7.877 (2.98), 7.890 (2.09), 7.896 (1.96), 7.899 (1.07), 8.480 (4.03), 8.481 (4.57), 8.730 (4.22), 10.140 (3.04). |
| 1-096 | 1H-NMR (400 MHz, DMSO-d6) δ [ppm]: -0.008 (1.12), 0.008 (1.08), 3.826 (16.00), 7.384 (1.49), 7.405 (2.04), 7.409 (1.79), 7.431 (1.75), 7.624 (0.94), 7.630 (1.11), 7.635 (1.14), 7.641 (1.12), 7.645 (1.04), 7.651 (1.02), 7.657 (0.92), 7.663 (0.88), 7.794 (1.35), 7.812 (1.84), 7.815 (2.57), 7.834 (2.38), 7.870 (2.38), 7.875 (2.36), 7.892 (2.61), 7.898 (2.24), 7.911 (1.76), 7.916 (1.64), 7.989 (1.95), 7.994 (1.81), 8.015 (1.92), 8.019 (1.80), 8.482 (6.69), 8.733 (6.04), 10.390 (4.26). |
| 1-097 | 1H-NMR (400 MHz, DMSO-d6) δ [ppm]: -0.008 (1.89), 0.008 (1.76), 2.228 (10.19), 3.827 (11.35), 3.881 (16.00), 7.293 (1.35), 7.296 (1.44), 7.314 (1.61), 7.316 (1.76), 7.366 (1.09), 7.387 (1.48), 7.392 (1.27), 7.413 (1.27), 7.527 (1.22), 7.531 (1.95), 7.540 (2.49), 7.545 (3.50), 7.601 (0.66), 7.607 (0.77), 7.613 (0.79), 7.619 (0.79), 7.623 (0.71), 7.629 (0.71), 7.634 (0.64), 7.640 (0.60), 7.878 (1.18), 7.883 (1.20), 7.896 (1.22), 7.902 (1.14), 8.487 (4.33), 8.489 (4.78), 8.739 (4.38), 10.130 (3.02). |
| 1-098 | 1H-NMR (400 MHz, DMSO-d6) δ [ppm]: -0.008 (2.47), 0.008 (2.26), 3.825 (16.00), 7.404 (1.42), 7.426 (1.96), 7.430 (1.71), 7.452 (1.67), 7.635 (0.94), 7.641 (1.12), 7.647 (1.14), 7.652 (1.14), 7.654 (1.00), 7.656 (1.05), 7.662 (1.00), 7.668 (0.91), 7.674 (0.84), 7.965 (1.64), 7.971 (1.69), 7.983 (1.69), 7.989 (1.60), 8.245 (2.42), 8.247 (2.56), 8.265 (2.90), 8.267 (2.94), 8.475 (6.00), 8.476 (6.28), 8.535 (1.92), 8.541 (1.92), 8.555 (1.64), 8.561 (1.67), 8.732 (6.07), 9.246 (2.42), 9.248 (2.81), 9.254 (2.53), 10.697 (2.37). |
| 1-099 | 1H-NMR (400 MHz, DMSO-d6) δ [ppm]: -0.008 (0.91), 0.000 (16.00), 0.008 (0.63), 1.989 (0.60), 3.826 (4.60), 7.393 (0.44), 7.415 (0.58), 7.419 (0.52), 7.440 (0.49), 7.726 (0.78), 7.728 (0.93), 7.747 (0.80), 7.749 (0.99), 7.936 (0.50), 7.942 (0.51), 7.954 (0.50), 7.960 (0.46), 8.367 (0.62), 8.373 (0.62), 8.387 (0.56), 8.394 (0.56), 8.479 (1.66), 8.480 (1.93), 8.734 (1.75), 8.980 (0.91), 8.987 (0.82), 10.519 (1.23). |
| 1-100 | 1H-NMR (400 MHz, DMSO-d6) δ [ppm]: -0.008 (1.56), 0.008 (1.39), 3.287 (0.43), 3.771 (1.39), 3.826 (16.00), 7.380 (1.69), 7.385 (1.71), 7.392 (1.72), 7.401 (1.86), 7.407 (1.89), 7.413 (2.21), 7.417 (1.86), 7.439 (1.82), 7.625 (0.99), 7.630 (1.10), 7.636 (1.17), 7.642 (1.15), 7.646 (1.05), 7.652 (1.02), 7.658 (0.92), 7.663 (0.88), 7.931 (1.65), 7.937 (1.69), 7.949 (1.71), 7.955 (1.61), 8.479 (6.19), 8.481 (6.45), 8.507 (0.95), 8.513 (1.01), 8.526 (1.40), 8.528 (1.47), 8.535 (1.45), 8.548 (0.92), 8.554 (0.93), 8.578 (0.43), 8.734 (6.14), 8.852 (2.56), 8.859 (2.50), 10.472 (4.29). |
| 1-101 | 1H-NMR (400 MHz, DMSO-d6) δ [ppm]: -0.008 (2.59), 0.008 (1.57), 1.377 (5.35), 1.394 (11.14), 1.412 (5.24), 3.826 (16.00), 4.181 (1.68), 4.199 (5.33), 4.216 (5.20), 4.234 (1.52), 5.754 (0.67), 7.363 (1.83), 7.374 (1.77), 7.384 (2.36), 7.391 (2.29), 7.395 (2.47), 7.399 (1.96), 7.412 (2.18), 7.421 (1.88), 7.608 (1.39), 7.611 (1.89), 7.613 (2.08), 7.618 (2.22), 7.624 (2.26), 7.629 (2.21), 7.633 (1.93), 7.639 (1.80), 7.645 (1.72), 7.650 (0.95), 7.749 (1.85), 7.755 (1.70), 7.770 (1.80), 7.775 (1.63), 7.872 (1.77), 7.878 (1.73), 7.890 (1.78), 7.896 (1.62), 8.486 (6.84), 8.736 (6.18), 10.216 (4.27). |
| 1-106 | 1H-NMR (500 MHz, DICHLOROMETHANE-d2) δ [ppm]: 1.205 (0.41), 3.400 (0.91), 3.402 (0.74), 3.477 (8.64), 3.788 (1.36), 3.832 (16.00), 3.846 (1.35), 7.073 (0.56), 7.093 (0.99), 7.111 (0.58), 7.416 (1.25), 7.432 (1.90), 7.441 (1.39), 7.449 (1.18), 7.459 (0.92), 7.463 (0.70), 7.584 (0.87), 7.601 (0.92), 7.848 (1.01), 7.855 (1.40), 7.875 (4.32), 7.890 (0.44), 8.063 (3.69), 8.622 (1.14). |
| 1-127 | 1H-NMR (400 MHz, DMSO-d6) δ [ppm]: -0.008 (1.49), 0.008 (1.07), 2.524 (0.76), 2.937 (12.88), 3.057 (16.00), 3.815 (10.31), 7.395 (0.71), 7.414 (2.03), 7.433 (1.83), 7.450 (1.07), 7.454 (1.92), 7.458 (1.22), 7.469 (0.58), 7.473 (0.84), 7.477 (0.45), 7.524 (0.99), 7.529 (1.31), 7.533 (0.90), 7.544 (0.71), 7.549 (0.94), 7.552 (0.63), 7.971 (1.43), 7.976 (2.27), 7.981 (1.29), 8.402 (4.14), 8.687 (3.86), 10.760 (2.12). |
| 1-128 | 1H-NMR (400 MHz, CHLOROFORM-d) δ [ppm]: 0.000 (1.41), 3.797 (8.35), 3.836 (12.86), 3.843 (16.00), 3.856 (3.05), 3.858 (2.78), 6.865 (1.32), 6.872 (1.95), 6.877 (2.99), 6.881 (1.58), 6.896 (3.91), 7.032 (0.59), 7.036 (0.57), 7.054 (1.24), 7.058 (1.21), 7.076 (0.68), 7.080 (0.66), 7.128 (1.23), 7.138 (1.66), 7.141 (1.54), 7.150 (1.36), 7.447 (0.52), 7.461 (0.64), 7.468 (0.90), 7.483 (0.89), 7.490 (0.59), 7.504 (0.50), 7.538 (1.99), 7.575 (1.70), 7.578 (1.58), 7.587 (1.70), 7.591 (1.47), 7.709 (2.45), 7.720 (1.83), 7.723 (1.71), 7.730 (1.82), 7.732 (1.60), 7.834 (2.81), 7.986 (2.38), 7.988 (2.65), 7.992 (2.50), 8.001 (2.17), 8.007 (1.97), 8.153 (4.22), 8.163 (3.69). |
| 1-129 | 1H-NMR (400 MHz, DMSO-d6) δ [ppm]: -0.149 (0.63), -0.008 (6.67), 0.008 (6.56), 0.146 (0.63), 1.147 (0.63), 2.328 (0.48), 2.665 (0.44), 2.670 (0.52), 3.771 (0.67), 3.800 (0.85), 3.814 (11.41), 3.937 (16.00), 5.754 (4.85), 7.339 (0.70), 7.359 (1.33), 7.362 (1.41), 7.387 (1.74), 7.408 (1.48), 7.415 (1.30), 7.436 (1.37), 7.630 (0.70), 7.635 (0.93), 7.646 (0.89), 7.651 (0.81), 7.656 (0.81), 7.667 (0.67), 7.765 (1.22), 7.771 (1.26), 7.787 (1.33), 7.792 (1.22), 7.849 (0.48), 7.863 (0.63), 7.870 (0.93), 7.885 (0.96), 7.892 (0.59), 7.906 (0.48), 8.531 (3.96), 8.737 (2.26), 8.742 (2.30), 10.272 (3.41). |
| 1-130 | 1H-NMR (400 MHz, CHLOROFORM-d) δ [ppm]: 0.000 (1.19), 1.622 (1.64), 3.799 (6.41), 3.844 (16.00), 3.856 (3.35), 3.961 (8.30), 6.866 (1.15), 6.875 (2.18), 6.878 (2.84), 6.898 (3.29), 7.012 (0.62), 7.033 (1.10), 7.043 (0.58), 7.047 (0.56), 7.049 |
| | (0.49), 7.055 (0.64), 7.065 (0.95), 7.069 (0.83), 7.087 (0.50), 7.091 (0.44), 7.464 (0.44), 7.471 (0.61), 7.484 (0.60), 7.492 (0.41), 7.533 (1.43), 7.668 (0.70), 7.674 (0.87), 7.698 (2.19), 7.703 (1.53), 7.709 (2.13), 7.717 (0.96), 7.723 (0.68), 7.725 (0.55), 7.836 (2.08), 7.986 (2.31), 7.988 (2.51), 7.991 (2.33), 8.005 (1.55), 8.008 (1.63), 8.010 (1.43), 8.153 (3.69), 8.167 (2.45). |
| 1-132 | 1H-NMR (400 MHz, DMSO-d6) δ [ppm]: -0.008 (2.68), 0.008 (2.24), 3.286 (0.41), 3.814 (16.00), 7.354 (0.96), 7.356 (0.92), 7.374 (1.74), 7.377 (1.99), 7.378 (1.93), 7.399 (2.65), 7.406 (1.80), 7.421 (1.70), 7.427 (1.70), 7.864 (0.67), 7.879 (0.84), 7.886 (1.35), 7.901 (1.30), 7.908 (0.84), 7.923 (0.66), 8.524 (1.09), 8.532 (6.02), 8.545 (1.61), 8.551 (1.52), 8.565 (0.88), 8.571 (0.88), 8.741 (3.12), 8.746 (3.05), 8.874 (2.62), 8.881 (2.61), 10.531 (4.56). |
| 1-133 | 1H-NMR (500 MHz, DICHLOROMETHANE-d2) δ [ppm]: 0.000 (0.53), 3.398 (0.43), 3.410 (16.00), 3.829 (15.10), 6.810 (1.17), 6.811 (1.23), 6.816 (1.24), 6.817 (1.19), 6.827 (1.23), 6.828 (1.30), 6.833 (1.26), 6.834 (1.17), 6.964 (0.43), 6.970 (0.75), 6.981 (0.69), 6.984 (0.98), 6.988 (1.23), 6.990 (0.81), 7.002 (0.94), 7.501 (0.66), 7.513 (0.87), 7.519 (1.05), 7.529 (0.95), 7.534 (0.91), 7.547 (0.62), 7.849 (0.70), 7.854 (0.76), 7.864 (1.05), 7.866 (1.14), 7.869 (1.14), 7.871 (1.07), 7.881 (0.71), 7.886 (0.68), 7.998 (2.52), 8.001 (2.50), 8.142 (3.92), 8.176 (1.98), 8.181 (1.88). |
| 1-134 | 1H-NMR (400 MHz, DMSO-d6) δ [ppm]: -0.008 (0.92), 0.008 (0.86), 3.804 (0.47), 3.821 (16.00), 7.311 (1.34), 7.331 (2.91), 7.351 (1.66), 7.376 (1.70), 7.382 (1.68), 7.397 (1.75), 7.404 (1.69), 7.563 (0.91), 7.567 (1.06), 7.582 (1.60), 7.584 (1.78), 7.586 (1.73), 7.601 (0.86), 7.603 (0.83), 7.605 (0.81), 7.736 (0.93), 7.741 (1.02), 7.757 (1.79), 7.773 (0.90), 7.775 (0.91), 7.778 (0.84), 8.504 (0.95), 8.510 (1.01), 8.524 (1.65), 8.530 (1.87), 8.537 (5.74), 8.545 (1.13), 8.551 (0.99), 8.749 (3.21), 8.755 (3.05), 8.849 (2.71), 8.856 (2.68), 10.470 (4.17). |
| 2-003 | 1H-NMR (400 MHz, DMSO-d6) δ [ppm]: -0.149 (0.64), -0.008 (5.69), 0.008 (5.19), 0.146 (0.67), 2.073 (2.68), 2.327 (0.74), 2.366 (1.31), 2.465 (16.00), 2.670 (0.74), 2.674 (0.53), 2.710 (1.31), 3.286 (0.71), 3.833 (15.26), 7.382 (2.65), 7.388 (0.88), 7.399 (1.13), 7.404 (5.55), 7.410 (1.20), 7.421 (0.88), 7.427 (2.93), 8.072 (3.89), 8.076 (5.33), 8.089 (3.11), 8.097 (3.00), 8.106 (1.13), 8.111 (2.65), 8.577 (5.72), 8.579 (6.53), 8.748 (3.64), 8.753 (3.57), 8.837 (5.93), 10.173 (4.06). |
| 2-004 | 1H-NMR (400 MHz, DMSO-d6) δ [ppm]: -0.149 (0.41), -0.008 (3.40), 0.008 (3.32), 0.146 (0.41), 2.366 (0.44), 2.476 (16.00), 2.710 (0.43), 3.834 (15.30), 7.906 (4.24), 7.910 (2.90), 7.917 (2.90), 7.921 (4.44), 8.103 (3.02), 8.108 (3.05), 8.583 (6.05), 8.774 (3.52), 8.780 (3.53), 8.821 (5.10), 8.825 (3.12), 8.832 (3.18), 8.836 (5.17), 8.843 (6.10), 10.439 (3.94). |
| 2-005 | 1H-NMR (400 MHz, DMSO-d6) δ [ppm]: -0.149 (0.64), -0.008 (6.72), 0.008 (5.43), 0.146 (0.55), 2.073 (0.42), 2.327 (0.82), 2.366 (1.18), 2.524 (4.01), 2.710 (0.67), 3.286 (1.88), 3.836 (16.00), 7.889 (5.94), 7.893 (3.94), 7.900 (3.85), 7.904 (6.14), 8.508 (2.06), 8.516 (7.67), 8.537 (2.13), 8.542 (1.57), 8.591 (6.58), 8.593 (6.41), 8.822 (6.10), 8.826 (3.99), 8.833 (3.68), 8.837 (5.87), 8.861 (6.20), 10.720 (3.64). |
| 2-008 | 1H-NMR (400 MHz, DMSO-d6) δ [ppm]: -0.008 (2.25), 0.008 (2.02), 2.073 (3.44), 3.286 (0.71), 3.836 (16.00), 8.273 (2.62), 8.275 (2.59), 8.293 (3.03), 8.295 (3.05), 8.439 (4.09), 8.445 (4.26), 8.557 (2.31), 8.562 (2.29), 8.577 (1.97), 8.583 (2.00), 8.635 (6.89), 8.775 (4.13), 8.781 (3.92), 8.917 (6.29), 9.267 (2.59), 9.269 (3.03), 9.273 (2.95), 9.275 (2.62), 10.839 (3.89). |

### LC-MS-Daten

### Methode M1:

Instrument: Waters ACQUITY SQD UPLC System; Säule: Waters Acquity UPLC HSS T3 1.8 µ 50 x 1 mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A Ofen: 50°C; Fluss: 0.40 ml/min; UV-Detektion: 208 - 400 nm.

### Methode M2:

Instrument: Agilent MS Quad 6150;HPLC: Agilent 1290; Säule: Waters Acquity UPLC HSS T3 1.8 µ 50 x 2.1 mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 0.3 min 90% A → 1.7 min 5% A → 3.0 min 5% A Ofen: 50°C; Fluss: 1,20 ml/min; UV-Detektion: 205 - 305 nm.

### Methode M3:

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril (ULC) + 0.025% Ameisensäure; Gradient: 0.0 min 98%A - 0.9 min 25%A - 1.0 min 5%A - 1.4 min 5%A - 1.41 min 98%A - 1.5 min 98%A; Ofen: 40°C; Fluss: 0.600 ml/min; UV-Detektion: DAD; 210 nm.

### Methode M4

Instrument: Waters ACQUITY SQD UPLC System; Säule: Waters Acquity UPLC HSS T3 1.8 µ 50 x 1 mm; Eluent A: 11 Wasser + 0.25 ml 99%ige Ameisensäure , Eluent B: 11 Acetonitril + 0.25 ml 99%ige Ameisensäure; Gradient: 0.0 min 95% A → 6.0 min 5% A → 7.5 min 5% A Ofen: 50°C; Fluss: 0.35 ml/min; UV-Detektion: 210 - 400 nm.

| **Bsp.-Nr.** | **Methode** | **Retentionszeit [min]** | **M+H** |
|---|---|---|---|
| 1-025 | M1 | 1,12 | 549 |
| 1-042 | M3 | 1,26 | 578 |
| 1-043 | M3 | 1,3 | 597 |
| 1-044 | M3 | 1,32 | 653 |
| 1-045 | M3 | 1,41 | 638 |
| 1-046 | M3 | 1,25 | 546 |
| 1-047 | M3 | 1,24 | 584 |
| 1-048 | M3 | 1,21 | 516 |
| 1-049 | M1 | 1,22 | 554 |
| 1-050 | M3 | 1,33 | 634 |
| 1-051 | M3 | 1,24 | 608 |
| 1-052 | M3 | 1,26 | 528 |
| 1-053 | M3 | 1,33 | 568 |
| 1-054 | M1 | 1,31 | 591 |
| 1-055 | M3 | 1,22 | 512 |
| 1-056 | M3 | 1,4 | 674 |
| 1-057 | M3 | 1,31 | 616 |
| 1-058 | M1 | 1,27 | 525 |
| 1-059 | M3 | 1,38 | 602 |
| 1-060 | M3 | 1,29 | 562 |
| 1-061 | M3 | 1,22 | 530 |
| 1-062 | M3 | 1,25 | 654 |
| 1-063 | M3 | 1,3 | 620 |
| 1-064 | M3 | 1,23 | 514 |
| 1-065 | M3 | 1,31 | 556 |
| 1-066 | M3 | 1,25 | 518 |
| 1-067 | M3 | 1,26 | 552 |
| 1-068 | M3 | 1,26 | 528 |
| 1-069 | M3 | 1,28 | 568 |
| 1-070 | M3 | 1,32 | 584 |
| 1-071 | M3 | 1,3 | 566 |
| 1-072 | M3 | 1,38 | 592 |
| 1-073 | M3 | 1,27 | 566 |
| 1-074 | M3 | 1,31 | 632 |
| 1-075 | M3 | 1,25 | 573 |
| 1-076 | M3 | 1,21 | 500 |
| 1-077 | M3 | 1,28 | 540 |
| 1-078 | M3 | 1,27 | 528 |
| 1-079 | M3 | 1,29 | 542 |
| 1-080 | M3 | 1,3 | 576 |
| 1-081 | M3 | 1,22 | 553 |
| 1-082 | M3 | 1,35 | 572 |
| 1-083 | M3 | 1,43 | 652 |
| 1-084 | M1 | 1,31 | 627 |
| 1-087 | M1 | 1,12 | 563 |
| 1-088 | M1 | 1,2 | 573 |
| 1-089 | M2 | 1,51 | 567 |
| 1-090 | M1 | 1,36 | 576 |
| 1-091 | M1 | 1,3 | 580 |
| 1-092 | M1 | 1,3 | 580 |
| 1-093 | M1 | 1,22 | 592 |
| 1-094 | M1 | 1,32 | 596 |
| 1-095 | M1 | 1,28 | 596 |
| 1-096 | M1 | 1,34 | 600 |
| 1-097 | M1 | 1,38 | 592 |
| 1-098 | M1 | 1,23 | 574 |
| 1-099 | M1 | 1,28 | 583 |
| 1-100 | M1 | 1,24 | 567 |
| 1-101 | M1 | 1,33 | 619 |
| 1-106 | M1 | 1,23 | 588 |
| 1-107 | M3 | 1,28 | 594 |
| 1-108 | M3 | 1,29 | 576 |
| 1-109 | M3 | 1,35 | 630 |
| 1-110 | M3 | 1,32 | 602 |
| 1-111 | M3 | 1,38 | 644 |
| 1-112 | M3 | 1,27 | 634 |
| 1-113 | M3 | 1,35 | 588 |
| 1-114 | M3 | 1,31 | 592 |
| 1-115 | M3 | 1,35 | 636 |
| 1-116 | M3 | 1,3 | 630 |
| 1-117 | M3 | 1,26 | 568 |
| 1-118 | M3 | 1,29 | 578 |
| 1-119 | M3 | 1,34 | 624 |
| 1-120 | M3 | 1,35 | 638 |
| 1-121 | M3 | 1,27 | 580 |
| 1-122 | M3 | 1,37 | 650 |
| 1-123 | M3 | 1,31 | 591 |
| 1-124 | M3 | 1,31 | 590 |
| 1-125 | M3 | 1,29 | 576 |
| 1-126 | M3 | 1,29 | 614 |
| 1-127 | M1 | 1,14 | 525 |
| 1-128 | M1 | 1,22 | 572 |
| 1-129 | M1 | 1,25 | 614 |
| 1-130 | M1 | 1,234 | 614 |
| 1-132 | M1 | 1,22 | 585 |
| 1-133 | M4 | 4,09 | 599 |
| 1-134 | M1 | 1,23 | 567 |
| 2-003 | M1 | 1,13 | 563 |
| 2-004 | M1 | 0,98 | 546 |
| 2-005 | M1 | 1,08 | 550 |
| 2-006 | M1 | 1,09 | 565 |
| 2-007 | M1 | 1,25 | 613 |
| 2-008 | M1 | 1,21 | 591 |

### Intermediate

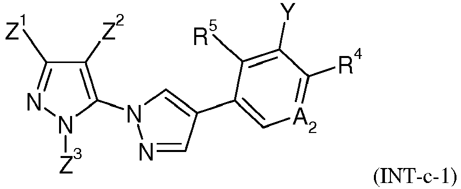

Verbindungen der Formel (INT-c-1) sind Verbindungen der allgemeinen Formel **7** (Y = NO₂) oder **8** (Y = NH₂), wobei A₁ = CH, A₃ = CR⁴, A₄ = CR⁵ und E₁ = N, E₂ = E₃ = CH sind.

| **Bsp.-Nr.** | **Z¹** | **Z²** | **Z³** | **A₂** | **R⁴** | **R⁵** | **Y** |
|---|---|---|---|---|---|---|---|
| INT-001 | CF₂CF₃ | CF₃ | CH₃ | CH | H | H | NO₂ |
| INT-002 | CF₂CF₃ | CF₃ | CH₃ | CH | H | H | NH₂ |
| INT-003 | CF₂CF₃ | CF₃ | CH₃ | CH | F | H | NO₂ |
| INT-004 | CF₂CF₃ | CF₃ | CH₃ | CH | F | H | NH₂ |
| INT-005 | CF₂CF₃ | CF₃ | CH₃ | CH | Cl | H | NO₂ |
| INT-006 | CF₂CF₃ | CF₃ | CH₃ | CH | Cl | H | NH₂ |
| INT-007 | CF₂CF₃ | CF₃ | CH₃ | CH | H | F | NO₂ |
| INT-008 | CF₂CF₃ | CF₃ | CH₃ | CH | H | F | NH₂ |
| INT-009 | CF₂CF₃ | CF₃ | CH₃ | CH | H | MeO | NO₂ |
| INT-010 | CF₂CF₃ | CF₃ | CH₃ | CH | H | MeO | NH₂ |
| INT-011 | CF₂CF₃ | CF₃ | CH₃ | N | H | H | NH₂ |
| INT-012 | CF₂CF₃ | CF₃ | CH₃ | N | Cl | H | NH₂ |
| INT-013 | CF₂CF₃ | CF₃ | CH₃ | CH | F | F | NO₂ |
| INT-014 | CF₂CF₃ | CF₃ | CH₃ | CH | F | F | NH₂ |
| INT-015 | CF₂CF₃ | CF₃ | CH₃ | N | F | H | NH₂ |
| INT-016 | CF₂CF₃ | CF₃ | CH₃ | N | CH₃ | H | NH₂ |

### Analytische Daten Intermediate

### NMR-Peak-Listen Intermediate

| |
|---|
| Beispiel INT-001: ¹H-NMR(400,0 MHz, DMSO): δ = 8,991(6,2); 8,699(6,9); 8,563(2,3); 8,559(3,6); 8,554(2,2); 8,201(1,9); 8,197(1,4); 8,181(2,0); 8,180(1,9); 8,177(1,7); 8,174(1,7); 8,171(1,6); 8,168(1,5); 8,166(1,4); 8,153(1,7); 8,151(1,5); 8,147(1,7); 8,145(1,3); 7,769(2,2); 7,749(3,6); 7,729(1,8); 3,846(16,0); 3,775(0,4); 3,328(9,6); 2,527(0,5); 2,514(9,5); 2,510(18,6); 2,505(23,9); 2,501(16,8); 2,496(7,9); 1,398(2,4); 0,008(2,4); 0,000(53,5); -0,009(1,9) |
| Beispiel INT-002: ¹H-NMR(400,0 MHz, DMSO): δ = 8,548(6,1); 8,299(6,8); 7,087(1,2); 7,068(2,8); 7,047(1,7); 6,827(5,3); 6,823(6,0); 6,806(1,9); 6,527(1,4); 6,523(2,0); 6,520(1,3); 6,505(1,5); 6,502(1,5); 5,158(2,6); 4,056(0,3); 4,038(1,1); 4,021(1,1); 4,003(0,4); 3,806(16,0); 3,327(20,5); 2,525(0,6); 2,511(13,9); 2,507(27,9); 2,503(36,3); 2,498(25,7); 2,494(12,2); 1,989(4,7); 1,398(0,4); 1,193(1,2); 1,175(2,5); 1,158(1,2); 0,008(1,0); 0,000(26,5); -0,009(0,9) |
| Beispiel INT-003: ¹H-NMR(400,0 MHz, DMSO): δ = 8,924(6,1); 8,651(6,7); 8,536(0,5); 8,530(0,5); 8,518(0,5); 8,512(0,6); 8,502(1,8); 8,496(1,9); 8,484(1,9); 8,478(1,9); 8,218(0,3); 8,180(1,0); 8,174(1,1); 8,170(1,2); 8,164(1,1); 8,158(1,2); 8,152(1,2); 8,148(1,2); 8,142(1,0); 7,778(0,4); 7,756(0,5); 7,750(0,5); 7,729(2,0); 7,708(1,8); 7,702(2,0); 7,680(1,7); 3,836(16,0); 3,733(0,9); 3,329(28,0); 2,509(25,6); 2,504(33,4); 2,500(25,5); 1,990(0,9); 1,398(14,1); 1,176(0,5); 0,000(5,0) |
| Beispiel INT-005: ¹H-NMR(400,0 MHz, DMSO): δ = 8,932(5,9); 8,656(6,2); 8,611(0,6); 8,607(0,6); 8,575(0,7); 8,463(4,1); 8,459(3,8); 8,289(0,9); 8,283(0,8); 8,235(0,8); 8,217(0,4); 8,211(0,3); 8,070(1,9); 8,065(1,7); 8,049(2,3); 8,044(2,1); 7,988(0,9); 7,963(0,6); 7,942(0,6); 7,912(0,5); 7,891(0,6); 7,873(3,7); 7,852(3,0); 7,800(0,5); 7,780(0,5); 7,767(0,4); 7,762(0,4); 6,691(0,6); 6,687(0,8); 3,833(16,0); 3,732(3,7); 3,330(49,3); 2,672(0,4); 2,503(63,4); 2,329(0,4); 1,990(1,2); 1,398(1,0); 1,193(0,3); 1,176(0,6); 1,158(0,3); |
| 0,000(2,9) |
| Beispiel INT-006: ¹H-NMR(400,0 MHz, DMSO): δ = 8,588(6,0); 8,318(6,6); 7,250(3,9); 7,230(4,3); 7,053(4,0); 7,048(4,2); 6,877(2,4); 6,872(2,3); 6,856(2,2); 6,851(2,1); 5,758(1,6); 5,427(5,0); 3,806(16,0); 3,331(66,3); 2,672(0,4); 2,525(0,9); 2,512(22,4); 2,507(45,9); 2,503(60,4); 2,498(43,4); 2,494(20,7); 2,329(0,4); 0,008(0,6); 0,000(18,7); - 0,009(0,7) |
| Beispiel INT-007: ¹H-NMR(400,0 MHz, DMSO): δ = 8,926(6,0); 8,653(6,7); 8,536(1,2); 8,530(1,3); 8,518(1,2); 8,512(1,3); 8,502(1,8); 8,497(1,9); 8,485(1,8); 8,479(1,8); 8,251(0,7); 8,244(0,7); 8,240(0,8); 8,234(0,7); 8,229(0,8); 8,222(0,8); 8,218(0,8); 8,212(0,7); 8,181(0,9); 8,175(1,0); 8,170(1,1); 8,164(1,0); 8,159(1,1); 8,153(1,1); 8,149(1,1); 8,143(0,9); 7,778(1,3); 7,756(1,2); 7,751(1,4); 7,730(2,5); 7,709(1,8); 7,703(1,9); 7,681(1,7); 3,837(16,0); 3,333(29,8); 2,527(0,7); 2,513(13,7); 2,509(27,5); 2,505(36,0); 2,500(25,7); 2,496(12,3); 1,991(0,6); 1,398(1,9); 0,000(4,9) |
| Beispiel INT-008: ¹H-NMR(400,0 MHz, DMSO): δ = 8,614(0,8); 8,537(5,9); 8,374(0,9); 8,291(6,5); 7,064(1,5); 7,043(2,0); 7,035(1,8); 7,029(2,0); 7,024(2,0); 7,014(2,1); 7,007(1,9); 7,002(1,9); 6,841(1,1); 6,835(1,1); 6,830(1,3); 6,824(1,2); 6,820(1,1); 6,814(1,0); 6,809(1,0); 6,804(0,9); 5,232(5,0); 3,835(0,5); 3,805(16,0); 3,332(43,9); 2,512(19,3); 2,508(37,2); 2,503(47,4); 2,499(33,8); 2,495(16,3); 1,990(1,0); 1,398(3,3); 1,175(0,5); 0,008(0,9); 0,000(16,5); -0,009(0,6) |
| Beispiel INT-009: ¹H-NMR(400,0 MHz, DMSO): δ = 8,823(4,1); 8,553(4,4); 8,095(1,4); 8,091(1,5); 8,075(1,6); 8,071(1,5); 7,880(1,5); 7,876(1,5); 7,860(1,7); 7,856(1,6); 7,459(1,6); 7,439(2,7); 7,419(1,4); 5,758(0,4); 3,826(10,4); 3,740(16,0); 3,331(21,6); 2,527(0,4); 2,513(9,2); 2,509(18,2); 2,504(23,4); 2,500(16,8); 2,495(8,1); 0,000(6,9) |
| Beispiel INT-012: ¹H-NMR(400,0 MHz, DMSO): δ = 8,718(3,0); 8,431(3,4); 8,430(2,9); 7,966(2,2); 7,961(2,2); 7,343(2,2); 7,337(2,1); 5,684(2,9); 3,933(2,3); 3,815(8,1); 3,329(29,3); 2,508(20,1); 2,503(24,5); 2,499(18,2); 1,990(1,1); 1,176(0,6); 1,070(16,0); 0,000(0,8) |

### LC-MS-Daten Intermediate

| **Bsp.-Nr.** | **Methode** | **Retentionszeit** | **M+H** |
|---|---|---|---|
| INT-014 | M1 | 1,20 | 462 |
| INT-015 | M1 | 1,09 | 445 |
| INT-016 | M1 | 0,80 | 441 |

### Biologische Beispiele

### Ctenocephalides felis - in-vitro Kontakttests mit adulten Katzenflöhen

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 cm² Innenoberfläche wird bei homogener Verteilung eine Flächendosis von 5 µg/cm² erreicht.

Nach Abdampfen des Lösungsmittels werden die Gläschen mit 5-10 adulten Katzenflöhen (*Ctenocephalides felis*) besetzt, mit einem gelochten Kunststoffdeckel verschlossen und liegend bei Raumtemperatur und Umgebungsfeuchte inkubiert. Nach 48 h wird die Wirksamkeit bestimmt. Hierzu werden die Gläschen aufrecht gestellt und die Flöhe auf den Boden des Gläschens geklopft. Flöhe, die unbeweglich auf dem Boden verbleiben oder sich unkoordiniert bewegen, gelten als tot bzw. angeschlagen.

Eine Substanz zeigt gute Wirkung gegen *Ctenocephalides felis,* wenn in diesem Test bei einer Aufwandmenge von 5 µg/cm² mindestens 80% Wirkung erzielt wurde. Dabei bedeutet 100% Wirkung, dass alle Flöhe angeschlagen oder tot waren. 0% Wirkung bedeutet, dass keine Flöhe geschädigt wurden.

Bei diesem Test zeigen z.B. die folgenden Verbindungen der Herstellungsbeispiele eine Wirkung von 100% bei einer Aufwandmenge von 5 µg/cm² (500g/ha): 1-001, 1-003, 1-004, 1-006, 1-010, 1-012, 1-013, 1-016, 1-022, 1-023, 1-024, 1-025, 1-026, 1-027, 1-028, 1-031, 1-034, 1-036, 1-037, 1-040, 1-041, 1-049, 1-058, 1-064, 1-066, 1-068, 1-069, 1-073, 1-076, 1-081, 1-085, 1-086, 1-087, 1-088, 1-089, 1-091, 1-094, 1-095, 1-096, 1-097, 1-098, 1-099, 1-100, 1-101, 1-127, 2-005, 2-006

Bei diesem Test zeigen z.B. die folgenden Verbindungen der Herstellungsbeispiele eine Wirkung von 80% bei einer Aufwandmenge von 5 µg/cm² (500 g/ha): 1-039, 1-060, 1-083

Bei diesem Test zeigen z.B. die folgenden Verbindungen der Herstellungsbeispiele eine Wirkung von 80% bei einer Aufwandmenge von 1 µg/cm² (100 g/ha): 1-014

### Rhipicephalus sanguineus - in-vitro Kontakttests mit Adulten der braunen Hundezecke

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 cm² Innenoberfläche wird bei homogener Verteilung eine Flächendosis von 5 µg/cm² erreicht.

Nach Abdampfen des Lösungsmittels werden die Gläschen mit 5-10 adulten Hundezecken (*Rhipicephalus sanguineus*) besetzt, mit einem gelochten Kunststoffdeckel verschlossen und liegend im Dunkeln bei Raumtemperatur und Umgebungsfeuchte inkubiert. Nach 48 h wird die Wirksamkeit bestimmt. Hierzu werden die Zecken auf den Boden des Gläschens geklopft und auf einer Wärmeplatte bei 45-50°C maximal 5 min. inkubiert. Zecken, die unbeweglich auf dem Boden verbleiben oder sich so unkoordiniert bewegen, dass sie nicht gezielt der Wärme durch nach oben klettern ausweichen können, gelten als tot bzw. angeschlagen.

Eine Substanz zeigt gute Wirkung gegen *Rhipicephalus sanguineus,* wenn in diesem Test bei einer Aufwandmenge von 5 µg/cm² mindestens 80% Wirkung erzielt wurde. Dabei bedeutet 100% Wirkung, dass alle Zecken angeschlagen oder tot waren. 0% Wirkung bedeutet, dass keine Zecken geschädigt wurden.

Bei diesem Test zeigen z.B. die folgenden Verbindungen der Herstellungsbeispiele eine Wirkung von 100% bei einer Aufwandmenge von 5 µg/cm² (500 g/ha): 1-001, 1-002, 1-003, 1-004, 1-007, 1-008, 1-009, 1-010, 1-012, 1-013, 1-014, 1-016, 1-017, 1-022, 1-023, 1-024, 1-025, 1-026, 1-027, 1-028, 1-029, 1-031, 1-032, 1-034, 1-036, 1-038, 1-040, 1-041, 1-042, 1-048, 1-049, 1-054, 1-058, 1-060, 1-064, 1-066, 1-068, 1-069, 1-073, 1-075, 1-076, 1-077, 1-078, 1-081, 1-085, 1-086, 1-088, 1-089, 1-090, 1-091, 1-092, 1-093, 1-094, 1-095, 1-096, 1-097, 1-099, 1-100, 1-101, 1-117, 1-121, 1-127, 2-001, 2-003

Bei diesem Test zeigen z.B. die folgenden Verbindungen der Herstellungsbeispiele eine Wirkung von 80% bei einer Aufwandmenge von 5 µg/cm² (500 g/ha): 1-011, 1-015, 1-030, 1-035, 1-055, 1-061, 1-070, 1-084, 1-087, 1-098, 2-006

Bei diesem Test zeigen z.B. die folgenden Verbindungen der Herstellungsbeispiele eine Wirkung von 100% bei einer Aufwandmenge von 1 µg/cm² (100 g/ha): 1-033

Bei diesem Test zeigen z.B. die folgenden Verbindungen der Herstellungsbeispiele eine Wirkung von 80% bei einer Aufwandmenge von 1 µg/cm² (100 g/ha): 1-043, 2-005, 2-007

### Amblyomma hebaraeum -Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Zeckennymphen (*Amblyomma hebraeum*) werden in perforierte Plastikbecher gesetzt und in der gewünschten Konzentration eine Minute getaucht. Die Zecken werden auf Filterpapier in eine Petrischale überführt und in einem Klimaschrank gelagert.

Nach 42 Tagen wird die Abtötung in % bestimmt. Dabei bedeutet 100 %, dass alle Zecken abgetötet wurden; 0 % bedeutet, dass keine der Zecken abgetötet wurde.

Bei diesem Test zeigen z. B. die folgenden Verbindungen der Herstellungsbeispiele eine Wirkung von 100% bei einer Aufwandmenge von 100 ppm: 1-022, 1-024, 1-025, 1-038, 1-040, 1-041, 1-088

Bei diesem Test zeigen z. B. die folgenden Verbindungen der Herstellungsbeispiele eine Wirkung von 90% bei einer Aufwandmenge von 100 ppm: 1-087

### Boophilus microplus - Diptest

| | |
|---|---|
| Testtiere: | Rinderzecken (*Boophilus microplus*) Stamm Parkhurst,SP-resistent |
| Lösungsmittel: | Dimethylsulfoxid |

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration.

Diese Wirkstoffzubereitung wird in Röhrchen pipettiert. 8-10 gesogene, adulte, weibliche Rinderzecken (*Boophilus microplus*) werden in ein weiteres Röhrchen mit Löchern überführt. Das Röhrchen wird in die Wirkstoffzubereitung getaucht wobei alle Zecken vollständig benetzt werden. Nach Ablaufen der Flüssigkeit werden die Zecken auf Filterscheiben in Kunststoffschalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 100 ppm: 1-004, 1-022, 1-024, 1-025, 1-026, 1-027, 1-038, 1-040, 1-041, 1-049, 1-060, 1-073, 1-080, 1-087, 1-088, 1-089, 1-094, 1-098, 1-099, 1-100

Bei diesem Test zeigen z.B. die folgenden Verbindungen der Herstellungsbeispiele eine Wirkung von 95% bei einer Aufwandmenge von 100 ppm: 1-001

Bei diesem Test zeigen z.B. die folgenden Verbindungen der Herstellungsbeispiele eine Wirkung von 90% bei einer Aufwandmenge von 100 ppm: 1-010, 1-042

Bei diesem Test zeigen z.B. die folgenden Verbindungen der Herstellungsbeispiele eine Wirkung von 80% bei einer Aufwandmenge von 100 ppm: 1-037, 2-001

### Boophilus microplus -Injektionstest

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 20 µg/Tier: 1-001, 1-002, 1-003, 1-004, 1-005, 1-006, 1-007, 1-008, 1-009, 1-010, 1-011, 1-012, 1-013, 1-014, 1-015, 1-016, 1-017, 1-018, 1-019, 1-020, 1-022, 1-023, 1-024, 1-025, 1-026, 1-027, 1-028, 1-029, 1-030, 1-031, 1-032, 1-033, 1-034, 1-035, 1-036, 1-037, 1-038, 1-040, 1-041, 1-042, 1-043, 1-046, 1-047, 1-048, 1-049, 1-050, 1-052, 1-054, 1-055, 1-057, 1-058, 1-059, 1-060, 1-061, 1-062, 1-063, 1-064, 1-065, 1-066, 1-067, 1-068, 1-069, 1-070, 1-071, 1-073, 1-075, 1-076, 1-077, 1-078, 1-079, 1-080, 1-081, 1-082, 1-084, 1-085, 1-086, 1-087, 1-088, 1-089, 1-091, 1-092, 1-093, 1-094, 1-095, 1-096, 1-097, 1-098, 1-099, 1-100, 1-101, 1-107, 1-108, 1-110, 1-114, 1-115, 1-116, 1-117, 1-118, 1-119, 1-121, 1-123, 1-124, 1-125, 1-126, 1-127, 2-001, 2-002, 2-003, 2-004, 2-005, 2-006, 2-007

Bei diesem Test zeigen z.B. die folgenden Verbindungen der Herstellungsbeispiele eine Wirkung von 95% bei einer Aufwandmenge von 20 µg/Tier: 1-112

Bei diesem Test zeigen z.B. die folgenden Verbindungen der Herstellungsbeispiele eine Wirkung von 90% bei einer Aufwandmenge von 20 µg/Tier: 1-074

Bei diesem Test zeigen z.B. die folgenden Verbindungen der Herstellungsbeispiele eine Wirkung von 80% bei einer Aufwandmenge von 20 µg/Tier: 1-083, 1-109, 1-111

### Ctenocephalides felis - Oraltest

| | |
|---|---|
| Lösungsmittel: | Dimethylsulfoxid |

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.

Ca. 20 nüchterne adulte Katzenflöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach 2 Tagen wird die Abtötung in % bestimmt. Dabei bedeutet 100 %, dass alle Flöhe abgetötet wurden; 0 % bedeutet, dass keiner der Flöhe abgetötet wurde.

Bei diesem Test zeigen z.B. die folgenden Verbindungen der Herstellungsbeispiele eine Wirkung von 100% bei einer Aufwandmenge von 100 ppm: 1-001, 1-002, 1-003, 1-004, 1-005, 1-006, 1-007, 1-008, 1-009, 1-010, 1-011, 1-012, 1-013, 1-014, 1-015, 1-016, 1-017, 1-019, 1-022, 1-023, 1-024, 1-025, 1-026, 1-027, 1-028, 1-029, 1-030, 1-031, 1-032, 1-033, 1-034, 1-035, 1-036, 1-037, 1-038, 1-040, 1-041, 1-042, 1-043, 1-047, 1-049, 1-055, 1-058, 1-060, 1-061, 1-062, 1-064, 1-066, 1-067, 1-068, 1-069, 1-070, 1-073, 1-075, 1-076, 1-077, 1-081, 1-084, 1-085, 1-086, 1-087, 1-088, 1-089, 1-091, 1-092, 1-093, 1-094, 1-095, 1-096, 1-097, 1-098, 1-099, 1-100, 1-101, 1-116, 1-117, 1-121, 1-126, 1-127, 2-001, 2-003, 2-004, 2-005, 2-006, 2-007

Bei diesem Test zeigen z.B. die folgenden Verbindungen der Herstellungsbeispiele eine Wirkung von 95% bei einer Aufwandmenge von 100 ppm: 1-020, 1-048, 1-057, 1-079, 1-080

Bei diesem Test zeigen z.B. die folgenden Verbindungen der Herstellungsbeispiele eine Wirkung von 90% bei einer Aufwandmenge von 100 ppm: 1-052, 1-063, 1-065, 1-071, 1-082, 1-115, 1-123

Bei diesem Test zeigen z.B. die folgenden Verbindungen der Herstellungsbeispiele eine Wirkung von 80% bei einer Aufwandmenge von 100 ppm: 1-021, 1-078

### Lucilia cuprina - Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 LI-Larven der Australischen Schafgoldfliege (*Lucilia cuprina*) werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden und 0 %, dass keine Larven abgetötet wurden.

Bei diesem Test zeigen z.B. die folgenden Verbindungen der Herstellungsbeispiele eine Wirkung von 100% bei einer Aufwandmenge von 100 ppm: 1-001, 1-002, 1-003, 1-004, 1-005, 1-006, 1-007, 1-008, 1-009, 1-010, 1-011, 1-012, 1-013, 1-014, 1-015, 1-016, 1-017, 1-019, 1-022, 1-023, 1-024, 1-025, 1-026, 1-027, 1-028, 1-029, 1-030, 1-031, 1-032, 1-033, 1-034, 1-035, 1-036, 1-037, 1-038, 1-040, 1-041, 1-042, 1-043, 1-046, 1-047, 1-049, 1-050, 1-052, 1-054, 1-055, 1-057, 1-058, 1-060, 1-062, 1-064, 1-067, 1-068, 1-069, 1-070, 1-071, 1-073, 1-075, 1-077, 1-078, 1-079, 1-081, 1-084, 1-085, 1-086, 1-087, 1-088, 1-089, 1-091, 1-092, 1-093, 1-094, 1-095, 1-096, 1-097, 1-098, 1-099, 1-100, 1-101, 1-116, 1-117, 1-119, 1-121, 1-126, 1-127, 2-001, 2-003, 2-004, 2-005, 2-006, 2-007

Bei diesem Test zeigen z.B. die folgenden Verbindungen der Herstellungsbeispiele eine Wirkung von 95% bei einer Aufwandmenge von 100 ppm: 1-048, 1-076

Bei diesem Test zeigen z.B. die folgenden Verbindungen der Herstellungsbeispiele eine Wirkung von 90% bei einer Aufwandmenge von 100 ppm: 1-020 Bei diesem Test zeigen z.B. die folgenden Verbindungen der Herstellungsbeispiele eine Wirkung von 80% bei einer Aufwandmenge von 100 ppm: 1-065, 1-066, 1-115

### Musca domestica-Test

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit Zuckerlösung und der Wirkstoffzubereitung der gewünschten Konzentration behandelt wurde, werden mit 10 adulten Stubenfliegen *(Musca domestica)* besetzt.

Nach 2 Tagen wird die Abtötung in % bestimmt. Dabei bedeutet 100 %, dass alle Fliegen abgetötet wurden; 0 % bedeutet, dass keine der Fliegen abgetötet wurde.

Bei diesem Test zeigen z.B. die folgenden Verbindungen der Herstellungsbeispiele eine Wirkung von 100% bei einer Aufwandmenge von 100 ppm: 1-001, 1-002, 1-003, 1-004, 1-006, 1-007, 1-010, 1-019, 1-024, 1-025, 1-027, 1-028, 1-034, 1-035, 1-036, 1-040, 1-055, 1-068, 1-075, 1-077, 1-084, 1-086, 1-087, 1-089, 1-094, 1-099, 1-100, 1-127, 2-001, 2-003

Bei diesem Test zeigen z.B. die folgenden Verbindungen der Herstellungsbeispiele eine Wirkung von 90% bei einer Aufwandmenge von 100 ppm: 1-013, 1-014, 1-026, 1-037, 1-073, 1-096, 1-126, 2-005, 2-006

Bei diesem Test zeigen z.B. die folgenden Verbindungen der Herstellungsbeispiele eine Wirkung von 80% bei einer Aufwandmenge von 100 ppm: 1-031, 1-032, 1-047, 1-049, 1-058, 1-071, 1-119, 2-004

### Myzus persicae - Sprühtest

| | |
|---|---|
| Lösungsmittel: | 78 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 90% bei einer Aufwandmenge von 500 g/ha: 1-016, 1-031

Bei diesem Test zeigen z. B. die folgenden Verbindungen der Herstellungsbeispiele Wirkung von 100% bei einer Aufwandmenge von 100 g/ha: 1-001, 1-006, 1-009, 1-010, 1-025, 1-026, 1-027, 1-036, 1-040, 1-041, 1-064, 1-076, 1-081, 1-086, 1-098, 1-099, 1-100

Bei diesem Test zeigen z. B. die folgenden Verbindungen der Herstellungsbeispiele Wirkung von 90% bei einer Aufwandmenge von 100 g/ha: 1-003, 1-012, 1-017, 1-019, 1-033, 1-048, 1-087, 1-089

### Phaedon cochleariae - Sprühtest

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 500 g/ha: 1-009, 1-016, 1-018, 1-031

Bei diesem Test zeigen z. B. die folgenden Verbindungen der Herstellungsbeispiele Wirkung von 83% bei einer Aufwandmenge von 500 g/ha: 1-029

Bei diesem Test zeigen z. B. die folgenden Verbindungen der Herstellungsbeispiele Wirkung von 100% bei einer Aufwandmenge von 100 g/ha: 1-001, 1-002, 1-003, 1-004, 1-005, 1-006, 1-007, 1-010, 1-011, 1-012, 1-013, 1-014, 1-015, 1-017, 1-019, 1-022, 1-023, 1-024, 1-025, 1-026, 1-027, 1-028, 1-034, 1-035, 1-036, 1-037, 1-038, 1-039, 1-040, 1-041, 1-042, 1-043, 1-047, 1-050, 1-054, 1-055, 1-060, 1-064, 1-067, 1-068, 1-069, 1-071, 1-073, 1-076, 1-081, 1-084, 1-085, 1-086, 1-087, 1-088, 1-089, 1-090, 1-091, 1-092, 1-093, 1-094, 1-095, 1-096, 1-097, 1-098, 1-099, 1-100, 1-101, 1-116, 1-121, 1-126, 1-127, 2-001, 2-003, 2-004, 2-005, 2-006, 2-007

Bei diesem Test zeigen z. B. die folgenden Verbindungen der Herstellungsbeispiele Wirkung von 83% bei einer Aufwandmenge von 100 g/ha: 1-033

### Spodoptera frugiperda - Sprühtest

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 500 g/ha: 1-009, 1-016, 1-018, 1-029, 1-030, 1-031

Bei diesem Test zeigen z. B. die folgenden Verbindungen der Herstellungsbeispiele Wirkung von 100% bei einer Aufwandmenge von 100 g/ha: 1-001, 1-003, 1-004, 1-005, 1-006, 1-007, 1-010, 1-012, 1-013, 1-014, 1-015, 1-017, 1-019, 1-022, 1-023, 1-024, 1-025, 1-026, 1-027, 1-028, 1-034, 1-035, 1-036, 1-037, 1-038, 1-039, 1-040, 1-041, 1-043, 1-052, 1-054, 1-055, 1-064, 1-067, 1-068, 1-069, 1-071, 1-073, 1-076, 1-078, 1-081, 1-084, 1-086, 1-087, 1-088, 1-089, 1-091, 1-092, 1-093, 1-095, 1-096, 1-097, 1-098, 1-099, 1-100, 1-101, 1-117, 1-121, 1-127, 2-001, 2-003, 2-004, 2-005, 2-006

Bei diesem Test zeigen z. B. die folgenden Verbindungen der Herstellungsbeispiele Wirkung von 83% bei einer Aufwandmenge von 100 g/ha: 1-002, 1-057, 1-058, 1-060, 1-085, 1-094, 1-116

### Tetranychus urticae - Sprühtest, OP-resistent

| | | |
|---|---|---|
| Lösungsmittel: | 78,0 | GewichtsteileAceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100% bei einer Aufwandmenge von 500 g/ha: 1-009, 1-016

Bei diesem Test zeigen z. B. die folgenden Verbindungen der Herstellungsbeispiele eine Wirkung von 90% bei einer Aufwandmenge von 500 g/ha: 1-031

Bei diesem Test zeigen z. B. die folgenden Verbindungen der Herstellungsbeispiele eine Wirkung von 100% bei einer Aufwandmenge von 100 g/ha: 1-001, 1-003, 1-004, 1-006, 1-010, 1-012, 1-013, 1-015, 1-017, 1-019, 1-022, 1-023, 1-024, 1-025, 1-026, 1-027, 1-028, 1-029, 1-033, 1-034, 1-035, 1-038, 1-039, 1-040, 1-041, 1-042, 1-043, 1-046, 1-047, 1-048, 1-052, 1-053, 1-054, 1-058, 1-060, 1-063, 1-064, 1-065, 1-067, 1-071, 1-073, 1-078, 1-081, 1-085, 1-087, 1-088, 1-089, 1-091, 1-092, 1-093, 1-094, 1-095, 1-096, 1-097, 1-098, 1-099, 1-100, 1-101, 1-108, 1-121, 2-001, 2-003, 2-004, 2-006

Bei diesem Test zeigen z. B. die folgenden Verbindungen der Herstellungsbeispiele eine Wirkung von 90% bei einer Aufwandmenge von 100 g/ha: 1-007, 1-011, 1-014, 1-018, 1-055, 1-068, 1-069, 1-076, 1-086

Bei diesem Test zeigen z. B. die folgenden Verbindungen der Herstellungsbeispiele eine Wirkung von 100% bei einer Aufwandmenge von 20 g/ha: 2-005

### Anopheles-Test (ANPHGB Oberflächenbehandlung)

| | |
|---|---|
| Lösungsmittel: | Aceton + 2000ppm Rapsölmethylester (RME) |

Zur Herstellung einer zweckmäßigen Wirkstoffzubereitung löst man den Wirkstoff im Lösungsmittel (2mg/ml). Die Wirkstoffzubereitung wird auf eine lasierte Kachel pipettiert und nach Abtrocknen werden adulte Mücken der Spezies Anopheles gambiae Stamm RSPH (homozygot kdr) auf die behandelte Kachel gesetzt. Die Expositionszeit beträgt 30 Minuten.

24 Stunden nach Kontakt zur behandelten Oberfläche wird die Mortalität in % bestimmt. Dabei bedeutet 100%, dass alle Mücken abgetötet wurden; 0% bedeutet, dass keine Mücken abgetötet wurden.

Bei diesem Test zeigen z.B. die folgenden Verbindungen der Herstellungsbeispiele Wirkung von 90-100% bei einer Aufwandmenge von 100mg/m²: 1-001, 1-023, 1-024, 1-036, 1-040

Bei diesem Test zeigen z.B. die folgenden Verbindungen der Herstellungsbeispiele Wirkung von 90-100% bei einer Aufwandmenge von 20mg/m²: 1-024, 1-127

### Anopheles-Test (ANPHFU Oberflächenbehandlung)

| | |
|---|---|
| Lösungsmittel: | Aceton + 2000ppm Rapsölmethylester (RME) |

Zur Herstellung einer zweckmäßigen Wirkstoffzubereitung löst man den Wirkstoff im Lösungsmittel (2mg/ml). Die Wirkstoffzubereitung wird auf eine lasierte Kachel pipettiert und nach Abtrocknen werden adulte Mücken der Spezies Anopheles funestus Stamm FUMOZ-R (Hunt et al., Med Vet Entomol. 2005 Sep; 19(3):271-5) auf die behandelte Kachel gesetzt. Die Expositionszeit beträgt 30 Minuten.

24 Stunden nach Kontakt zur behandelten Oberfläche wird die Mortalität in % bestimmt. Dabei bedeutet 100%, dass alle Mücken abgetötet wurden; 0% bedeutet, dass keine Mücken abgetötet wurden.

Bei diesem Test zeigen z.B. die folgenden Verbindungen der Herstellungsbeispiele Wirkung von 90-100% bei einer Aufwandmenge von 100mg/m²: 1-001, 1-022, 1-023, 1-024, 1-025, 1-036, 1-037, 1-040, 1-073

Bei diesem Test zeigen z.B. die folgenden Verbindungen der Herstellungsbeispiele Wirkung von 90-100% bei einer Aufwandmenge von 20mg/m²: 1-001, 1-022, 1-023, 1-024, 1-025, 1-026, 1-027, 1-036, 1-040, 1-081, 1-127

### Aedes-Test (AEDSAE Oberflächenbehandlung)

| | |
|---|---|
| Lösungsmittel: | Aceton + 2000ppm Rapsölmethylester (RME) |

Zur Herstellung einer zweckmäßigen Wirkstoffzubereitung löst man den Wirkstoff im Lösungsmittel (2mg/ml). Die Wirkstoffzubereitung wird auf eine lasierte Kachel pipettiert und nach Abtrocknen werden adulte Mücken der Spezies Aedes aegypti Stamm MONHEIM auf die behandelte Kachel gesetzt. Die Expositionszeit beträgt 30 Minuten.

24 Stunden nach Kontakt zur behandelten Oberfläche wird die Mortalität in % bestimmt. Dabei bedeutet 100%, dass alle Mücken abgetötet wurden; 0% bedeutet, dass keine Mücken abgetötet wurden.

Bei diesem Test zeigen z.B. die folgenden Verbindungen der Herstellungsbeispiele Wirkung von 90-100% bei einer Aufwandmenge von 100mg/m²: 1-001, 1-002, 1-003, 1-006, 1-010, 1-012, 1-013, 1-014, 1-016, 1-017, 1-019, 1-022, 1-023, 1-024, 1-025, 1-026, 1-027, 1-034, 1-035, 1-036, 1-037, 1-073, 1-081, 1-085, 1-094, 1-099, 1-100, 1-127, 2-001

Bei diesem Test zeigen z.B. die folgenden Verbindungen der Herstellungsbeispiele Wirkung von 90-100% bei einer Aufwandmenge von 20mg/m²: 1-001, 1-002, 1-003, 1-006, 1-010, 1-012, 1-013, 1-014, 1-016, 1-017, 1-019, 1-022, 1-023, 1-024, 1-025, 1-026, 1-027, 1-034, 1-035, 1-036, 1-037, 1-040, 1-073, 1-081, 1-086, 1-094, 1-099, 1-100, 1-127, 2-001

## Patentansprüche

1. Verbindungen der allgemeinen Formel (Ic), 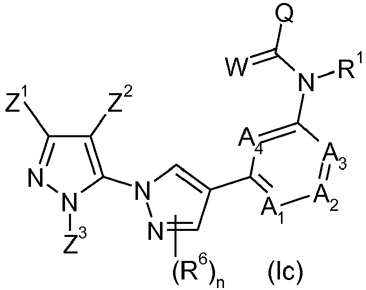 worin
R¹ für Wasserstoff, gegebenenfalls substituiertes C₁-C₆-Alkyl, C₃-C₆-Alkenyl, C₃-C₆-Alkinyl, C₃-C₇-Cycloaₗkyl, C₁-C₆-Alkylcarbonyl, C₁-C₆-Alkoxycarbonyl, Aryl(C₁-C₃)-alkyl, Heteroaryl(C₁-C₃)-alkyl steht;
die chemischen Gruppierungen
A₁ für CR² oder Stickstoff,
A₂ für CR³ oder Stickstoff,
A₃ für CR⁴ oder Stickstoff, und
A₄ für CR⁵ oder Stickstoff stehen,
wobei aber nicht mehr als drei der chemischen Gruppierungen A₁ bis A₄ gleichzeitig für Stickstoff stehen;
R², R³, R⁴ und R⁵ unabhängig voneinander für Wasserstoff, Halogen, Cyano, Nitro, gegebenenfalls substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N*-Di-C₁-C₆-alkylamino, stehen;
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält;
W für Sauerstoff oder Schwefel steht;
Q für Wasserstoff, Amino oder eine der gegebenenfalls substituierten Gruppierungen Alkyl, Alkyloxy, Alkenyl, Alkinyl, Cycloalkyl, Heterocyclyl, Cycloalkylalkyl, Arylalkyl, Heteroarylalkyl, Alkoxycarbonyl, oder für eine Gruppierung *N-*Alkylamino, *N*-Alkylcarbonylamino, *N,N-*Dialkylamino, Alkylsulfonylamino steht; oder
Q für ein gegebenenfalls ein- bis fünffach mit V substituiertes Aryl steht, oder für ein gegebenenfalls ein- bis fünffach mit V substituiertes Heterorayl steht, wobei
V für Halogen, Cyano, Nitro, gegebenenfalls substituiertes Alkyl, Alkenyl, Alkinyl, Cycloalkyl, Alkoxy, *N*-Alkoxyiminoalkyl, Alkylsulfanyl, Alkylsulfinyl, Alkylsulfonyl, *N,N-*Dialkylamino steht;
R⁶ unabhängig voneinander für Halogen, Cyano, Nitro, Amino oder ein gegebenenfalls substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, und
n für die Werte 0-2 steht;
Z¹ für ein gegebenenfalls substituiertes Alkyl oder Cycloalkyl, und
Z² für Wasserstoff, Halogen, Cyano, Nitro, Amino oder ein gegebenenfalls substituiertes Alkyl, Alkylcarbonyl, Alkylsulfanyl, Alkylsulfinyl, Alkylsulfonyl, und
Z³ für Wasserstoff oder ein gegebenenfalls substituiertes Alkyl, Cycloalkyl, Alkenyl, Alkinyl, Aryl oder Hetaryl stehen.

2. Verbindungen gemäß Anspruch 1, wobei
R¹ für Wasserstoff oder für ein gegebenenfalls ein- bis siebenfach unabhängig voneinander mit Halogen, Cyano, Alkoxy oder Alkoxycarbonyl substituiertes C₁-C₆-Alkyl, C₃-C₆-Alkenyl, C₃-C₆-Alkinyl, C₃-C₇-Cycloalkyl, C₃-C₇-Cycloalkyl-C₁-C₃-alkyl, C₁-C₆-Alkylcarbonyl, C₁-C₆-Alkoxycarbonyl, Aryl(C₁-C₃)-alkyl, Heteroaryl(C₁-C₃)-alkyl steht;
die chemischen Gruppierungen
A₁ für CR² oder Stickstoff,
A₂ für CR³ oder Stickstoff,
A₃ für CR⁴ oder Stickstoff, und
A₄ für CR⁵ oder Stickstoff stehen,
wobei aber nicht mehr als drei der chemischen Gruppierungen A₁ bis A₄ gleichzeitig für Stickstoff stehen;
R², R³, R⁴ und R⁵ unabhängig voneinander für Wasserstoff, Halogen, Cyano, Nitro, oder für ein gegebenenfalls ein- bis siebenfach unabhängig voneinander mit Halogen, Cyano, Alkoxy oder Alkoxycarbonyl substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N*-Di-C₁-C₆-alkylamino stehen;
W für Sauerstoff oder Schwefel steht;
Q für Wasserstoff, Amino oder eine der gegebenenfalls unabhängig von einander ein-bis siebenfach mit Hydoxy, Nitro, Amino, Halogen, Alkoxy, Cyano, Hydroxycarbonyl, Alkoxycarbonyl, Alkylcarbamoyl, Cycloalkylcarbamoyl, oder Phenyl substituierten Gruppierungen C₁-C₆-Alkyl, C₂-C₆-Alkenyl, C₂-C₆-Alkinyl, C₃-C₆-Cycloalkyl, C₂-C₅-Heterocyclyl, C₁-C₄-Alkoxy, C₁-C₆-Alkyl-C₃-C₆-cycloalkyl, C₃-C₆-Cycloalkyl-C₁-C₆-alkyl, C₁-C₆-Hydroxyalkyl, Aryl(C₁-C₃)-alkyl, Heteroaryl(C₁-C₃)-alkyl, C₁-C₄-Alkoxycarbonyl, *N*-C₁-C₄-Alkylamino, *N*-C₁-C₄-Alkylcarbonylamino, *N,N*-Di-C₁-C₄-alkylamino, C₁-C₄-Alkylsulfonylamino steht; oder
Q für Wasserstoff, Amino oder eine der gegebenenfalls unabhängig von einander mit 1, 2, 3, 4, 5, 6 oder 7 Substituenten ausgewählt aus der Gruppe bestehend aus Hydoxy, Nitro, Amino, Halogen, Oxo, Benzyloxy, C₃-C₆-Cycloalkyl, C₁-C₆-Alkoxy, C₁-C₆-Halogenalkoxy, Cyano, Hydroxycarbonyl, C₁-C₄-Alkoxycarbonyl, C₁-C₆-Alkylcarbamoyl, C₃-C₆-Cycloalkylcarbamoyl, *N,N*-Di-C₁-C₄-alkylamino, gegebenenfalls mit 1, 2 oder 3 Substituenten unabhängig voneinander ausgewählt aus der Gruppe von Substituenten bestehend aus Halogen, C₁-C₆-Alkoxy, C₁-C₆-Halogenalkoxy und C₁-C₆-Alkyl substituierten Phenyl, gegebenenfalls mit 1, 2 oder 3 Substituenten unabhängig voneinander ausgewählt aus der Gruppe von Substituenten bestehend aus Halogen, C₁-C₆-Alkoxy, C₁-C₆-Halogenalkoxy nd C₁-C₆-Alkyl substituierten Phenylthio, gegebenenfalls mit 1, 2 oder 3 Substituenten unabhängig voneinander ausgewählt aus der Gruppe von Substituenten bestehend aus Halogen, C₁-C₆-Alkoxy, C₁-C₆-Halogenalkoxy und C₁-C₆-Alkyl substituierten Phenyloxy, gegebenenfalls mit 1, 2 oder 3 Substituenten unabhängig voneinander ausgewählt aus der Gruppe von Substituenten bestehend aus Halogen, C₁-C₆-Alkoxy, C₁-C₆-Halogenalkoxy und C₁-C₆-Alkyl substituierten 5- oder 6-gliedrigen Heteroaryl (z. B. Pyrazolyl) substituierten Gruppierungen ausgewählt aus C₁-C₆-Alkyl, C₁-C₆-Alkyloxy, C₂-C₆-Alkenyl, C₂-C₆-Alkinyl, C₃-C₆-Cycloalkyl, 5 oder 6 gliedriges Heterocyclyl, C₁-C₆-Alkyl-C₃-C₆-cycloalkyl, C₃-C₆-Cycloalkyl-C₁-C₆-alkyl, C₆-C₁₀-Aryl-(C₁-C₃)-alkyl, 5 oder 6 gliedriges Heteroaryl-(C₁-C₃)-alkyl, C₁-C₄-Alkoxycarbonyl, oder für eine Gruppierung *N*-C₁-C₄-Alkylamino, *N*-C₁-C₄-Alkylcarbonylamino, *N,N*-Di-C₁-C₄-alkylamino, C₁-C₄-Alkylsulfonylamino steht; oder
Q für ein mit 0, 1, 2, 3 oder 4 Substituenten V substituiertes Aryl oder für ein mit 0, 1, 2, 3 oder 4 Substituenten V substituiertes 5- bzw. 6-gliedriges Heteroaryl steht, oder
Q für einen mit 0, 1, 2, 3 oder 4 Substituenten V substituierten bicyclischen Heterocyclus oder für einen mit 0, 1, 2, 3 oder 4 Substituenten V substituierten bicyclische Carbocyclus steht; wobei
V unabhängig voneinander für Halogen, Cyano, Nitro oder für ein gegebenenfalls einbis siebenfach unabhängig voneinander mit Halogen, Cyano, Alkoxy oder Alkoxycarbonyl substituiertes C₁-C₆-Alkyl, C₁-C₆-Halogenalkyl, C₂-C₄-Alkenyl, C₂-C₄-Alkinyl, C₃-C₆-Cycloalkyl, C₁-C₆-Alkoxy, C₁-C₆-Halogenalkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N,N*-Di-(C₁-C₆-alkyl)amino steht;
R⁶ unabhängig voneinander für Halogen, Cyano, Nitro, Amino oder ein gegebenenfalls ein- bis siebenfach mit Halogen substituiertes C₁-C₆-Alkyl, C₁-C₆-Alkyloxy, C₁-C₆-Alkylcarbonyl, C₁-C₆-Alkylsulfanyl, C₁-C₆-Alkylsulfinyl, C₁-C₆-Alkylsulfonyl, und
n für die Werte 0-1 steht;
Z¹ für ein gegebenenfalls ein- bis siebenfach unabhängig voneinander mit Halogen, Cyano, Alkoxy oder Alkoxycarbonyl substituiertes C₁-C₆-Alkyl, C₃-C₆-Cycloalkyl, und
Z² für Wasserstoff, Halogen, Cyano, Nitro, Amino oder ein ein- bis siebenfach unabhängig voneinander mit Halogen, Cyano, Alkoxy oder Alkoxycarbonyl substituiertes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl, und
Z³ für Wasserstoff oder für ein gegebenenfalls ein- bis siebenfach unabhängig voneinander mit Halogen, Cyano, Alkoxy oder Alkoxycarbonyl substituiertes C₁-C₆-Alkyl, C₃-C₆-Cycloalkyl C₃-C₄-Alkenyl, C₃-C₄-Alkinyl, oder für ein gegebenenfalls ein- bis fünffach unabhängig voneinander mit Halogen, Cyano, Alkoxy und Alkoxycarbonyl substituiertes Aryl und Hetaryl stehen.

3. Verbindungen gemäß Anspruch 1 oder 2, worin
R¹ für Wasserstoff, C₁-C₄-Alkyl, C₃-C₄-Alkenyl, C₃-C₄-Alkinyl steht;
die chemischen Gruppierungen
A₁ für CR²,
A₂ für CR³ oder Stickstoff,
A₃ für CR⁴, und
A₄ für CR⁵ stehen;
W für Sauerstoff steht;
R⁶ für C₁-C₄-Alkyl steht;
n für die Werte 0-1 steht;
Z¹ für ein jeweils gegebenenfalls ein- bis siebenfach unabhängig voneinander mit Halogen oder Cyano substituiertes C₁-C₆-Alkyl oder C₃-C₆-Cycloalkyl steht;
Z² für ein jeweils gegebenenfalls ein- bis siebenfach unabhängig voneinander mit Halogen oder Cyano substituiertes C₁-C₆-Alkyl oder C₃-C₆-Cycloalkyl steht;
Z³ für Wasserstoff oder C₁-C₆-Alkyl steht.

4. Verbindungen gemäß Anspruch 1, 2 oder 3, worin
R¹ für Wasserstoff, Methyl, Ethyl, n-Propyl, Isopropyl, n-Butyl, Isobutyl, s-Butyl, oder t-Butyl steht;
die chemischen Gruppierungen
A₁ für CR²,
A₂ für CR³ oder Stickstoff,
A₃ für CR⁴, und
A₄ für CR⁵ stehen;
W für Sauerstoff steht;
R⁶ für Wasserstoff steht;
Z¹ für ein jeweils gegebenenfalls ein- bis siebenfach unabhängig voneinander mit Halogen substituiertes C₁-C₆-Alkyl steht;
Z² für ein jeweils gegebenenfalls ein- bis siebenfach unabhängig voneinander mit Halogen substituiertes C₁-C₆-Alkyl steht;
Z³ für C₁-C₆-Alkyl steht.

5. Verbindungen gemäß einem der vorhergehenden Ansprüche, worin
R² und R⁵ unabhängig voneinander für Wasserstoff, Fluor Chlor, Brom, Iod, Methyl und Methoxy stehen;
R³ und R⁴ unabhängig voneinander für Wasserstoff, Fluor, Chlor, Brom, Iod, Cyano, Nitro, C₁-C₄-Alkyl, C₁-C₄-Haloalkyl stehen;

6. Verbindungen gemäß einem der vorhergehenden Ansprüche, worin
R² und R⁵ unabhängig voneinander für Wasserstoff oder Fluor stehen;
R³ für Wasserstoff steht; und
R⁴ für Wasserstoff, Fluor, Chlor, oder Methyl steht.

7. Verbindungen gemäß einem der vorhergehenden Ansprüche, worin
Q für Wasserstoff; oder
C₁-C₆-Alkyl optional substituiert mit 1, 2, 3 oder 4 Substituenten unabhängig voneinander ausgewählt aus der Gruppe bestehend aus
Fluor,
Chlor,
Brom,
Iod,
Cyano,
Oxo,
Methoxy,
Benzyloxy,
Ethoxy,
*N,N*-Dimethylamino,
optional unabhängig voneinander mit 1, 2, 3, oder 4 Subtituenten ausgewählt aus der Gruppe bestehend aus Fluor, Chlor, Brom, Iod, Methoxy, Methyl, und Trifluormethyl substituiertem Phenylthio,
optional unabhängig voneinander mit 1, 2, 3, oder 4 Subtituenten ausgewählt aus der Gruppe bestehend aus Fluor, Chlor, Brom, Iod, Methoxy, Methyl, und Trifluormethyl substituiertem Phenyloxy,
optional unabhängig voneinander mit 1, 2, 3, oder 4 Subtituenten ausgewählt aus der Gruppe bestehend aus Fluor, Chlor, Brom, Iod, Methoxy, Methyl, und Trifluormethyl substituiertem Phenyl,
optional unabhängig voneinander mit 1, 2, 3, oder 4 Subtituenten ausgewählt aus der Gruppe bestehend aus Fluor, Chlor, Brom, Iod, Methoxy, Methyl, und Trifluormethyl substituiertem Thiophenyl,
optional unabhängig voneinander mit 1, 2, 3, oder 4 Subtituenten ausgewählt aus der Gruppe bestehend aus Fluor, Chlor, Brom, Iod, Methoxy, Methyl, und Trifluormethyl substituiertem Pyrazolyl, und
C₃-C₆-Cycloalkyl; oder C₃-C₆-Cycloalkyl optional substituiert mit 1, 2, 3 oder 4 Substituenten unabhängig voneinander ausgewählt aus der Gruppe bestehend aus
Methoxy,
Fluor,
Chlor,
Brom
Iod,
Cyano,
Methyl,
optional unabhängig voneinander mit 1, 2, 3, oder 4 Subtituenten ausgewählt aus der Gruppe bestehend aus Fluor, Chlor, Brom, Iod, Methoxy, Methyl, Trifluormethyl, substituiertem Phenyl;
oder
Q für ein mit 0, 1, 2, 3 oder 4 Substituenten V substituiertes Phenyl, Naphthyl, Pyridazinyl, Pyrazinyl, Pyrimidinyl, Triazinyl, Pyridinyl, Pyrazolyl, Thiazolyl, Isothiazolyl, Oxazolyl, Isoxazolyl, Triazolyl, Imidazolyl, Furanyl, Thiophenyl, Pyrrolyl, Oxadiazolyl, Thiadiazolyl, Benzothiophenyl, Benzofuranyl, [1,2,4]Triazolo[1,5-a]pyrimidinyl, 1,3-Benzodioxolyl oder Tetralinyl steht, wobei
V unabhängig voneinander für Fluor, Chlor, Brom, Iod, Cyano, Nitro, Methyl, Ethyl, Propyl, Butyl, Difluormethyl, , Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N*-Dimethylamino steht.

8. Verbindungen gemäß einem der vorhergehenden Ansprüche, worin
Q C₁-C₆-Alkyl optional substituiert mit 1, 2, 3 oder 4 Substituenten unabhängig voneinander ausgewählt aus der Gruppe bestehend aus
Fluor,
Oxo,
Methoxy,
Benzyloxy,
Ethoxy,
*N,N*-Dimethylamino
Phenylthio,
Phenyloxy,
C₃-C₆-Cycloalkyl,
optional unabhängig voneinander mit 1, 2, 3, oder 4 Subtituenten ausgewählt aus der Gruppe bestehend aus Fluor, Chlor, und Trifluormethyl substituiertem Phenyl,
optional mit 1, 2, 3, oder 4 Trifluormethyl substituiertem Thiophenyl, und optional unabhängig voneinander mit 1, 2, 3, oder 4 Subtituenten ausgewählt aus der Gruppe bestehend aus Methyl und Trifluormethyl substituiertem Pyrazolyl;
oder
C₃-C₆-Cycloalkyl optional substituiert mit 1, 2, 3 oder 4 Substituenten unabhängig voneinander ausgewählt aus der Gruppe bestehend aus
Methoxy,
Chlor,
Cyano,
Methyl,
optional unabhängig voneinander mit 1, 2, 3, oder 4 Subtituenten ausgewählt aus der Gruppe bestehend aus Chlor und Methyl, und Trifluormethyl substituiertem Phenyl;
oder
Q für ein mit 0, 1, 2, 3 oder 4 Substituenten V substituiertes Phenyl, Naphthyl, Pyridazinyl, Pyrazinyl, Pyrimidinyl, Triazinyl, Pyridinyl, Pyrazolyl, Thiazolyl, Isothiazolyl, Oxazolyl, Isoxazolyl, Triazolyl, Imidazolyl, Furanyl, Thiophenyl, Pyrrolyl, Oxadiazolyl, Thiadiazolyl, Benzothiophenyl, Benzofuranyl, [1,2,4]Triazolo[1,5-a]pyrimidinyl, 1,3-Benzodioxolyl oder Tetralinyl steht, wobei
V unabhängig voneinander für Fluor, Chlor, Brom, Iod, Cyano, Nitro, Methyl, Ethyl, Propyl, Butyl, Difluormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N-*Dimethylamino steht.

9. Verbindungen gemäß einem der vorhergehenden Ansprüche, worin die Verbindung eine Verbindung der Formel (Ic-1) ist

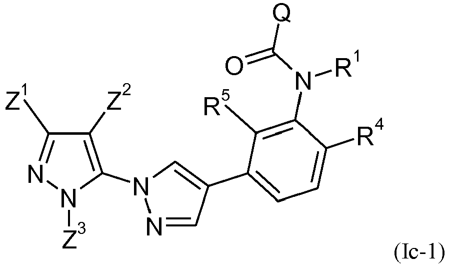

10. Verbindungen gemäß einem der vorhergehenden Ansprüche, worin die Verbindung eine Verbindung der Formel (Ic-2) ist

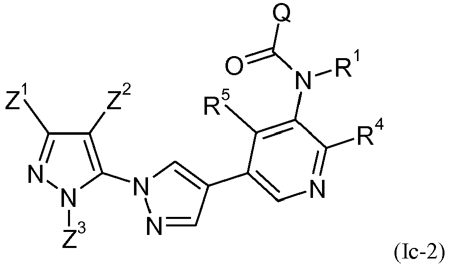

11. Verwendung von Verbindungen der allgemeinen Formeln (I), (Ia), (Ib), (Ic), (Id), (Ie), (If), (Ig), (Ih), (Ii) und (Ij) gemäß einem der Ansprüche 1 bis 10 zur nicht-therapeutischen Bekämpfung von Insekten, Spinnentieren und Nematoden.

12. Pharmazeutische Zusammensetzungen, enthaltend wenigstens eine Verbindung gemäß einem der Ansprüche 1 bis 10.

13. Verbindungen gemäß einem der Ansprüche 1 bis 10 zur Verwendung als Arzneimittel.

14. Verbindungen gemäß einem der Ansprüche 1 bis 10 zur Verwendung zur Bekämpfung von Parasiten auf Tieren.

15. Verfahren zur Herstellung von Pflanzenschutzmitteln enthaltend Verbindungen gemäß einem der Ansprüche 1 bis 10, sowie übliche Streckmittel und/oder oberflächenaktive Substanzen.

16. Nicht-therapeutisches Verfahren zum Bekämpfen von Schädlingen, **dadurch gekennzeichnet, dass** man eine Verbindung gemäß einem der Ansprüche 1 bis 10 auf die Schädlinge und/oder ihren Lebensraum einwirken lässt.

17. Verwendung von Verbindungen gemäß einem der Ansprüche 1 bis 10 zum Schutz des Vermehrungsmaterials von Pflanzen.

## Claims

1. Compounds of the general formula (Ic) 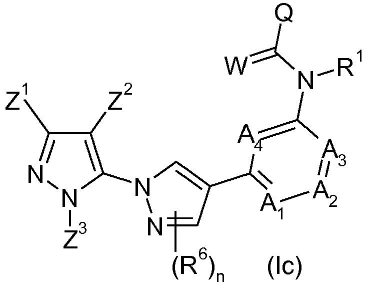 in which
R¹ is hydrogen, optionally substituted C₁-C₆-alkyl, C₃-C₆-alkenyl, C₃-C₆-alkynyl, C₃-C₇-cycloalkyl, C₁-C₆-alkylcarbonyl, C₁-C₆-alkoxycarbonyl, aryl(C₁-C₃)-alkyl, heteroaryl (C₁-C₃) -alkyl;
the following chemical moieties are as follows:
A₁ is CR² or nitrogen,
A₂ is CR³ or nitrogen,
A₃ is CR⁴ or nitrogen, and
A₄ is CR⁵ or nitrogen,
but not more than three of the chemical moieties A₁ to A₄ are simultaneously nitrogen;
R², R³, R⁴ and R⁵ are each independently hydrogen, halogen, cyano, nitro, optionally substituted C₁-C₆-alkyl, C₃-C₆-cycloalkyl, C₁-C₆-alkoxy, *N-*C₁-C₆-alkoxyimino-C₁-C₃-alkyl, C₁-C₆-alkylsulphanyl, C₁-C₆-alkylsulphinyl, C₁-C₆-alkylsulphonyl, *N*-C₁-C₆-alkylamino or *N,N*-di-C₁-C₆-alkylamino;
if neither of the A₂ and A₃ moieties is nitrogen, R³ and R⁴ together with the carbon atom to which they are bonded may form a 5-or 6-membered ring containing 0, 1 or 2 nitrogen atoms and/or 0 or 1 oxygen atom and/or 0 or 1 sulphur atom, or
if neither of the A₁ and A₂ moieties is nitrogen, R² and R³ together with the carbon atom to which they are bonded may form a 5-or 6-membered ring containing 0, 1 or 2 nitrogen atoms and/or 0 or 1 oxygen atom and/or 0 or 1 sulphur atom;
W is oxygen or sulphur;
Q is hydrogen, amino or one of the following optionally substituted moieties: alkyl, alkyloxy, alkenyl, alkynyl, cycloalkyl, heterocyclyl, cycloalkylalkyl, arylalkyl, heteroarylalkyl, alkoxycarbonyl, or an *N-*alkylamino, *N*-alkylcarbonylamino, *N,N-*dialkylamino, alkylsulphonylamino moiety; or
Q is an optionally mono- to penta-V-substituted aryl, or an optionally mono- to penta-V-substituted heteroaryl, where
V is halogen, cyano, nitro, optionally substituted alkyl, alkenyl, alkynyl, cycloalkyl, alkoxy, *N*-alkoxyiminoalkyl, alkylsulphanyl, alkylsulphinyl, alkylsulphonyl, N,N-dialkylamino;
R⁶ is independently halogen, cyano, nitro, amino or an optionally substituted C₁-C₆-alkyl, C₁-C₆-alkyloxy, C₁-C₆-alkylcarbonyl, C₁-C₆-alkylsulphanyl, C₁-C₆-alkylsulphinyl, C₁-C₆-alkylsulphonyl, and
n has the values of 0-2;
Z¹ is an optionally substituted alkyl or cycloalkyl, and
Z² is hydrogen, halogen, cyano, nitro, amino or an optionally substituted alkyl, alkylcarbonyl, alkylsulphanyl, alkylsulphinyl, alkylsulphonyl, and
Z³ is hydrogen or an optionally substituted alkyl, cycloalkyl, alkenyl, alkynyl, aryl or hetaryl.

2. Compounds according to Claim 1, where
R¹ is hydrogen or an optionally independently mono- to hepta-halogen-, -cyano-, -alkoxy- or -alkoxycarbonyl-substituted C₁-C₆-alkyl, C₃-C₆-alkenyl, C₃-C₆-alkynyl, C₃-C₇-cycloalkyl, C₃-C₇-cycloalkyl-C₁-C₃-alkyl, C₁-C₆-alkylcarbonyl, C₁-C₆-alkoxycarbonyl, aryl (C₁-C₃) -alkyl, heteroaryl(C₁-C₃)-alkyl;
the following chemical moieties are as follows:
A₁ is CR² or nitrogen,
A₂ is CR³ or nitrogen,
A₃ is CR⁴ or nitrogen, and
A₄ is CR⁵ or nitrogen,
but not more than three of the chemical moieties A₁ to A₄ are simultaneously nitrogen;
R², R³, R⁴ and R⁵ are each independently hydrogen, halogen, cyano, nitro, or an optionally independently mono- to hepta-halogen-, -cyano-, -alkoxy- or -alkoxycarbonyl-substituted C₁-C₆-alkyl, C₃-C₆-cycloalkyl, C₁-C₆-alkoxy, *N*-C₁-C₆-alkoxyimino-C₁-C₃-alkyl, C₁-C₆-alkylsulphanyl, C₁-C₆-alkylsulphinyl, C₁-C₆-alkylsulphonyl, *N*-C₁-C₆-alkylamino or *N,N-*di-C₁-C₆-alkylamino;
W is oxygen or sulphur;
Q is hydrogen, amino or one of the following independently mono- to hepta-hydroxy-, -nitro-, -amino-, -halogen-, -alkoxy-,-cyano-, -hydroxycarbonyl-, -alkoxycarbonyl-, -alkylcarbamoyl-, -cycloalkylcarbamoyl-, or-phenyl-substituted moieties: C₁-C₆-alkyl, C₂-C₆-alkenyl, C₂-C₆-alkynyl, C₃-C₆-cycloalkyl, C₂-C₅-heterocyclyl, C₁-C₄-alkoxy, C₁-C₆-alkyl-C₃-C₆-cycloalkyl, C₃-C₆-cycloalkyl-C₁-C₆-alkyl, C₁-C₆-hydroxyalkyl, aryl (C₁-C₃) -alkyl, heteroaryl(C₁-C₃)-alkyl, C₁-C₄-alkoxycarbonyl, *N*-C₁-C₄-alkylamino, *N*-C₁-C₄-alkylcarbonylamino, *N,N*-di-C₁-C₄-alkylamino, C₁-C₄-alkylsulphonylamino; or
Q is hydrogen, amino or one of the moieties selected from C₁-C₆-alkyl, C₁-C₆-alkyloxy, C₂-C₆-alkenyl, C₂-C₆-alkynyl, C₃-C₆-cycloalkyl, 5-or 6-membered heterocyclyl, C₁-C₆-alkyl-C₃-C₆-cycloalkyl, C₃-C₆-cycloalkyl-C₁-C₆-alkyl, C₆-C₁₀-aryl-(C₁-C₃)-alkyl, 5- or 6-membered heteroaryl-(C₁-C₃)-alkyl, C₁-C₄-alkoxycarbonyl, each of which is optionally independently substituted by 1, 2, 3, 4, 5, 6 or 7 substituents selected from the group consisting of hydroxy, nitro, amino, halogen, oxo, benzyloxy, C₃-C₆-cycloalkyl, C₁-C₆-alkoxy, C₁-C₆-haloalkoxy, cyano, hydroxycarbonyl, C₁-C₄-alkoxycarbonyl, C₁-C₆-alkylcarbamoyl, C₃-C₆-cycloalkylcarbamoyl, *N,N*-di-C₁-C₄-alkylamino, phenyl optionally substituted by 1, 2 or 3 substituents selected independently from the group of substituents consisting of halogen, C₁-C₆-alkoxy, C₁-C₆-haloalkoxy and C₁-C₆-alkyl, phenylthio optionally substituted by 1, 2 or 3 substituents selected independently from the group of substituents consisting of halogen, C₁-C₆-alkoxy, C₁-C₆-haloalkoxy and C₁-C₆-alkyl, phenyloxy optionally substituted by 1, 2 or 3 substituents selected independently from the group of substituents consisting of halogen, C₁-C₆-alkoxy, C₁-C₆-haloalkoxy and C₁-C₆-alkyl, 5- or 6-membered heteroaryl (e.g. pyrazolyl) optionally substituted by 1, 2 or 3 substituents selected independently from the group of substituents consisting of halogen, C₁-C₆-alkoxy, C₁-C₆-haloalkoxy and C₁-C₆-alkyl, or is an *N*-C₁-C₄-alkylamino, *N*-C₁-C₄-alkylcarbonylamino, *N,N*-di-C₁-C₄-alkylamino, C₁-C₄-alkylsulphonylamino moiety; or
Q is an aryl substituted by 0, 1, 2, 3 or 4 V substituents or a 5- or 6-membered heteroaryl substituted by 0, 1, 2, 3 or 4 V substituents, or
Q is a bicyclic heterocycle substituted by 0, 1, 2, 3 or 4 V substituents or a bicyclic carbocycle substituted by 0, 1, 2, 3 or 4 V substituents; where
V is independently halogen, cyano, nitro or an optionally independently mono- to hepta-halogen, -cyano-, -alkoxy- or-alkoxycarbonyl-substituted C₁-C₆-alkyl, C₁-C₆-haloalkyl, C₂-C₄-alkenyl, C₂-C₄-alkynyl, C₃-C₆-cycloalkyl, C₁-C₆-alkoxy, C₁-C₆-haloalkoxy, *N-*C₁-C₆-alkoxyimino-C₁-C₃-alkyl, C₁-C₆-alkylsulphanyl, C₁-C₆-alkylsulphinyl, C₁-C₆-alkylsulphonyl, *N,N*-di-(C₁-C₆-alkyl)amino;
R⁶ is independently halogen, cyano, nitro, amino or an optionally mono- to hepta-halogen-substituted C₁-C₆-alkyl, C₁-C₆-alkyloxy, C₁-C₆-alkylcarbonyl, C₁-C₆-alkylsulphanyl, C₁-C₆-alkylsulphinyl, C₁-C₆-alkylsulphonyl, and
n has the values of 0-1;
Z¹ is an optionally independently mono- to hepta-halogen-, -cyano-, -alkoxy-or -alkoxycarbonyl-substituted C₁-C₆-alkyl, C₃-C₆-cycloalkyl, and
Z² is hydrogen, halogen, cyano, nitro, amino or an independently mono- to hepta-halogen-,-cyano-, -alkoxy- or -alkoxycarbonyl-substituted C₁-C₆-alkyl, C₁-C₆-alkylcarbonyl, C₁-C₆-alkylsulphanyl, C₁-C₆-alkylsulphinyl, C₁-C₆-alkylsulphonyl, and
Z³ is hydrogen or an optionally independently mono- to hepta-halogen-, -cyano-, -alkoxy- or -alkoxycarbonyl-substituted C₁-C₆-alkyl, C₃-C₆-cycloalkyl, C₃-C₄-alkenyl, C₃-C₄-alkynyl, or an optionally independently mono-to penta-halogen-, -cyano-, -alkoxy- and -alkoxycarbonyl-substituted aryl or hetaryl.

3. Compounds according to Claim 1 or 2, in which
R¹ is hydrogen, C₁-C₄-alkyl, C₃-C₄-alkenyl, C₃-C₄-alkynyl;
the following chemical moieties are as follows:
A₁ is CR²,
A₂ is CR³ or nitrogen,
A₃ is CR⁴, and
A₄ is CR⁵;
W is oxygen;
R⁶ is C₁-C₄-alkyl;
n has the values of 0-1;
Z¹ is an in each case optionally mono- to hepta-halogen- or -cyano-substituted C₁-C₆-alkyl or C₃-C₆-cycloalkyl;
Z² is an in each case optionally mono- to hepta-halogen- or -cyano-substituted C₁-C₆-alkyl or C₃-C₆-cycloalkyl;
Z³ is hydrogen or C₁-C₆-alkyl.

4. Compounds according to Claim 1, 2 or 3, in which
R¹ is hydrogen, methyl, ethyl, n-propyl, isopropyl, n-butyl, isobutyl, s-butyl or t-butyl;
the following chemical moieties are as follows:
A₁ is CR²,
A₂ is CR³ or nitrogen,
A₃ is CR⁴, and
A₄ is CR⁵;
W is oxygen;
R⁶ is hydrogen;
Z¹ is an in each case optionally mono- to hepta-halogen-substituted C₁-C₆-alkyl;
Z² is an in each case optionally mono- to hepta-halogen-substituted C₁-C₆-alkyl;
Z³ is C₁-C₆-alkyl.

5. Compounds according to any of the preceding claims, in which
R² and R⁵ are each independently hydrogen, fluorine, chlorine, bromine, iodine, methyl and methoxy;
R³ and R⁴ are each independently hydrogen, fluorine, chlorine, bromine, iodine, cyano, nitro, C₁-C₄-alkyl, C₁-C₄-haloalkyl.

6. Compounds according to any of the preceding claims, in which
R² and R⁵ are each independently hydrogen or fluorine;
R³ is hydrogen; and
R⁴ is hydrogen, fluorine, chlorine or methyl.

7. Compounds according to any of the preceding claims, in which
Q is hydrogen;
or
C₁-C₆-alkyl optionally substituted by 1, 2, 3 or 4 substituents selected independently from the group consisting of
fluorine,
chlorine,
bromine,
iodine,
cyano,
oxo,
methoxy,
benzyloxy,
ethoxy,
*N,N*-dimethylamino, phenylthio optionally independently substituted by 1, 2, 3 or 4 substituents selected from the group consisting of fluorine, chlorine, bromine, iodine, methoxy, methyl and trifluoromethyl, phenyloxy optionally independently substituted by 1, 2, 3 or 4 substituents selected from the group consisting of fluorine, chlorine, bromine, iodine, methoxy, methyl and trifluoromethyl, phenyl optionally independently substituted by 1, 2, 3 or 4 substituents selected from the group consisting of fluorine, chlorine, bromine, iodine, methoxy, methyl and trifluoromethyl, thiophenyl optionally independently substituted by 1, 2, 3 or 4 substituents selected from the group consisting of fluorine, chlorine, bromine, iodine, methoxy, methyl and trifluoromethyl, pyrazolyl optionally independently substituted by 1, 2, 3 or 4 substituents selected from the group consisting of fluorine, chlorine, bromine, iodine, methoxy, methyl and trifluoromethyl, and C₃-C₆-cycloalkyl;
or
C₃-C₆-cycloalkyl optionally substituted by 1, 2, 3 or 4 substituents selected independently from the group consisting of
methoxy,
fluorine,
chlorine,
bromine,
iodine,
cyano,
methyl, phenyl optionally independently substituted by 1, 2, 3 or 4 substituents selected from the group consisting of fluorine, chlorine, bromine, iodine, methoxy, methyl, trifluoromethyl;
or
Q is one of the following groups substituted by 0, 1, 2, 3 or 4 V substituents: phenyl, naphthyl, pyridazinyl, pyrazinyl, pyrimidinyl, triazinyl, pyridinyl, pyrazolyl, thiazolyl, isothiazolyl, oxazolyl, isoxazolyl, triazolyl, imidazolyl, furanyl, thiophenyl, pyrrolyl, oxadiazolyl, thiadiazolyl, benzothiophenyl, benzofuranyl, [1,2,4]triazolo[1,5-a]pyrimidinyl, 1,3-benzodioxolyl or tetralinyl, where
V is independently fluorine, chlorine, bromine, iodine, cyano, nitro, methyl, ethyl, propyl, butyl, difluoromethyl, chlorodifluoromethyl, dichlorofluoromethyl, trifluoromethyl, chloromethyl, bromomethyl, 1-fluoroethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, pentafluoro-tert-butyl, heptafluoro-n-propyl, heptafluoroisopropyl, nonafluoro-n-butyl, cyclopropyl, cyclobutyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, *N-*methoxyiminomethyl, 1-(*N*-methoxyimino)ethyl, methylsulphanyl, methylsulphonyl, methylsulphinyl, trifluoromethylsulphonyl, trifluoromethylsulphinyl, trifluoromethylsulphanyl, N,N-dimethylamino.

8. Compounds according to any of the preceding claims, in which
Q is C₁-C₆-alkyl optionally substituted by 1, 2, 3 or 4 substituents selected independently from the group consisting of
fluorine,
oxo,
methoxy,
benzyloxy,
ethoxy,
*N,N*-dimethylamino
phenylthio,
phenyloxy,
C₃-C₆-cycloalkyl,
phenyl optionally independently substituted by 1, 2, 3 or 4 substituents selected from the group consisting of fluorine, chlorine and trifluoromethyl, thiophenyl optionally substituted by 1, 2, 3 or 4 trifluoromethyl, and pyrazolyl optionally independently substituted by 1, 2, 3 or 4 substituents selected from the group consisting of methyl and trifluoromethyl;
or
C₃-C₆-cycloalkyl optionally substituted by 1, 2, 3 or 4 substituents selected independently from the group consisting of
methoxy,
chlorine,
cyano,
methyl,
phenyl optionally independently substituted by 1, 2, 3 or 4 substituents selected from the group consisting of chlorine and methyl, and trifluoromethyl;
or
Q is one of the following groups substituted by 0, 1, 2, 3 or 4 V substituents: phenyl, naphthyl, pyridazinyl, pyrazinyl, pyrimidinyl, triazinyl, pyridinyl, pyrazolyl, thiazolyl, isothiazolyl, oxazolyl, isoxazolyl, triazolyl, imidazolyl, furanyl, thiophenyl, pyrrolyl, oxadiazolyl, thiadiazolyl, benzothiophenyl, benzofuranyl, [1,2,4]triazolo[1,5-a]pyrimidinyl, 1,3-benzodioxolyl or tetralinyl , where
V is independently fluorine, chlorine, bromine, iodine, cyano, nitro, methyl, ethyl, propyl, butyl, difluoromethyl, chlorodifluoromethyl, dichlorofluoromethyl, trifluoromethyl, chloromethyl, bromomethyl, 1-fluoroethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, pentafluoro-tert-butyl, heptafluoro-n-propyl, heptafluoroisopropyl, nonafluoro-n-butyl, cyclopropyl, cyclobutyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, *N-*methoxyiminomethyl, 1-(*N*-methoxyimino)ethyl, methylsulphanyl, methylsulphonyl, methylsulphinyl, trifluoromethylsulphonyl, trifluoromethylsulphinyl, trifluoromethylsulphanyl, N,N-dimethylamino.

9. Compounds according to any of the preceding claims, in which the compound is a compound of the formula (Ic-1)

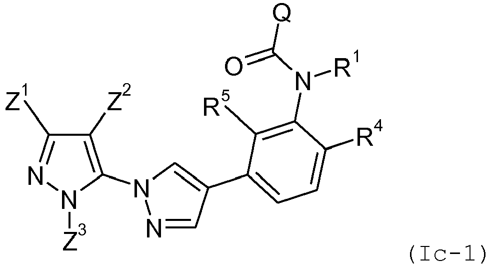

10. Compounds according to any of the preceding claims, in which the compound is a compound of the formula (Ic-2)

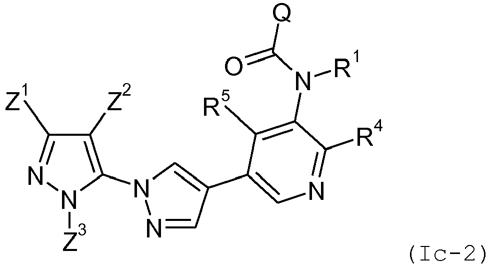

11. Use of compounds of the general formulae (I), (Ia), (Ib), (Ic), (Id), (Ie), (If), (Ig), (Ih), (Ii) and (Ij) according to any of Claims 1 to 10 for non-therapeutic control of insects, arachnids and nematodes.

12. Pharmaceutical compositions comprising at least one compound according to any of Claims 1 to 10.

13. Compounds according to any of Claims 1 to 10 for use as medicaments.

14. Compounds according to any of Claims 1 to 10 for use for controlling parasites in animals.

15. Process for producing crop protection compositions comprising compounds according to any of Claims 1 to 10 and customary extenders and/or surfactants.

16. Non-therapeutic method for controlling pests, **characterized in that** a compound according to any of Claims 1 to 10 is allowed to act on the pests and/or their habitat.

17. Use of compounds according to any of Claims 1 to 10 for protection of propagation material of plants.

## Revendications

1. Composés de formule générale (Ic), 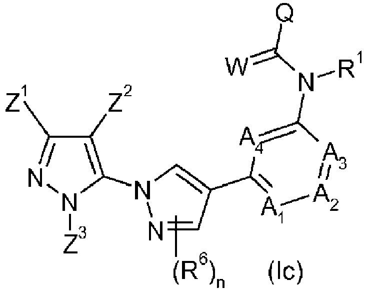 dans laquelle
R¹ représente hydrogène ; C₁-C₆-alkyle, C₃-C₆-alcényle, C₃-C₆-alcynyle, C₃-C₇-cycloalkyle, C₁-C₆-alkylcarbonyle, C₁-C₆-alcoxycarbonyle, aryl(C₁-C₃)-alkyle, hétéroaryl(C₁-C₃)-alkyle le cas échéant substitués ;
les groupements chimiques
A₁ représente CR² ou azote,
A₂ représente CR³ ou azote,
A₃ représente CR⁴ ou azote et
A₄ représente CR⁵ ou azote, pas plus de trois des groupements chimiques A₁ à A₄ représentant cependant simultanément azote ;
R², R³, R⁴ et R⁵ représentent, indépendamment les uns des autres, hydrogène, halogène, cyano, nitro ; C₁-C₆-alkyle, C₃-C₆-cycloalkyle, C₁-C₆-alcoxy, N-C₁-C₆-alcoxyimino-C₁-C₃-alkyle, C₁-C₆-alkylsulfanyle, C₁-C₆-alkylsulfinyle, C₁-C₆-alkylsulfonyle, N-C₁-C₆-alkylamino ou N,N-di-C₁-C₆-alkylamino le cas échéant substitués ;
lorsqu'aucun des groupements A₂ et A₃ ne représente azote, R³ et R⁴ peuvent former, ensemble avec l'atome de carbone auquel ils sont liés, un cycle de 5 ou 6 chaînons, qui contient 0, 1 ou 2 atomes d'azote et/ou 0 ou 1 atome d'oxygène et/ou 0 ou 1 atome de soufre, ou
lorsqu'aucun des groupements A₁ et A₂ ne représente azote, R² et R³ peuvent former, ensemble avec l'atome de carbone auquel ils sont liés, un cycle de 5 ou 6 chaînons, qui contient 0, 1 ou 2 atomes d'azote et/ou 0 ou 1 atome d'oxygène et/ou 0 ou 1 atome de soufre, ou
W représente oxygène ou soufre ;
Q représente hydrogène, amino ; ou un des groupements alkyle, alkyloxy, alcényle, alcynyle, cycloalkyle, hétérocyclyle, cycloalkylalkyle, arylalkyle, hétéroarylalkyle, alcoxycarbonyle le cas échéant substitués ou un groupement N-alkylamino, N-alkylcarbonylamino, N,N-dialkylamino, alkylsulfonylamino ; ou
Q représente aryle le cas échéant monosubstitué à pentasubstitué par V ou hétéroaryle le cas échéant monosubstitué à pentasubstitué par V
V représentant halogène, cyano, nitro ; alkyle, alcényle, alcynyle, cycloalkyle, alcoxy, N-alcoxyiminoalkyle, alkylsulfanyle, alkylsulfinyle, alkylsulfonyle, N,N-dialkylamino le cas échéant substitués ;
R⁶ représente, indépendamment, halogène, cyano, nitro, amino ; ou C₁-C₆-alkyle, C₁-C₆-alkyloxy, C₁-C₆-alkylcarbonyle, C₁-C₆-alkylsulfanyle, C₁-C₆-alkylsulfinyle, C₁-C₆-alkylsulfonyle le cas échéant substitués et
n représente les valeurs 0-2 ;
Z¹ représente alkyle ou cycloalkyle le cas échéant substitués et
Z² représente hydrogène, halogène, cyano, nitro, amino ; ou alkyle, alkylcarbonyle, alkylsulfanyle, alkylsulfinyle, alkylsulfonyle le cas échéant substitués et
Z³ représente hydrogène ; ou alkyle, cycloalkyle, alcényle, alcynyle, aryle ou hétéroaryle le cas échéant substitués.

2. Composés selon la revendication 1, où
R¹ représente hydrogène ; ou C₁-C₆-alkyle, C₃-C₆-alcényle, C₃-C₆-alcynyle, C₃-C₇-cycloalkyle, C₃-C₇-cycloalkyl-C₁-C₃-alkyle, C₁-C₆-alkylcarbonyle, C₁-C₆-alcoxycarbonyle, aryl(C₁-C₃)-alkyle, hétéroaryl(C₁-C₃)-alkyle le cas échéant monosubstitués à heptasubstitués, indépendamment les uns des autres, par halogène, cyano, alcoxy ou alcoxycarbonyle ;
les groupements chimiques
A₁ représente CR² ou azote,
A₂ représente CR³ ou azote,
A₃ représente CR⁴ ou azote et
A₄ représente CR⁵ ou azote, pas plus de trois des groupements chimiques A₁ à A₄ représentant cependant simultanément azote ;
R², R³, R⁴ et R⁵ représentent, indépendamment les uns des autres, hydrogène, halogène, cyano, nitro ; ou C₁-C₆-alkyle, C₃-C₆-cycloalkyle, C₁-C₆-alcoxy, N-C₁-C₆-alcoxyimino-C₁-C₃-alkyle, C₁-C₆-alkylsulfanyle, C₁-C₆-alkylsulfinyle, C₁-C₆-alkylsulfonyle, N-C₁-C₆-alkylamino ou N,N-di-C₁-C₆-alkylamino le cas échéant monosubstitués à heptasubstitués, indépendamment les uns des autres, par halogène, cyano, alcoxy ou alcoxycarbonyle ;
W représente oxygène ou soufre ;
Q représente hydrogène, amino ; ou un des groupements C₁-C₆-alkyle, C₂-C₆-alcényle, C₂-C₆-alcynyle, C₃-C₆-cycloalkyle, C₂-C₅-hétérocyclyle, C₁-C₄-alcoxy, C₁-C₆-alkyl-C₃-C₆-cycloalkyle, C₃-C₆-cycloalkyl-C₁-C₆-alkyle, C₁-C₆-hydroxyalkyle, aryl (C₁-C₃) -alkyle, hétéroaryl (C₁-C₃) -alkyle, C₁-C₄-alcoxycarbonyle, N-C₁-C₄-alkylamino, N-C₁-C₄-alkylcarbonylamino, N,N-di-C₁-C₄-alkylamino, C₁-C₄-alkylsulfonylamino le cas échéant monosubstitués à heptasubstitués, indépendamment les uns des autres, par hydroxy, nitro, amino, halogène, alcoxy, cyano, hydroxycarbonyle, alcoxycarbonyle, alkylcarbamoyle, cycloalkylcarbamoyle ou phényle ; ou
Q représente hydrogène, amino ; ou un des groupements choisis parmi C₁-C₆-alkyle, C₁-C₆-alkyloxy, C₂-C₆-alcényle, C₂-C₆-alcynyle, C₃-C₆-cycloalkyle, hétérocyclyle de 5 à 6 chaînons, C₁-C₆-alkyl-C₃-C₆-cycloalkyle, C₃-C₆-cycloalkyl-C₁-C₆-alkyle, C₆-C₁₀-aryl- (C₁-C₃) -alkyle, hétéroaryl-(C₁-C₃)-alkyle de 5 à 6 chaînons, C₁-C₄-alcoxycarbonyle, le cas échéant substitués, indépendamment les uns des autres, par 1, 2, 3, 4, 5, 6 ou 7 substituants choisis dans le groupe constitué par hydroxy, nitro, amino, halogène, oxo, benzyloxy, C₃-C₆-cycloalkyle, C₁-C₆-alcoxy, C₁-C₆-halogénoalcoxy, cyano, hydroxycarbonyle, C₁-C₄-alcoxycarbonyle, C₁-C₆-alkylcarbamoyle, C₃-C₆-cycloalkylcarbamoyle, N,N-di-C₁-C₄-alkylamino ; par phényle le cas échéant substitué par 1, 2 ou 3 substituants choisis, indépendamment les uns des autres, dans le groupe de substituants constitué par halogène, C₁-C₆-alcoxy, C₁-C₆-halogénoalcoxy et C₁-C₆-alkyle ; par phénylthio le cas échéant substitué par 1, 2 ou 3 substituants choisis indépendamment les uns des autres dans le groupe de substituants constitué par halogène, C₁-C₆-alcoxy, C₁-C₆-halogénoalcoxy et C₁-C₆-alkyle ; par phényloxy, le cas échéant substitué par 1, 2 ou 3 substituants choisis indépendamment les uns des autres dans le groupe de substituants constitué par halogène, C₁-C₆-alcoxy, C₁-C₆-halogénoalcoxy et C₁-C₆-alkyle ; par hétéroaryle de 5 à 6 chaînons (par exemple pyrazolyle) le cas échéant substitué par 1, 2 ou 3 substituants choisis indépendamment les uns des autres dans le groupe de substituants constitué par halogène, C₁-C₆-alcoxy, C₁-C₆-halogénoalcoxy et C₁-C₆-alkyle ; ou un groupement N-C₁-C₄-alkylamino, N-C₁-C₄-alkylcarbonylamino, N,N-di-C₁-C₄-alkylamino, C₁-C₄-alkylsulfonylamino ; ou
Q représente aryle, substitué par 0, 1, 2, 3 ou 4 substituants V ou hétéroaryle de 5 ou 6 chaînons, substitué par 0, 1, 2, 3 ou 4 substituants V ou
Q représente un hétérocycle bicyclique substitué par 0, 1, 2, 3 ou 4 substituants V ou un carbocycle bicyclique substitué par 0, 1, 2, 3 ou 4 substituants V ;
V représentant, indépendamment, halogène, cyano, nitro ; ou C₁-C₆-alkyle, C₁-C₆-halogénoalkyle, C₂-C₄-alcényle, C₂-C₄-alcynyle, C₃-C₆-cycloalkyle, C₁-C₆-alcoxy, C₁-C₆-halogénoalcoxy, N-C₁-C₆-alcoxyimino-C₁-C₃-alkyle, C₁-C₆-alkylsulfanyle, C₁-C₆-alkylsulfinyle, C₁-C₆-alkylsulfonyle, N,N-di-(C₁-C₆-alkyl)amino le cas échéant monosubstitués à heptasubstitués, indépendamment les uns des autres, par halogène, cyano, alcoxy ou alcoxycarbonyle ;
R⁶ représente, indépendamment, halogène, cyano, nitro, amino ; ou C₁-C₆-alkyle, C₁-C₆-alkyloxy, C₁-C₆-alkylcarbonyle, C₁-C₆-alkylsulfanyle, C₁-C₆-alkylsulfinyle, C₁-C₆-alkylsulfonyle le cas échéant monosubstitués à heptasubstitués par halogène et
n représente les valeurs 0-1 ;
Z¹ représente C₁-C₆-alkyle, C₃-C₆-cycloalkyle le cas échéant monosubstitués à heptasubstitués, indépendamment les uns des autres, par halogène, cyano, alcoxy ou alcoxycarbonyle ;
Z² représente hydrogène, halogène, cyano, nitro, amino ; ou C₁-C₆-alkyle, C₁-C₆-alkylcarbonyle, C₁-C₆-alkylsulfanyle, C₁-C₆-alkylsulfinyle, C₁-C₆-alkylsulfonyle monosubstitués à heptasubstitués, indépendamment les uns des autres, par halogène, cyano, alcoxy ou alcoxycarbonyle et
Z³ représente hydrogène ; ou C₁-C₆-alkyle, C₃-C₆-cycloalkyle, C₃-C₄-alcényle, C₃-C₄-alcynyle le cas échéant monosubstitués à heptasubstitués, indépendamment les uns des autres, par halogène, cyano, alcoxy ou alcoxycarbonyle ; ou aryle et hétéroaryle le cas échéant monosubstitués à pentasubstitués, indépendamment les uns des autres, par halogène, cyano, alcoxy et alcoxycarbonyle.

3. Composés selon la revendication 1 ou 2, où
R¹ représente hydrogène, C₁-C₄-alkyle, C₃-C₄-alcényle, C₃-C₄-alcynyle ;
les groupements chimiques
A₁ représente CR²,
A₂ représente CR³ ou azote,
A₃ représente CR⁴ et
A₄ représente CR⁵ ;
W représente oxygène ;
R⁶ représente C₁-C₄-alkyle ;
n représente les valeurs 0-1 ;
Z¹ représente C₁-C₆-alkyle ou C₃-C₆-cycloalkyle, à chaque fois, le cas échéant, monosubstitué à heptasubstitué, indépendamment les uns des autres, par halogène ou cyano ;
Z² représente C₁-C₆-alkyle ou C₃-C₆-cycloalkyle, à chaque fois, le cas échéant, monosubstitué à heptasubstitué, indépendamment les uns des autres, par halogène ou cyano ;
Z³ représente hydrogène ou C₁-C₆-alkyle.

4. Composés selon la revendication 1, 2 ou 3, où
R¹ représente hydrogène, méthyle, éthyle, n-propyle, isopropyle, n-butyle, isobutyle, s-butyle, ou t-butyle ;
les groupements chimiques
A₁ représente CR²,
A₂ représente CR³ ou azote,
A₃ représente CR⁴ et
A₄ représente CR⁵ ;
W représente oxygène ;
R⁶ représente hydrogène ;
Z¹ représente C₁-C₆-alkyle, à chaque fois, le cas échéant, monosubstitué à heptasubstitué, indépendamment, par halogène ;
Z² représente C₁-C₆-alkyle, à chaque fois, le cas échéant, monosubstitué à heptasubstitué, indépendamment, par halogène ;
Z³ représente C₁-C₆-alkyle.

5. Composés selon l'une quelconque des revendications précédentes, où
R² et R⁵ représentent, indépendamment l'un de l'autre, hydrogène, fluor, chlore, brome, iode, méthyle et méthoxy ;
R³ et R⁴ représentent, indépendamment l'un de l'autre, hydrogène, fluor, chlore, brome, iode, cyano, nitro, C₁-C₄-alkyle, C₁-C₄-halogénoalkyle.

6. Composés selon l'une quelconque des revendications précédentes, où
R² et R⁵ représentent, indépendamment, hydrogène ou fluor ;
R³ représente hydrogène ; et
R⁴ représente hydrogène, chlore, fluor ou méthyle.

7. Composés selon l'une quelconque des revendications précédentes, où
Q représente hydrogène ; ou C₁-C₆-alkyle, éventuellement substitué par 1, 2, 3 ou 4 substituants choisis indépendamment les uns des autres dans le groupe constitué par
fluor,
chlore,
brome,
iode,
cyano,
oxo,
méthoxy,
benzyloxy,
éthoxy,
N,N-diméthylamino,
phénylthio, éventuellement substitué, indépendamment, par 1, 2, 3 ou 4 substituants choisis dans le groupe constitué par fluor, chlore, brome, iode, méthoxy, méthyle et trifluorométhyle,
phényloxy, éventuellement substitué, indépendamment, par 1, 2, 3 ou 4 substituants choisis dans le groupe constitué par fluor, chlore, brome, iode, méthoxy, méthyle et trifluorométhyle,
phényle, éventuellement substitué, indépendamment, par 1, 2, 3 ou 4 substituants choisis dans le groupe constitué par fluor, chlore, brome, iode, méthoxy, méthyle et trifluorométhyle,
thiophényle, éventuellement substitué, indépendamment, par 1, 2, 3 ou 4 substituants choisis dans le groupe constitué par fluor, chlore, brome, iode, méthoxy, méthyle et trifluorométhyle,
pyrazolyle, éventuellement substitué, indépendamment, par 1, 2, 3 ou 4 substituants choisis dans le groupe constitué par fluor, chlore, brome, iode, méthoxy, méthyle et trifluorométhyle,
C₃-C₆-cycloalkyle ; ou
C₃-C₆-cycloalkyle, éventuellement substitué par 1, 2, 3 ou 4 substituants choisis indépendamment les uns des autres dans le groupe constitué par
méthoxy,
fluor,
chlore,
brome,
iode,
cyano,
méthyle,
phényle, éventuellement substitué, indépendamment, par 1, 2, 3 ou 4 substituants choisis dans le groupe constitué par fluor, chlore, brome, iode, méthoxy, méthyle et trifluorométhyle ; ou
Q représente phényle, naphtyle, pyridazinyle, pyrazinyle, pyrimidinyle, triazinyle, pyridinyle, pyrazolyle, thiazolyle, isothiazolyle, oxazolyle, isoxazolyle, triazolyle, imidazolyle, furannyle, thiophényle, pyrrolyle, oxadiazolyle, thiadiazolyle, benzothiophényle, benzofurannyle, [1,2,4]triazolo[1,5-a]pyrimidinyle, 1,3-benzodioxolyle ou tétralinyle substitués par 0, 1, 2, 3 ou 4 substituants V,
V représentant, indépendamment, fluor, chlore, brome, iode, cyano, nitro, méthyle, éthyle, propyle, butyle, difluorométhyle, chlorodifluorométhyle, dichlorofluorométhyle, trifluorométhyle, chlorométhyle, bromométhyle, 1-fluoroéthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle, pentafluoro-tert-butyle, heptafluoro-n-propyle, heptafluoro-isopropyle, nonafluoro-n-butyle, cyclopropyle, cyclobutyle, méthoxy, éthoxy, n-propoxy, 1-méthyléthoxy, fluorométhoxy, difluorométhoxy, chlorodifluorométhoxy, dichlorofluorométhoxy, trifluorométhoxy, 2,2,2-trifluoroéthoxy, 2-chloro-2,2-difluoroéthoxy, pentafluoroéthoxy, N-méthoxyiminométhyle, 1-(N-méthoxyimino)-éthyle, méthylsulfanyle, méthylsulfonyle, méthylsulfinyle, trifluorométhylsulfonyle, trifluorométhylsulfinyle, trifluorométhylsulfanyle, N,N-diméthylamino.

8. Composés selon l'une quelconque des revendications précédentes, où
Q représente C₁-C₆-alkyle, éventuellement substitué par 1, 2, 3 ou 4 substituants choisis indépendamment les uns des autres dans le groupe constitué par
fluor,
oxo,
méthoxy,
benzyloxy,
éthoxy,
N,N-diméthylamino,
phénylthio,
phényloxy,
C₃-C₆-cycloalkyle,
phényle, éventuellement substitué, indépendamment, par 1, 2 3, ou 4 substituants choisis dans le groupe constitué par fluor, chlore et trifluorométhyle,
thiophényle éventuellement substitué par 1, 2, 3, ou 4 substituants trifluorométhyle et
pyrazolyle, éventuellement substitué, indépendamment, par 1, 2, 3 ou 4 substituants choisis dans le groupe constitué par méthyle et trifluorométhyle ; ou
C₃-C₆-cycloalkyle, éventuellement substitué par 1, 2, 3 ou 4 substituants choisis indépendamment l'un de l'autre dans le groupe constitué par
méthoxy,
chlore,
cyano,
méthyle,
phényle, éventuellement substitué, indépendamment, par 1, 2, 3, ou 4 substituants choisis dans le groupe constitué par chlore, méthyle et trifluorométhyle ; ou
Q représente phényle, naphtyle, pyridazinyle, pyrazinyle, pyrimidinyle, triazinyle, pyridinyle, pyrazolyle, thiazolyle, isothiazolyle, oxazolyle, isoxazolyle, triazolyle, imidazolyle, furannyle, thiophényle, pyrrolyle, oxadiazolyle, thiadiazolyle, benzothiophényle, benzofurannyle, [1,2,4]triazolo[1,5-a]pyrimidinyle, 1,3-benzodioxolyle ou tétralinyle substitués par 0, 1, 2, 3 ou 4 substituants V,
V représentant, indépendamment, fluor, chlore, brome, iode, cyano, nitro, méthyle, éthyle, propyle, butyle, difluorométhyle, chlorodifluorométhyle, dichlorofluorométhyle, trifluorométhyle, chlorométhyle, bromométhyle, 1-fluoroéthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle, pentafluoro-tert-butyle, heptafluoro-n-propyle, heptafluoro-isopropyle, nonafluoro-n-butyle, cyclopropyle, cyclobutyle, méthoxy, éthoxy, n-propoxy, 1-méthyléthoxy, fluorométhoxy, difluorométhoxy, chlorodifluorométhoxy, dichlorofluorométhoxy, trifluorométhoxy, 2,2,2-trifluoroéthoxy, 2-chloro-2,2-difluoroéthoxy, pentafluoroéthoxy, N-méthoxyiminométhyle, 1-(N-méthoxyimino)-éthyle, méthylsulfanyle, méthylsulfonyle, méthylsulfinyle, trifluorométhylsulfonyle, trifluorométhylsulfinyle, trifluorométhylsulfanyle, N,N-diméthylamino.

9. Composés selon l'une quelconque des revendications précédentes, le composé étant un composé de formule (Ic-1)

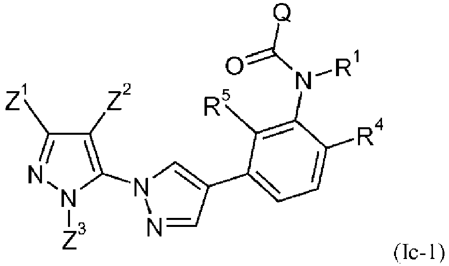

10. Composés selon l'une quelconque des revendications précédentes, le composé étant un composé de formule (Ic-2)

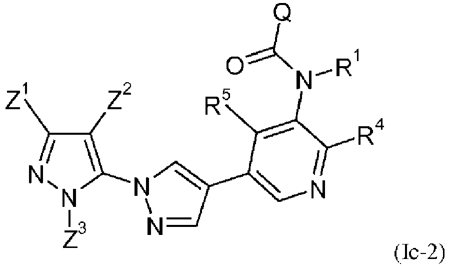

11. Utilisation de composés des formules générales (I), (Ia), (Ib), (Ic), (Id), (Ie), (If), (Ig), (Ih), (Ii) et (Ij) selon l'une quelconque des revendications 1 à 10 pour la lutte non thérapeutique contre les insectes, les arachnides et les nématodes.

12. Compositions pharmaceutiques contenant au moins un composé selon l'une quelconque des revendications 1 à 10.

13. Composés selon l'une quelconque des revendications 1 à 10 pour une utilisation comme médicament.

14. Composés selon l'une quelconque des revendications 1 à 10 pour une utilisation pour la lutte contre des parasites sur des animaux.

15. Procédé pour la préparation d'agents de phytoprotection, contenant des composés selon l'une quelconque des revendications 1 à 10, ainsi que des agents d'allongement et/ou des substances tensioactives usuel(le)s.

16. Procédé non thérapeutique pour lutter contre les organismes nuisibles, **caractérisé en ce qu'**on laisse agir un composé selon l'une quelconque des revendications 1 à 10 sur les organismes nuisibles et/ou leur espace de vie.

17. Utilisation de composés selon l'une quelconque des revendications 1 à 10 pour la protection de matériel de reproduction de plantes.
